# EUROPEAN PATENT SPECIFICATION

(11) **EP 1 169 347 B1**
(45) Date of publication and mention of the grant of the patent: **02.07.2008**
(21) Application number: 00920102.1
(22) Date of filing: 03.04.2000
(51) Int. Cl.: C07K 14/47, C12N 15/12, C12N 15/10, C12N 15/62, C07K 19/00, G01N 33/53, C12N 15/11, C12Q 1/68, A61K 39/395, A61K 38/17, A61P 35/00

(54) **COMPOUNDS AND METHODS FOR THERAPY AND DIAGNOSIS OF LUNG CANCER**
VERBINDUNGEN UND VERFAHREN FÜR THERAPIE UND DIAGNOSE VON LUNGENKREBS
COMPOSES ET PROCEDES DE THERAPIE ET DE DIAGNOSTIC DU CANCER DU POUMON

(30) Priority: 02.04.1999 US 285479; 17.12.1999 US 466396; 30.12.1999 US 476496; 10.01.2000 US 480884; 22.02.2000 US 510376
(43) Date of publication of application: 09.01.2002
(73) Proprietor: CORIXA CORPORATION, Seattle, WA 98104 (US)
(72) Inventor: WANG, Tongtong, Medina, WA 98039 (US); FAN, Liqun, Bellevue, WA 98006 (US)
(74) Representative: Gowshall, Jonathan Vallance
(86) International application number: PCT/US2000/008896
(87) International publication number: WO 2000/061612

(56) References cited:
- WO-A-99/47674
- WO-A-99/54738
- MÜELLER-PILLASCH ET AL: "Cloning of a gene highly overexpressed in cancer coding for a novel KH-domain containing protein" ONCOGENE, BASINGSTOKE, HANTS, GB, vol. 14, no. 22, 5 June 1997 (1997-06-05), pages 2729-2733, XP002118534 ISSN: 0950-9232 -& DATABASE EMBL 11 September 1998 (1998-09-11) "Homo sapiens putative RNA binding protein KOC (koc) mRNA, complete cds." retrieved from EMBL Database accession no. HSU97188 XP002290879 -& DATABASE UNIPROT/TREMBL 1 July 1997 (1997-07-01) "Putattive RNA binding protein KOC (Koc1)" retrieved from UNIPROT/TREMBL Database accession no. O00425 XP002290880
- NIELSEN JACOB ET AL: "A family of insulin-like growth factor II mRNA-binding proteins represses translation in late development" MOLECULAR AND CELLULAR BIOLOGY, vol. 19, no. 2, February 1999 (1999-02), pages 1262-1270, XP002290878 ISSN: 0270-7306 -& DATABASE EMBL 26 January 1999 (1999-01-26) "Homo sapiens IGF-II mRNA-binding protein 3 (IMP-3) mRNA, complete cds" retrieved from EMBL Database accession no. AF117108 XP002290881 -& DATABASE EMBL 26 January 1999 (1999-01-26) "IGF-II mRNA-binding protein 3 [Homo sapiens]" retrieved from EMBL Database accession no. AAD09828 XP002290882

### SEQUENCE LISTING

<110> Corixa Corporation et al.
<120> COMPOUNDS AND METHODS FOR THERAPY AND DIAGNOSIS OF LUNG CANCER
<130> 210121.45501PC
<140> PCT
   <141> 2000-04-03
<160> 350
<170> FastSEQ for Windows Version 3.0
<210> 1
   <211> 315
   <212> DNA
   <213> Homo sapien
<220>
   <221> misc_feature
   <222> (1)...(315)
   <223> n = A,T,C or G
<400> 1
   gcagagacag actggtggtt gaacctggag gtgccaaaaa agccagctgc gggcccagga      60
   cagctgccgt gagactcccg atgtcacagg cagtctgtgt ggttacagcg cccctcagtg     120
   ttcatctcca gcagagacaa cggaggaggc tcccaccagg acggttctca ttatttatat     180
   gttaatatgt ttgtaaactc atgtacagtt ttttttgggg gggaagcaat gggaanggta     240
   naaattacaa atagaatcat ttgctgtaat ccttaaatgg caaacggtca ggccacgtga     300
   aaaaaaaaaa aaaaa                                                      315
<210> 2
   <211> 380
   <212> DNA
   <213> Homo sapien
<400> 2
   atttaggctt aagattttgt ttacccttgt tactaaggag caaattagta ttaaagtata      60
   atatatataa acaaatacaa aaagttttga gtggttcagc ttttttattt tttttaatgg      120
   cataactttt aacaacactg ctctgtaatg ggttgaactg tggtactcag actgagataa    180
   ctgaaatgag tggatgtata gtgttattgc ataattatcc cactatgaag caaagggact    240
   ggataaattc ccagtctaga ttattagcct ttgttaacca tcaagcacct agaagaagaa    300
   ttattggaaa ttttgtcctc tgtaactggc actttggggt gtgacttatc ttttgccttt    360
   gtaaaaaaaa aaaaaaaaaa                                                 380
<210> 3
   <211> 346
   <212> DNA
   <213> Homo sapien
<220>
   <221> misc_feature
<220>
   <221> misc_feature
   <222> (1)...(346)
   <223> n = A,T,C or G
<400> 3
   ttgtaagtat acaattttag aaaggattaa atgttattga tcattttact gaatactgca     60
   catcctcacc atacaccatc cactttccaa taacatttaa tcctttctaa aattgtaagt    120
   atacaattgt actttctttg gattttcata acaaatatac catagactgt taattttatt    180
   gaagtttcct taatggaatg agtcattttt gtcttgtgct tttgaggtta cctttgcttt    240
   gacttccaac aatttgatca tatagtgttg agctgtggaa atctttaagt ttattctata    300
   gcaataattt ctattnnnag annccnggnn naaaannann annaaa                   346
<210> 4
   <211> 372
   <212> DNA
   <213> Homo sapien
<220>
   <221> misc_feature
   <222> (1)...(372)
   <223> n = A,T,C or G
<400> 4
   actagtctca ttactccaga attatgctct tgtacctgtg tggctgggtt tcttagtcgt     60
   tggtttggtt tggttttttg aactggtatg tagggtggtt cacagttcta atgtaagcac  120
   tctcttctcc aagttgtgct ttgtggggac aatcattctt tgaacattag agaggaaggc    180
   agttcaagct gttgaaaaga ctattgctta tttttgtttt taaagaccta cttgacgtca    240
   tgtggacagt gcacgtgcct tacgctacat cttgttttct aggaagaagg ggatgcnggg    300
   aaggantggg tgctttgtga tggataaaac gnctaaataa cacaccttta cattttgaaa    360
   aaaacaaaac aa                                                         372
<210> 5
   <211> 698
   <212> DNA
   <213> Homo sapien
<220>
   <221> misc_feature
   <222> (1)...(698)
   <223> n = A,T,C or G
<400> 5
   actagtanga tagaaacact gtgtcccgag agtaaggaga gaagctacta ttgattagag 60
   cctaacccag gttaactgca agaagaggcg ggatactttc agctttccat gtaactgtat 120
   gcataaagcc aatgtagtcc agtttctaag atcatgttcc aagctaactg aatcccactt 180
   caatacacac tcatgaactc ctgatggaac aataacaggc ccaagcctgt ggtatgatgt 240
   gcacacttgc tagactcaga aaaaatacta ctctcataaa tgggtgggag tattttgggt 300
   gacaacctac tttgcttggc tgagtgaagg aatgatattc atatnttcat ttattccatg 360
   gacatttagt tagtgctttt tatataccag gcatgatgct gagtgacact cttgtgtata 420
   tntccaaatn ttngtncngt cgctgcacat atctgaaatc ctatattaag antttcccaa 480
   natgangtcc ctggtttttc cacgccactt gatcngtcaa ngatctcacc tctgtntgtc 540
   ctaaaaccnt ctnctnnang gttagacngg acctctcttc tcccttcccg aanaatnaag 600
   tgtgngaaga nanccncncn cccccctncn tncnncctng ccngctnnnc cncntgtngg 660
   gggngccgcc cccgcggggg gacccccccn ttttcccc 698
<210> 6
   <211> 740
   <212> DNA
   <213> Homo sapien
<220>
   <221> misc_feature
   <222> (1)...(740)
   <223> n = A,T,C or G
<400> 6
   actagtcaaa aatgctaaaa taatttggga gaaaatattt tttaagtagt gttatagttt 60
   catgtttatc ttttattatg tnttgtgaag ttgtgtcttt tcactaatta cctatactat 120
   gccaatattt ccttatatct atccataaca tttatactac atttgtaaga gaatatgcac 180
   gtgaaactta acactttata aggtaaaaat gaggtttcca agatttaata atctgatcaa 240
   gttcttgtta tttccaaata gaatggactt ggtctgttaa ggggctaagg gagaagaaga 300
   agataaggtt aaaagttgtt aatgaccaaa cattctaaaa gaaatgcaaa aaaaaattta 360
   ttttcaagcc ttcgaactat ttaaggaaag caaaatcatt tcctanatgc atatcatttg 420
   tgagantttc tcantaatat cctgaatcat tcatttcagc tnaggcttca tgttgactcg 480
   atatgtcatc tagggaaagt ctatttcatg gtccaaacct gttgccatag ttggtnaggc 540
   tttcctttaa ntgtgaanta ttnacangaa attttctctt tnanagttct tnatagggtt 600
   aggggtgtgg gaaaagcttc taacaatctg tagtgttncg tgttatctgt ncagaaccan 660
   aatnacggat cgnangaagg actgggtcta tttacangaa cgaatnatct ngttnnntgt 720
   gtnnncaact ccngggagcc 740
<210> 7
   <211> 670
   <212> DNA
   <213> Homo sapien
<220>
   <221> misc_feature
   <222> (1)...(670)
   <223> n = A,T,C or G
<400> 7
   gctggggagc tcggcatggc ggtccccgct gcagccatgg ggccctcggc gttgggccag      60
   agcggccccg gctcgatggc cccgtggtgc tcagtgagca gcggcccgtc gcgctacgtg     120
   cttgggatgc aggagctgtt ccggggccac agcaagaccg cgagttcctg gcgcacagcg     180
   ccaaggtgca ctcggtggcc tggagttgcg acgggcgtcg cctacctcgg ggtcttcgac     240
   aagacgccac gtcttcttgc tgganaanga ccgttggtca aagaaaacaa ttatcgggga     300
   catggggata gtgtggacca ctttgttggc atccaagtaa tcctgaccta tttgttacgg     360
   cgtctggaga taaaaccatt cgcatctggg atgtgaggac tacaaaatgc attgccactg     420
   tgaacactaa aggggagaac attaatatct gctggantcc tgatgggcan accattgctg     480
   tagcnacaag gatgatgtgg tgactttatt gatgccaaga aaccccgttc caaagcaaaa     540
   aaacanttcc aanttcgaag tcaccnaaat ctcctggaac aatgaacatn aatatnttct     600
   tcctgacaat ggnccttggg tgtntcacat cctcagctnc cccaaaactg aancctgtnc     660
   natccacccc                                                            670
<210> 8
   <211> 689
   <212> DNA
   <213> Homo sapien
<220>
   <221> misc_feature
   <222> (1)...(689)
   <223> n = A,T,C or G
<400> 8
   actagtatct aggaatgaac agtaaaagag gagcagttgg ctacttgatt acaacagagt      60
   aaatgaagta ctggatttgg gaaaacctgg ttttattaga acatatggaa tgaaagccta     120
   cacctagcat tgcctactta gccccctgaa ttaacagagc ccaattgaga caaacccctg     180
   gcaacaggaa attcaaggga gaaaaagtaa gcaacttggg ctaggatgag ctgactccct     240
   tagagcaaag ganagacagc ccccattacc aaataccatt tttgcctggg gcttgtgcag     300
   ctggcagtgt tcctgcccca gcatggcacc ttatngtttt gatagcaact tcgttgaatt     360
   ttcaccaact tattacttga aattataata tagcctgtcc gtttgctgtn tccaggctgt     420
   gatatatntt cctagtggtt tgactttnaa aataaatnag gtttantttt ctccccccnn      480
   cnntnctncc nntcnctcnn cnntcccccc cnctcngtcc tccnnnnttn gggggggccn     540
   cccccncggn ggacccccct ttggtccctt agtggaggtt natggcccct ggnnttatcc     600
   nggccntann tttccccgtn nnaaatgntt ccccctccca ntcccnccac ctcaanccgg     660
   aagcctaagt ttntaccctg ggggtcccc                                       689
<210> 9
   <211> 674
   <212> DNA
   <213> Homo sapien
<220>
   <221> misc_feature
   <222> (1)...(674)
   <223> n = A,T,C or G
<400> 9
   gtccactctc ctttgagtgt actgtcttac tgtgcactct gtttttcaac tttctagata     60
   taaaaaatgc ttgttctata gtggagtaag agctcacaca cccaaggcag caagataact    120
   gaaaaaagcg aggctttttt gccaccttgg taaaggccag ttcactgcta tagaactgct    180
   ataagcctga agggaagtag ctatgagact ttccattttt cttagttctc ccaataggct    240
   ccttcatgga aaaaggcttc ctgtaataat tttcacctaa tgaattagca gtgtgattat    300
   ttctgaaata agagacaaat tgggccgcag agtcttcctg tgatttaaaa taaacaaccc    360
   aaagttttgt ttggtcttca ccaaaggaca tactctaggg ggtatgttgt tgaagacatt    420
   caaaaacatt agctgttctg tctttcaatt tcaagttatt ttggagactg cctccatgtg    480
   agttaattac tttgctctgg aactagcatt attgtcatta tcatcacatt ctgtcatcat    540
   catctgaata atattgtgga tttccccctc tgcttgcatc ttcttttgac tcctctggga    600
   anaaatgtca aaaaaaaagg tcgatctact cngcaaggnc catctaatca ctgcgctgga    660
   aggacccnct gccc                                                      674
<210> 10
   <211> 346
   <212> DNA
   <213> Homo sapien
<220>
   <221> misc_feature
   <222> (1)...(346)
   <223> n = A,T,C or G
<400> 10
   actagtctgc tgatagaaag cactatacat cctattgttt ctttctttcc aaaatcagcc     60
   ttctgtctgt aacaaaaatg tactttatag agatggagga aaaggtctaa tactacatag    120
   ccttaagtgt ttctgtcatt gttcaagtgt attttctgta acagaaacat atttggaatg    180
   tttttctttt ccccttataa attgtaattc ctgaaatact gctgctttaa aaagtcccac    240
   tgtcagatta tattatctaa caattgaata ttgtaaatat acttgtctta cctctcaata    300
   aaagggtact tttctattan nnagnngnnn gnnnnataaa anaaaa                   346
<210> 11
   <211> 602
   <212> DNA
   <213> Homo sapien
<400> 11
   actagtaaaa agcagcattg ccaaataatc cctaattttc cactaaaaat ataatgaaat     60
   gatgttaagc tttttgaaaa gtttaggtta aacctactgt tgttagatta atgtatttgt    120
   tgcttccctt tatctggaat gtggcattag cttttttatt ttaaccctct ttaattctta    180
   ttcaattcca tgacttaagg ttggagagct aaacactggg atttttggat aacagactga    240
   cagttttgca taattataat cggcattgta catagaaagg atatggctac cttttgttaa    300
   atctgcactt tctaaatatc aaaaaaggga aatgaagtta taaatcaatt tttgtataat    360
   ctgtttgaaa catgagtttt atttgcttaa tattagggct ttgccccttt tctgtaagtc    420
   tcttgggatc ctgtgtagaa ctgttctcat taaacaccaa acagttaagt ccattctctg    480
   gtactagcta caaattcggt ttcatattct acttaacaat ttaaataaac tgaaatattt    540
   ctagatggtc tacttctgtt catataaaaa caaaacttga tttccaaaaa aaaaaaaaaa    600
   aa                                                                   602
<210> 12
   <211> 685
   <212> DNA
   <213> Homo sapien
<220>
   <221> misc_feature
   <222> (1)...(685)
   <223> n = A,T,C or G
<400> 12
   actagtcctg tgaaagtaca actgaaggca gaaagtgtta ggattttgca tctaatgttc      60
   attatcatgg tattgatgga cctaagaaaa taaaaattag actaagcccc caaataagct     120
   gcatgcattt gtaacatgat tagtagattt gaatatatag atgtagtatn ttgggtatct     180
   aggtgtttta tcattatgta aaggaattaa agtaaaggac tttgtagttg tttttattaa     240
   atatgcatat agtagagtgc aaaaatatag caaaaatana aactaaaggt agaaaagcat     300
   tttagatatg ccttaatnta nnaactgtgc caggtggccc tcggaataga tgccaggcag     360
   agaccagtgc ctgggtggtg cctccccttg tctgcccccc tgaagaactt ccctcacgtg     420
   angtagtgcc ctcgtaggtg tcacgtggan tantggganc aggccgnncn gtnanaagaa     480
   ancanngtga nagtttcncc gtngangcng aactgtccct gngccnnnac gctcccanaa     540
   cntntccaat ngacaatcga gtttccnnnc tccngnaacc tngccgnnnn cnngcccnnc     600
   cantntgnta accccgcgcc cggatcgctc tcnnntcgtt ctcncncnaa ngggntttcn     660
   cnnccgccgt cncnnccccg cnncc                                           685
<210> 13
   <211> 694
   <212> DNA
   <213> Homo sapien
<220>
   <221> misc_feature
   <222> (1)...(694)
   <223> n = A,T,C or G
<400> 13
   cactagtcac tcattagcgt tttcaatagg gctcttaagt ccagtagatt acgggtagtc      60
   agttgacgaa gatctggttt acaagaacta attaaatgtt tcattgcatt tttgtaagaa     120
   cagaataatt ttataaaatg tttgtagttt ataattgccg aaaataattt aaagacactt     180
   tttctctgtg tgtgcaaatg tgtgtttgtg atccattttt tttttttttt taggacacct     240
   gtttactagc tagctttaca atatgccaaa aaaggatttc tccctgaccc catccgtggt     300
   tcaccctctt ttccccccat gctttttgcc ctagtttata acaaaggaat gatgatgatt     360
   taaaaagtag ttctgtatct tcagtatctt ggtcttccag aaccctctgg ttgggaaggg     420
   gatcattttt tactggtcat ttccctttgg agtgtactac tttaacagat ggaaagaact     480
   cattggccat ggaaacagcc gangtgttgg gagccagcag tgcatggcac cgtccggcat     540
   ctggcntgat tggtctggct gccgtcattg tcagcacagt gccatgggac atggggaana     600
   ctgactgcac ngccaatggt tttcatgaag aatacngcat ncncngtgat cacgtnancc     660
   angacgctat gggggncana gggccanttg cttc                                 694
<210> 14
   <211> 679
   <212> DNA
   <213> Homo sapien
<220>
   <221> misc_feature
   <222> (1)...(679)
   <223> n = A,T,C or G
<400> 14
   cagccgcctg catctgtatc cagcgccang tcccgccagt cccagctgcg cgcgcccccc     60
   agtcccgnac ccgttcggcc cangctnagt tagncctcac catnccggtc aaaggangca    120
   ccaagtgcat caaatacctg cngtncggat ntaaattcat cttctggctt gccgggattg    180
   ctgtccntgc cattggacta nggctccgat ncgactctca gaccanganc atcttcganc    240
   naganactaa tnatnattnt tccagcttct acacaggagt ctatattctg atcggatccg    300
   gcnccctcnt gatgctggtg ggcttcctga gctgctgcgg ggctgtgcaa gagtcccant    360
   gcatgctggg actgttcttc ggcttcntct tggtgatatn cgccattgaa atacctgcgg    420
   ccatctgggg atattccact ncgatnatgt gattaaggaa ntccacggag ttttacaagg    480
   acacgtacaa cnacctgaaa accnnggatg anccccaccg ggaancnctg aangccatcc    540
   actatgcgtt gaactgcaat ggtttggctg gggnccttga acaatttaat cncatacatc    600
   tggccccann aaaggacntn ctcganncct tcnccgtgna attcngttct gatnccatca    660
   cagaagtctc gaacaatcc                                                 679
<210> 15
   <211> 695
   <212> DNA
   <213> Homo sapien
<220>
   <221> misc_feature
   <222> (1)...(695)
   <223> n = A,T,C or G
<400> 15
   actagtggat aaaggccagg gatgctgctc aacctcctac catgtacagg gacgtctccc     60
   cattacaact acccaatccg aagtgtcaac tgtgtcagga ctaanaaacc ctggttttga    120
   ttaaaaaagg gcctgaaaaa aggggagcca caaatctgtc tgcttcctca cnttantcnt    180
   tggcaaatna gcattctgtc tcnttggctg cngcctcanc ncaaaaaanc ngaactcnat    240
   cnggcccagg aatacatctc ncaatnaacn aaattganca aggcnntggg aaatgccnga    300
   tgggattatc ntccgcttgt tgancttcta agtttcnttc ccttcattcn accctgccag    360
   ccnagttctg ttagaaaaat gccngaattc naacnccggt tttcntactc ngaatttaga    420
   tctncanaaa cttcctggcc acnattcnaa ttnanggnca cgnacanatn ccttccatna    480
   ancncacccc acntttgana gccangacaa tgactgcntn aantgaaggc ntgaaggaan    540
   aactttgaaa ggaaaaaaaa ctttgtttcc ggccccttcc aacncttctg tgttnancac    600
   tgccttctng naaccctgga agcccngnga cagtgttaca tgttgttcta nnaaacngac    660
   ncttnaatnt cnatcttccc nanaacgatt ncncc                               695
<210> 16
   <211> 669
   <212> DNA
   <213> Homo sapien
<220>
   <221> mise feature
   <222> (1)... (669)
   <223> n = A,T,C or G
<400> 16
   cgccgaagca gcagcgcagg ttgtccccgt ttcccctccc ccttcccttc tccggttgcc      60
   ttcccgggcc ccttacactc cacagtcccg gtcccgccat gtcccagaaa caagaagaag     120
   agaaccctgc ggaggagacc ggcgaggaga agcaggacac gcaggagaaa gaaggtattc     180
   tgcctgagag agctgaagag gcaaagctaa aggccaaata cccaagccta ggacaaaagc     240
   ctggaggctc cgacttcctc atgaagagac tccagaaagg gcaaaagtac tttgactcng     300
   gagactacaa catggccaaa gccaacatga agaataagca gctgccaagt gcangaccag     360
   acaagaacct ggtgactggt gatcacatcc ccaccccaca ggatctgccc agagaaagtc     420
   ctcgctcgtc accagcaagc ttgcgggtgg ccaagttgaa tgatgctgcc ggggctctgc     480
   canatctgag acgcttccct ccctgcccca cccgggtcct gtgctggctc ctgcccttcc     540
   tgcttttgca gccangggtc aggaagtggc ncnggtngtg gctggaaagc aaaacccttt     600
   cctgttggtg tcccacccat ggagcccctg gggcgagccc angaacttga ncctttttgt      660
   tntcttncc                                                             669
<210> 17
   <211> 697
   <212> DNA
   <213> Homo sapien
<220>
   <221> mise feature
   <222> (1)...(697)
   <223> n = A,T,C or G
<400> 17
   gcaagatatg gacaactaag tgagaaggta atnctctact gctctagntn ctccnggcnn      60
   gacgcgctga ggagannnac gctggcccan ctgccggcca cacacgggga tcntggtnat     120
   gcctgcccan gggancccca ncnctcggan cccatntcac acccgnnccn tncgcccacn    180
   ncctggctcn cncngcccng nccagctcnc gnccccctcc gccnnnctcn ttnncntctc    240
   cncnccctcc ncnacnacct cctacccncg gctccctccc cagccccccc ccgcaancct     300
   ccacnacncc ntcnncncga ancnccnctc gcnctcngcc ccngccccct gccccccgcc     360
   cncnacnncg cgntcccccg cgcncgcngc ctcnccccct cccacnacag ncncacccgc    420
   agncacgcnc tccgcccnct gacgccccnn cccgccgcgc tcaccttcat ggnccnacng     480
   ccccgctcnc nccnctgcnc gccgncnngg cgccccgccc cnnccgngtn ccncncgnng     540
   ccccngcngn angcngtgcg cnncangncc gngccgnncn ncaccctccg nccnccgccc     600
   cgcccgctgg gggctcccgc cncgcggntc antccccncc cntncgccca ctntccgntc     660
   cnncnctcnc gctcngcgcn cgcccnccnc ccccccc                              697
<210> 18
   <211> 670
   <212> DNA
   <213> Homo sapien
<220>
   <221> misc_feature
   <222> (1)...(670)
   <223> n = A,T,C or G
<400> 18
   ctcgtgtgaa gggtgcagta cctaagccgg agcggggtag aggcgggccg gcaccccctt     60
   ctgacctcca gtgccgccgg cctcaagatc agacatggcc cagaacttga acgacttggc    120
   gggacggctg cccgccgggc cccggggcat gggcacggcc ctgaagctgt tgctgggggc    180
   cggcgccgtg gcctacggtg tgcgcgaatc tgtgttcacc gtggaaggcg ggcncagagc    240
   catcttcttc aatcggatcg gtggagtgca caggacacta tcctgggccg anggccttca    300
   cttcaggatc cttggttcca gtaccccanc atctatgaca ttcgggccag acctcgaaaa    360
   aatctcctcc ctacaggctc caaagaccta cagatggtga atatctccct gcgagtgttg    420
   tctcgaccaa tgctcangaa cttcctaaca tgttccancg cctaagggct ggactacnaa    480
   gaacgantgt tgccgtccat tgtcacgaag tgctcaagaa tttnggtggc caagttcaat    540
   gncctcacnn ctgatcnccc agcggggcca agttanccct ggttgatccc cgggganctg   600
   acnnaaaagg gccaaggact tcccctcatc ctggataatg tggccntcac aaagctcaac    660
   tttanccacc                                                           670
<210> 19
   <211> 606
   <212> DNA
   <213> Homo sapien
<220>
   <221> misc_feature
   <222> (1)...(606)
   <223> n = A,T,C or G
<400> 19
   actagtgcca acctcagctc ccaggccagt tctctgaatg tcgaggagtt ccaggatctc     60
   tggcctcagt tgtccttggt tattgatggg ggacaaattg gggatggcca gagccccgag    120
   tgtcgccttg gctcaactgt ggttgatttg tctgtgcccg gaaagtttgg catcattcgt    180
   ccaggctgtg ccctggaaag tactacagcc atcctccaac agaagtacgg actgctcccc    240
   tcacatgcgt cctacctgtg aaactctggg aagcaggaag gcccaagacc tggtgctgga    300
   tactatgtgt ctgtccactg acgactgtca aggcctcatt tgcagaggcc accggagcta    360
   gggcactagc ctgactttta aggcagtgtg tctttctgag cactgtagac caagcccttg    420
   gagctgctgg tttagccttg cacctgggga aaggatgtat ttatttgtat tttcatatat    480
   cagccaaaag ctgaatggaa aagttnagaa cattcctagg tggccttatt ctaataagtt    540
   tcttctgtct gttttgtttt tcaattgaaa agttattaaa taacagattt agaatctagt    600
   gagacc                                                               606
<210> 20
   <211> 449
   <212> DNA
   <213> Homo sapien
<400> 20
   actagtaaac aacagcagca gaaacatcag tatcagcagc gtcgccagca ggagaatatg     60
   cagcgccaga gccgaggaga acccccgctc cctgaggagg acctgtccaa actcttcaaa    120
   ccaccacagc cgcctgccag gatggactcg ctgctcattg caggccagat aaacacttac    180
   tgccagaaca tcaaggagtt cactgcccaa aacttaggca agctcttcat ggcccaggct    240
   cttcaagaat acaacaacta agaaaaggaa gtttccagaa aagaagttaa catgaactct    300
   tgaagtcaca ccagggcaac tcttggaaga aatatatttg catattgaaa agcacagagg    360
   atttctttag tgtcattgcc gattttggct ataacagtgt ctttctagcc ataataaaat    420
   aaaacaaaat cttgactgct tgctcaaaa                                      449
<210> 21
   <211> 409
   <212> DNA
   <213> Homo sapien
<400> 21
   tatcaatcaa ctggtgaata attaaacaat gtgtggtgtg atcatacaaa gggtaccact      60
   caatgataaa aggaacaagc tgcctatatg tggaacaaca tggatgcatt tcagaaactt     120
   tatgttgagt gaaagaacaa acacggagaa catactatgt ggttctcttt atgtaacatt     180
   acagaaataa aaacagaggc aaccaccttt gaggcagtat ggagtgagat agactggaaa     240
   aaggaaggaa ggaaactcta cgctgatgga aatgtctgtg tcttcattgg gtggtagtta     300
   tgtggggata tacatttgtc aaaatttatt gaactatata ctaaagaact ctgcatttta     360
   ttgggatgta aataatacct caattaaaaa gacaaaaaaa aaaaaaaaa                 409
<210> 22
   <211> 649
   <212> DNA
   <213> Homo sapien
<220>
   <221> misc_feature
   <222> (1)...(649)
   <223> n = A,T,C or G
<400> 22
   acaattttca ttatcttaag cacattgtac atttctacag aacctgtgat tattctcgca      60
   tgataaggat ggtacttgca tatggtgaat tactactgtt gacagtttcc gcagaaatcc     120
   tatttcagtg gaccaacatt gtggcatggc agcaaatgcc aacattttgt ggaatagcag     180
   caaatctaca agagaccctg gttggttttt cgttttgttt tctttgtttt ttcccccttc      240
   tcctgaatca gcagggatgg aangagggta gggaagttat gaattactcc ttccagtagt     300
   agctctgaag tgtcacattt aatatcagtt ttttttaaac atgattctag ttnaatgtag     360
   aagagagaag aaagaggaag tgttcacttt tttaatacac tgatttagaa atttgatgtc     420
   ttatatcagt agttctgagg tattgatagc ttgctttatt tctgccttta cgttgacagt     480
   gttgaagcag ggtgaataac taggggcata tatatttttt ttttttgtaa gctgtttcat      540
   gatgttttct ttggaatttc cggataagtt caggaaaaca tctgcatgtt gttatctagt     600
   ctgaagttcn tatccatctc attacaacaa aaacncccag aacggnttg                 649
<210> 23
   <211> 669
   <212> DNA
   <213> Homo sapien
<220>
   <221> misc_feature
   <222> (1)...(669)
   <223> n = A,T,C or G
<400> 23
   actagtgccg tactggctga aatccctgca ggaccaggaa gagaaccagt tcagactttg      60
   tactctcagt caccagctct ggaattagat aaattccttg aagatgtcag gaatgggatc     120
   tatcctctga cagcctttgg gctgcctcgg ccccagcagc cacagcagga ggaggtgaca     180
   tcacctgtcg tgcccccctc tgtcaagact ccgacacctg aaccagctga ggtggagact     240
   cgcaaggtgg tgctgatgca gtgcaacatt gagtcggtgg aggagggagt caaacaccac     300
   ctgacacttc tgctgaagtt ggaggacaaa ctgaaccggc acctgagctg tgacctgatg     360
   ccaaatgaga atatccccga gttggcggct gagctggtgc agctgggctt cattagtgag     420
   gctgaccaga gccggttgac ttctctgcta gaagagactt gaacaagttc aattttgcca     480
   ggaacagtac cctcaactca gccgctgtca ccgtctcctc ttagagctca ctcgggccag     540
   gccctgatct gcgctgtggc tgtcctggac gtgctgcacc ctctgtcctt ccccccagtc     600
   agtattacct gtgaagccct tccctccttt attattcagg anggctgggg gggctccttg     660
   nttctaacc                                                             669
<210> 24
   <211> 442
   <212> DNA
   <213> Homo sapien
<400> 24
   actagtacca tcttgacaga ggatacatgc tcccaaaacg tttgttacca cacttaaaaa      60
   tcactgccat cattaagcat cagtttcaaa attatagcca ttcatgattt actttttcca     120
   gatgactatc attattctag tcctttgaat ttgtaagggg aaaaaaaaca aaaacaaaaa     180
   cttacgatgc acttttctcc agcacatcag atttcaaatt gaaaattaaa gacatgctat     240
   ggtaatgcac ttgctagtac tacacacttt ggtacaacaa aaaacagagg caagaaacaa     300
   cggaaagaga aaagccttcc tttgttggcc cttaaactga gtcaagatct gaaatgtaga     360
   gatgatctct gacgatacct gtatgttctt attgtgtaaa taaaattgct ggtatgaaat     420
   gacctaaaaa aaaaaaaaga aa                                              442
<210> 25
   <211> 656
   <212> DNA
   <213> Homo sapien
<220>
   <221> mise feature
   <222> (1) .. (656)
   <223> n = A,T,C or G
<400> 25
   tgcaagtacc acacactgtt tgaattttgc acaaaaagtg actgtaggat caggtgatag      60
   ccccggaatg tacagtgtct tggtgcacca agatgccttc taaaggctga cataccttgg   120
   accctaatgg ggcagagagt atagccctag cccagtggtg acatgaccac tccctttggg     180
   aggcctgagg tagaggggag tggtatgtgt tttctcagtg gaagcagcac atgagtgggt     240
   gacaggatgt tagataaagg ctctagttag ggtgtcattg tcatttgaga gactgacaca     300
   ctcctagcag ctggtaaagg ggtgctggan gccatggagg anctctagaa acattagcat     360
   gggctgatct gattacttcc tggcatcccg ctcactttta tgggaagtct tattagangg     420
   atgggacagt tttccatatc cttgctgtgg agctctggaa cactctctaa atttccctct     480
   attaaaaatc actgccctaa ctacacttcc tccttgaagg aatagaaatg gaactttctc     540
   tgacatantt cttggcatgg ggagccagcc acaaatgana atctgaacgt gtccaggttt     600
   ctcctganac tcatctacat agaattggtt aaaccctccc ttggaataag gaaaaa         656
<210> 26
   <211> 434
   <212> DNA
   <213> Homo sapien
<220>
   <221> misc_feature
   <222> (1)...(434)
   <223> n = A,T,C or G
<400> 26
   actagttcag actgccacgc caaccccaga aaatacccca catgccagaa aagtgaagtc       60
   ctaggtgttt ccatctatgt ttcaatctgt ccatctacca ggcctcgcga taaaaacaaa      120
   acaaaaaaac gctgccaggt tttagaagca gttctggtct caaaaccatc aggatcctgc      180
   caccagggtt cttttgaaat agtaccacat gtaaaaggga atttggcttt cacttcatct      240
   aataactgaa ttgtcaggct ttgattgata attgtagaaa taagtagcct tctgttgtgg      300
   gaataagtta taatcagtat tcatctcttt gttttttgtc actcttttct ctctaattgt      360
   gtcatttgta ctgtttgaaa aatatttctt ctatnaaatt aaactaacct gccttaaaaa      420
   aaaaaaaaaa aaaa                                                        434
<210> 27
   <211> 654
   <212> DNA
   <213> Homo sapien
<220>
   <221> misc_feature
   <222> (1)...(654)
   <223> n = A,T,C or G
<400> 27
   actagtccaa cacagtcaga aacattgttt tgaatcctct gtaaaccaag gcattaatct       60
   taataaacca ggatccattt aggtaccact tgatataaaa aggatatcca taatgaatat      120
   tttatactgc atcctttaca ttagccacta aatacgttat tgcttgatga agacctttca      180
   cagaatccta tggattgcag catttcactt ggctacttca tacccatgcc ttaaagaggg      240
   gcagtttctc aaaagcagaa acatgccgcc agttctcaag ttttcctcct aactccattt      300
   gaatgtaagg gcagctggcc cccaatgtgg ggaggtccga acattttctg aattcccatt      360
   ttcttgttcg cggctaaatg acagtttctg tcattactta gattccgatc tttcccaaag      420
   gtgttgattt acaaagaggc cagctaatag cagaaatcat gaccctgaaa gagagatgaa      480
   attcaagctg tgagccaggc agganctcag tatggcaaag gtcttgagaa tcngccattt      540
   ggtacaaaaa aaattttaaa gcntttatgt tataccatgg aaccatagaa anggcaaggg      600
   aattgttaag aanaatttta agtgtccaga cccanaanga aaaaaaaaaa aaaa            654
<210> 28
   <211> 670
   <212> DNA
   <213> Homo sapien
<220>
   <221> misc_feature
   <222> (1)...(670)
   <223> n = A,T,C or G
<400> 28
   cgtgtgcaca tactgggagg atttccacag ctgcacggtc acagccctta cggattgcca 60
   ggaaggggcg aaagatatgt gggataaact gagaaaagaa nccaaaaacc tcaacatcca 120
   aggcagctta ttcgaactct gcggcagcgg caacggggcg gcggggtccc tgctcccggc 180
   gttcccggtg ctcctggtgt ctctctcggc agctttagcg acctgncttt ccttctgagc 240
   gtggggccag ctccccccgc ggcgcccacc cacnctcact ccatgctccc ggaaatcgag 300
   aggaagatca ttagttcttt ggggacgttn gtgattctct gtgatgctga aaaacactca 360
   tatagggaat gtgggaaatc ctganctctt tnttatntcg tntgatttct tgtgttttat 420
   ttgccaaaat gttaccaatc agtgaccaac cnagcacagc caaaaatcgg acntcngctt 480
   tagtccgtct tcacacacag aataagaaaa cggcaaaccc accccacttt tnantttnat 540
   tattactaan ttttttctgt tgggcaaaag aatctcagga acngccctgg ggccnccgta 600
   ctanagttaa ccnagctagt tncatgaaaa atgatgggct ccncctcaat gggaaagcca 660
   agaaaaagnc 670
<210> 29
   <211> 551
   <212> DNA
   <213> Homo sapien
<220>
   <221> mise feature
   <222> (1)...(551)
   <223> n = A,T,C or G
<400> 29
   actagtcctc cacagcctgt gaatccccct agacctttca agcatagtga gcggagaaga 60
   agatctcagc gtttagccac cttacccatg cctgatgatt ctgtagaaaa ggtttcttct 120
   ccctctccag ccactgatgg gaaagtattc tccatcagtt ctcaaaatca gcaagaatct 180
   tcagtaccag aggtgcctga tgttgcacat ttgccacttg agaagctggg accctgtctc 240
   cctcttgact taagtcgtgg ttcagaagtt acagcaccgg tagcctcaga ttcctcttac 300
   cgtaatgaat gtcccagggc agaaaaagag gatacncaga tgcttccaaa tccttcttcc 360
   aaagcaatag ctgatgggaa gaggagctcc agcagcagca ggaatatcga aaacagaaaa 420
   aaaagtgaaa ttgggaagac aaaagctcaa cagcatttgg taaggagaaa aganaagatg 480
   aggaaggaag agagaagaga gacnaagatc nctacggacc gnnncggaag aagaagaagn 540
   aaaaaanaaa a 551
<210> 30
   <211> 684
   <212> DNA
   <213> Homo sapien
<220>
   <221> misc_feature
   <222> (1)...(684)
   <223> n = A,T,C or G
<400> 30
   actagttcta tctggaaaaa gcccgggttg gaagaagctg tggagagtgc gtgtgcaatg      60
   cgagactcat ttcttggaag catccctggc aaaaatgcag ctgagtacaa ggttatcact     120
   gtgatagaac ctggactgct ttttgagata atagagatgc tgcagtctga agagacttcc     180
   agcacctctc agttgaatga attaatgatg gcttctgagt caactttact ggctcaggaa     240
   ccacgagaga tgactgcaga tgtaatcgag cttaaaggga aattcctcat caacttagaa     300
   ggtggtgata ttcgtgaaga gtcttcctat aaagtaattg tcatgccgac tacgaaagaa     360
   aaatgccccc gttgttggaa gtatacagcg ggagtcttca gatacactgt gtcctcgatg     420
   tgcagaagtt gtcagtggga aaatagtatt aacagctcac tcgagcaaga accctcctga     480
   cagtactggg ctagaagttt ggatggatta tttacaatat aggaaagaaa gccaagaatt     540
   aggtnatgag tggatgagta aatggtggan gatggggaat tcaaatcaga attatggaag     600
   aagttnttcc tgttactata gaaaggaatt atgtttattt acatgcagaa aatatanatg     660
   tgtggtgtgt accgtggatg gaan                                            684
<210> 31
   <211> 654
   <212> DNA
   <213> Homo sapien
<220>
   <221> misc_feature
   <222> (1)...(654)
   <223> n = A,T,C or G
<400> 31
   gcgcagaaaa ggaaccaata tttcagaaac aagcttaata ggaacagctg cctgtacatc      60
   aacatcttct cagaatgacc cagaagttat catcgtggga gctggcgtgc ttggctctgc     120
   tttggcagct gtgctttcca gagatggaag aaaggtgaca gtcattgaga gagacttaaa     180
   agagcctgac agaatagttg gagaattcct gcagccgggt ggttatcatg ttctcaaaga     240
   ccttggtctt ggagatacag tggaaggtct tgatgcccag gttgtaaatg gttacatgat     300
   tcatgatcag ggaaagcaaa tcagangttc agattcctta ccctctgtca gaaaacaatc     360
   aagtgcagag tggaagagct ttccatcacg gaagattcat catgagtctc cggaaagcag     420
   ctatggcaga gcccaatgca aagtttattg aaggtgttgt gttacagtta ttagaggaag     480
   atgatgttgt gatgggagtt cagtacaagg ataaagagac tgggagatat caaggaactc     540
   catgctccac tgactgttgt tgcagatggg cttttctcca anttcaggaa aagcctggtc     600
   tcaataaagt ttctgtatca ctcatttggt tggcttctta tgaagaatgc nccc           654
<210> 32
   <211> 673
   <212> DNA
   <213> Homo sapien
<220>
   <221> misc_feature
   <222> (1) .. (673)
   <223> n = A,T,C or G
<400> 32
   actagtgaag aaaaagaaat tctgatacgg gacaaaaatg ctcttcaaaa catcattctt      60
   tatcacctga caccaggagt tttcattgga aaaggatttg aacctggtgt tactaacatt     120
   ttaaagacca cacaaggaag caaaatcttt ctgaaagaag taaatgatac acttctggtg     180
   aatgaattga aatcaaaaga atctgacatc atgacaacaa atggtgtaat tcatgttgta     240
   gataaactcc tctatccagc agacacacct gttggaaatg atcaactgct ggaaatactt     300
   aataaattaa tcaaatacat ccaaattaag tttgttcgtg gtagcacctt caaagaaatc     360
   cccgtgactg tctatnagcc aattattaaa aaatacacca aaatcattga tgggagtgcc     420
   tgtgggaaat aactgaaaaa gagaccgaga agaacgaatc attacaggtc ctgaaataaa     480
   atacctagga tttctactgg aggtggagaa acagaagaac tctgaagaaa ttgttacaag     540
   aagangtccc aaggtcacca aattcattga aggtggtgat ggtctttatt tgaagatgaa     600
   gaaattaaaa gacgcttcag ggagacnccc catgaaggaa ttgccagcca caaaaaaatt     660
   cagggattag aaa                                                        673
<210> 33
   <211> 673
   <212> DNA
   <213> Homo sapien
<220>
   <221> misc_feature
   <222> (1)...(673)
   <223> n = A,T,C or G
<400> 33
   actagttatt tactttcctc cgcttcagaa ggtttttcag actgagagcc taagcatact      60
   ggatctgttg tttcttttgg gtctcacctc atcagtgtgc atagtggcag aaattataaa     120
   gaaggttgaa aggagcaggg aaaagatcca gaagcatgtt agttcgacat catcatcttt     180
   tcttgaagta tgatgcatat tgcattattt tatttgcaaa ctaggaattg cagtctgagg     240
   atcatttaga agggcaagtt caagaggata tgaagatttg agaacttttt aactattcat     300
   tgactaaaaa tgaacattaa tgttnaagac ttaagacttt aacctgctgg cagtcccaaa     360
   tgaaattatg caactttgat atcatattcc ttgatttaaa ttgggctttt gtgattgant     420
   gaaactttat aaagcatatg gtcagttatt tnattaaaaa ggcaaaacct gaaccacctt     480
   ctgcacttaa agaagtctaa cagtacaaat acctatctat cttagatgga tntatttntt     540
   tntattttta aatattgtac tatttatggt nggtggggct ttcttactaa tacacaaatn     600
   aatttatcat ttcaanggca ttctatttgg gtttagaagt tgattccaag nantgcatat     660
   ttcgctactg tnt                                                        673
<210> 34
   <211> 684
   <212> DNA
   <213> Homo sapien
<220>
   <221> misc_feature
   <222> (1)...(684)
   <223> n = A,T,C or G
<400> 34
   actagtttat tcaagaaaag aacttactga ttcctctgtt cctaaagcaa gagtggcagg      60
   tgatcagggc tggtgtagca tccggttcct ttagtgcagc taactgcatt tgtcactgat     120
   gaccaaggag gaaatcacta agacatttga gaagcagtgg tatgaacgtt cttggacaag     180
   ccacagttct gagccttaac cctgtagttt gcacacaaga acgagctcca cctccccttc     240
   ttcaggagga atctgtgcgg atagattggc tggacttttc aatggttctg ggttgcaagt     300
   gggcactgtt atggctgggt atggagcgga cagccccagg aatcagagcc tcagcccggc     360
   tgcctggttg gaaggtacag gtgttcagca ccttcggaaa aagggcataa agtngtgggg     420
   gacaattctc agtccaagaa gaatgcattg accattgctg gctatttgct tncctagtan     480
   gaattggatn catttttgac cangatnntt ctnctatgct ttnttgcaat gaaatcaaat     540
   cccgcattat ctacaagtgg tatgaagtcc tgcnnccccc agagaggctg ttcaggcnat     600
   gtcttccaag ggcagggtgg gttacaccat tttacctccc ctctcccccc agattatgna     660
   cncagaagga atttntttcc tccc                                            684
<210> 35
   <211> 614
   <212> DNA
   <213> Homo sapien
<220>
   <221> misc_feature
   <222> (1)...(614)
   <223> n = A,T,C or G
<400> 35
   actagtccaa cgcgttngcn aatattcccc tggtagccta cttccttacc cccgaatatt      60
   ggtaagatcg agcaatggct tcaggacatg ggttctcttc tcctgtgatc attcaagtgc     120
   tcactgcatg aagactggct tgtctcagtg tntcaacctc accagggctg tctcttggtc     180
   cacacctcgc tccctgttag tgccgtatga cagcccccat canatgacct tggccaagtc     240
   acggtttctc tgtggtcaat gttggtnggc tgattggtgg aaagtanggt ggaccaaagg     300
   aagncncgtg agcagncanc nccagttctg caccagcagc gcctccgtcc tactngggtg     360
   ttccngtttc tcctggccct gngtgggcta nggcctgatt cgggaanatg cctttgcang     420
   gaaggganga taantgggat ctaccaattg attctggcaa aacnatntct aagattnttn     480
   tgctttatgt ggganacana tctanctctc atttnntgct gnanatnaca ccctactcgt     540
   gntcgancnc gtcttcgatt ttcgganaca cnccantnaa tactggcgtt ctgttgttaa     600
   aaaaaaaaaa aaaa                                                       614
<210> 36
   <211> 686
   <212> DNA
   <213> Homo sapien
<220>
   <221> mise feature
   <222> (1)...(686)
   <223> n = A,T,C or G
<400> 36
   gtggctggcc cggttctccg cttctcccca tcccctactt tcctccctcc ctccctttcc     60
   ctccctcgtc gactgttgct tgctggtcgc agactccctg acccctccct cacccctccc    120
   taacctcggt gccaccggat tgcccttctt ttcctgttgc ccagcccagc cctagtgtca    180
   gggcgggggc ctggagcagc ccgaggcact gcagcagaag ananaaaaga cacgacnaac    240
   ctcagctcgc cagtccggtc gctngcttcc cgccgcatgg caatnagaca gacgccgctc    300
   acctgctctg ggcacacgcg acccgtggtt gatttggcct tcagtggcat cacccttatg    360
   ggtatttctt aatcagcgct tgcaaagatg gttaacctat gctacgccag ggagatacag    420
   gagactggat tggaacattt ttggggtcta aaggtctgtt tggggtgcaa cactgaataa    480
   ggatgccacc aaagcagcta cagcagctgc agatttcaca gcccaagtgt gggatgctgt    540
   ctcagganat naattgataa cctggctcat aacacattgt caagaatgtg gatttcccca    600
   ggatattatt atttgtttac cggggganag gataactgtt tcncntattt taattgaaca    660
   aactnaaaca aaanctaagg aaatcc                                          686
<210> 37
   <211> 681
   <212> DNA
   <213> Homo sapien
<220>
   <221> misc_feature
   <222> (1)...(681)
   <223> n = A,T,C or G
<400> 37
   gagacanacn naacgtcang agaanaaaag angcatggaa cacaanccag gcncgatggc     60
   caccttccca ccagcancca gcgcccccca gcngccccca ngnccggang accangactc    120
   cancctgnat caatctganc tctattcctg gcccatncct acctcggagg tggangccgn    180
   aaaggtcgca cnnncagaga agctgctgcc ancaccancc gccccnnccc tgncgggctn    240
   nataggaaac tggtgaccnn gctgcanaat tcatacagga gcacgcgang ggcacnnnct    300
   cacactgagt tnnngatgan gcctnaccan ggacctnccc cagcnnattg annacnggac    360
   tgcggaggaa ggaagacccc gnacnggatc ctggccggcn tgccaccccc ccacccctag    420
   gattatnccc cttgactgag tctctgaggg gctacccgaa cccgcctcca ttccctacca    480
   natnntgctc natcgggact gacangctgg ggatnggagg ggctatcccc cancatcccc    540
   tnanaccaac agcnacngan natnggggct ccccngggtc ggngcaacnc tcctncaccc    600
   cggcgcnggc cttcggtgnt gtcctccntc aacnaattcc naaanggcgg gccccccngt    660
   ggactcctcn ttgttccctc c                                               681
<210> 38
   <211> 687
   <212> DNA
   <213> Homo sapien
<220>
   <221> misc_feature
   <222> (1)...(687)
   <223> n = A,T,C or G
<400> 38
   canaaaaaaa aaaacatggc cgaaaccagn aagctgcgcg atggcgccac ggcccctctt    60
   ctcccggcct gtgtccggaa ggtttccctc cgaggcgccc cggctcccgc aagcggagga   120
   gagggcggga cntgccgggg ccggagctca naggccctgg ggccgctctg ctctcccgcc   180
   atcgcaaggg cggcgctaac ctnaggcctc cccgcaaagg tccccnangc ggnggcggcg   240
   gggggctgtg anaaccgcaa aaanaacgct gggcgcgcng cgaacccgtc cacccccgcg   300
   aaggananac ttccacagan gcagcgtttc cacagcccan agccacnttt ctagggtgat   360
   gcaccccagt aagttcctgn cggggaagct caccgctgtc aaaaaanctc ttcgctccac   420
   cggcgcacna aggggangan ggcangangc tgccgcccgc acaggtcatc tgatcacgtc   480
   gcccgcccta ntctgctttt gtgaatctcc actttgttca accccacccg ccgttctctc   540
   ctccttgcgc cttcctctna ccttaanaac cagcttcctc tacccnatng tanttnctct   600
   gcncnngtng aaattaattc ggtccnccgg aacctcttnc ctgtggcaac tgctnaaaga   660
   aactgctgtt ctgnttactg cngtccc                                       687
<210> 39
   <211> 695
   <212> DNA
   <213> Homo sapien
<220>
   <221> mise_feature
   <222> (1)...(695)
   <223> n = A,T,C or G
<400> 39
   actagtctgg cctacaatag tgtgattcat gtaggacttc tttcatcaat tcaaaacccc    60
   tagaaaaacg tatacagatt atataagtag ggataagatt tctaacattt ctgggctctc   120
   tgacccctgc gctagactgt ggaaagggag tattattata gtatacaaca ctgctgttgc   180
   cttattagtt ataacatgat aggtgctgaa ttgtgattca caatttaaaa acactgtaat   240
   ccaaactttt ttttttaact gtagatcatg catgtgaatg ttaatgttaa tttgttcaan   300
   gttgttatgg gtagaaaaaa ccacatgcct taaaatttta aaaagcaggg cccaaactta   360
   ttagtttaaa attaggggta tgtttccagt ttgttattaa ntggttatag ctctgtttag   420
   aanaaatcna ngaacangat ttngaaantt aagntgacat tatttnccag tgacttgtta   480
   atttgaaatc anacacggca ccttccgttt tggtnctatt ggnntttgaa tccaancngg   540
   ntccaaatct tnttggaaac ngtccnttta acttttttac nanatcttat ttttttattt   600
   tggaatggcc ctatttaang ttaaaagggg ggggnnccac naccattcnt gaataaaact   660
   naatatatat ccttggtccc ccaaaattta aggng                              695
<210> 40
   <211> 674
   <212> DNA
   <213> Homo sapien
<220>
   <221> misc feature
   <222> (1) ... (674)
   <223> n = A,T,C or G
<400> 40
   actagtagtc agttgggagt ggttgctata ccttgacttc atttatatga atttccactt      60
   tattaaataa tagaaaagaa aatcccggtg cttgcagtag agttatagga cattctatgc     120
   ttacagaaaa tatagccatg attgaaatca aatagtaaag gctgttctgg ctttttatct     180
   tcttagctca tcttaaataa gtagtacact tgggatgcag tgcgtctgaa gtgctaatca     240
   gttgtaacaa tagcacaaat cgaacttagg atgtgtttct tctcttctgt gtttcgattt     300
   tgatcaattc tttaattttg ggaacctata atacagtttt cctattcttg gagataaaaa     360
   ttaaatggat cactgatatt taagtcattc tgcttctcat ctnaatattc catattctgt     420
   attagganaa antacctccc agcacagccc cctctcaaac cccacccaaa accaagcatt     480
   tggaatgagt ctcctttatt tccgaantgt ggatggtata acccatatcn ctccaatttc     540
   tgnttgggtt gggtattaat ttgaactgtg catgaaaagn ggnaatcttt nctttgggtc     600
   aaantttncc ggttaatttg nctngncaaa tccaatttnc tttaagggtg tctttataaa     660
   atttgctatt cngg                                                       674
<210> 41
   <211> 657
   <212> DNA
   <213> Homo sapien
<220>
   <221> misc_feature
   <222> (1)...(657)
   <223> n = A,T,C or G
<400> 41
   gaaacatgca agtaccacac actgtttgaa ttttgcacaa aaagtgactg tagggatcag      60
   gtgatagccc cggaatgtac agtgtcttgg tgcaccaaga tgccttctaa aggctgacat     120
   accttgggac cctaatgggg cagagagtat agccctagcc cagtggtgac atgaccactc     180
   cctttgggag gctgaagtta aagggaatgg tatgtgtttt ctcatggaag cagcacatga     240
   atnggtnaca ngatgttaaa ntaaggntct antttgggtg tcttgtcatt tgaaaaantg     300
   acacactcct ancanctggt aaaggggtgc tggaagccat ggaagaactc taaaaacatt     360
   agcatgggct gatctgatta cttcctggca tcccgctcac ttttatggga agtcttatta     420
   naaggatggg ananttttcc atatccttgc tgttggaact ctggaacact ctctaaattt     480
   ccctctatta aaaatcactg nccttactac acttcctcct tganggaata gaaatggacc     540
   tttctctgac ttagttcttg gcatggganc cagcccaaat taaaatctga cttntccggt     600
   ttctccngaa ctcacctact tgaattggta aaacctcctt tggaattagn aaaaacc        657
<210> 42
   <211> 389
   <212> DNA
   <213> Homo sapien
<220>
   <221> misc_feature
   <222> (1)...(389)
   <223> n = A,T,C or G
<400> 42
   actagtgctg aggaatgtaa acaagtttgc tgggccttgc gagacttcac caggttgttt     60
   cgatagctca cactcctgca ctgtgcctgt cacccaggaa tgtctttttt aattagaaga    120
   caggaagaaa acaaaaacca gactgtgtcc cacaatcaga aacctccgtt gtggcagang    180
   ggccttcacc gccaccaggg tgtcccgcca gacagggaga gactccagcc ttctgaggcc    240
   atcctgaaga attcctgttt gggggttgtg aaggaaaatc acccggattt aaaaagatgc    300
   tgttgcctgc ccgcgtngtn gggaagggac tggtttcctg gtgaatttct taaaagaaaa    360
   atattttaag ttaagaaaaa aaaaaaaaa                                      389
<210> 43
   <211> 279
   <212> DNA
   <213> Homo sapien
<400> 43
   actagtgaca agctcctggt cttgagatgt cttctcgtta aggagatggg ccttttggag     60
   gtaaaggata aaatgaatga gttctgtcat gattcactat tctagaactt gcatgacctt    120
   tactgtgtta gctctttgaa tgttcttgaa attttagact ttctttgtaa acaaataata    180
   tgtccttatc attgtataaa agctgttatg tgcaacagtg tggagatcct tgtctgattt    240
   aataaaatac ttaaacactg aaaaaaaaaa aaaaaaaaa                           279
<210> 44
   <211> 449
   <212> DNA
   <213> Homo sapien
<220>
   <221> misc_feature
   <222> (1)...(449)
   <223> n = A,T,C or G
<400> 44
   actagtagca tcttttctac aacgttaaaa ttgcagaagt agcttatcat taaaaaacaa     60
   caacaacaac aataacaata aatcctaagt gtaaatcagt tattctaccc cctaccaagg    120
   atatcagcct gttttttccc ttttttctcc tgggaataat tgtgggcttc ttcccaaatt    180
   tctacagcct ctttcctctt ctcatgcttg agcttccctg tttgcacgca tgcgttgtgc    240
   aagantgggc tgtttngctt ggantncggt ccnagtggaa ncatgctttc ccttgttact    300
   gttggaagaa actcaaacct tcnancccta ggtgttncca ttttgtcaag tcatcactgt    360
   atttttgtac tggcattaac aaaaaaagaa atnaaatatt gttccattaa actttaataa    420
   aactttaaaa gggaaaaaaa aaaaaaaaa                                      449
<210> 45
   <211> 559
   <212> DNA
   <213> Homo sapien
<220>
   <221> misc_feature
   <222> (1)...(559)
   <223> n = A,T,C or G
<400> 45
   actagtgtgg gggaatcacg gacacttaaa gtcaatctgc gaaataattc ttttattaca      60
   cactcactga agtttttgag tcccagagag ccattctatg tcaaacattc caagtactct     120
   ttgagagccc agcattacat caacatgccc gtgcagttca aaccgaagtc cgcaggcaaa     180
   tttgaagctt tgcttgtcat tcaaacagat gaaggcaaga gtattgctat tcgactaatt     240
   ggtgaagctc ttggaaaaaa ttnactagaa tactttttgt gttaagttaa ttacataagt     300
   tgtattttgt taactttatc tttctacact acaattatgc ttttgtatat atattttgta     360
   tgatggatat ctataattgt agattttgtt tttacaagct aatactgaag actcgactga     420
   aatattatgt atctagccca tagtattgta cttaactttt acagggtgaa aaaaaaattc     480
   tgtgtttgca ttgattatga tattctgaat aaatatggga atatatttta atgtgggtaa     540
   aaaaaaaaaa aaaaaggaa                                                  559
<210> 46
   <211> 731
   <212> DNA
   <213> Homo sapien
<220>
   <221> misc_feature
   <222> (1)...(731)
   <223> n = A,T,C or G
<400> 46
   actagttcta gtaccatggc tgtcatagat gcaaccatta tattccattt agtttcttcc      60
   tcaggttccc taacaattgt ttgaaactga atatatatgt ttatgtatgt gtgtgtgttc     120
   actgtcatgt atatggtgta tatgggatgt gtgcagtttt cagttatata tatattcata     180
   tatacatatg catatatatg tataatatac atatatacat gcatacactt gtataatata     240
   catatatata cacatatatg cacacatatn atcactgagt tccaaagtga gtctttattt     300
   ggggcaattg tattctctcc ctctgtctgc tcactgggcc tttgcaagac atagcaattg     360
   cttgatttcc tttggataag agtcttatct tcggcactct tgactctagc cttaacttta     420
   gatttctatt ccagaatacc tctcatatct atcttaaaac ctaaganggg taaagangtc     480
   ataagattgt agtatgaaag antttgctta gttaaattat atctcaggaa actcattcat     540
   ctacaaatta aattgtaaaa tgatggtttg ttgtatctga aaaaatgttt agaacaagaa     600
   atgtaactgg gtacctgtta tatcaaagaa cctcnattta ttaagtctcc tcatagccan     660
   atccttatat ngccctctct gacctgantt aatananact tgaataatga atagttaatt     720
   taggnttggg c                                                          731
<210> 47
   <211> 640
   <212> DNA
   <213> Homo sapien
<220>
   <221> misc_feature
   <222> (1)...(640)
   <223> n = A,T,C or G
<400> 47
   tgcgngccgg tttggccctt ctttgtanga cactttcatc cgccctgaaa tcttcccgat      60
   cgttaataac tcctcaggtc cctgcctgca cagggttttt tcttantttg ttgcctaaca     120
   gtacaccaaa tgtgacatcc tttcaccaat atngattnct tcataccaca tcntcnatgg     180
   anacgactnc aacaattttt tgatnacccn aaanactggg ggctnnaana agtacantct     240
   ggagcagcat ggacctgtcn gcnactaang gaacaanagt nntgaacatt tacacaacct     300
   ttggtatgtc ttactgaaag anagaaacat gcttctnncc ctagaccacg aggncaaccg     360
   caganattgc caatgccaag tccgagcggt tagatcaggt aatacattcc atggatgcat     420
   tacatacntt gtccccgaaa nanaagatgc cctaanggct tcttcanact ggtccngaaa     480
   acanctacac ctggtgcttg ganaacanac tctttggaag atcatctggc acaagttccc     540
   cccagtgggt tttnccttgg cacctanctt accanatcna ttcggaancc attctttgcc     600
   ntggcnttnt nttgggacca ntcttctcac aactgnaccc                           640
<210> 48
   <211> 257
   <212> DNA
   <213> Homo sapien
<400> 48
   actagtatat gaaaatgtaa atatcacttg tgtactcaaa caaaagttgg tcttaagctt      60
   ccaccttgag cagccttgga aacctaacct gcctctttta gcataatcac attttctaaa     120
   tgattttctt tgttcctgaa aaagtgattt gtattagttt tacatttgtt ttttggaaga     180
   ttatatttgt atatgtatca tcataaaata tttaaataaa aagtatcttt agagtgaaaa     240
   aaaaaaaaaa aaaaaaa                                                    257
<210> 49
   <211> 652
   <212> DNA
   <213> Homo sapien
<220>
   <221> misc_feature
   <222> (1)...(652)
   <223> n = A,T,C or G
<400> 49
   actagttcag atgagtggct gctgaagggg cccccttgtc attttcatta taacccaatt      60
   tccacttatt tgaactctta agtcataaat gtataatgac ttatgaatta gcacagttaa     120
   gttgacacta gaaactgccc atttctgtat tacactatca aataggaaac attggaaaga     180
   tggggaaaaa aatcttattt taaaatggct tagaaagttt tcagattact ttgaaaattc     240
   taaacttctt tctgtttcca aaacttgaaa atatgtagat ggactcatgc attaagactg     300
   ttttcaaagc tttcctcaca tttttaaagt gtgattttcc ttttaatata catatttatt     360
   ttctttaaag cagctatatc ccaacccatg actttggaga tatacctatn aaaccaatat     420
   aacagcangg ttattgaagc agctttctca aatgttgctt cagatgtgca agttgcaaat     480
   tttattgtat ttgtanaata caatttttgt tttaaactgt atttcaatct atttctccaa     540
   gatgcttttc atatagagtg aaatatccca ngataactgc ttctgtgtcg tcgcatttga     600
   cgcataactg cacaaatgaa cagtgtatac ctcttggttg tgcattnacc cc             652
<210> 50
   <211> 650
   <212> DNA
   <213> Homo sapien
<220>
   <221> mise feature
   <222> (1) ... (650)
   <223> n = A,T,C or G
<400> 50
   ttgcgctttg atttttttag ggcttgtgcc ctgtttcact tatagggtct agaatgcttg     60
   tgttgagtaa aaaggagatg cccaatattc aaagctgcta aatgttctct ttgccataaa    120
   gactccgtgt aactgtgtga acacttggga tttttctcct ctgtcccgag gtcgtcgtct    180
   gctttctttt ttgggttctt tctagaagat tgagaaatgc atatgacagg ctgagancac    240
   ctccccaaac acacaagctc tcagccacan gcagcttctc cacagcccca gcttcgcaca    300
   ggctcctgga nggctgcctg ggggaggcag acatgggagt gccaaggtgg ccagatggtt    360
   ccaggactac aatgtcttta tttttaactg tttgccactg ctgccctcac ccctgcccgg    420
   ctctggagta ccgtctgccc canacaagtg ggantgaaat gggggtgggg gggaacactg    480
   attcccantt agggggtgcc taactgaaca gtagggatan aaggtgtgaa cctgngaant    540
   gcttttataa attatnttcc ttgttanatt tattttttaa tttaatctct gttnaactgc    600
   ccngggaaaa ggggaaaaaa aaaaaaaaat tctntttaaa cacatgaaca               650

<210> 51
   <211> 545
   <212> DNA
   <213> Homo sapien
<220>
   <221> misc_feature
   <222> (1)...(545)
   <223> n = A,T,C or G
<400> 51
   tggcgtgcaa ccagggtagc tgaagtttgg gtctgggact ggagattggc cattaggcct     60
   cctganattc cagctccctt ccaccaagcc cagtcttgct acgtggcaca gggcaaacct    120
   gactcccttt gggcctcagt ttcccctccc cttcatgana tgaaaagaat actacttttt    180
   cttgttggtc taacnttgct ggacncaaag tgtngtcatt attgttgtat tgggtgatgt    240
   gtncaaaact gcagaagctc actgcctatg agaggaanta agagagatag tggatganag    300
   ggacanaagg agtcattatt tggtatagat ccacccntcc caacctttct ctcctcagtc    360
   cctgcncctc atgtntctgg tntggtgagt cctttgtgcc accanccatc atgctttgca    420
   ttgctgccat cctgggaagg gggtgnatcg tctcacaact tgttgtcatc gtttganatg    480
   catgctttct tnatnaaaca aanaaannaa tgtttgacag ngtttaaaat aaaaaanaaa    540
   caaaa                                                                545
<210> 52
   <211> 678
   <212> DNA
   <213> Homo sapien
<220>
   <221> misc_feature
   <222> (1)...(678)
   <223> n = A,T,C or G
<400> 52
   actagtagaa gaactttgcc gcttttgtgc ctctcacagg cgcctaaagt cattgccatg      60
   ggaggaagac gatttggggg gggagggggg gggggcangg tccgtggggc tttccctant     120
   ntatctccat ntccantgnn cnntgtcgcc tcttccctcg tcncattnga anttantccc     180
   tggnccccnn nccctctccn ncctncncct cccccctccg ncncctccnn ctttttntan     240
   ncttccccat ctccntcccc cctnanngtc ccaacnccgn cagcaatnnc ncacttnctc     300
   nctccncncc tccnnccgtt cttctnttct cnacntntnc ncnnntnccn tgccnntnaa     360
   annctctccc cnctgcaanc gattctctcc ctccncnnan ctntccactc cntncttctc     420
   ncncgctcct nttcntcnnc ccacctctcn ccttcgnccc cantacnctc nccncccttn     480
   cgnntcnttn nnntcctcnn accncccncc tcccttcncc cctcttctcc ccggtntntc     540
   tctctcccnc nncncnncct cnncccntcc nngcgnccnt ttccgccccn cnccnccntt     600
   ccttcntcnc cantccatcn cntntnccat nctncctncc nctcacnccc gctncccccn      660
   ntctctttca cacngtcc                                                   678
<210> 53
   <211> 502
   <212> DNA
   <213> Homo sapien
<220>
   <221> misc_feature
   <222> (1)...(502)
   <223> n = A,T,C or G
<400> 53
   tgaagatcct ggtgtcgcca tgggccgccg ccccgcccgt tgttaccggt attgtaagaa      60
   caagccgtac ccaaagtctc gcttctgccg aggtgtccct gatgccaaaa ttcgcatttt     120
   tgacctgggg cggaaaaang caaaantgga tgagtctccg ctttgtggcc acatggtgtc     180
   agatcaatat gagcagctgt cctctgaagc cctgnangct gcccgaattt gtgccaataa     240
   gtacatggta aaaagtngtg gcnaagatgc ttccatatcc gggtgcggnt ccaccccttc     300
   cacgtcatcc gcatcaacaa gatgttgtcc tgtgctgggg ctgacaggct cccaacaggc     360
   atgcgaagtg cctttggaaa acccanggca ctgtggccag ggttcacatt gggccaattn     420
   atcatgttca tccgcaccaa ctgcagaaca angaacntgt naattnaagc cctgcccagg     480
   gncaanttca aatttcccgg cc                                              502
<210> 54
   <211> 494
   <212> DNA
   <213> Homo sapien
<220>
   <221> misc_feature
   <222> (1)...(494)
   <223> n = A,T,C or G
<400> 54
   actagtccaa gaaaaatatg cttaatgtat attacaaagg ctttgtatat gttaacctgt 60
   tttaatgcca aaagtttgct ttgtccacaa tttccttaag acctcttcag aaagggattt 120
   gtttgcctta atgaatactg ttgggaaaaa acacagtata atgagtgaaa agggcagaag 180
   caagaaattt ctacatctta gcgactccaa gaagaatgag tatccacatt tagatggcac 240
   attatgagga ctttaatctt tccttaaaca caataatgtt ttcttttttc ttttattcac 300
   atgatttcta agtatatttt tcatgcagga cagtttttca accttgatgt acagtgactg 360
   tgttaaattt ttctttcagt ggcaacctct ataatcttta aaatatggtg agcatcttgt 420
   ctgttttgaa ngggatatga cnatnaatct atcagatggg aaatcctgtt tccaagttag 480
   aaaaaaaaaa aaaa 494
<210> 55
   <211> 606
   <212> DNA
   <213> Homo sapien
<220>
   <221> misc_feature
   <222> (1)...(606)
   <223> n = A,T,C or G
<400> 55
   actagtaaaa agcagcattg ccaaataatc cctaattttc cactaaaaat ataatgaaat 60
   gatgttaagc tttttgaaaa gtttaggtta aacctactgt tgttagatta atgtatttgt 120
   tgcttccctt tatctggaat gtggcattag cttttttatt ttaaccctct ttaattctta 180
   ttcaattcca tgacttaagg ttggagagct aaacactggg atttttggat aacagactga 240
   cagttttgca taattataat cggcattgta catagaaagg atatggctac cttttgttaa 300
   atctgcactt tctaaatatc aaaaaaggga aatgaagtat aaatcaattt ttgtataatc 360
   tgtttgaaac atgantttta tttgcttaat attanggctt tgcccttttc tgttagtctc 420
   ttgggatcct gtgtaaaact gttctcatta aacaccaaac agttaagtcc attctctggt 480
   actagctaca aattccgttt catattctac ntaacaattt aaattaactg aaatatttct 540
   anatggtcta cttctgtcnt ataaaaacna aacttgantt nccaaaaaaa aaaaaaaaaa 600
   aaaaaa 606
<210> 56
   <211> 183
   <212> DNA
   <213> Homo sapien
<400> 56
   actagtatat ttaaacttac aggcttattt gtaatgtaaa ccaccatttt aatgtactgt 60
   aattaacatg gttataatac gtacaatcct tccctcatcc catcacacaa ctttttttgt 120
   gtgtgataaa ctgattttgg tttgcaataa aaccttgaaa aataaaaaaa aaaaaaaaaa 180
   aaa 183
<210> 57
   <211> 622
   <212> DNA
   <213> Homo sapien
<220>
   <221> misc_feature
   <222> (1)...(622)
   <223> n = A,T,C or G
<400> 57
   actagtcact actgtcttct ccttgtagct aatcaatcaa tattcttccc ttgcctgtgg      60
   gcagtggaga gtgctgctgg gtgtacgctg cacctgccca ctgagttggg gaaagaggat     120
   aatcagtgag cactgttctg ctcagagctc ctgatctacc ccacccccta ggatccagga     180
   ctgggtcaaa gctgcatgaa accaggccct ggcagcaacc tgggaatggc tggaggtggg     240
   agagaacctg acttctcttt ccctctccct cctccaacat tactggaact ctatcctgtt     300
   agggatcttc tgagcttgtt tccctgctgg gtgggacaga agacaaagga gaagggangg     360
   tctacaanaa gcagcccttc tttgtcctct ggggttaatg agcttgacct ananttcatg     420
   gaganaccan aagcctctga tttttaattt ccntnaaatg tttgaagtnt atatntacat     480
   atatatattt ctttnaatnt ttgagtcttt gatatgtctt aaaatccant ccctctgccn     540
   gaaacctgaa ttaaaaccat gaanaaaaat gtttncctta aagatgttan taattaattg     600
   aaacttgaaa aaaaaaaaaa aa                                              622
<210> 58
   <211> 433
   <212> DNA
   <213> Homo sapien
<400> 58
   gaacaaattc tgattggtta tgtaccgtca aaagacttga agaaatttca tgattttgca      60
   gtgtggaagc gttgaaaatt gaaagttact gcttttccac ttgctcatat agtaaaggga     120
   tcctttcagc tgccagtgtt gaataatgta tcatccagag tgatgttatc tgtgacagtc     180
   accagcttta agctgaacca ttttatgaat accaaataaa tagacctctt gtactgaaaa     240
   catatttgtg actttaatcg tgctgcttgg atagaaatat ttttactggt tcttctgaat     300
   tgacagtaaa cctgtccatt atgaatggcc tactgttcta ttatttgttt tgacttgaat     360
   ttatccacca aagacttcat ttgtgtatca tcaataaagt tgtatgtttc aactgaaaaa     420
   aaaaaaaaaa aaa                                                        433
<210> 59
   <211> 649
   <212> DNA
   <213> Homo sapien
<220>
   <221> misc_feature
   <222> (1) ... (649)
   <223> n = A,T,C or G
<400> 59
   actagttatt atctgacttt cnggttataa tcattctaat gagtgtgaag tagcctctgg      60
   tgtcatttgg atttgcattt ctctgatgag tgatgctatc aagcaccttt gctggtgctg     120
   ttggccatat gtgtatgttc cctggagaag tgtctgtgct gagccttggc ccacttttta     180
   attaggcgtn tgtcttttta ttactgagtt gtaaganttc tttatatatt ctggattcta     240
   gacccttatc agatacatgg tttgcaaata ttttctccca ttctgtgggt tgtgttttca     300
   ctttatcgat aatgtcctta gacatataat aaatttgtat tttaaaagtg acttgatttg     360
   ggctgtgcaa ggtgggctca cgcttgtaat cccagcactt tgggagactg aggtgggtgg     420
   atcatatgan gangctagga gttcgaggtc agcctggcca gcatagcgaa aacttgtctc     480
   tacnaaaaat acaaaaatta gtcaggcatg gtggtgcacg tctgtaatac cagcttctca     540
   ggangctgan gcacaaggat cacttgaacc ccagaangaa gangttgcag tganctgaag     600
   atcatgccag ggcaacaaaa atgagaactt gtttaaaaaa aaaaaaaaa                649
<210> 60
   <211> 423
   <212> DNA
   <213> Homo sapien
<220>
   <221> misc_feature
   <222> (1)...(423)
   <223> n = A,T,C or G
<400> 60
   actagttcag gccttccagt tcactgacaa acatggggaa gtgtgcccag ctggctggaa      60
   acctggcagt gataccatca agcctgatgt ccaaaagagc aaagaatatt tctccaagca     120
   gaagtgagcg ctgggctgtt ttagtgccag gctgcggtgg gcagccatga gaacaaaacc     180
   tcttctgtat tttttttttc cattagtana acacaagact cngattcagc cgaattgtgg     240
   tgtcttacaa ggcagggctt tcctacaggg ggtgganaaa acagcctttc ttcctttggt     300
   aggaatggcc tgagttggcg ttgtgggcag gctactggtt tgtatgatgt attagtagag     360
   caacccatta atcttttgta gtttgtatna aacttganct gagaccttaa acaaaaaaaa     420
   aaa                                                                   423
<210> 61
   <211> 423
   <212> DNA
   <213> Homo sapien
<220>
   <221> misc_feature
   <222> (1)...(423)
   <223> n = A,T,C or G
<400> 61
   cgggactgga atgtaaagtg aagttcggag ctctgagcac gggctcttcc cgccgggtcc      60
   tccctcccca gaccccagag ggagaggccc accccgccca gccccgcccc agcccctgct     120
   caggtctgag tatggctggg agtcgggggc cacaggcctc tagctgtgct gctcaagaag     180
   actggatcag ggtanctaca agtggccggg ccttgccttt gggattctac cctgttccta      240
   atttggtgtt ggggtgcggg gtccctggcc cccttttcca cactncctcc ctccngacag      300
   caacctccct tggggcaatt gggcctggnt ctccncccgn tgttgcnacc ctttgttggt      360
   ttaaggnctt taaaaatgtt annttttccc ntgccngggt taaaaaagga aaaaactnaa      420
   aaa                                                                   423
<210> 62
   <211> 683
   <212> DNA
   <213> Homo sapien
<220>
   <221> mise feature
   <222> (1)...(683)
   <223> n = A,T,C or G
<400> 62
   gctggagagg ggtacggact ttcttggagt tgtcccaggt tggaatgaga ctgaactcaa       60
   gaagagaccc taagagactg gggaatggtt cctgccttca ggaaagtgaa agacgcttag      120
   gctgtcaaca cttaaaggaa gtccccttga agcccagagt ggacagacta gacccattga      180
   tggggccact ggccatggtc cgtggacaag acattccngt gggccatggc acaccggggg      240
   ggatcaaaat gtgtacttgt ggggtctcgc cccttgccaa aaccaaacca ntcccactcc      300
   tgtcnttgga ctttcttccc attccctcct ccccaaatgc acttcccctc ctccctctgc      360
   ccctcctgtg tttttggaat tctgtttccc tcaaaattgt taatttttta nttttngacc      420
   atgaacttat gtttggggtc nangttcccc ttnccaatgc atactaatat attaatggtt      480
   atttattttt gaaatatttt ttaatgaact tggaaaaaat tnntggaatt tccttncttc      540
   cnttttnttt ggggggggtg gggggntggg ttaaaatttt tttggaancc cnatnggaaa      600
   ttnttacttg gggcccccct naaaaaantn anttccaatt cttnnatngc ccctnttccn      660
   ctaaaaaaaa ananannaaa aan                                             683
<210> 63
   <211> 731
   <212> DNA
   <213> Homo sapien
<220>
   <221> misc_feature
   <222> (1)...(731)
   <223> n = A,T,C or G
<400> 63
   actagtcata aagggtgtgc gcgtcttcga cgtggcggtc ttggcgccac tgctgcgaga      60
   cccggccctg gacctcaagg tcatccactt ggtgcgtgat ccccgcgcgg tggcgagttc     120
   acggatccgc tcgcgccacg gcctcatccg tgagagccta caggtggtgc gcagccgaga     180
   ccgcgagctc accgcatgcc cttcttggag gccgcgggcc acaagcttgg cgcccanaaa     240
   gaaggcgtng ggggcccgca aantaccacg ctctgggcgc tatggaangt cctcttgcaa     300
   taatattggt tnaaaanctg canaanagcc cctgcanccc cctgaactgg gntgcagggc     360
   cncttacctn gtttggntgc ggttacaaag aacctgtttn ggaaaaccct nccnaaaacc     420
   ttccgggaaa attntncaaa tttttnttgg ggaattnttg ggtaaacccc ccnaaaatgg     480
   gaaacntttt tgccctnnaa antaaaccat tnggttccgg gggccccccc ncaaaaccct     540
   tttttntttt tttntgcccc cantnncccc ccggggcccc tttttttngg ggaaaanccc    600
   cccccctncc nanantttta aaagggnggg anaatttttn nttncccccc gggncccccn   660
   ggngntaaaa nggtttcncc cccccgaggg gnggggnnnc ctcnnaaacc cntntcnnna     720
   ccncnttttn n                                                          731
<210> 64
   <211> 313
   <212> DNA
   <213> Homo sapien
<220>
   <221> misc_feature
   <222> (1)...(313)
   <223> n = A,T,C or G
<400> 64
   actagttgtg caaaccacga ctgaagaaag acgaaaagtg ggaaataact tgcaacgtct      60
   gttagagatg gttgctacac atgttgggtc tgtagagaaa catcttgagg agcagattgc     120
   taaagttgat agagaatatg aagaatgcat gtcagaagat ctctcggaaa atattaaaga     180
   gattagagat aagtatgaga agaaagctac tctaattaag tcttctgaag aatgaagatn     240
   aaatgttgat catgtatata tatccatagt gaataaaatt gtctcagtaa agttgtaaaa     300
   aaaaaaaaaa aaa                                                        313
<210> 65
   <211> 420
   <212> DNA
   <213> Homo sapien
<220>
   <221> misc_feature
   <222> (1)...(420)
   <223> n = A,T,C or G
<400> 65
   actagttccc tggcaggcaa gggcttccaa ctgaggcagt gcatgtgtgg cagagagagg      60
   caggaagctg gcagtggcag cttctgtgtc tagggagggg tgtggctccc tccttccctg     120
   tctgggaggt tggagggaag aatctaggcc ttagcttgcc ctcctgccac ccttcccctt     180
   gtagatactg ccttaacact ccctcctctc tcagctgtgg ctgccaccca agccaggttt     240
   ctccgtgctc actaatttat ttccaggaaa ggtgtgtgga agacatgagc cgtgtataat     300
   atttgtttta acattttcat tgcaagtatt gaccatcatc cttggttgtg tatcgttgta     360
   acacaaatta atgatattaa aaagcatcca aacaaagccn annnnnaana nnannngaaa     420
<210> 66
   <211> 676
   <212> DNA
   <213> Homo sapien
<220>
   <221> misc_feature
   <222> (1)...(676)
   <223> n = A,T,C or G
<400> 66
   actagtttcc tatgatcatt aaactcattc tcagggttaa gaaaggaatg taaatttctg      60
   cctcaatttg tacttcatca ataagttttt gaagagtgca gatttttagt caggtcttaa     120
   aaataaactc acaaatctgg atgcatttct aaattctgca aatgtttcct ggggtgactt     180
   aacaaggaat aatcccacaa tatacctagc tacctaatac atggagctgg ggctcaaccc     240
   actgttttta aggatttgcg cttacttgtg gctgaggaaa aataagtagt tccgagggaa     300
   gtagttttta aatgtgagct tatagatngg aaacagaata tcaacttaat tatggaaatt     360
   gttagaaacc tgttctcttg ttatctgaat cttgattgca attactattg tactggatag     420
   actccagccc attgcaaagt ctcagatatc ttanctgtgt agttgaattc cttggaaatt     480
   ctttttaaga aaaaattgga gtttnaaaga aataaacccc tttgttaaat gaagcttggc     540
   tttttggtga aaaanaatca tcccgcaggg cttattgttt aaaaanggaa ttttaagcct     600
   ccctggaaaa anttgttaat taaatgggga aaatgntggg naaaaattat ccgttagggt     660
   ttaaagggaa aactta                                                     676
<210> 67
   <211> 620
   <212> DNA
   <213> Homo sapien
<220>
   <221> misc_feature
   <222> (1)...(620)
   <223> n = A,T,C or G
<400> 67
   caccattaaa gctgcttacc aagaacttcc ccagcatttt gacttccttg tttgatagct      60
   gaattgtgag caggtgatag aagagccttt ctagttgaac atacagataa tttgctgaat     120
   acattccatt taatgaaggg gttacatctg ttacgaagct actaagaagg agcaagagca     180
   taggggaaaa aaatctgatc agaacgcatc aaactcacat gtgccccctc tactacaaac     240
   agattgtagt gctgtggtgg tttattccgt tgtgcagaac ttgcaagctg agtcactaaa     300
   cccaaagaga ggaaattata ggttagttaa acattgtaat cccaggaact aagtttaatt     360
   cacttttgaa gtgttttgtt ttttattttt ggtttgtctg atttactttg ggggaaaang     420
   ctaaaaaaaa aggatatcaa atctctaatt cagtgcccac taaaagttgt ccctaaaaag     480
   tctttactgg aanttatggg actttttaag ctccaggtnt tttggtcctc caaattaacc     540
   ttgcatgggc cccttaaaat tgttgaangg cattcctgcc tctaagtttg gggaaaattc     600
   ccccnttttn aaaatttgga                                                 620
<210> 68
   <211> 551
   <212> DNA
   <213> Homo sapien
<220>
   <221> misc_feature
   <222> (1)...(551)
   <223> n = A,T,C or G
<400> 68
   actagtagct ggtacataat cactgaggag ctatttctta acatgctttt atagaccatg      60
   ctaatgctag accagtattt aagggctaat ctcacacctc cttagctgta agagtctggc     120
   ttagaacaga cctctctgtg caataacttg tggccactgg aaatccctgg gccggcattt     180
   gtattggggt tgcaatgact cccaagggcc aaaagagtta aaggcacgac tgggatttct     240
   tctgagactg tggtgaaact ccttccaagg ctgagggggt cagtangtgc tctgggaggg     300
   actcggcacc actttgatat tcaacaagcc acttgaagcc caattataaa attgttattt     360
   tacagctgat ggaactcaat ttgaaccttc aaaactttgt tagtttatcc tattatattg     420
   ttaaacctaa ttacatttgt ctagcattgg atttggttcc tgtngcatat gtttttttcn     480
   cctatgtgct cccctccccc nnatcttaat ttaaaccnca attttgcnat tcnccnnnnn     540
   nannnannna a                                                          551
<210> 69
   <211> 396
   <212> DNA
   <213> Homo sapien
<220>
   <221> misc_feature
   <222> (1)...(396)
   <223> n = A,T,C or G
<400> 69
   cagaaatgga aagcagagtt ttcatttctg tttataaacg tctccaaaca aaaatggaaa      60
   gcagagtttt cattaaatcc ttttaccttt tttttttctt ggtaatcccc tcaaataaca     120
   gtatgtggga tattgaatgt taaagggata tttttttcta ttatttttat aattgtacaa     180
   aattaagcaa atgttaaaag ttttatatgc tttattaatg ttttcaaaag gtatnataca     240
   tgtgatacat tttttaagct tcagttgctt gtcttctggt actttctgtt atgggctttt     300
   ggggagccan aaaccaatct acnatctctt tttgtttgcc aggacatgca ataaaattta     360
   aaaaataaat aaaaactatt nagaaattga aaaaaa                               396
<210> 70
   <211> 536
   <212> DNA
   <213> Homo sapien
<220>
   <221> misc_feature
   <222> (1)...(536)
   <223> n = A,T,C or G
<400> 70
   actagtgcaa aagcaaatat aaacatcgaa aaggcgttcc tcacgttagc tgaagatatc      60
   cttcgaaaga cccctgtaaa agagcccaac agtgaaaatg tagatatcag cagtggagga     120
   ggcgtgacag gctggaagag caaatgctgc tgagcattct cctgttccat cagttgccat     180
   ccactacccc gttttctctt cttgctgcaa aataaaccac tctgtccatt tttaactcta     240
   aacagatatt tttgtttctc atcttaacta tccaagccac ctattttatt tgttctttca     300
   tctgtgactg cttgctgact ttatcataat tttcttcaaa caaaaaaatg tatagaaaaa     360
   tcatgtctgt gacttcattt ttaaatgnta cttgctcagc tcaactgcat ttcagttgtt     420
   ttatagtcca gttcttatca acattnaaac ctatngcaat catttcaaat ctattctgca     480
   aattgtataa gaataaaagt tagaatttaa caattaaaaa aaaaaaaaaa aaaaaa         536
<210> 71
   <211> 865
   <212> DNA
   <213> Homo sapien
<220>
   <221> misc_feature
   <222> (1)...(865)
   <223> n = A,T,C or G
<400> 71
   gacaaagcgt taggagaaga anagaggcag ggaanactnc ccaggcacga tggccncctt      60
   cccaccagca accagcgccc cccaccagcc cccaggcccg gacgacgaag actccatcct     120
   ggattaatct nacctctntc gcctgnccca ttcctacctc ggaggtggag gccggaaagg     180
   tcncaccaag aganaanctg ctgccaacac caaccgcccc agccctggcg ggcacganag     240
   gaaactggtg accaatctgc agaattctna gaggaanaag cnaggggccc cgcgctnaga     300
   cagagctgga tatgangcca gaccatggac nctacncccn ncaatncana cgggactgcg     360
   gaagatggan gacccncgac nngatcaggc cngctnncca nccccccacc cctatgaatt     420
   attcccgctg aangaatctc tganngcctt ccannaaagc gcctccccnc cnaacgnaan     480
   tncaacatng ggattanang ctgggaactg naaggggcaa ancctnnaat atccccagaa     540
   acaanctctc ccnaanaaac tggggcncct catnggtggn accaactatt aactaaaccg     600
   cacgccaagn aantataaaa gggggccccc tccncggnng accccctttt gtcccttaat     660
   ganggttatc cnccttgcgt accatggtnc ccnnttctgt ntgnatgttt ccnctcccct     720
   ccncctatnt cnagccgaac tcnnatttnc ccgggggtgc natcnantng tncncctttn     780
   ttngttgncc cngccctttc cgncggaacn cgtttccccg ttantaacgg cacccggggn     840
   aagggtgntt ggccccctcc ctccc                                           865
<210> 72
   <211> 560
   <212> DNA
   <213> Homo sapien
<220>
   <221> mise feature
   <222> (1)...(560)
   <223> n = A,T,C or G
<400> 72
   cctggacttg tcttggttcc agaacctgac gacccggcga cggcgacgtc tcttttgact 60
   aaaagacagt gtccagtgct ccngcctagg agtctacggg gaccgcctcc cgcgccgcca 120
   ccatgcccaa cttctctggc aactggaaaa tcatccgatc ggaaaacttc gangaattgc 180
   tcnaantgct gggggtgaat gtgatgctna ngaanattgc tgtggctgca gcgtccaagc 240
   cagcagtgga gatcnaacag gagggagaca ctttctacat caaaacctcc accaccgtgc 300
   gcaccacaaa gattaacttc nnngttgggg aggantttga ggancaaact gtggatngga 360
   ngcctgtnaa aacctggtga aatgggagaa tganaataaa atggtctgtg ancanaaact 420
   cctgaaagga gaaggccccc anaactcctg gaccngaaaa actgacccnc cnatngggga 480
   actgatnctt gaaccctgaa cgggcgggat ganccttttt tnttgccncc naangggttc 540
   tttccntttc cccaaaaaaa 560
<210> 73
   <211> 379
   <212> DNA
   <213> Homo sapien
<220>
   <221> misc_feature
   <222> (1)...(379)
   <223> n = A,T,C or G
<400> 73
   ctggggancc ggcggtnngc nccatntcnn gncgcgaagg tggcaataaa aanccnctga 60
   aaccgcncaa naaacatgcc naagatatgg acgaggaaga tngngctttc nngnacaanc 120
   gnanngagga acanaacaaa ctcnangagc tctcaagcta atgccgcggg gaaggggccc 180
   ttggccacnn gtggaattaa gaaatctggc aaanngtann tgttccttgt gcctnangag 240
   ataagngacc ctttatttca tctgtattta aacctctctn ttccctgnca taacttcttt 300
   tnccacgtan agntggaant anttgttgtc ttggactgtt gtncatttta gannaaactt 360
   ttgttcaaaa aaaaaataa 379
<210> 74
   <211> 437
   <212> DNA
   <213> Homo sapien
<220>
   <221> misc_feature
   <222> (1)...(437)
   <223> n = A,T,C or G
<400> 74
   actagttcag actgccacgc caaccccaga aaatacccca catgccagaa aagtgaagtc 60
   ctaggtgttt ccatctatgt ttcaatctgt ccatctacca ggcctcgcga taaaaacaaa 120
   acaaaaaaac gctgccaggt tttanaagca gttctggtct caaaaccatc aggatcctgc 180
   caccagggtt cttttgaaat agtaccacat gtaaaaggga atttggcttt cacttcatct 240
   aatcactgaa ttgtcaggct ttgattgata attgtagaaa taagtagcct tctgttgtgg 300
   gaataagtta taatcagtat tcatctcttt gttttttgtc actcttttct ctctnattgt 360
   gtcatttgta ctgtttgaaa aatatttctt ctataaaatt aaactaacct gccttaaaaa 420
   aaaaaaaaaa aaaaaaa 437
<210> 75
   <211> 579
   <212> DNA
   <213> Homo sapien
<220>
   <221> misc_feature
   <222> (1) ... (579)
   <223> n = A,T,C or G
<400> 75
   ctccgtcgcc gccaagatga tgtgcggggc gccctccgcc acgcagccgg ccaccgccga      60
   gacccagcac atcgccgacc aggtgaggtc ccagcttgaa gagaaagaaa acaagaagtt     120
   ccctgtgttt aaggccgtgt cattcaagag ccaggtggtc gcggggacaa actacttcat     180
   caaggtgcac gtcggcgacg aggacttcgt acacctgcga gtgttccaat ctctccctca     240
   tgaaaacaag cccttgacct tatctaacta ccagaccaac aaagccaagc atgatgagct     300
   gacctatttc tgatcctgac tttggacaag gcccttcagc cagaagactg acaaagtcat     360
   cctccgtcta ccagagcgtg cacttgtgat cctaaaataa gcttcatctc cgggctgtgc     420
   ccttggggtg gaaggggcan gatctgcact gcttttgcat ttctcttcct aaatttcatt     480
   gtgttgattc tttccttcca ataggtgatc ttnattactt tcagaatatt ttccaaatna     540
   gatatatttt naaaatcctt aaaaaaaaaa aaaaaaaaa                            579
<210> 76
   <211> 666
   <212> DNA
   <213> Homo sapien
<220>
   <221> misc_feature
   <222> (1)...(666)
   <223> n = A,T,C or G
<400> 76
   gtttatccta tctctccaac cagattgtca gctccttgag ggcaagagcc acagtatatt      60
   tccctgtttc ttccacagtg cctaataata ctgtggaact aggttttaat aattttttaa     120
   ttgatgttgt tatgggcagg atggcaacca gaccattgtc tcagagcagg tgctggctct     180
   ttcctggcta ctccatgttg gctagcctct ggtaacctct tacttattat cttcaggaca     240
   ctcactacag ggaccaggga tgatgcaaca tccttgtctt tttatgacag gatgtttgct     300
   cagcttctcc aacaataaaa agcacgtggt aaaacacttg cggatattct ggactgtttt     360
   taaaaaatat acagtttacc gaaaatcata ttatcttaca atgaaaagga ntttatagat     420
   cagccagtga acaacctttt cccaccatac aaaaattcct tttcccgaan gaaaanggct     480
   ttctcaataa ncctcacttt cttaanatct tacaagatag ccccganatc ttatcgaaac     540
   tcattttagg caaatatgan ttttattgtn cgttacttgt ttcaaaattt ggtattgtga     600
   atatcaatta ccacccccat ctcccatgaa anaaanggga aanggtgaan ttcntaancg     660
   cttaaa                                                                666
<210> 77
   <211> 396
   <212> DNA
   <213> Homo sapien
<220>
   <221> misc_feature
   <222> (1)...(396)
   <223> n = A,T,C or G
<400> 77
   ctgcagcccg ggggatccac taatctacca nggttatttg gcagctaatt ctanatttgg      60
   atcattgccc aaagttgcac ttgctggtct cttgggattt ggccttggaa aggtatcata     120
   catanganta tgccanaata aattccattt ttttgaaaat canctccntg gggctggttt     180
   tggtccacag cataacangc actgcctcct tacctgtgag gaatgcaaaa taaagcatgg     240
   attaagtgag aagggagact ctcagccttc agcttcctaa attctgtgtc tgtgactttc     300
   gaagtttttt aaacctctga atttgtacac atttaaaatt tcaagtgtac tttaaaataa     360
   aatacttcta atgggaacaa aaaaaaaaaa aaaaaa                               396
<210> 78
   <211> 793
   <212> DNA
   <213> Homo sapien
<220>
   <221> misc_feature
   <222> (1)...(793)
   <223> n = A,T,C or G
<400> 78
   gcatcctagc cgccgactca cacaaggcag gtgggtgagg aaatccagag ttgccatgga      60
   gaaaattcca gtgtcagcat tcttgctcct tgtggccctc tcctacactc tggccagaga     120
   taccacagtc aaacctggag ccaaaaagga cacaaaggac tctcgaccca aactgcccca     180
   gaccctctcc agaggttggg gtgaccaact catctggact cagacatatg aagaagctct     240
   atataaatcc aagacaagca acaaaccctt gatgattatt catcacttgg atgagtgccc     300
   acacagtcna gctttaaaga aagtgtttgc tgaaaataaa gaaatccaga aattggcaga     360
   gcagtttgtc ctcctcaatc tggtttatga aacaactgac aaacaccttt ctcctgatgg     420
   ccagtatgtc ccaggattat gtttgttgac ccatctctga cagttgaagc cgatatcctg     480
   ggaagatatt cnaaccgtct ctatgcttac aaactgcaga tacgctctgt tgcttgacac     540
   atgaaaaagc tctcaagttg ctnaaaatga attgtaagaa aaaaaatctc cagccttctg     600
   tctgtcggct tgaaaattga aaccagaaaa atgtgaaaaa tggctattgt ggaacanatn     660
   gacacctgat taggttttgg ttatgttcac cactattttt aanaaaanan nttttaaaat     720
   ttggttcaat tntctttttn aaacaatntg tttctacntt gnganctgat ttctaaaaaa     780
   aataatnttt ggc                                                        793
<210> 79
   <211> 456
   <212> DNA
   <213> Homo sapien
<220>
   <221> misc_feature
   <222> (1)...(456)
   <223> n = A,T,C or G
<400> 79
   actagtatgg ggtgggaggc cccacccttc tcccctaggc gctgttcttg ctccaaaggg      60
   ctccgtggag agggactggc agagctgang ccacctgggg ctggggatcc cactcttctt     120
   gcagctgttg agcgcaccta accactggtc atgcccccac ccctgctctc cgcacccgct     180
   tcctcccgac cccangacca ggctacttct cccctcctct tgcctccctc ctgcccctgc     240
   tgcctctgat cgtangaatt gangantgtc ccgccttgtg gctganaatg gacagtggca     300
   ggggctggaa atgggtgtgt gtgtgtgtgt gtgtgtgtgt gtgtgtgtgt gcnccccccc     360
   tgcaagaccg agattgaggg aaancatgtc tgctgggtgt gaccatgttt cctctccata     420
   aantncccct gtgacnctca naaaaaaaaa aaaaaa                               456
<210> 80
   <211> 284
   <212> DNA
   <213> Homo sapien
<220>
   <221> mise feature
   <222> (1)...(284)
   <223> n = A,T,C or G
<400> 80
   ctttgtacct ctagaaaaga taggtattgt gtcatgaaac ttgagtttaa attttatata      60
   taaaactaaa agtaatgctc actttagcaa cacatactaa aattggaacc atactgagaa     120
   gaatagcatg acctccgtgc aaacaggaca agcaaatttg tgatgtgttg attaaaaaga     180
   aataaataaa tgtgtatatg tgtaacttgt atgtttatgt ggaatacaga ttgggaaata     240
   aaatgtattt cttactgtga aaaaaaaaaa aaaaaaaaaa aana                      284
<210> 81
   <211> 671
   <212> DNA
   <213> Homo sapien
<220>
   <221> misc_feature
   <222> (1)...(671)
   <223> n = A,T,C or G
<400> 81
   gccaccaaca ttccaagcta ccctgggtac ctttgtgcag tagaagctag tgagcatgtg      60
   agcaagcggt gtgcacacgg agactcatcg ttataattta ctatctgcca agagtagaaa     120
   gaaaggctgg ggatatttgg gttggcttgg ttttgatttt ttgcttgttt gtttgttttg     180
   tactaaaaca gtattatctt ttgaatatcg tagggacata agtatataca tgttatccaa     240
   tcaagatggc tagaatggtg cctttctgag tgtctaaaac ttgacacccc tggtaaatct     300
   ttcaacacac ttccactgcc tgcgtaatga agttttgatt catttttaac cactggaatt     360
   tttcaatgcc gtcattttca gttagatnat tttgcacttt gagattaaaa tgccatgtct     420
   atttgattag tcttattttt ttatttttac aggcttatca gtctcactgt tggctgtcat     480
   tgtgacaaag tcaaataaac ccccnaggac aacacacagt atgggatcac atattgtttg     540
   acattaagct ttggccaaaa aatgttgcat gtgttttacc tcgacttgct aaatcaatan     600
   canaaaggct ggctnataat gttggtggtg aaataattaa tnantaacca aaaaaaaaan     660
   aaaaaaaaaa a                                                          671
<210> 82
   <211> 217
   <212> DNA
   <213> Homo sapien
<220>
   <221> misc_feature
   <222> (1)...(217)
   <223> n = A,T,C or G
<400> 82
   ctgcagatgt ttcttgaatg ctttgtcaaa ttaanaaagt taaagtgcaa taatgtttga      60
   agacaataag tggtggtgta tcttgtttct aataagataa acttttttgt ctttgcttta      120
   tcttattagg gagttgtatg tcagtgtata aaacatactg tgtggtataa caggcttaat     180
   aaattcttta aaaggaaaaa aaaaaaaaaa aaaaaaa                                 217
<210> 83
   <211> 460
   <212> DNA
   <213> Homo sapien
<220>
   <221> misc_feature
   <222> (1)...(460)
   <223> n = A,T,C or G
<400> 83
   cgcgagtggg agcaccagga tctcgggctc ggaacgagac tgcacggatt gttttaagaa      60
   aatggcagac aaaccagaca tgggggaaat cgccagcttc gatnaggcca agctgaanaa     120
   aacggagacg caggagaaga acaccctgcc gaccaaagag accattgagc angagaagcg     180
   gagtgaaatt tcctaagatc ctggaggatt tcctaccccc gtcctcttcg agaccccagt     240
   cgtgatgtgg aggaagagcc acctgcaaga tggacacgag ccacaagctg cactgtgaac     300
   ctgggcactc cgcgccgatg ccaccggcct gtgggtctct gaagggaccc cccccaatcg     360
   gactgccaaa ttctccggtt tgccccggga tattatacaa nattatttgt atgaataatg     420
   annataaaac acacctcgtg gcancaaana aaaaaaaaaa                           460
<210> 84
   <211> 323
   <212> DNA
   <213> Homo sapien
<220>
   <221> mise feature
   <222> (1)...(323)
   <223> n = A,T,C or G
<400> 84
   tggtggatct tggctctgtg gagctgctgg gacgggatct aaaagactat tctggaagct      60
   gtggtccaan gcattttgct ggcttaacgg gtcccggaac aaaggacacc agctctctaa     120
   aattgaagtt tacccganat aacaatcttt tgggcagaga tgcctatttt aacaaacncc     180
   gtccctgcgc aacaacnaac aatctctggg aaataccggc catgaacntg ctgtctcaat     240
   cnancatctc tctagctgac cgatcatatc gtcccagatt actacanatc ataataattg     300
   atttcctgta naaaaaaaaa aaa                                             323
<210> 85
   <211> 771
   <212> DNA
   <213> Homo sapien
<220>
   <221> misc_feature
   <222> (1)...(771)
   <223> n = A,T,C or G
<400> 85
   aaactgggta ctcaacactg agcagatctg ttctttgagc taaaaaccat gtgctgtacc     60
   aanagtttgc tcctggctgc tttgatgtca gtgctgctac tccacctctg cggcgaatca    120
   gaagcaagca actttgactg ctgtcttgga tacacagacc gtattcttca tcctaaattt    180
   attgtgggct tcacacggca gctggccaat gaaggctgtg acatcaatgc tatcatcttt    240
   cacacaaaga aaaagttgtc tgtgtgcgca aatccaaaac agacttgggt gaaatatatt    300
   gtgcgtctcc tcagtaaaaa agtcaagaac atgtaaaaac tgtggctttt ctggaatgga    360
   attggacata gcccaagaac agaaagaact tgctggggtt ggaggtttca cttgcacatc    420
   atgganggtt tagtgcttat cttatttgtg cctcctggac ttgtccaatt natgaagtta    480
   atcatattgc atcatanttt gctttgttta acatcacatt naaattaaac tgtattttat    540
   gttatttata gctntaggtt ttctgtgttt aactttttat acnaantttc ctaaactatt    600
   ttggtntant gcaanttaaa aattatattt ggggggggaa taaatattgg antttctgca    660
   gccacaagct ttttttaaaa aaccantaca nccnngttaa atggtnggtc ccnaatggtt    720
   tttgcttttn antagaaaat ttnttagaac natttgaaaa aaaaaaaaaa a             771
<210> 86
   <211> 628
   <212> DNA
   <213> Homo sapien
<220>
   <221> misc_feature
   <222> (1)...(628)
   <223> n = A,T,C or G
<400> 86
   actagtttgc tttacatttt tgaaaagtat tatttttgtc caagtgctta tcaactaaac     60
   cttgtgttag gtaagaatgg aatttattaa gtgaatcagt gtgacccttc ttgtcataag    120
   attatcttaa agctgaagcc aaaatatgct tcaaaagaaa angactttat tgttcattgt    180
   agttcataca ttcaaagcat ctgaactgta gtttctatag caagccaatt acatccataa    240
   gtggagaang aaatagatta atgtcnaagt atgattggtg gagggagcaa ggttgaagat    300
   aatctggggt tgaaattttc tagttttcat tctgtacatt tttagttnga catcagattt    360
   gaaatattaa tgtttacctt tcaatgtgtg gtatcagctg gactcantaa cacccctttc    420
   ttccctnggg gatggggaat ggattattgg aaaatggaaa gaaaaaagta cttaaagcct    480
   tcctttcnca gtttctggct cctaccctac tgatttancc agaataagaa aacattttat    540
   catcntctgc tttattccca ttaatnaant tttgatgaat aaatctgctt ttatgcnnac    600
   ccaaggaatt nagtggnttc ntcnttgt                                       628
<210> 87
   <211> 518
   <212> DNA
   <213> Homo sapien
<220>
   <221> misc_feature
   <222> (1)...(518)
   <223> n = A,T,C or G
<400> 87
   ttttttattt tttttagaga gtagttcagc ttttatttat aaatttattg cctgttttat     60
   tataacaaca ttatactgtt tatggtttaa tacatatggt tcaaaatgta taatacatca    120
   agtagtacag ttttaaaatt ttatgcttaa aacaagtttt gtgtaaaaaa tgcagataca    180
   ttttacatgg caaatcaatt tttaagtcat cctaaaaatt gatttttttt tgaaatttaa    240
   aaacacattt aatttcaatt tctctcttat ataaccttta ttactatagc atggtttcca    300
   ctacagttta acaatgcagc aaaattccca tttcacggta aattgggttt taagcggcaa    360
   ggttaaaatg ctttgaggat cctnaatacc ctttgaactt caaatgaagg ttatggttgt    420
   naatttaacc ctcatgccat aagcagaagc acaagtttag ctgcattttg ctctaaactg    480
   taaaancgag ccccccgttg aaaaagcaaa agggaccc                            518
<210> 88
   <211> 1844
   <212> DNA
   <213> Homo sapien
<400> 88
   gagacagtga atcctagtat caaaggattt ttggcctcag aaaaagttgt tgattatttt     60
   tattttattt tatttttcga gactccgtct caaaaaaaaa aaaaaaaaaa agaatcacaa    120
   ggtatttgct aaagcatttt gagctgcttg gaaaaaggga agtagttgca gtagagtttc    180
   ttccatcttc ttggtgctgg gaagccatat atgtgtcttt tactcaagct aaggggtata    240
   agcttatgtg ttgaatttgc tacatctata tttcacatat tctcacaata agagaatttt    300
   gaaatagaaa tatcatagaa catttaagaa agtttagtat aaataatatt ttgtgtgttt    360
   taatcccttt gaagggatct atccaaagaa aatattttac actgagctcc ttcctacacg    420
   tctcagtaac agatcctgtg ttagtctttg aaaatagctc attttttaaa tgtcagtgag    480
   tagatgtagc atacatatga tgtataatga cgtgtattat gttaacaatg tctgcagatt    540
   ttgtaggaat acaaaacatg gcctttttta taagcaaaac gggccaatga ctagaataac    600
   acatagggca atctgtgaat atgtattata agcagcattc cagaaaagta gttggtgaaa    660
   taattttcaa gtcaaaaagg gatatggaaa gggaattatg agtaacctct attttttaag    720
   ccttgctttt aaattaaacg ctacagccat ttaagccttg aggataataa agcttgagag    780
   taataatgtt aggttagcaa aggtttagat gtatcacttc atgcatgcta ccatgatagt    840
   aatgcagctc ttcgagtcat ttctggtcat tcaagatatt cacccttttg cccatagaaa    900
   gcaccctacc tcacctgctt actgacattg tcttagctga tcacaagatc attatcagcc    960
   tccattattc cttactgtat ataaaataca gagttttata ttttcctttc ttcgtttttc   1020
   accatattca aaacctaaat ttgtttttgc agatggaatg caaagtaatc aagtgttcgt   1080
   gctttcacct agaagggtgt ggtcctgaag gaaagaggtc cctaaatatc ccccaccctg   1140
   ggtgctcctc cttccctggt accctgacta ccagaagtca ggtgctagag cagctggaga   1200
   agtgcagcag cctgtgcttc cacagatggg ggtgctgctg caacaaggct ttcaatgtgc   1260
   ccatcttagg gggagaagct agatcctgtg cagcagcctg gtaagtcctg aggaggttcc   1320
   attgctcttc ctgctgctgt cctttgcttc tcaacggggc tcgctctaca gtctagagca   1380
   catgcagcta acttgtgcct ctgcttatgc atgagggtta aattaacaac cataaccttc   1440
   atttgaagtt caaaggtgta ttcaggatcc tcaaagcatt ttaaccttgc cgcttaaaac   1500
   ccaatttacc gtgaaatggg aattttgctg cattgttaaa ctgtagtgga aaccatgcta   1560
   tagtaataaa ggttatataa gagagaaatt gaaattaaat gtgtttttaa atttcaaaaa   1620
   aaaatcaatc tttaggatga cttaaaaatt gatttgccat gtaaaatgta tctgcatttt   1680
   ttacacaaaa cttgttttaa gcataaaatt ttaaaactgt actacttgat gtattataca   1740
   ttttgaacca tatgtattaa accataaaca gtataatgtt gttataataa aacaggcaat   1800
   aaatttataa ataaaagctg aaaaaaaaaa aaaaaaaaaa aaaa                   1844
<210> 89
   <211> 523
   <212> DNA
   <213> Homo sapien
<220>
   <221> misc_feature
   <222> (1)...(523)
   <223> n = A,T,C or G
<400> 89
   tttttttttt tttttttagt caatccacat ttattgatca cttattatgt accaggcact      60
   gggataaaga tgactgttag tcactcacag taaggaagaa aactagcaaa taagacgatt    120
   acaatatgat gtagaaaatg ctaagccaga gatatagaaa ggtcctattg ggtccttctg    180
   tcaccttgtc tttccacatc cctacccttc acaggccttc cctccagctt cctgcccccg    240
   ctccccactg cagatcccct gggattttgc ctagagctaa acgagganat gggccccctg    300
   gccctggcat gacttgaacc caaccacaga ctgggaaagg gagcctttcg anagtggatc    360
   actttgatna gaaaacacat agggaattga agagaaantc cccaaatggc cacccgtgct    420
   ggtgctcaag aaaagtttgc agaatggata aatgaaggat caagggaatt aatanatgaa    480
   taattgaatg gtggctcaat aagaatgact ncnttgaatg acc                      523
<210> 90
   <211> 604
   <212> DNA
   <213> Homo sapien
<220>
   <221> misc_feature
   <222> (1)...(604)
   <223> n = A,T,C or G
<400> 90
   ccagtgtggt ggaatgcaaa gattaccccg gaagctttcg agaagctggg attccctgca     60
   gcaaaggaaa tagccaatat gtgtcgtttc tatgaaatga agccagaccg agatgtcaat    120
   ctcacccacc aactaaatcc caaagtcaaa agcttcagcc agtttatctc agagaaccag    180
   gggagccttc aagggcatgt agaaaatcag ctgttcagat aggcctctgc accacacagc    240
   ctctttcctc tctgatcctt ttcctcttta cggcacaaca ttcatgtttg acagaacatg    300
   ctggaatgca attgtttgca acaccgaagg atttcctgcg gtcgcctctt cagtaggaag    360
   cactgcattg gtgataggac acggtaattt gattcacatt taacttgcta gttagtgata    420
   aggggtggta cacctgtttg gtaaaatgag aagcctcgga aacttgggag cttctctcct    480
   accactaatg gggagggcag attattactg ggatttctcc tggggtgaat taatttcaag    540
   ccctaattgc tgaaattccc ctnggcaggc tccagttttc tcaactgcat tgcaaaattc    600
   cccc                                                                 604
<210> 91
   <211> 858
   <212> DNA
   <213> Homo sapien
<220>
   <221> misc_feature
   <222> (1)...(858)
   <223> n = A,T,C or G
<400> 91
   tttttttttt ttttttttta tgattattat tttttttatt gatctttaca tcctcagtgt 60
   tggcagagtt tctgatgctt aataaacatt tgttctgatc agataagtgg aaaaaattgt 120
   catttcctta ttcaagccat gcttttctgt gatattctga tcctagttga acatacagaa 180
   ataaatgtct aaaacagcac ctcgattctc gtctataaca ggactaagtt cactgtgatc 240
   ttaaataagc ttggctaaaa tgggacatga gtggaggtag tcacacttca gcgaagaaag 300
   agaatctcct gtataatctc accaggagat tcaacgaatt ccaccacact ggactagtgg 360
   atcccccggg ctgcaggaat tcgatatcaa gcttatcgat accgtcgacc tcgagggggg 420
   gcccggtacc caattcgccc tatagtgagt cgtattacgc gcgctcactg gccgtcgttt 480
   tacaacgtcg tgactgggaa aaccctggcg ttacccaact taatcgcctt gcagcacatc 540
   cccctttcgc cagctggcgt aatagcgaan agcccgcacc gatcgccctt ncaacagttg 600
   cgcagcctga atggcgaatg ggacgcgccc tgtagcggcg cattaaagcg cggcngggtg 660
   tggnggntcc cccacgtgac cgntacactt ggcagcgcct tacgccggtc nttcgctttc 720
   ttcccttcct ttctcgcacc gttcgccggg tttccccgnn agctnttaat cgggggnctc 780
   cctttanggg tncnaattaa nggnttacng gaccttngan cccaaaaact ttgattaggg 840
   ggaaggtccc cgaagggg 858
<210> 92
   <211> 585
   <212> DNA
   <213> Homo sapien
<220>
   <221> misc_feature
   <222> (1)...(585)
   <223> n = A,T,C or G
<400> 92
   gttgaatctc ctggtgagat tatacaggag attctctttc ttcgctgaag tgtgactacc 60
   tccactcatg tcccatttta gccaagctta tttaagatca cagtgaactt agtcctgtta 120
   tagacgagaa tcgaggtgct gttttagaca tttatttctg tatgttcaac taggatcaga 180
   atatcacaga aaagcatggc ttgaataagg aaatgacaat tttttccact tatctgatca 240
   gaacaaatgt ttattaagca tcagaaactc tgccaacact gaggatgtaa agatcaataa 300
   aaaaaataat aatcatnann naaanannan nngaagggcg gccgccaccg cggtggagct 360
   ccagcttttg ttccctttag tgagggttaa ttgcgcgctt ggcgttaatc atggtcatag 420
   ctgtttcctg tgtgaaattg ttatccggct cacaattccn cncaacatac gagccgggaa 480
   gcntnangtg taaaagcctg gggtgcctaa attgagtgag ctnactcaca ttaattgngt 540
   tgcgctccac ttgcccgctt ttccantccg ggaaacctgt tcgnc 585
<210> 93
   <211> 567
   <212> DNA
   <213> Homo sapien
<220>
   <221> misc_feature
   <222> (1)...(567)
   <223> n = A,T,C or G
<400> 93
   cggcagtgtt gctgtctgcg tgtccacctt ggaatctggc tgaactggct gggaggacca 60
   agactgcggc tggggtgggc anggaaggga accgggggct gctgtgaagg atcttggaac 120
   ttccctgtac ccaccttccc cttgcttcat gtttgtanag gaaccttgtg ccggccaagc 180
   ccagtttcct tgtgtgatac actaatgtat ttgctttttt tgggaaatan anaaaaatca 240
   attaaattgc tantgtttct ttgaannnnn nnnnnnnnnn nnnnnnnggg ggggncgccc 300
   ccncggngga aacnccccct tttgttccct ttaattgaaa ggttaattng cncncntggc 360
   gttaanccnt gggccaaanc tngttncccg tgntgaaatt gttnatcccc tcccaaattc 420
   ccccccnncc ttccaaaccc ggaaancctn annntgttna ancccggggg gttgcctaan 480
   ngnaattnaa ccnaaccccc ntttaaatng nntttgcncn ccacnngccc cnctttccca 540
   nttcggggaa aaccctntcc gtgccca 567
<210> 94
   <211> 620
   <212> DNA
   <213> Homo sapien
<220>
   <221> misc_feature
   <222> (1)...(620)
   <223> n = A,T,C or G
<400> 94
   actagtcaaa aatgctaaaa taatttggga gaaaatattt tttaagtagt gttatagttt 60
   catgtttatc ttttattatg ttttgtgaag ttgtgtcttt tcactaatta cctatactat 120
   gccaatattt ccttatatct atccataaca tttatactac atttgtaana naatatgcac 180
   gtgaaactta acactttata aggtaaaaat gaggtttcca anatttaata atctgatcaa 240
   gttcttgtta tttccaaata gaatggactt ggtctgttaa gggctaagga gaagaggaag 300
   ataaggttaa aagttgttaa tgaccaaaca ttctaaaaga aatgcaaaaa aaaagtttat 360
   tttcaagcct tcgaactatt taaggaaagc aaaatcattt cctaaatgca tatcatttgt 420
   gagaatttct cattaatatc ctgaatcatt catttcacta aggctcatgt tnactccgat 480
   atgtctctaa gaaagtacta tttcatggtc caaacctggt tgccatantt gggtaaaggc 540
   tttcccttaa gtgtgaaant atttaaaatg aaattttcct ctttttaaaa attctttana 600
   agggttaagg gtgttgggga 620
<210> 95
   <211> 470
   <212> DNA
   <213> Homo sapien
<220>
   <221> mise_feature
   <222> (1)...(470)
   <223> n = A,T,C or G
<400> 95
   ctcgaccttc tctgcacagc ggatgaaccc tgagcagctg aagaccagaa aagccactat      60
   nactttntgc ttaattcang agcttacang attcttcaaa gagtgngtcc agcatccttt     120
   gaaacatgag ttcttaccag cagaagcaga cctttacccc accacctcag cttcaacagc     180
   agcaggtgaa acaacccatc cagcctccac ctnaggaaat atttgttccc acaaccaagg     240
   agccatgcca ctcaaaggtt ccacaacctg naaacacaaa nattccagag ccaggctgta     300
   ccaaggtccc tgagccaggg ctgtaccaan gtccctgagc caggttgtac caangtccct     360
   gagccaggat gtaccaaggt ccctgancca ggttgtccaa ggtccctgag ccaggctaca     420
   ccaagggcct gngccaggca gcatcaangt ccctgaccaa ggcttatcaa                470
<210> 96
   <211> 660
   <212> DNA
   <213> Homo sapien
<220>
   <221> misc_feature
   <222> (1)...(660)
   <223> n = A,T,C or G
<400> 96
   tttttttttt tttttttttt ggaattaaaa gcaatttaat gagggcagag caggaaacat      60
   gcatttcttt tcattcgaat cttcagatga accctgagca gccgaagacc agaaaagcca     120
   tgaagacttt ctgcttaatt caggggctta caggattctt cagagtgtgt gtgaacaaaa     180
   gctttatagt acgtattttt aggatacaaa taagagagag actatggctt ggggtgagaa     240
   tgtactgatt acaaggtcta cagacaatta agacacagaa acagatggga agagggtgnc     300
   cagcatctgg nggttggctt ctcaagggct tgtctgtgca ccaaattact tctgcttggn     360
   cttctgctga gctgggcctg gagtgaccgt tgaaggacat ggctctggta cctttgtgta     420
   gcctgncaca ggaactttgg tgtatccttg ctcaggaact ttgatggcac ctggctcagg     480
   aaacttgatg aagccttggt caagggacct tgatgcttgc tggctcaggg accttggngn     540
   ancctgggct canggacctt tgncncaacc ttggcttcaa gggacccttg gnacatcctg     600
   gcnnagggac ccttgggncc aaccctgggc ttnagggacc ctttggntnc nanccttggc     660
<210> 97
   <211> 441
   <212> DNA
   <213> Homo sapien
<220>
   <221> misc_feature
   <222> (1)...(441)
   <223> n = A,T,C or G
<400> 97
   gggaccatac anagtattcc tctcttcaca ccaggaccag ccactgttgc agcatgagtt      60
   cccagcagca gaagcagccc tgcatcccac cccctcagct tcagcagcag caggtgaaac     120
   agccttgcca gcctccacct caggaaccat gcatccccaa aaccaaggag ccctgccacc     180
   ccaaggtgcc tgagccctgc caccccaaag tgcctgagcc ctgccagccc aaggttccag     240
   agccatgcca ccccaaggtg cctgagccct gcccttcaat agtcactcca gcaccagccc     300
   agcagaanac caagcagaag taatgtggtc cacagccatg cccttgagga gccggccacc     360
   agatgctgaa tcccctatcc cattctgtgt atgagtccca tttgccttgc aattagcatt     420
   ctgtctcccc caaaaaaaaa a                                               441
<210> 98
   <211> 600
   <212> DNA
   <213> Homo sapien
<220>
   <221> misc_feature
   <222> (1) ... (600)
   <223> n = A,T,C or G
<400> 98
   gtattcctct cttcacacca ggaccagcca ctgttgcagc atgagttccc agcagcagaa      60
   gcagccctgc atcccacccc ctcagcttca gcagcagcag gtgaaacagc cttgccagcc     120
   tccacctcag gaaccatgca tccccaaaac caaggagccc tgccacccca aggtgcctga     180
   gccctgccac cccaaagtgc ctgagccctg ccagcccaag gttccagagc catgccaccc     240
   caaggtgcct gagccctgcc cttcaatagt cactccagca ccagcccagc agaanaccaa     300
   gcagaagtaa tgtggtccac agccatgccc ttgaggagcc ggccaccana tgctgaatcc     360
   cctatcccat tctgtgtatg agtcccattt gccttgcaat tagcattctg tctcccccaa     420
   aaaagaatgt gctatgaagc tttctttcct acacactctg agtctctgaa tgaagctgaa     480
   ggtcttaant acaganctag ttttcagctg ctcagaattc tctgaagaaa agatttaaga     540
   tgaaaggcaa atgattcagc tccttattac cccattaaat tcnctttcaa ttccaaaaaa     600
<210> 99
   <211> 667
   <212> DNA
   <213> Homo sapien
<220>
   <221> misc_feature
   <222> (1)...(667)
   <223> n = A,T,C or G
<400> 99
   actagtgact gagttcctgg caaagaaatt tgacctggac cagttgataa ctcatgtttt      60
   accatttaaa aaaatcagtg aaggatttga gctgctcaat tcaggacaaa gcattcgaac     120
   ggtcctgacg ttttgagatc caaagtggca ggaggtctgt gttgtcatgg tgaactggag     180
   tttctcttgt gagagttccc tcatctgaaa tcatgtatct gtctcacaaa tacaagcata     240
   agtagaagat ttgttgaaga catagaaccc ttataaagaa ttattaacct ttataaacat     300
   ttaaagtctt gtgagcacct gggaattagt ataataacaa tgttnatatt tttgatttac     360
   attttgtaag gctataattg tatcttttaa gaaaacatac cttggatttc tatgttgaaa     420
   tggagatttt taagagtttt aaccagctgc tgcagatata ttactcaaaa cagatatagc     480
   gtataaagat atagtaaatg catctcctag agtaatattc acttaacaca ttggaaacta     540
   ttatttttta gatttgaata tnaatgttat tttttaaaca cttgttatga gttacttggg      600
   attacatttt gaaatcagtt cattccatga tgcanattac tgggattaga ttaagaaaga     660
   cggaaaa                                                               667
<210> 100
   <211> 583
   <212> DNA
   <213> Homo sapien
<220>
   <221> misc_feature
   <222> (1)...(583)
   <223> n = A,T,C or G
<400> 100
   gttttgtttg taagatgatc acagtcatgt tacactgatc taaaggacat atatataacc      60
   ctttaaaaaa aaaatcactg cctcattctt atttcaagat gaatttctat acagactaga     120
   tgtttttctg aagatcaatt agacattttg aaaatgattt aaagtgtttt ccttaatgtt     180
   ctctgaaaac aagtttcttt tgtagtttta accaaaaaag tgcccttttt gtcactggat     240
   tctcctagca ttcatgattt ttttttcata caatgaaatt aaaattgcta aaatcatgga     300
   ctggctttct ggttggattt caggtaagat gtgtttaagg ccagagcttt tctcagtatt     360
   tgattttttt ccccaatatt tgatttttta aaaatataca catnggtgct gcatttatat     420
   ctgctggttt aaaattctgt catatttcac ttctagcctt ttagttatgg caaatcatat     480
   tttactttta cttaaagcat ttggtnattt ggantatctg gttctannct aaaaaaanta     540
   attctatnaa ttgaantttt ggtactcnnc catatttgga tcc                       583
<210> 101
   <211> 592
   <212> DNA
   <213> Homo sapien
<220>
   <221> misc_feature
   <222> (1)...(592)
   <223> n = A,T,C or G
<400> 101
   gtggagacgt acaaagagca gccgctcaag acacctggga agaaaaagaa aggcaagccc      60
   gggaaacgca aggagcagga aaagaaaaaa cggcgaactc gctctgcctg gttagactct     120
   ggagtgactg ggagtgggct agaaggggac cacctgtctg acacctccac aacgtcgctg     180
   gagctcgatt cacggaggca ttgaaatttt cagcaganac cttccaagga catattgcag     240
   gattctgtaa tagtgaacat atggaaagta ttagaaatat ttattgtctg taaatactgt     300
   aaatgcattg gaataaaact gtctccccca ttgctctatg aaactgcaca ttggtcattg    360
   tgaatatttt tttttttgcc aaggctaatc caattattat tatcacattt accataattt     420
   attttgtcca ttgatgtatt tattttgtaa atgtatcttg gtgctgctga atttctatat     480
   tttttgtaca taatgcnttt anatatacct atcaagtttg ttgataaatg acncaatgaa     540
   gtgncncnan ttggnggttg aatttaatga atgcctaatt ttattatccc aa             592

<210> 102
   <211> 587
   <212> DNA
   <213> Homo sapien
<220>
   <221> misc_feature
   <222> (1)...(587)
   <223> n = A,T,C or G
<400> 102
   cgtcctaagc acttagacta catcagggaa gaacacagac cacatccctg tcctcatgcg      60
   gcttatgttt tctggaagaa agtggagacc nagtccttgg ctttagggct ccccggctgg     120
   gggctgtgca ntccggtcag ggcgggaagg gaaatgcacc gctgcatgtg aacttacagc     180
   ccaggcggat gccccttccc ttagcactac ctggcctcct gcatcccctc gcctcatgtt     240
   cctcccacct tcaaanaatg aanaacccca tgggcccagc cccttgccct ggggaaccaa     300
   ggcagccttc caaaactcag gggctgaagc anactattag ggcaggggct gactttgggt      360
   gacactgccc attccctctc agggcagctc angtcacccn ggnctcttga acccagcctg     420
   ttcctttgaa aaagggcaaa actgaaaagg gcttttccta naaaaagaaa aaccagggaa     480
   ctttgccagg gcttcnntnt taccaaaacn ncttctcnng gatttttaat tccccattng     540
   gcctccactt accnggggcn atgccccaaa attaanaatt tcccatc                   587
<210> 103
   <211> 496
   <212> DNA
   <213> Homo sapien
<220>
   <221> misc_feature
   <222> (1)...(496)
   <223> n = A,T,C or G
<400> 103
   anaggactgg ccctacntgc tctctctcgt cctacctatc aatgcccaac atggcagaac      60
   ctgcanccct tggncactgc anatggaaac ctctcagtgt cttgacatca ccctacccnt    120
   gcggtgggtc tccaccacaa ccactttgac tctgtggtcc ctgnanggtg gnttctcctg     180
   actggcagga tggaccttan ccnacatatc cctctgttcc ctctgctnag anaaagaatt     240
   cccttaacat gatataatcc acccatgcaa ntngctactg gcccagctac catttaccat     300
   ttgcctacag aatttcattc agtctacact ttggcattct ctctggcgat agagtgtggc     360
   tgggctgacc gcaaaaggtg ccttacacac tggcccccac cctcaaccgt tgacncatca     420
   gangcttgcc tcctccttct gattnncccc catgttggat atcagggtgc tcnagggatt     480
   ggaaaagaaa caaaac                                                     496
<210> 104
   <211> 575
   <212> DNA
   <213> Homo sapien
<220>
   <221> misc_feature
   <222> (1)...(575)
   <223> n = A,T,C or G
<400> 104
   gcacctgctc tcaatccnnc tctcaccatg atcctccgcc tgcanaaact cctctgccaa      60
   ctatggangt ggtttcnggg gtggctcttg ccaactggga agaagccgtg gtgtctctac     120
   ctgttcaact cngtttgtgt ctgggggatc aactnggggc tatggaagcg gctnaactgt     180
   tgttttggtg gaagggctgg taattggctt tgggaagtng cttatngaag ttggcctngg     240
   gaagttgcta ttgaaagtng ccntggaagt ngntttggtg gggggttttg ctggtggcct     300
   ttgttnaatt tgggtgcttt gtnaatggcg gccccctcnc ctgggcaatg aaaaaaatca     360
   ccnatgcngn aaacctcnac nnaacagcct gggcttccct cacctcgaaa aaagttgctc     420
   cccccccaaa aaaggncaan cccctcaann tggaangttg aaaaaatcct cgaatgggga     480
   ncccnaaaac aaaaancccc ccntttcccn gnaanggggg aaataccncc cccccactta     540
   cnaaaaccct tntaaaaaac cccccgggaa aaaaa                                575
<210> 105
   <211> 619
   <212> DNA
   <213> Homo sapien
<220>
   <221> misc_feature
   <222> (1)...(619)
   <223> n = A,T,C or G
<400> 105
   cactagtagg atagaaacac tgtgtcccga gagtaaggag agaagctact attgattaga      60
   gcctaaccca ggttaactgc aagaagaggc gggatacttt cagctttcca tgtaactgta     120
   tgcataaagc caatgtagtc cagtttctaa gatcatgttc caagctaact gaatcccact     180
   tcaatacaca ctcatgaact cctgatggaa caataacagg cccaagcctg tggtatgatg     240
   tgcacacttg ctagactcan aaaaaatact actctcataa atgggtggga gtattttggt     300
   gacaacctac tttgcttggc tgagtgaagg aatgatattc atatattcat ttattccatg     360
   gacatttagt tagtgctttt tatataccag gcatgatgct gagtgacact cttgtgtata     420
   tttccaaatt tttgtacagt cgctgcacat atttgaaatc atatattaag acttccaaaa     480
   aatgaagtcc ctggtttttc atggcaactt gatcagtaaa ggattcncct ctgtttggta     540
   cttaaaacat ctactatatn gttnanatga aattcctttt ccccncctcc cgaaaaaana     600
   aagtggtggg gaaaaaaaa                                                 619
<210> 106
   <211> 506
   <212> DNA
   <213> Homo sapien
<220>
   <221> misc_feature
   <222> (1)...(506)
   <223> n = A,T,C or G
<400> 106
   cattggtnct ttcatttgct ntggaagtgt nnatctctaa cagtggacaa agttcccngt 60
   gccttaaact ctgtnacact tttgggaant gaaaanttng tantatgata ggttattctg 120
   angtanagat gttctggata ccattanatn tgcccccngt gtcagaggct catattgtgt 180
   tatgtaaatg gtatntcatt cgctactatn antcaattng aaatanggtc tttgggttat 240
   gaatantnng cagcncanct nanangctgt ctgtngtatt cattgtggtc atagcacctc 300
   acancattgt aacctcnatc nagtgagaca nactagnaan ttcctagtga tggctcanga 360
   ttccaaatgg nctcatntcn aatgtttaaa agttanttaa gtgtaagaaa tacagactgg 420
   atgttccacc aactagtacc tgtaatgacn ggcctgtccc aacacatctc ccttttccat 480
   gactgtggta ncccgcatcg gaaaaa 506
<210> 107
   <211> 452
   <212> DNA
   <213> Homo sapien
<220>
   <221> misc_feature
   <222> (1)...(452)
   <223> n = A,T,C or G
<400> 107
   gttgagtctg tactaaacag taagatatct caatgaacca taaattcaac tttgtaaaaa 60
   tcttttgaag catagataat attgtttggt aaatgtttct tttgtttggt aaatgtttct 120
   tttaaagacc ctcctattct ataaaactct gcatgtagag gcttgtttac ctttctctct 180
   ctaaggttta caataggagt ggtgatttga aaaatataaa attatgagat tggttttcct 240
   gtggcataaa ttgcatcact gtatcatttt cttttttaac cggtaagant ttcagtttgt 300
   tggaaagtaa ctgtganaac ccagtttccc gtccatctcc cttagggact acccatagaa 360
   catgaaaagg tccccacnga agcaagaaga taagtctttc atggctgctg gttgcttaaa 420
   ccactttaaa accaaaaaat tccccttgga aa 452
<210> 108
   <211> 502
   <212> DNA
   <213> Homo sapien
<220>
   <221> misc_feature
   <222> (1)...(502)
   <223> n = A,T,C or G
<400> 108
   atcttcttcc cttaattagt tnttatttat ntattaaatt ttattgcatg tcctggcaaa 60
   caaaaagaga ttgtagattg gcttctggct ccccaaaagc ccataacaga aagtaccaca 120
   agaccncaac tgaagcttaa aaaatctatc acatgtataa tacctttnga agaacattaa 180
   tanagcatat aaaactttta acatntgctt aatgttgtnc aattataaaa ntaatngaaa 240
   aaaatgtccc tttaacatnc aatatcccac atagtgttat ttnaggggat taccnngnaa 300
   naaaaaaagg gtagaaggga tttaatgaaa actctgcttn ccatttctgt ttanaaacgt 360
   ctccagaaca aaaacttntc aantctttca gctaaccgca tttgagctna ggccactcaa 420
   aaactccatt agncccactt tctaanggtc tctanagctt actaancctt ttgacccctt 480
   accctggnta ctcctgccct ca 502
<210> 109
   <211> 1308
   <212> DNA
   <213> Homo sapien
<400> 109
   acccgaggtc tcgctaaaat catcatggat tcacttggcg ccgtcagcac tcgacttggg      60
   tttgatcttt tcaaagagct gaagaaaaca aatgatggca acatcttctt ttcccctgtg     120
   ggcatcttga ctgcaattgg catggtcctc ctggggaccc gaggagccac cgcttcccag     180
   ttggaggagg tgtttcactc tgaaaaagag acgaagagct caagaataaa ggctgaagaa     240
   aaagaggtga ttgagaacac agaagcagta catcaacaat tccaaaagtt tttgactgaa     300
   ataagcaaac tcactaatga ttatgaactg aacataacca acaggctgtt tggagaaaaa     360
   acatacctct tccttcaaaa atacttagat tatgttgaaa aatattatca tgcatctctg     420
   gaacctgttg attttgtaaa tgcagccgat gaaagtcgaa agaagattaa ttcctgggtt     480
   gaaagcaaaa caaatgaaaa aatcaaggac ttgttcccag atggctctat tagtagctct     540
   accaagctgg tgctggtgaa catggtttat tttaaagggc aatgggacag ggagtttaag     600
   aaagaaaata ctaaggaaga gaaattttgg atgaataaga gcacaagtaa atctgtacag     660
   atgatgacac agagccattc ctttagcttc actttcctgg aggacttgca ggccaaaatt     720
   ctagggattc catataaaaa caacgaccta agcatgtttg tgcttctgcc caacgacatc     780
   gatggcctgg agaagataat agataaaata agtcctgaga aattggtaga gtggactagt     840
   ccagggcata tggaagaaag aaaggtgaat ctgcacttgc cccggtttga ggtggaggac     900
   agttacgatc tagaggcggt cctggctgcc atggggatgg gcgatgcctt cagtgagcac     960
   aaagccgact actcgggaat gtcgtcaggc tccgggttgt acgcccagaa gttcctgcac    1020
   agttcctttg tggcagtaac tgaggaaggc accgaggctg cagctgccac tggcataggc    1080
   tttactgtca catccgcccc aggtcatgaa aatgttcact gcaatcatcc cttcctgttc    1140
   ttcatcaggc acaatgaatc caacagcatc ctcttcttcg gcagattttc ttctccttaa    1200
   gatgatcgtt gccatggcat tgctgctttt agcaaaaaac aactaccagt gttactcata    1260
   tgattatgaa aatcgtccat tcttttaaat ggtggctcac ttgcattt                 1308
<210> 110
   <211> 391
   <212> PRT
   <213> Homo sapien
<400> 110
<210> 111
   <211> 1419
   <212> DNA
   <213> Homo sapien
<400> 111
   ggagaactat aaattaagga tcccagctac ttaattgact tatgcttcct agttcgttgc      60
   ccagccacca ccgtctctcc aaaaacccga ggtctcgcta aaatcatcat ggattcactt     120
   ggcgccgtca gcactcgact tgggtttgat cttttcaaag agctgaagaa aacaaatgat     180
   ggcaacatct tcttttcccc tgtgggcatc ttgactgcaa ttggcatggt cctcctgggg     240
   acccgaggag ccaccgcttc ccagttggag gaggtgtttc actctgaaaa agagacgaag     300
   agctcaagaa taaaggctga agaaaaagag gtggtaagaa taaaggctga aggaaaagag     360
   attgagaaca cagaagcagt acatcaacaa ttccaaaagt ttttgactga aataagcaaa      420
   ctcactaatg attatgaact gaacataacc aacaggctgt ttggagaaaa aacatacctc     480
   ttccttcaaa aatacttaga ttatgttgaa aaatattatc atgcatctct ggaacctgtt     540
   gattttgtaa atgcagccga tgaaagtcga aagaagatta attcctgggt tgaaagcaaa     600
   acaaatgaaa aaatcaagga cttgttccca gatggctcta ttagtagctc taccaagctg     660
   gtgctggtga acatggttta ttttaaaggg caatgggaca gggagtttaa gaaagaaaat     720
   actaaggaag agaaattttg gatgaataag agcacaagta aatctgtaca gatgatgaca     780
   cagagccatt cctttagctt cactttcctg gaggacttgc aggccaaaat tctagggatt     840
   ccatataaaa acaacgacct aagcatgttt gtgcttctgc ccaacgacat cgatggcctg     900
   gagaagataa tagataaaat aagtcctgag aaattggtag agtggactag tccagggcat     960
   atggaagaaa gaaaggtgaa tctgcacttg ccccggtttg aggtggagga cagttacgat    1020
   ctagaggcgg tcctggctgc catggggatg ggcgatgcct tcagtgagca caaagccgac    1080
   tactcgggaa tgtcgtcagg ctccgggttg tacgcccaga agttcctgca cagttccttt    1140
   gtggcagtaa ctgaggaagg caccgaggct gcagctgcca ctggcatagg ctttactgtc    1200
   acatccgccc caggtcatga aaatgttcac tgcaatcatc ccttcctgtt cttcatcagg    1260
   cacaatgaat ccaacagcat cctcttcttc ggcagatttt cttctcctta agatgatcgt    1320
   tgccatggca ttgctgcttt tagcaaaaaa caactaccag tgttactcat atgattatga    1380
   aaatcgtcca ttcttttaaa tggtggctca cttgcattt                            1419
<210> 112
   <211> 400
   <212> PRT
   <213> Homo sapien
<400> 112
<210> 113
   <211> 957
   <212> DNA
   <213> Homo sapien
<400> 113
   ctcgaccttc tctgcacagc ggatgaaccc tgagcagctg aagaccagaa aagccactat      60
   gactttctgc ttaattcagg agcttacagg attcttcaaa gagtgtgtcc agcatccttt     120
   gaaacatgag ttcttaccag cagaagcaga cctttacccc accacctcag cttcaacagc     180
   agcaggtgaa acaacccagc cagcctccac ctcaggaaat atttgttccc acaaccaagg     240
   agccatgcca ctcaaaggtt ccacaacctg gaaacacaaa gattccagag ccaggctgta     300
   ccaaggtccc tgagccaggc tgtaccaagg tccctgagcc aggttgtacc aaggtccctg     360
   agccaggatg taccaaggtc cctgagccag gttgtaccaa ggtccctgag ccaggctaca     420
   ccaaggtccc tgagccaggc agcatcaagg tccctgacca aggcttcatc aagtttcctg     480
   agccaggtgc catcaaagtt cctgagcaag gatacaccaa agttcctgtg ccaggctaca     540
   caaaggtacc agagccatgt ccttcaacgg tcactccagg cccagctcag cagaagacca     600
   agcagaagta atttggtgca cagacaagcc cttgagaagc caaccaccag atgctggaca     660
   ccctcttccc atctgtttct gtgtcttaat tgtctgtaga ccttgtaatc agtacattct     720
   caccccaagc catagtctct ctcttatttg tatcctaaaa atacggtact ataaagcttt     780
   tgttcacaca cactctgaag aatcctgtaa gcccctgaat taagcagaaa gtcttcatgg     840
   cttttctggt cttcggctgc tcagggttca tctgaagatt cgaatgaaaa gaaatgcatg     900
   tttcctgctc tgccctcatt aaattgcttt taattccaaa aaaaaaaaaa aaaaaaa        957
<210> 114
   <211> 161
   <212> PRT
   <213> Homo sapien
<400> 114
   Met Ser Ser Tyr Gln Gln Lys Gln Thr Phe Thr Pro Pro Pro Gln Leu
   1               5                   10                  15
   Gln Gln Gln Gln Val Lys Gln Pro Ser Gln Pro Pro Pro Gln Glu Ile
               20                  25                  30
   Phe Val Pro Thr Thr Lys Glu Pro Cys His Ser Lys Val Pro Gln Pro
           35                  40                  45
   Gly Asn Thr Lys Ile Pro Glu Pro Gly Cys Thr Lys Val Pro Glu Pro
       50                  55                  60
   Gly Cys Thr Lys Val Pro Glu Pro Gly Cys Thr Lys Val Pro Glu Pro
   65                  70                  75                  80
   Gly Cys Thr Lys Val Pro Glu Pro Gly Cys Thr Lys Val Pro Glu Pro
                   85                  90                  95
   Gly Tyr Thr Lys Val Pro Glu Pro Gly Ser Ile Lys Val Pro Asp Gln
               100                 105                 110
   Gly Phe Ile Lys Phe Pro Glu Pro Gly Ala Ile Lys Val Pro Glu Gln
           115                 120                 125
   Gly Tyr Thr Lys Val Pro Val Pro Gly Tyr Thr Lys Val Pro Glu Pro
       130                 135                 140
   Cys Pro Ser Thr Val Thr Pro Gly Pro Ala Gln Gln Lys Thr Lys Gln
   145                 150                 155                 160
   Lys
<210> 115
   <211> 506
   <212> DNA
   <213> Homo sapien
<220>
   <221> misc_feature
   <222> (1)...(506)
   <223> n = A,T,C or G
<400> 115
   cattggtnct ttcatttgct ntggaagtgt nnatctctaa cagtggacaa agttcccngt     60
   gccttaaact ctgtnacact tttgggaant gaaaanttng tantatgata ggttattctg    120
   angtanagat gttctggata ccattanatn tgcccccngt gtcagaggct catattgtgt    180
   tatgtaaatg gtatntcatt cgctactatn antcaattng aaatanggtc tttgggttat    240
   gaatantnng cagcncanct nanangctgt ctgtngtatt cattgtggtc atagcacctc    300
   acancattgt aacctcnatc nagtgagaca nactagnaan ttcctagtga tggctcanga    360
   ttccaaatgg nctcatntcn aatgtttaaa agttanttaa gtgtaagaaa tacagactgg    420
   atgttccacc aactagtacc tgtaatgacn ggcctgtccc aacacatctc ccttttccat    480
   gactgtggta ncccgcatcg gaaaaa                                         506
<210> 116
   <211> 3079
   <212> DNA
   <213> Homo sapien
<400> 116
   ggatccccgg gtttcctaaa ccccccacag agtcctgccc aggccaaaga gcaaggaaaa     60
   ggtcaaaggg cagaaaaaat gctgagttag gaggagctat ggaaggataa acctggcctt    120
   aaagaggtca aagtggttta tagggggcgc tgagggcttc ccacattctc tggcctaaac    180
   cttgcaggca gatctgccca gtgggctctg ggatagctgt gccttcccta acaaaaaaat    240
   tgtgcacaaa aggatgaaac tctattttcc ctctagcaca taaccaagaa tataaggcta    300
   cagattgcct ttcccagagg gaaaaccctg cagcaacctg ctgcctggaa aagtgtaaga    360
   gcagatcact ggggaatcgt ttgccccccg ctgatggaca gcttccccaa gctccaaggg    420
   caggtgctca gcatgtaccg tactgggatg gttgtcaata ctcctggtcc tgtaagagtc    480
   ccaggacact gccatgccaa tgccccctca gttcctggca tcctttttgg gctgctcaca    540
   gccccagcct ctatggtgaa gacatacttg ctagcagcgt caccaacttg ttgccaagag    600
   atcagtgctc gaaggcaagg ttatttctaa ctgagcagag cctgccagga agaaagcgtt    660
   tgcaccccac accactgtgc aggtgtgacc ggtgagctca cagctgcccc ccaggcatgc    720
   ccagcccact taatcatcac agctcgacag ctctctcgcc cagcccagtt ctggaaggga    780
   taaaaagggg catcaccgtt cctgggtaac agagccacct tctgcgtcct gctgagctct    840
   gttctctcca gcacctccca acccactagt gcctggttct cttgctccac caggaacaag    900
   ccaccatgtc tcgccagtca agtgtgtctt ccggagcggg gggcagtcgt agcttcagca    960
   ccgcctctgc catcaccccg tctgtctccc gcaccagctt cacctccgtg tcccggtccg   1020
   ggggtggcgg tggtggtggc ttcggcaggg tcagccttgc gggtgcttgt ggagtgggtg   1080
   gctatggcag ccggagcctc tacaacctgg ggggctccaa gaggatatcc atcagcacta   1140
   gtggtggcag cttcaggaac cggtttggtg ctggtgctgg aggcggctat ggctttggag   1200
   gtggtgccgg tagtggattt ggtttcggcg gtggagctgg tggtggcttt gggctcggtg   1260
   gcggagctgg ctttggaggt ggcttcggtg gccctggctt tcctgtctgc cctcctggag   1320
   gtatccaaga ggtcactgtc aaccagagtc tcctgactcc cctcaacctg caaatcgacc   1380
   ccagcatcca gagggtgagg accgaggagc gcgagcagat caagaccctc aacaataagt   1440
   ttgcctcctt catcgacaag gtgcggttcc tggagcagca gaacaaggtt ctggaaacaa    1500
   agtggaccct gctgcaggag cagggcacca agactgtgag gcagaacctg gagccgttgt    1560
   tcgagcagta catcaacaac ctcaggaggc agctggacag catcgtgggg gaacggggcc    1620
   gcctggactc agagctgaga aacatgcagg acctggtgga agacttcaag aacaagtatg    1680
   aggatgaaat caacaagcgt accactgctg agaatgagtt tgtgatgctg aagaaggatg    1740
   tagatgctgc ctacatgaac aaggtggagc tggaggccaa ggttgatgca ctgatggatg    1800
   agattaactt catgaagatg ttctttgatg cggagctgtc ccagatgcag acgcatgtct    1860
   ctgacacctc agtggtcctc tccatggaca acaaccgcaa cctggacctg gatagcatca    1920
   tcgctgaggt caaggcccag tatgaggaga ttgccaaccg cagccggaca gaagccgagt    1980
   cctggtatca gaccaagtat gaggagctgc agcagacagc tggccggcat ggcgatgacc    2040
   tccgcaacac caagcatgag atctctgaga tgaaccggat gatccagagg ctgagagccg    2100
   agattgacaa tgtcaagaaa cagtgcgcca atctgcagaa cgccattgcg gatgccgagc    2160
   agcgtgggga gctggccctc aaggatgcca ggaacaagct ggccgagctg gaggaggccc    2220
   tgcagaaggc caagcaggac atggcccggc tgctgcgtga gtaccaggag ctcatgaaca    2280
   ccaagctggc cctggacgtg gagatcgcca cttaccgcaa gctgctggag ggcgaggaat    2340
   gcagactcag tggagaagga gttggaccag tcaacatctc tgttgtcaca agcagtgttt    2400
   cctctggata tggcagtggc agtggctatg gcggtggcct cggtggaggt cttggcggcg    2460
   gcctcggtgg aggtcttgcc ggaggtagca gtggaagcta ctactccagc agcagtgggg    2520
   gtgtcggcct aggtggtggg ctcagtgtgg ggggctctgg cttcagtgca agcagtagcc    2580
   gagggctggg ggtgggcttt ggcagtggcg ggggtagcag ctccagcgtc aaatttgtct    2640
   ccaccacctc ctcctcccgg aagagcttca agagctaaga acctgctgca agtcactgcc    2700
   ttccaagtgc agcaacccag cccatggaga ttgcctcttc taggcagttg ctcaagccat    2760
   gttttatcct tttctggaga gtagtctaga ccaagccaat tgcagaacca cattctttgg    2820
   ttcccaggag agccccattc ccagcccctg gtctcccgtg ccgcagttct atattctgct    2880
   tcaaatcagc cttcaggttt cccacagcat ggcccctgct gacacgagaa cccaaagttt    2940
   tcccaaatct aaatcatcaa aacagaatcc ccaccccaat cccaaatttt gttttggttc    3000
   taactacctc cagaatgtgt tcaataaaat gttttataat ataagctggt gtgcagaatt    3060
   gttttttttt tctacccaa                                                  3079
<210> 117
   <211> 6921
   <212> DNA
   <213> Homo sapien
<400> 117
   gaattctgac tgtccactca aaacttctat tccgatcaaa gctatctgtg actacagaca      60
   aattgagata accatttaca aagacgatga atgtgttttg gcgaataact ctcatcgtgc     120
   taaatggaag gtcattagtc ctactgggaa tgaggctatg gtcccatctg tgtgcttcac     180
   cgttcctcca ccaaacaaag aagcggtgga ccttgccaac agaattgagc aacagtatca     240
   gaatgtcctg actctttggc atgagtctca cataaacatg aagagtgtag tatcctggca     300
   ttatctcatc aatgaaattg atagaattcg agctagcaat gtggcttcaa taaagacaat     360
   gctacctggt gaacatcagc aagttctaag taatctacaa tctcgttttg aagattttct     420
   ggaagatagc caggaatccc aagtcttttc aggctcagat ataacacaac tggaaaagga     480
   ggttaatgta tgtaagcagt attatcaaga acttcttaaa tctgcagaaa gagaggagca     540
   agaggaatca gtttataatc tctacatctc tgaagttcga aacattagac ttcggttaga     600
   gaactgtgaa gatcggctga ttagacagat tcgaactccc ctggaaagag atgatttgca     660
   tgaaagtgtg ttcagaatca cagaacagga gaaactaaag aaagagctgg aacgacttaa     720
   agatgatttg ggaacaatca caaataagtg tgaggagttt ttcagtcaag cagcagcctc     780
   ttcatcagtc cctaccctac gatcagagct taatgtggtc cttcagaaca tgaaccaagt     840
   ctattctatg tcttccactt acatagataa gttgaaaact gttaacttgg tgttaaaaaa     900
   cactcaagct gcagaagccc tcgtaaaact ctatgaaact aaactgtgtg aagaagaagc     960
   agttatagct gacaagaata atattgagaa tctaataagt actttaaagc aatggagatc    1020
   tgaagtagat gaaaagagac aggtattcca tgccttagag gatgagttgc agaaagctaa    1080
   agccatcagt gatgaaatgt ttaaaacgta taaagaacgg gaccttgatt ttgactggca    1140
   caaagaaaaa gcagatcaat tagttgaaag gtggcaaaat gttcatgtgc agattgacaa    1200
   caggttacgg gacttagagg gcattggcaa atcactgaag tactacagag acacttacca 1260
   tcctttagat gattggatcc agcaggttga aactactcag agaaagattc aggaaaatca 1320
   gcctgaaaat agtaaaaccc tagccacaca gttgaatcaa cagaagatgc tggtgtccga 1380
   aatagaaatg aaacagagca aaatggacga gtgtcaaaaa tatgcagaac agtactcagc 1440
   tacagtgaag gactatgaat tacaaacaat gacctaccgg gccatggtag attcacaaca 1500
   aaaatctcca gtgaaacgcc gaagaatgca gagttcagca gatctcatta ttcaagagtt 1560
   catggaccta aggactcgat atactgccct ggtcactctc atgacacaat atattaaatt 1620
   tgctggtgat tcattgaaga ggctggaaga ggaggagatt aaaaggtgta aggagacttc 1680
   tgaacatggg gcatattcag atctgcttca gcgtcagaag gcaacagtgc ttgagaatag 1740
   caaacttaca ggaaagataa gtgagttgga aagaatggta gctgaactaa agaaacaaaa 1800
   gtcccgagta gaggaagaac ttccgaaggt cagggaggct gcagaaaatg aattgagaaa 1860
   gcagcagaga aatgtagaag atatctctct gcagaagata agggctgaaa gtgaagccaa 1920
   gcagtaccgc agggaacttg aaaccattgt gagagagaag gaagccgctg aaagagaact 1980
   ggagcgggtg aggcagctca ccatagaggc cgaggctaaa agagctgccg tggaagagaa 2040
   cctcctgaat tttcgcaatc agttggagga aaacaccttt accagacgaa cactggaaga 2100
   tcatcttaaa agaaaagatt taagtctcaa tgatttggag caacaaaaaa ataaattaat 2160
   ggaagaatta agaagaaaga gagacaatga ggaagaactc ttgaagctga taaagcagat 2220
   ggaaaaagac cttgcatttc agaaacaggt agcagagaaa cagttgaaag aaaagcagaa 2280
   aattgaattg gaagcaagaa gaaaaataac tgaaattcag tatacatgta gagaaaatgc 2340
   attgccagtg tgtccgatca cacaggctac atcatgcagg gcagtaacgg gtctccagca 2400
   agaacatgac aagcagaaag cagaagaact caaacagcag gtagatgaac taacagctgc 2460
   caatagaaag gctgaacaag acatgagaga gctgacatat gaacttaatg ccctccagct 2520
   tgaaaaaacg tcatctgagg aaaaggctcg tttgctaaaa gataaactag atgaaacaaa 2580
   taatacactc agatgcctta agttggagct ggaaaggaag gatcaggcgg agaaagggta 2640
   ttctcaacaa ctcagagagc ttggtaggca attgaatcaa accacaggta aagctgaaga 2700
   agccatgcaa gaagctagtg atctcaagaa aataaagcgc aattatcagt tagaattaga 2760
   atctcttaat catgaaaaag ggaaactaca aagagaagta gacagaatca caagggcaca 2820
   tgctgtagct gagaagaata ttcagcattt aaattcacaa attcattctt ttcgagatga 2880
   gaaagaatta gaaagactac aaatctgcca gagaaaatca gatcatctaa aagaacaatt 2940
   tgagaaaagc catgagcagt tgcttcaaaa tatcaaagct gaaaaagaaa ataatgataa 3000
   aatccaaagg ctcaatgaag aattggagaa aagtaatgag tgtgcagaga tgctaaaaca 3060
   aaaagtagag gagcttacta ggcagaataa tgaaaccaaa ttaatgatgc agagaattca 3120
   ggcagaatca gagaatatag ttttagagaa acaaactatc cagcaaagat gtgaagcact 3180
   gaaaattcag gcagatggtt ttaaagatca gctacgcagc acaaatgaac acttgcataa 3240
   acagacaaaa acagagcagg attttcaaag aaaaattaaa tgcctagaag aagacctggc 3300
   gaaaagtcaa aatttggtaa gtgaatttaa gcaaaagtgt gaccaacaga acattatcat 3360
   ccagaatacc aagaaagaag ttagaaatct gaatgcggaa ctgaatgctt ccaaagaaga 3420
   gaagcgacgc ggggagcaga aagttcagct acaacaagct caggtgcaag agttaaataa 3480
   caggttgaaa aaagtacaag acgaattaca cttaaagacc atagaggagc agatgaccca 3540
   cagaaagatg gttctgtttc aggaagaatc tggtaaattc aaacaatcag cagaggagtt 3600
   tcggaagaag atggaaaaat taatggagtc caaagtcatc actgaaaatg atatttcagg 3660
   cattaggctt gactttgtgt ctcttcaaca agaaaactct agagcccaag aaaatgctaa 3720
   gctttgtgaa acaaacatta aagaacttga aagacagctt caacagtatc gtgaacaaat 3780
   gcagcaaggg cagcacatgg aagcaaatca ttaccaaaaa tgtcagaaac ttgaggatga 3840
   gctgatagcc cagaagcgtg aggttgaaaa cctgaagcaa aaaatggacc aacagatcaa 3900
   agagcatgaa catcaattag ttttgctcca gtgtgaaatt caaaaaaaga gcacagccaa 3960
   agactgtacc ttcaaaccag attttgagat gacagtgaag gagtgccagc actctggaga 4020
   gctgtcctct agaaacactg gacaccttca cccaacaccc agatcccctc tgttgagatg 4080
   gactcaagaa ccacagccat tggaagagaa gtggcagcat cgggttgttg aacagatacc 4140
   caaagaagtc caattccagc caccaggggc tccactcgag aaagagaaaa gccagcagtg 4200
   ttactctgag tacttttctc agacaagcac cgagttacag ataacttttg atgagacaaa 4260
   ccccattaca agactgtctg aaattgagaa gataagagac caagccctga acaattctag 4320
   accacctgtt aggtatcaag ataacgcatg tgaaatggaa ctggtgaagg ttttgacacc 4380
   cttagagata gctaagaaca agcagtatga tatgcataca gaagtcacaa cattaaaaca 4440
   agaaaagaac ccagttccca gtgctgaaga atggatgctt gaagggtgca gagcatctgg 4500
   tggactcaag aaaggggatt tccttaagaa gggcttagaa ccagagacct tccagaactt   4560
   tgatggtgat catgcatgtt cagtcaggga tgatgaattt aaattccaag ggcttaggca   4620
   cactgtgact gccaggcagt tggtggaagc taagcttctg gacatgagaa caattgagca   4680
   gctgcgactc ggtcttaaga ctgttgaaga agttcagaaa actcttaaca agtttctgac   4740
   gaaagccacc tcaattgcag ggctttacct agaatctaca aaagaaaaga tttcatttgc   4800
   ctcagcggcc gagagaatca taatagacaa aatggtggct ttggcatttt tagaagctca   4860
   ggctgcaaca ggttttataa ttgatcccat ttcaggtcag acatattctg ttgaagatgc   4920
   agttcttaaa ggagttgttg accccgaatt cagaattagg cttcttgagg cagagaaggc   4980
   agctgtggga tattcttatt cttctaagac attgtcagtg tttcaagcta tggaaaatag   5040
   aatgcttgac agacaaaaag gtaaacatat cttggaagcc cagattgcca gtgggggtgt   5100
   cattgaccct gtgagaggca ttcgtgttcc tccagaaatt gctctgcagc aggggttgtt   5160
   gaataatgcc atcttacagt ttttacatga gccatccagc aacacaagag ttttccctaa   5220
   tcccaataac aagcaagctc tgtattactc agaattactg cgaatgtgtg tatttgatgt   5280
   agagtcccaa tgctttctgt ttccatttgg ggagaggaac atttccaatc tcaatgtcaa   5340
   gaaaacacat agaatttctg tagtagatac taaaacagga tcagaattga ccgtgtatga   5400
   ggctttccag agaaacctga ttgagaaaag tatatatctt gaactttcag ggcagcaata   5460
   tcagtggaag gaagctatgt tttttgaatc ctatgggcat tcttctcata tgctgactga   5520
   tactaaaaca ggattacact tcaatattaa tgaggctata gagcagggaa caattgacaa   5580
   agccttggtc aaaaagtatc aggaaggcct catcacactt acagaacttg ctgattcttt   5640
   gctgagccgg ttagtcccca agaaagattt gcacagtcct gttgcagggt attggctgac   5700
   tgctagtggg gaaaggatct ctgtactaaa agcctcccgt agaaatttgg ttgatcggat   5760
   tactgccctc cgatgccttg aagcccaagt cagtacaggg ggcataattg atcctcttac   5820
   tggcaaaaag taccgggtgg ccgaagcttt gcatagaggc ctggttgatg aggggtttgc   5880
   ccagcagctg cgacagtgtg aattagtaat cacagggatt ggccatccca tcactaacaa   5940
   aatgatgtca gtggtggaag ctgtgaatgc aaatattata aataaggaaa tgggaatccg   6000
   atgtttggaa tttcagtact tgacaggagg gttgatagag ccacaggttc actctcggtt   6060
   atcaatagaa gaggctctcc aagtaggtat tatagatgtc ctcattgcca caaaactcaa   6120
   agatcaaaag tcatatgtca gaaatataat atgccctcag acaaaaagaa agttgacata   6180
   taaagaagcc ttagaaaaag ctgattttga tttccacaca ggacttaaac tgttagaagt   6240
   atctgagccc ctgatgacag gaatttctag cctctactat tcttcctaat gggacatgtt   6300
   taaataactg tgcaaggggt gatgcaggct ggttcatgcc actttttcag agtatgatga   6360
   tatcggctac atatgcagtc tgtgaattat gtaacatact ctatttcttg agggctgcaa   6420
   attgctaagt gctcaaaata gagtaagttt taaattgaaa attacataag atttaatgcc   6480
   cttcaaatgg tttcatttag ccttgagaat ggttttttga aacttggcca cactaaaatg   6540
   tttttttttt tttacgtaga atgtgggata aacttgatga actccaagtt cacagtgtca   6600
   tttcttcaga actccccttc attgaatagt gatcatttat taaatgataa attgcactcg   6660
   ctgaaagagc acgtcatgaa gcaccatgga atcaaagaga aagatataaa ttcgttccca   6720
   cagccttcaa gctgcagtgt tttagattgc ttcaaaaaat gaaaaagttt tgcctttttc    6780
   gatatagtga ccttctttgc atattaaaat gtttaccaca atgtcccatt tctagttaag   6840
   tcttcgcact tgaaagctaa cattatgaat attatgtgtt ggaggagggg aaggattttc   6900
   ttcattctgt gtattttccg g                                             6921
<210> 118
   <211> 946
   <212> DNA
   <213> Homo sapien
<400> 118
   cttctgactg ggctcaggct gacaggtaga gctcaccatg gcttcttgtg tccttgtccc      60
   ctccccatca cagctgtggt gcagtccacc gtctccagtg gctatggcgg tgccagtggt     120
   gtcggcagtg gcttaggcct gggtggagga agcagctact cctatggcag tggtcttggc     180
   gttggaggtg gcttcagttc cagcagtggc agagccattg ggggtggcct cagctctgtt      240
   ggaggcggca gttccaccat caagtacacc accacctcct cctccagcag gaagagctat     300
   aagcactaaa gtgcgtctgc tagctctcgg tcccacagtc ctcaggcccc tctctggctg     360
   cagagccctc tcctcaggtt gcctgtcctc tcctggcctc cagtctcccc tgctgtccca     420
   ggtagagctg gggatgaatg cttagtgccc tcacttcttc tctctctctc tataccatct      480
   gagcacccat tgctcaccat cagatcaacc tctgatttta catcatgatg taatcaccac     540
   tggagcttca ctgttactaa attattaatt tcttgcctcc agtgttctat ctctgaggct     600
   gagcattata agaaaatgac ctctgctcct tttcattgca gaaaattgcc aggggcttat     660
   ttcagaacaa cttccactta ctttccactg gctctcaaac tctctaactt ataagtgttg     720
   tgaaccccca cccaggcagt atccatgaaa gcacaagtga ctagtcctat gatgtacaaa     780
   gcctgtatct ctgtgatgat ttctgtgctc ttcactgttt gcaattgcta aataaagcag     840
   atttataata catatattct tttactttgc cttgctttgg ggccaaagtt ttgggcttaa     900
   acttttttat ctgataagtg aatagttgtt tttaaaagat aatcta                    946
<210> 119
   <211> 8948
   <212> DNA
   <213> Homo sapien
<400> 119
<210> 120
   <211> 587
   <212> DNA
   <213> Homo sapien
<220>
   <221> misc_feature
   <222> (1)...(587)
   <223> n = A,T,C or G
<400> 120
   cgtcctaagc acttagacta catcagggaa gaacacagac cacatccctg tcctcatgcg      60
   gcttatgttt tctggaagaa agtggagacc nagtccttgg ctttagggct ccccggctgg     120
   gggctgtgca ntccggtcag ggcgggaagg gaaatgcacc gctgcatgtg aacttacagc     180
   ccaggcggat gccccttccc ttagcactac ctggcctcct gcatcccctc gcctcatgtt     240
   cctcccacct tcaaanaatg aanaacccca tgggcccagc cccttgccct ggggaaccaa     300
   ggcagccttc caaaactcag gggctgaagc anactattag ggcaggggct gactttgggt      360
   gacactgccc attccctctc agggcagctc angtcacccn ggnctcttga acccagcctg     420
   ttcctttgaa aaagggcaaa actgaaaagg gcttttccta naaaaagaaa aaccagggaa     480
   ctttgccagg gcttcnntnt taccaaaacn ncttctcnng gatttttaat tccccattng     540
   gcctccactt accnggggcn atgccccaaa attaanaatt tcccatc                  587
<210> 121
   <211> 619
   <212> DNA
   <213> Homo sapien
<220>
   <221> misc_feature
   <222> (1)...(619)
   <223> n = A,T,C or G
<400> 121
   cactagtagg atagaaacac tgtgtcccga gagtaaggag agaagctact attgattaga      60
   gcctaaccca ggttaactgc aagaagaggc gggatacttt cagctttcca tgtaactgta     120
   tgcataaagc caatgtagtc cagtttctaa gatcatgttc caagctaact gaatcccact     180
   tcaatacaca ctcatgaact cctgatggaa caataacagg cccaagcctg tggtatgatg     240
   tgcacacttg ctagactcan aaaaaatact actctcataa atgggtggga gtattttggt     300
   gacaacctac tttgcttggc tgagtgaagg aatgatattc atatattcat ttattccatg     360
   gacatttagt tagtgctttt tatataccag gcatgatgct gagtgacact cttgtgtata     420
   tttccaaatt tttgtacagt cgctgcacat atttgaaatc atatattaag acttccaaaa     480
   aatgaagtcc ctggtttttc atggcaactt gatcagtaaa ggattcncct ctgtttggta     540
   cttaaaacat ctactatatn gttnanatga aattcctttt ccccncctcc cgaaaaaana     600
   aagtggtggg gaaaaaaaa                                                619
<210> 122
   <211> 1475
   <212> DNA
   <213> Homo sapien
<400> 122
   tccacctgtc cccgcagcgc cggctcgcgc cctcctgccg cagccaccga gccgccgtct      60
   agcgccccga cctcgccacc atgagagccc tgctggcgcg cctgcttctc tgcgtcctgg     120
   tcgtgagcga ctccaaaggc agcaatgaac ttcatcaagt tccatcgaac tgtgactgtc     180
   taaatggagg aacatgtgtg tccaacaagt acttctccaa cattcactgg tgcaactgcc     240
   caaagaaatt cggagggcag cactgtgaaa tagataagtc aaaaacctgc tatgagggga     300
   atggtcactt ttaccgagga aaggccagca ctgacaccat gggccggccc tgcctgccct     360
   ggaactctgc cactgtcctt cagcaaacgt accatgccca cagatctgat gctcttcagc     420
   tgggcctggg gaaacataat tactgcagga acccagacaa ccggaggcga ccctggtgct     480
   atgtgcaggt gggcctaaag ccgcttgtcc aagagtgcat ggtgcatgac tgcgcagatg     540
   gaaaaaagcc ctcctctcct ccagaagaat taaaatttca gtgtggccaa aagactctga     600
   ggccccgctt taagattatt gggggagaat tcaccaccat cgagaaccag ccctggtttg     660
   cggccatcta caggaggcac cgggggggct ctgtcaccta cgtgtgtgga ggcagcctca     720
   tcagcccttg ctgggtgatc agcgccacac actgcttcat tgattaccca aagaaggagg     780
   actacatcgt ctacctgggt cgctcaaggc ttaactccaa cacgcaaggg gagatgaagt     840
   ttgaggtgga aaacctcatc ctacacaagg actacagcgc tgacacgctt gctcaccaca     900
   acgacattgc cttgctgaag atccgttcca aggagggcag gtgtgcgcag ccatcccgga     960
   ctatacagac catctgcctg ccctcgatgt ataacgatcc ccagtttggc acaagctgtg    1020
   agatcactgg ctttggaaaa gagaattcta ccgactatct ctatccggag cagctgaaga    1080
   tgactgttgt gaagctgatt tcccaccggg agtgtcagca gccccactac tacggctctg    1140
   aagtcaccac caaaatgctg tgtgctgctg acccacagtg gaaaacagat tcctgccagg    1200
   gagactcagg gggacccctc gtctgttccc tccaaggccg catgactttg actggaattg    1260
   tgagctgggg ccgtggatgt gccctgaagg acaagccagg cgtctacacg agagtctcac    1320
   acttcttacc ctggatccgc agtcacacca aggaagagaa tggcctggcc ctctgagggt    1380
   ccccagggag gaaacgggca ccacccgctt tcttgctggt tgtcattttt gcagtagagt    1440
   catctccatc agctgtaaga agagactggg aagat                               1475
<210> 123
   <211> 2294
   <212> DNA
   <213> Homo sapien
<400> 123
   cagcgccggc tcgcgccctc ctgccgcagc caccgagccg ccgtctagcg ccccgacctc    60
   gccaccatga gagccctgct ggcgcgcctg cttctctgcg tcctggtcgt gagcgactcc   120
   aaaggcagca atgaacttca tcaagttcca tcgaactgtg actgtctaaa tggaggaaca   180
   tgtgtgtcca acaagtactt ctccaacatt cactggtgca actgcccaaa gaaattcgga   240
   gggcagcact gtgaaataga taagtcaaaa acctgctatg aggggaatgg tcacttttac   300
   cgaggaaagg ccagcactga caccatgggc cggccctgcc tgccctggaa ctctgccact   360
   gtccttcagc aaacgtacca tgcccacaga tctgatgctc ttcagctggg cctggggaaa   420
   cataattact gcaggaaccc agacaaccgg aggcgaccct ggtgctatgt gcaggtgggc   480
   ctaaagccgc ttgtccaaga gtgcatggtg catgactgcg cagatggaaa aaagccctcc   540
   tctcctccag aagaattaaa atttcagtgt ggccaaaaga ctctgaggcc ccgctttaag   600
   attattgggg gagaattcac caccatcgag aaccagccct ggtttgcggc catctacagg   660
   aggcaccggg ggggctctgt cacctacgtg tgtggaggca gcctcatcag cccttgctgg   720
   gtgatcagcg ccacacactg cttcattgat tacccaaaga aggaggacta catcgtctac   780
   ctgggtcgct caaggcttaa ctccaacacg caaggggaga tgaagtttga ggtggaaaac   840
   ctaatcctac acaaggacta cagcgctgac acgcttgctc accacaacga cattgccttg   900
   ctgaagatcc gttccaagga gggcaggtgt gcgcagccat cccggactat acagaccatc   960
   tgcctgccct cgatgtataa cgatccccag tttggcacaa gctgtgagat cactggcttt  1020
   ggaaaagaga attctaccga ctatctctat ccggagcagc tgaaaatgac tgttgtgaag  1080
   ctgatttccc accgggagtg tcagcagccc cactactacg gctctgaagt caccaccaaa  1140
   atgctgtgtg ctgctgaccc acagtggaaa acagattcct gccagggaga ctcaggggga  1200
   cccctcgtct gttccctcca aggccgcatg actttgactg gaattgtgag ctggggccgt  1260
   ggatgtgccc tgaaggacaa gccaggcgtc tacacgagag tctcacactt cttaccctgg  1320
   atccgcagtc acaccaagga agagaatggc ctggccctct gagggtcccc agggaggaaa  1380
   cgggcaccac ccgctttctt gctggttgct attttgcagt agagtcatct ccatcagctg  1440
   taagaagagc tgggaatata ggctctgcac agatggattt gcctgtgcca ccaccagggc  1500
   gaacgacaat agctttaccc tcaggcatag gcctgggtgc tggctgccca gacccctctg  1560
   gccaggatgg aggggtggtc ctgactcaac atgttactga ccagcaactt gtctttttct  1620
   ggactgaagc ctgcaggagt taaaaagggc agggcatctc ctgtgcatgg gctcgaaggg  1680
   agagccagct cccccgaccg gtgggcattt gtgaggccca tggttgagaa atgaataatt  1740
   tcccaattag gaagtgtaag cagctgaggt ctcttgaggg agcttagcca atgtgggagc  1800
   agcggtttgg ggagcagaga cactaacgac ttcagggcag ggctctgata ttccatgaat  1860
   gtatcaggaa atatatatgt gtgtgtatgt ttgcacactt gtgtgtgggc tgtgagtgta  1920
   agtgtgagta agagctggtg tctgattgtt aagtctaaat atttccttaa actgtgtgga  1980
   ctgtgatgcc acacagagtg gtctttctgg agaggttata ggtcactcct ggggcctctt  2040
   gggtccccca cgtgacagtg cctgggaatg tattattctg cagcatgacc tgtgaccagc  2100
   actgtctcag tttcactttc acatagatgt ccctttcttg gccagttatc ccttcctttt  2160
   agcctagttc atccaatcct cactgggtgg ggtgaggacc actcctgtac actgaatatt  2220
   tatatttcac tatttttatt tatatttttg taattttaaa taaaagtgat caataaaatg  2280
   tgatttttct gatg                                                      2294
<210> 124
   <211> 956
   <212> DNA
   <213> Homo sapien
<400> 124
   gatgagttcc gcaccaagtt tgagacagac caggccctgc gcctgagtgt ggaggccgac      60
   atcaatggcc tgcgcagggt gctggatgag ctgaccctgg ccagagccga cctggagatg     120
   cagattgaga acctcaagga ggagctggcc tacctgaaga agaaccacga ggaggagatg     180
   aacgccctgc gaggccaggt gggtggtgag atcaatgtgg agatggacgc tgccccaggc     240
   gtggacctga gccgcatcct caacgagatg cgtgaccagt atgagaagat ggcagagaag     300
   aaccgcaagg atgccgagga ttggttcttc agcaagacag aggaactgaa ccgcgaggtg     360
   gccaccaaca gtgagctggt gcagagtggc aagagtgaga tctcggagct ccggcgcacc     420
   atgcaggcct tggagataga gctgcagtcc cagctcagca tgaaagcatc cctggagggc     480
   aacctggcgg agacagagaa ccgctactgc gtgcagctgt cccagatcca ggggctgatt     540
   ggcagcgtgg aggagcagct ggcccagctt cgctgcgaga tggagcagca gaaccaggaa     600
   tacaaaatcc tgctggatgt gaagacgcgg ctggagcagg agattgccac ctaccgccgc     660
   ctgctggagg gagaggatgc ccacctgact cagtacaaga aagaaccggt gaccacccgt     720
   caggtgcgta ccattgtgga agaggtccag gatggcaagg tcatctcctc ccgcgagcag     780
   gtccaccaga ccacccgctg aggactcagc taccccggcc ggccacccag gaggcaggga     840
   cgcagccgcc ccatctgccc cacagtctcc ggcctctcca gcctcagccc cctgcttcag     900
   tcccttcccc atgcttcctt gcctgatgac aataaaagct tgttgactca gctatg         956
<210> 125
   <211> 486
   <212> DNA
   <213> Homo sapien
<220>
   <221> misc_feature
   <222> (1) ... (486)
   <223> n = A,T,C or G
<400> 125
   aaattatata tagtgnttca gctcccattg tggtgttcat agtcttctag gaacagataa      60
   acttaagtat tcaattcact cttggcattt tttctttaat ataggctttt tagcctattt     120
   ttggaaaact gcttttcttc tgagaacctt attctgaatg tcatcaactt taccaaacct     180
   tctaagtcca gagctaactt agtactgttt aagttactat tgactgaatt ttcttcattt     240
   tctgtttagc cagtgttacc aaggtaagct ggggaatgaa gtataccaac ttctttcaga     300
   gcattttagg acattatggc agctttagaa ggctgtcttg tttctagcca agggagagcc     360
   agcgcaggtt ttggatacta gagaaagtca tttgcttgta ctattgccat tttagaaagc     420
   tctgatgtga attcaaattt tacctctgtt acttaaagcc aacaatttta aggcagtagt     480
   tttact                                                                486
<210> 126
   <211> 3552
   <212> DNA
   <213> Homo sapien
<400> 126
   cggcaggcag gtctcgtctc ggcaccctcc cggcgcccgc gttctcctgg ccctgcccgg     60
   catcccgatg gccgccgctg ggccccggcg ctccgtgcgc ggagccgtct gcctgcatct    120
   gctgctgacc ctcgtgatct tcagtcgtgc tggtgaagcc tgcaaaaagg tgatacttaa    180
   tgtaccttct aaactagagg cagacaaaat aattggcaga gttaatttgg aagagtgctt    240
   caggtctgca gacctcatcc ggtcaagtga tcctgatttc agagttctaa atgatgggtc    300
   agtgtacaca gccagggctg ttgcgctgtc tgataagaaa agatcattta ccatatggct    360
   ttctgacaaa aggaaacaga cacagaaaga ggttactgtg ctgctagaac atcagaagaa    420
   ggtatcgaag acaagacaca ctagagaaac tgttctcagg cgtgccaaga ggagatgggc    480
   acctattcct tgctctatgc aagagaattc cttgggccct ttcccattgt ttcttcaaca    540
   agttgaatct gatgcagcac agaactatac tgtcttctac tcaataagtg gacgtggagt    600
   tgataaagaa cctttaaatt tgttttatat agaaagagac actggaaatc tattttgcac    660
   tcggcctgtg gatcgtgaag aatatgatgt ttttgatttg attgcttatg cgtcaactgc    720
   agatggatat tcagcagatc tgcccctccc actacccatc agggtagagg atgaaaatga    780
   caaccaccct gttttcacag aagcaattta taattttgaa gttttggaaa gtagtagacc    840
   tggtactaca gtgggggtgg tttgtgccac agacagagat gaaccggaca caatgcatac    900
   gcgcctgaaa tacagcattt tgcagcagac accaaggtca cctgggctct tttctgtgca     960
   tcccagcaca ggcgtaatca ccacagtctc tcattatttg gacagagagg ttgtagacaa   1020
   gtactcattg ataatgaaag tacaagacat ggatggccag ttttttggat tgataggcac    1080
   atcaacttgt atcataacag taacagattc aaatgataat gcacccactt tcagacaaaa   1140
   tgcttatgaa gcatttgtag aggaaaatgc attcaatgtg gaaatcttac gaatacctat   1200
   agaagataag gatttaatta acactgccaa ttggagagtc aattttacca ttttaaaggg   1260
   aaatgaaaat ggacatttca aaatcagcac agacaaagaa actaatgaag gtgttctttc   1320
   tgttgtaaag ccactgaatt atgaagaaaa ccgtcaagtg aacctggaaa ttggagtaaa   1380
   caatgaagcg ccatttgcta gagatattcc cagagtgaca gccttgaaca gagccttggt   1440
   tacagttcat gtgagggatc tggatgaggg gcctgaatgc actcctgcag cccaatatgt   1500
   gcggattaaa gaaaacttag cagtggggtc aaagatcaac ggctataagg catatgaccc   1560
   cgaaaataga aatggcaatg gtttaaggta caaaaaattg catgatccta aaggttggat    1620
   caccattgat gaaatttcag ggtcaatcat aacttccaaa atcctggata gggaggttga   1680
   aactcccaaa aatgagttgt ataatattac agtcctggca atagacaaag atgatagatc   1740
   atgtactgga acacttgctg tgaacattga agatgtaaat gataatccac cagaaatact   1800
   tcaagaatat gtagtcattt gcaaaccaaa aatggggtat accgacattt tagctgttga   1860
   tcctgatgaa cctgtccatg gagctccatt ttatttcagt ttgcccaata cttctccaga   1920
   aatcagtaga ctgtggagcc tcaccaaagt taatgataca gctgcccgtc tttcatatca   1980
   gaaaaatgct ggatttcaag aatataccat tcctattact gtaaaagaca gggccggcca   2040
   agctgcaaca aaattattga gagttaatct gtgtgaatgt actcatccaa ctcagtgtcg   2100
   tgcgacttca aggagtacag gagtaatact tggaaaatgg gcaatccttg caatattact   2160
   gggtatagca ctgctctttt ctgtattgct aactttagta tgtggagttt ttggtgcaac   2220
   taaagggaaa cgttttcctg aagatttagc acagcaaaac ttaattatat caaacacaga   2280
   agcacctgga gacgatagag tgtgctctgc caatggattt atgacccaaa ctaccaacaa   2340
   ctctagccaa ggtttttgtg gtactatggg atcaggaatg aaaaatggag ggcaggaaac   2400
   cattgaaatg atgaaaggag gaaaccagac cttggaatcc tgccgggggg ctgggcatca   2460
   tcataccctg gactcctgca ggggaggaca cacggaggtg gacaactgca gatacactta   2520
   ctcggagtgg cacagtttta ctcaaccccg tctcggtgaa aaattgcatc gatgtaatca   2580
   gaatgaagac cgcatgccat cccaagatta tgtcctcact tataactatg agggaagagg   2640
   atctccagct ggttctgtgg gctgctgcag tgaaaagcag gaagaagatg gccttgactt   2700
   tttaaataat ttggaaccca aatttattac attagcagaa gcatgcacaa agagataatg   2760
   tcacagtgct acaattaggt ctttgtcaga cattctggag gtttccaaaa ataatattgt   2820
   aaagttcaat ttcaacatgt atgtatatga tgattttttt ctcaattttg aattatgcta   2880
   ctcaccaatt tatattttta aagcaagttg ttgcttatct tttccaaaaa gtgaaaaatg   2940
   ttaaaacaga caactggtaa atctcaaact ccagcactgg aattaaggtc tctaaagcat   3000
   ctgctctttt ttttttttac agatatttta gtaataaata tgctggataa atattagtcc   3060
   aacaatagct aagttatgct aatatcacat tattatgtat tcactttaag tgatagttta   3120
   aaaaataaac aagaaatatt gagtatcact atgtgaagaa agttttggaa aagaaacaat   3180
   gaagactgaa ttaaattaaa aatgttgcag ctcataaaga attggactca cccctactgc   3240
   actaccaaat tcatttgact ttggaggcaa aatgtgttga agtgccctat gaagtagcaa   3300
   ttttctatag gaatatagtt ggaaataaat gtgtgtgtgt atattattat taatcaatgc   3360
   aatatttaaa tgaaatgaga acaaagagga aaatggtaaa aacttgaaat gaggctgggg   3420
   tatagtttgt cctacaatag aaaaaagaga gagcttccta ggcctgggct cttaaatgct   3480
   gcattataac tgagtctatg aggaaatagt tcctgtccaa tttgtgtaat ttgtttaaaa   3540
   ttgtaaataa at                                                         3552
<210> 127
   <211> 754
   <212> DNA
   <213> Homo sapien
<400> 127
   tttttttttt ttgtcattgt tcattgattt taatgagaaa gctaagagag gaaataagta     60
   gcctttcaaa ggtcacacag aagtaagtga cagatccagg attcatatcc aagcattctg    120
   gctctagtgt ccatgcttct caaccattat gacccaatat tcaaccaaat caatactgaa    180
   ggacacgtga aatgtatccg gtattttact attacaaaca aaaatccaat gaacattctt    240
   gaagacatac acaaaaataa tggttacaat agaagttact ggaattgaaa ttttggttca    300
   acctatatta aaatgtaagg cttttgatat agctaataga tttttgaaat gatcagtctt    360
   aacgtttgta ggggagcaca ctcctgcatg ggaaaaagat tcactgtgaa gcacagagca    420
   cctttatggt tggatcatct tgtcattaaa gttcaggcgt tatctatcct gtaagtggca    480
   gaatcaagac tgcaatatcg cctgcttttc tttttaactc atgttttccc ttgactacac    540
   tggtcctcaa agtaaaaccc ctgtgtcagt gtactattca tggaatactc tgcaattata    600
   accaccttct aatactttta atacccaatc aaaatttatt atacatatgt atcatagata    660
   ctcatctgta aagctgtgct tcaaaatagt gatctcttcc caacattaca atatatatta    720
   atgatgtcga acctgcccgg gcggccgctc gaag                                754
<210> 128
   <211> 374
   <212> DNA
   <213> Homo sapien
<400> 128
   aggttttgat taaaaaggca aatgatttta ttgttcgata atcttttaaa aaaataagag     60
   gaaggagtaa aattaaagat gaaagatgat ttttatttcc ttgtgacctc tatatccccc    120
   ttcccctgcc cttggtaagt aactcttgat ggagaaagga ttaaagactc ttatttaacc    180
   aaaaaacaga gccagctaat catttccaaa ggttagtatc tccctgctga cctcttcttt    240
   ggtttaattg aataaaacta tatgttcata tatgtattaa aacaactcag aataacatct    300
   tttcttcctt agttaaggca ttataagggc tatactatca tccataataa ccaaggcaat    360
   aacttaaaaa gctg                                                      374
<210> 129
   <211> 546
   <212> DNA
   <213> Homo sapien
<400> 129
   agtgtgatgg atatctgcag aattcgggct aagcgtggtc gcggcccgag gtctggaact      60
   tcccagcacy tgaaaaggag cctcctgagc tgactcggct aaagccccac tttcgctcct     120
   cctcatttct gcctactgat ttccttggag cattcatctg aatattaccg tttgctgtgt     180
   aacctggtac atacatagca tgactccctg gaatagagtg ggctggggtg cttatgctgg     240
   gagagtgatt gacatgcact ttcaagctat atctaccatt tgcagcaaag gagaaaaaat     300
   acctcgagta aattccatca ttttttataa catcagcacc tgctccatca tcaaggagtc     360
   tcagcgtaac aggatctcca gtctctggct caactgtggc agtgacagtg gcattaagaa     420
   tgggataaaa tccctgtttc acattggcat aaatcatcac aggatgagga aaatggaggc     480
   tgtctctttc cacaaaggct tccacagtgg ctgggggcac agacctgccc gggcggccgc     540
   tcgaaa                                                                546
<210> 130
   <211> 5156
   <212> DNA
   <213> Homo sapien
<400> 130

<210> 131
   <211> 671
   <212> DNA
   <213> Homo sapien
<400> 131
   aggtctggag ggcccacagc cggatgtggg acaccgggaa aaagtggtca tagcacacat      60
   ttttgcatcc cggttgcagt gtgttgcaga cgaagtcctc ttgctcgtca ccccacactt     120
   cctgggcagc caycacgagg atcatgactc ggaaaataaa gatgactgtg atccacacct     180
   tcccgatgct ggtggagtgt ttgttgacac ccccgatgaa agtgtgcagc gtcccccaat      240
   ccattgcgct ggtttatccc tgagtcctgt ttccaacgac tgccagtgtt tcagacccaa     300
   agaatgaggg caagatccct ctgcgagggt ttcagacctc cttctcctac cccactggag     360
   tgcctagaag ccaatgggtg cacagtgatg atacgaatgt caatctttgc tcggtcagtg     420
   aggatgtcgc ctggaatatt caaattgaat tacagatgca tgaagagggc gtacaagtta     480
   gaatttttct ttcgccatac agaaattgtt tagccagatc ttctgtactt cttttccttc     540
   cctgaccctt cctgctcccc aggaagggag gtcagccccg tttgcaaaac acaggatgcc     600
   cgtgacaccg gagacaggtc ttcttcaccg acaggaagtg ccttctggtg cctgcacgtt     660
   ttaactgcta t                                                          671
<210> 132
   <211> 590
   <212> DNA
   <213> Homo sapien
<400> 132
   ctgaatggaa aagcttatgg ctctgtgatg atattagtga ccagcggaga tgataagctt      60
   cttggcaatt gcttacccac tgtgctcagc agtggttcaa caattcactc cattgccctg     120
   ggttcatctg cagccccaaa tctggaggaa ttatcacgtc ttacaggagg tttaaagttc     180
   tttgttccag atatatcaaa ctccaatagc atgattgatg ctttcagtag aatttcctct     240
   ggaactggag acattttcca gcaacatatt cagcttgaaa gtacaggtga aaatgtcaaa     300
   cctcaccatc aattgaaaaa cacagtgact gtggataata ctgtgggcaa cgacactatg     360
   tttctagtta cgtggcaggc cagtggtcct cctgagatta tattatttga tcctgatgga     420
   cgaaaatact acacaaataa ttttatcacc aatctaactt ttcggacagc tagtctttgg     480
   attccaggaa cagctaagcc tgggcactgg acttacaccc tgaacaatac ccatcattct     540
   ctgcaagccc tgaaagtgac agtgacctct cgcgcctcca actcagacct                590
<210> 133
   <211> 581
   <212> DNA
   <213> Homo sapien
<400> 133
   aggtcctgtc cgggggcact gagaactccc tctggaattc ttggggggtg ttggggagag      60
   actgtgggcc tggagataaa acttgtctcc tctaccacca ccctgtaccc tagcctgcac     120
   ctgtcctcat ctctgcaaag ttcagcttcc ttccccaggt ctctgtgcac tctgtcttgg     180
   atgctctggg gagctcatgg gtggaggagt ctccaccaga gggaggctca ggggactggt     240
   tgggccaggg atgaatattt gagggataaa aattgtgtaa gagccaaaga attggtagta     300
   gggggagaac agagaggagc tgggctatgg gaaatgattt gaataatgga gctgggaata     360
   tggctggata tctggtacta aaaaagggtc tttaagaacc tacttcctaa tctcttcccc     420
   aatccaaacc atagctgtct gtccagtgct ctcttcctgc ctccagctct gccccaggct     480
   cctcctagac tctgtccctg ggctagggca ggggaggagg gagagcaggg ttgggggaga     540
   ggctgaggag agtgtgacat gtggggagag gaccagacct c                         581
<210> 134
   <211> 4797
   <212> DNA
   <213> Homo sapien
<220>
   <221> misc_feature
   <222> (1)...(4797)
   <223> n = A,T,C or G
<400> 134
<210> 135
   <211> 2856
   <212> DNA
   <213> Homo sapien
<400> 135
   tagtcgcggg tccccgagtg agcacgccag ggagcaggag accaaacgac gggggtcgga      60
   gtcagagtcg cagtgggagt ccccggaccg gagcacgagc ctgagcggga gagcgccgct     120
   cgcacgcccg tcgccacccg cgtacccggc gcagccagag ccaccagcgc agcgctgcca     180
   tggagcccag cagcaagaag ctgacgggtc gcctcatgct ggctgtggga ggagcagtgc     240
   ttggctccct gcagtttggc tacaacactg gagtcatcaa tgccccccag aaggtgatcg     300
   aggagttcta caaccagaca tgggtccacc gctatgggga gagcatcctg cccaccacgc     360
   tcaccacgct ctggtccctc tcagtggcca tcttttctgt tgggggcatg attggctcct     420
   tctctgtggg ccttttcgtt aaccgctttg gccggcggaa ttcaatgctg atgatgaacc     480
   tgctggcctt cgtgtccgcc gtgctcatgg gcttctcgaa actgggcaag tcctttgaga     540
   tgctgatcct gggccgcttc atcatcggtg tgtactgcgg cctgaccaca ggcttcgtgc     600
   ccatgtatgt gggtgaagtg tcacccacag cctttcgtgg ggccctgggc accctgcacc     660
   agctgggcat cgtcgtcggc atcctcatcg cccaggtgtt cggcctggac tccatcatgg     720
   gcaacaagga cctgtggccc ctgctgctga gcatcatctt catcccggcc ctgctgcagt     780
   gcatcgtgct gcccttctgc cccgagagtc cccgcttcct gctcatcaac cgcaacgagg     840
   agaaccgggc caagagtgtg ctaaagaagc tgcgcgggac agctgacgtg acccatgacc     900
   tgcaggagat gaaggaagag agtcggcaga tgatgcggga gaagaaggtc accatcctgg     960
   agctgttccg ctcccccgcc taccgccagc ccatcctcat cgctgtggtg ctgcagctgt    1020
   cccagcagct gtctggcatc aacgctgtct tctattactc cacgagcatc ttcgagaagg    1080
   cgggggtgca gcagcctgtg tatgccacca ttggctccgg tatcgtcaac acggccttca    1140
   ctgtcgtgtc gctgtttgtg gtggagcgag caggccggcg gaccctgcac ctcataggcc    1200
   tcgctggcat ggcgggttgt gccatactca tgaccatcgc gctagcactg ctggagcagc    1260
   taccctggat gtcctatctg agcatcgtgg ccatctttgg ctttgtggcc ttctttgaag    1320
   tgggtcctgg ccccatccca tggttcatcg tggctgaact cttcagccag ggtccacgtc    1380
   cagctgccat tgccgttgca ggcttctcca actggacctc aaatttcatt gtgggcatgt    1440
   gcttccagta tgtggagcaa ctgtgtggtc cctacgtctt catcatcttc actgtgctcc    1500
   tggttctgtt cttcatcttc acctacttca aagttcctga gactaaaggc cggaccttcg    1560
   atgagatcgc ttccggcttc cggcaggggg gagccagcca aagtgataag acacccgagg    1620
   agctgttcca tcccctgggg gctgattccc aagtgtgagt cgccccagat caccagcccg    1680
   gcctgctccc agcagcccta aggatctctc aggagcacag gcagctggat gagacttcca    1740
   aacctgacag atgtcagccg agccgggcct ggggctcctt tctccagcca gcaatgatgt    1800
   ccagaagaat attcaggact taacggctcc aggattttaa caaaagcaag actgttgctc    1860
   aaatctattc agacaagcaa caggttttat aattttttta ttactgattt tgttattttt    1920
   atatcagcct gagtctcctg tgcccacatc ccaggcttca ccctgaatgg ttccatgcct    1980
   gagggtggag actaagccct gtcgagacac ttgccttctt cacccagcta atctgtaggg    2040
   ctggacctat gtcctaagga cacactaatc gaactatgaa ctacaaagct tctatcccag    2100
   gaggtggcta tggccacccg ttctgctggc ctggatctcc ccactctagg ggtcaggctc    2160
   cattaggatt tgccccttcc catctcttcc tacccaacca ctcaaattaa tctttcttta    2220
   cctgagacca gttgggagca ctggagtgca gggaggagag gggaagggcc agtctgggct    2280
   gccgggttct agtctccttt gcactgaggg ccacactatt accatgagaa gagggcctgt    2340
   gggagcctgc aaactcactg ctcaagaaga catggagact cctgccctgt tgtgtataga    2400
   tgcaagatat ttatatatat ttttggttgt caatattaaa tacagacact aagttatagt     2460
   atatctggac aagccaactt gtaaatacac cacctcactc ctgttactta cctaaacaga    2520
   tataaatggc tggtttttag aaacatggtt ttgaaatgct tgtggattga gggtaggagg    2580
   tttggatggg agtgagacag aagtaagtgg ggttgcaacc actgcaacgg cttagacttc    2640
   gactcaggat ccagtccctt acacgtacct ctcatcagtg tcctcttgct caaaaatctg    2700
   tttgatccct gttacccaga gaatatatac attctttatc ttgacattca aggcatttct    2760
   atcacatatt tgatagttgg tgttcaaaaa aacactagtt ttgtgccagc cgtgatgctc    2820
   aggcttgaaa tcgcattatt ttgaatgtga agggaa                              2856
<210> 136
   <211> 356
   <212> DNA
   <213> Homo sapien
<400> 136
   ggtggagcca aatgaagaaa atgaagatga aagagacaga cacctcagtt tttctggatc      60
   aggcattgat gatgatgaag attttatctc cagcaccatt tcaaccacac cacgggcttt     120
   tgaccacaca aaacagaacc aggactggac tcagtggaac ccaagccatt caaatccgga     180
   agtgctactt cagacaacca caaggatgac tgatgtagac agaaatggca ccactgctta     240
   tgaaggaaac tggaacccag aagcacaccc tcccctcatt caccatgagc atcatgagga     300
   agaagagacc ccacattcta caagcacaat ccaggcaact cctagtagta caacgg         356
<210> 137
   <211> 356
   <212> DNA
   <213> Homo sapien
<220>
   <221> misc_feature
   <222> (1)...(356)
   <223> n = A,T,C or G
<400> 137
   gcaggtggag aagacatttt attgttcctg gggtctctgg aggcccattg gtggggctgg      60
   gtcactggct gcccccggaa cagggcgctg ctccatggct ctgcttgtgg tagtctgtgg     120
   ctatgtctcc cagcaaggac agaaactcag aaaaatcaat cttcttatcc tcattcttgt     180
   cctttttctc aaagacatcg gcgaggtaat ttgtgccctt tttacctcgg cccgcgacca      240
   cgctaaggcc aaanttccag acanayggcc gggccggtnc nataggggan cccaacttgg      300
   ggacccaaac tctggcgcgg aaacacangg gcataagctt gnttcctgtg gggaaa         356
<210> 138
   <211> 353
   <212> DNA
   <213> Homo sapien
<400> 138
   aggtccagtc ctccacttgg cctgatgaga gtggggagtg gcaagggacg tttctcctgc      60
   aatagacact tagatttctc tcttgtggga agaaaccacc tgtccatcca ctgactcttc     120
   tacattgatg tggaaattgc tgctgctacc accacctcct gaagaggctt ccctgatgcc     180
   aatgccagcc atcttggcat cctggccctc gagcaggctg cggtaagtag cgatctcctg     240
   ctccagccgt gtctttatgt caagcagcat cttgtactcc tggttctgag cctccatctc     300
   gcatcggagc tcactcagac ctcgsccgsg mssmcgctam gccgaattcc agc            353
<210> 139
   <211> 371
   <212> DNA
   <213> Homo sapien
<400> 139
   agcgtggtcg cggccgaggt ccatccgaag caagattgca gatggcagtg tgaagagaga      60
   agacatattc tacacttcaa agctttggtg caattcccat cgaccagagt tggtccgacc     120
   agccttggaa aggtcactga aaaatcttca attggattat gttgacctct accttattca     180
   ttttccagtg tctgtaaagc caggtgagga agtgatccca aaagatgaaa atggaaaaat     240
   actatttgac acagtggatc tctgtgccac gtgggaggcc gtggagaagt gtaaagatgc     300
   aggattggac ctgcccgggc ggccgctcga aagccgaatt ccagcacact ggcggccgtt     360
   actagtggat c                                                          371
<210> 140
   <211> 370
   <212> DNA
   <213> Homo sapien
<400> 140
   tagcgtggtc gcggccgagg tccatctccc tttgggaact agggggctgc tggtgggaaa      60
   tgggagccag ggcagatgtt gcattccttt gtgtccctgt aaatgtggga ctacaagaag     120
   aggagctgcc tgagtggtac tttctcttcc tggtaatcct ctggcccagc ctcatggcag     180
   aatagaggta tttttaggct atttttgtaa tatggcttct ggtcaaaatc cctgtgtagc     240
   tgaattccca agccctgcat tgtacagccc cccactcccc tcaccaccta ataaaggaat     300
   agttaacact caaaaaaaaa aaaaaacctg cccgggcggc cgctcgaaag ccgaattcca     360
   gcacactggc                                                            370
<210> 141
   <211> 371
   <212> DNA
   <213> Homo sapien
<400> 141
   tagcgtggtc gcggccgagg tcctctgtgc tgcctgtcac agcccgatgg taccagcgca      60
   gggtgtaggc agtgcaggag ccctcatcca gtggcaggga acaggggtca tcactatccc     120
   aaggagcttc agggtcctgg tactcctcca cagaatactc ggagtattca gagtactcat     180
   catcctcagg gggtacccgc tcttcctcct ctgcatgaga gacgcggagc acaggcacag     240
   catggagctg ggagccggca gtgtctgcag cataactagg gaggggtcgt gatccagatg     300
   cgatgaactg gccctggcag gcacagtgct gactcatctc ttggcgacct gcccgggcgg     360
   ccgctcgaag c                                                          371
<210> 142
   <211> 343
   <212> DNA
   <213> Homo sapien
<400> 142
   gcgttttgag gccaatggtg taaaaggaaa tatcttcaca taaaaactag atggaagcat    60
   tgtcagaaac ctctttgtga tgtttgcttt caactcacag agttgaacat tccttttcat   120
   agagcagttt tgaaacactc ttttgtagaa tttgcaagcg gatgattgga tcgctatgag   180
   gtcttcattg gaaacgggat acctttacat aaaaactaga cagtagcatt ctcagaaatt   240
   tctttgggat gtgggcattc aacccacaga ggagaacttc atttgataga gcagttttga   300
   aacacccttt ttgtagaatc tacaggtgga catttagagt gct                     343
<210> 143
   <211> 354
   <212> DNA
   <213> Homo sapien
<400> 143
   aggtctgatg gcagaaaaac tcagactgtc tgcaacttta cagatggtgc attggttcag    60
   catcaggagt gggatgggaa ggaaagcaca ataacaagaa aattgaaaga tgggaaatta   120
   gtggtggagt gtgtcatgaa caatgtcacc tgtactcgga tctatgaaaa agtagaataa   180
   aaattccatc atcactttgg acaggagtta attaagagaa tgaccaagct cagttcaatg   240
   agcaaatctc catactgttt ctttcttttt tttttcatta ctgtgttcaa ttatctttat   300
   cataaacatt ttacatgcag ctatttcaaa gtgtgttgga ttaattagga tcat         354
<210> 144
   <211> 353
   <212> DNA
   <213> Homo sapien
<400> 144
   ggtcaaggac ctgggggacc cccaggtcca gcagccacat gattctgcag cagacaggga    60
   cctagagcac atctggatct cagccccacc cctggcaacc tgcctgccta gagaactccc   120
   aagatgacag actaagtagg attctgccat ttagaataat tctggtatcc tgggcgttgc   180
   gttaagttgc ttaactttca ttctgtctta cgatagtctt cagaggtggg aacagatgaa   240
   gaaaccatgc cccagagaag gttaagtgac ttcctcttta tggagccagt gttccaacct   300
   aggtttgcct gataccagac ctgtggcccc acctcccatg caggtctctg tgg          353
<210> 145
   <211> 371
   <212> DNA
   <213> Homo sapien
<400> 145
   caggtctgtc ataaactggt ctggagtttc tgacgactcc ttgttcacca aatgcaccat    60
   ttcctgagac ttgctggcct ctccgttgag tccacttggc tttctgtcct ccacagctcc   120
   attgccactg ttgatcacta gctttttctt ctgcccacac cttcttcgac tgttgactgc   180
   aatgcaaact gcaagaatca aagccaaggc caagagggat gccaagatga tcagccattc   240
   tggaatttgg ggtgtcctta taggaccaga ggttgtgttt gctccacctt cttgactccc   300
   atgtgagacc tcggccgcga ccacgctaag ccgaattcca gcacactggc ggcccgttac   360
   tagtggatcc g                                                        371
<210> 146
   <211> 355
   <212> DNA
   <213> Homo sapien
<400> 146
   ggtcctccgt cctcttccca gaggtgtcgg ggcttggccc cagcctccat cttcgtctct      60
   caggatggcg agtagcagcg gctccaaggc tgaattcatt gtcggaggga aatataaact     120
   ggtacggaag atcgggtctg gctccttcgg ggacatctat ttggcgatca acatcaccaa     180
   cggcgaggaa gtggcagtga agctagaatc tcagaaggcc aggcatcccc agttgctgta     240
   cgagagcaag ctctataaga ttcttcaagg tggggttggc atcccccaca tacggtggta     300
   tggtcaggaa aaagactaca atgtactagt catggatctt ctgggaccta gcctc          355
<210> 147
   <211> 355
   <212> DNA
   <213> Homo sapien
<400> 147
   ggtctgttac aaaatgaaga cagacaacac aacatttact ctgtggagat atcctactca      60
   tactatgcac gtgctgtgat tttgaacata actcgtccca aaaacttgtc acgatcatcc     120
   tgacttttta ggttggctga tccatcaatc ttgcactcaa ctgttacttc tttcccagtg     180
   ttgttaggag caaagctgac ctgaacagca accaatggct gtagataccc aacatgcagt     240
   tttttcccat aatatgggaa atattttaag tctatcattc cattatgagg ataaactgct     300
   acatttggta tatcttcatt ctttgaaaca caatctatcc ttggcactcc ttcag          355
<210> 148
   <211> 369
   <212> DNA
   <213> Homo sapien
<400> 148
   aggtctctct ccccctctcc ctctcctgcc agccaagtga agacatgctt acttcccctt      60
   caccttcctt catgatgtgg gaagagtgct gcaacccagc cctagccaac accgcatgag     120
   agggagtgtg ccgagggctt ctgagaaggt ttctctcaca tctagaaaga agcgcttaag     180
   atgtggcagc ccctcttctt caagtggctc ttgtcctgtt gccctgggag ttctcaaatt     240
   gctgcagcag cctccatcca gcctgaggat gacatcaata cacagaggaa gaagagtcag     300
   gaaaagatga gagaagttac agactctcct gggcgacccc gagagcttac cattcctcag     360
   acttcttca                                                             369
<210> 149
   <211> 620
   <212> DNA
   <213> Homo sapien
<220>
   <221> mise feature
   <222> (1)...(620)
   <223> n = A,T,C or G
<400> 149
   actagtcaaa aatgctaaaa taatttggga gaaaatattt tttaagtagt gttatagttt      60
   catgtttatc ttttattatg ttttgtgaag ttgtgtcttt tcactaatta cctatactat     120
   gccaatattt ccttatatct atccataaca tttatactac atttgtaana naatatgcac     180
   gtgaaactta acactttata aggtaaaaat gaggtttcca anatttaata atctgatcaa     240
   gttcttgtta tttccaaata gaatggactt ggtctgttaa gggctaagga gaagaggaag     300
   ataaggttaa aagttgttaa tgaccaaaca ttctaaaaga aatgcaaaaa aaaagtttat     360
   tttcaagcct tcgaactatt taaggaaagc aaaatcattt cctaaatgca tatcatttgt     420
   gagaatttct cattaatatc ctgaatcatt catttcacta aggctcatgt tnactccgat     480
   atgtctctaa gaaagtacta tttcatggtc caaacctggt tgccatantt gggtaaaggc     540
   tttcccttaa gtgtgaaant atttaaaatg aaattttcct ctttttaaaa attctttana     600
   agggttaagg gtgttgggga                                                 620
<210> 150
   <211> 371
   <212> DNA
   <213> Homo sapien
<400> 150
   ggtccgatca aaacctgcta cctccccaag actttactag tgccgataaa ctttctcaaa      60
   gagcaaccag tatcacttcc ctgtttataa aacctctaac catctctttg ttctttgaac     120
   atgctgaaaa ccacctggtc tgcatgtatg cccgaatttg yaattctttt ctctcaaatg     180
   aaaatttaat tttagggatt catttctata ttttcacata tgtagtatta ttatttcctt     240
   atatgtgtaa ggtgaaattt atggtatttg agtgtgcaag aaaatatatt tttaaagctt     300
   tcatttttcc cccagtgaat gatttagaat tttttatgta aatatacaga atgttttttc      360
   ttacttttat a                                                          371
<210> 151
   <211> 4655
   <212> DNA
   <213> Homo sapien
<400> 151
   gggacttgag ttctgttatc ttcttaagta gattcatatt gtaagggtct cggggtgggg      60
   gggttggcaa aatcctggag ccagaagaaa ggacagcagc attgatcaat cttacagcta     120
   acatgttgta cctggaaaac aatgcccaga ctcaatttag tgagccacag tacacgaacc     180
   tggggctcct gaacagcatg gaccagcaga ttcagaacgg ctcctcgtcc accagtccct     240
   ataacacaga ccacgcgcag aacagcgtca cggcgccctc gccctacgca cagcccagct     300
   ccaccttcga tgctctctct ccatcacccg ccatcccctc caacaccgac tacccaggcc     360
   cgcacagttt cgacgtgtcc ttccagcagt cgagcaccgc caagtcggcc acctggacgt     420
   attccactga actgaagaaa ctctactgcc aaattgcaaa gacatgcccc atccagatca     480
   aggtgatgac cccacctcct cagggagctg ttatccgcgc catgcctgtc tacaaaaaag    540
   ctgagcacgt cacggaggtg gtgaagcggt gccccaacca tgagctgagc cgtgaattca     600
   acgagggaca gattgcccct yctagtcatt tgattcgagt agaggggaac agccatgccc     660
   agtatgtaga agatcccatc acaggaagac agagtgtgct ggtaccttat gagccacccc     720
   aggttggcac tgaattcacg acagtcttgt acaatttcat gtgtaacagc agttgtgttg     780
   gagggatgaa ccgccgtcca attttaatca ttgttactct ggaaaccaga gatgggcaag     840
   tcctgggccg acgctgcttt gaggcccgga tctgtgcttg cccaggaaga gacaggaagg     900
   cggatgaaga tagcatcaga aagcagcaag tttcggacag tacaaagaac ggtgatggta     960
   cgaagcgccc gtttcgtcag aacacacatg gtatccagat gacatccatc aagaaacgaa    1020
   gatccccaga tgatgaactg gtatacttac cagtgagggg ccgtgagact tatgaaatgc    1080
   tggtgaagat caaagagtcc ctggaactca tgcagtacct tcttcagcac acaattgaaa    1140
   cgtacaggca acagcaacag cagcagcacc agcacttact tcagaaacag acctcaatac    1200
   agtctccatc ttcatatggt aacagctccc cacctctgaa caaaatgaac agcatgaaca    1260
   agctgccttc tgtgagccag cttatcaacc ctcagcagcg caacgccctc actcctacaa    1320
   ccattcctga tggcatggga gccaacattc ccatgatggg cacccacatg ccaatggctg    1380
   gagacatgaa tggactcagc cccacccagg cactccctcc cccactctcc atgccatcca 1440
   cctcccactg cacaccccca cctccgtatc ccacagattg cagcattgtc agtttcttag 1500
   cgaggttggg ctgttcatca tgtctggact atttcacgac ccaggggctg accaccatct 1560
   atcagattga gcattactcc atggatgatc tggcaagtct gaaaatccct gagcaatttc 1620
   gacatgcgat ctggaagggc atcctggacc accggcagct ccacgaattc tcctcccctt 1680
   ctcatctcct gcggacccca agcagtgcct ctacagtcag tgtgggctcc agtgagaccc 1740
   ggggtgagcg tgttattgat gctgtgcgat tcaccctccg ccagaccatc tctttcccac 1800
   cccgagatga gtggaatgac ttcaactttg acatggatgc tcgccgcaat aagcaacagc 1860
   gcatcaaaga ggagggggag tgagcctcac catgtgagct cttcctatcc ctctcctaac 1920
   tgccagcccc ctaaaagcac tcctgcttaa tcttcaaagc cttctcccta gctcctcccc 1980
   ttcctcttgt ctgatttctt aggggaagga gaagtaagag gcttacttct taccctaacc 2040
   atctgacctg gcatctaatt ctgattctgg ctttaagcct tcaaaactat agcttgcaga 2100
   actgtagctt gccatggcta ggtagaagtg agcaaaaaag agttgggtgt ctccttaagc 2160
   tgcagagatt tctcattgac ttttataaag catgttcacc cttatagtct aagactatat 2220
   atataaatgt ataaatatac agtatagatt tttgggtggg gggcattgag tattgtttaa 2280
   aatgtaattt aaatgaaaga aaattgagtt gcacttattg accatttttt aatttacttg 2340
   ttttggatgg cttgtctata ctccttccct taaggggtat catgtatggt gataggtatc 2400
   tagagcttaa tgctacatgt gagtgacgat gatgtacaga ttctttcagt tctttggatt 2460
   ctaaatacat gccacatcaa acctttgagt agatccattt ccattgctta ttatgtaggt 2520
   aagactgtag atatgtattc ttttctcagt gttggtatat tttatattac tgacatttct 2580
   tctagtgatg atggttcacg ttggggtgat ttaatccagt tataagaaga agttcatgtc 2640
   caaacgtcct ctttagtttt tggttgggaa tgaggaaaat tcttaaaagg cccatagcag 2700
   ccagttcaaa aacacccgac gtcatgtatt tgagcatatc agtaaccccc ttaaatttaa 2760
   taccagatac cttatcttac aatattgatt gggaaaacat ttgctgccat tacagaggta 2820
   ttaaaactaa atttcactac tagattgact aactcaaata cacatttgct actgttgtaa 2880
   gaattctgat tgatttgatt gggatgaatg ccatctatct agttctaaca gtgaagtttt 2940
   actgtctatt aatattcagg gtaaatagga atcattcaga aatgttgagt ctgtactaaa 3000
   cagtaagata tctcaatgaa ccataaattc aactttgtaa aaatcttttg aagcatagat 3060
   aatattgttt ggtaaatgtt tcttttgttt ggtaaatgtt tcytttaaag accctcctat 3120
   tctataaaac tctgcatgta gaggcttgtt tacctttctc tctctaaggt ttacaatagg 3180
   agtggtgatt tgaaaaatat aaaattatga gattggtttt cctgtggcat aaattgcatc 3240
   actgtatcat tttctttttt aaccggtaag agtttcagtt tgttggaaag taactgtgag 3300
   aacccagttt cccgtccatc tcccttaggg actacccata gacatgaaag gtccccacag 3360
   agcaagagat aagtctttca tggctgctgt tgcttaaacc acttaaacga agagttccct 3420
   tgaaactttg ggaaaacatg ttaatgacaa tattccagat ctttcagaaa tataacacat 3480
   ttttttgcat gcatgcaaat gagctctgaa atcttcccat gcattctggt caagggctgt 3540
   cattgcacat aagcttccat tttaatttta aagtgcaaaa gggccagcgt ggctctaaaa 3600
   ggtaatgtgt ggattgcctc tgaaaagtgt gtatatattt tgtgtgaaat tgcatacttt 3660
   gtattttgat tatttttttt ttcttcttgg gatagtggga tttccagaac cacacttgaa 3720
   accttttttt atcgtttttg tattttcatg aaaataccat ttagtaagaa taccacatca 3780
   aataagaaat aatgctacaa ttttaagagg ggagggaagg gaaagttttt ttttttatta 3840
   tttttttaaa attttgtatg ttaaagagaa tgagtccttg atttcaaagt tttgttgtac 3900
   ttaaatggta ataagcactg taaacttctg caacaagcat gcagctttgc aaacccatta 3960
   aggggaagaa tgaaagctgt tccttggtcc tagtaagaag acaaactgct tcccttactt 4020
   tgctgagggt ttgaataaac ctaggacttc cgagctatgt cagtactatt caggtaacac 4080
   tagggccttg gaaatccctg tactgtgtct catggatttg gcactagcca aagcgaggca 4140
   ccccttactg gcttacctcc tcatggcagc ctactctcct tgagtgtatg agtagccagg 4200
   gtaaggggta aaaggatagt aagcatagaa accactagaa agtgggctta atggagttct 4260
   tgtggcctca gctcaatgca gttagctgaa gaattgaaaa gtttttgttt ggagacgttt 4320
   ataaacagaa atggaaagca gagttttcat taaatccttt tacctttttt ttttcttggt 4380
   aatcccctaa aataacagta tgtgggatat tgaatgttaa agggatattt ttttctatta 4440
   tttttataat tgtacaaaat taagcaaatg ttaaaagttt tatatgcttt attaatgttt 4500
   tcaaaaggta ttatacatgt gatacatttt ttaagcttca gttgcttgtc ttctggtact 4560
   ttctgttatg ggcttttggg gagccagaag ccaatctaca atctcttttt gtttgccagg 4620
   acatgcaata aaatttaaaa aataaataaa aacta 4655
<210> 152
   <211> 586
   <212> PRT
   <213> Homo sapien
<400> 152
   Met Leu Tyr Leu Glu Asn Asn Ala Gln Thr Gln Phe Ser Glu Pro Gln
   1               5                   10                  15
   Tyr Thr Asn Leu Gly Leu Leu Asn Ser Met Asp Gln Gln Ile Gln Asn
               20                  25                  30
   Gly Ser Ser Ser Thr Ser Pro Tyr Asn Thr Asp His Ala Gln Asn Ser
           35                  40                  45
   Val Thr Ala Pro Ser Pro Tyr Ala Gln Pro Ser Ser Thr Phe Asp Ala
       50                  55                  60
   Leu Ser Pro Ser Pro Ala Ile Pro Ser Asn Thr Asp Tyr Pro Gly Pro
   65                  70                  75                  80
   His Ser Phe Asp Val Ser Phe Gln Gln Ser Ser Thr Ala Lys Ser Ala
                   85                  90                  95
   Thr Trp Thr Tyr Ser Thr Glu Leu Lys Lys Leu Tyr Cys Gln Ile Ala
               100                 105                 110
   Lys Thr Cys Pro Ile Gln Ile Lys Val Met Thr Pro Pro Pro Gln Gly
           115                 120                 125
   Ala Val Ile Arg Ala Met Pro Val Tyr Lys Lys Ala Glu His Val Thr
       130                 135                 140
   Glu Val Val Lys Arg Cys Pro Asn His Glu Leu Ser Arg Glu Phe Asn
   145                 150                 155                 160
   Glu Gly Gln Ile Ala Pro Ser Ser His Leu Ile Arg Val Glu Gly Asn
                   165                 170                 175
   Ser His Ala Gln Tyr Val Glu Asp Pro Ile Thr Gly Arg Gln Ser Val
               180                 185                 190
   Leu Val Pro Tyr Glu Pro Pro Gln Val Gly Thr Glu Phe Thr Thr Val
           195                 200                 205
   Leu Tyr Asn Phe Met Cys Asn Ser Ser Cys Val Gly Gly Met Asn Arg
       210                 215                 220
   Arg Pro Ile Leu Ile Ile Val Thr Leu Glu Thr Arg Asp Gly Gln Val
   225                 230                 235                 240
   Leu Gly Arg Arg Cys Phe Glu Ala Arg Ile Cys Ala Cys Pro Gly Arg
                   245                 250                 255
   Asp Arg Lys Ala Asp Glu Asp Ser Ile Arg Lys Gln Gln Val Ser Asp
               260                 265                 270
   Ser Thr Lys Asn Gly Asp Gly Thr Lys Arg Pro Phe Arg Gln Asn Thr
           275                 280                 285
   His Gly Ile Gln Met Thr Ser Ile Lys Lys Arg Arg Ser Pro Asp Asp
       290                 295                 300
   Glu Leu Val Tyr Leu Pro Val Arg Gly Arg Glu Thr Tyr Glu Met Leu
   305                 310                 315                 320
   Val Lys Ile Lys Glu Ser Leu Glu Leu Met Gln Tyr Leu Leu Gln His
                   325                 330                 335
   Thr Ile Glu Thr Tyr Arg Gln Gln Gln Gln Gln Gln His Gln His Leu
               340                 345                 350
   Leu Gln Lys Gln Thr Ser Ile Gln Ser Pro Ser Ser Tyr Gly Asn Ser
           355                 360                 365
   Ser Pro Pro Leu Asn Lys Met Asn Ser Met Asn Lys Leu Pro Ser Val
       370                 375                 380
   Ser Gln Leu Ile Asn Pro Gln Gln Arg Asn Ala Leu Thr Pro Thr Thr
   385                 390                 395                 400
   Ile Pro Asp Gly Met Gly Ala Asn Ile Pro Met Met Gly Thr His Met
                   405                 410                 415
   Pro Met Ala Gly Asp Met Asn Gly Leu Ser Pro Thr Gln Ala Leu Pro
               420                 425                 430
   Pro Pro Leu Ser Met Pro Ser Thr Ser His Cys Thr Pro Pro Pro Pro
           435                 440                 445
   Tyr Pro Thr Asp Cys Ser Ile Val Ser Phe Leu Ala Arg Leu Gly Cys
       450                 455                 460
   Ser Ser Cys Leu Asp Tyr Phe Thr Thr Gln Gly Leu Thr Thr Ile Tyr
   465                 470                 475                 480
   Gln Ile Glu His Tyr Ser Met Asp Asp Leu Ala Ser Leu Lys Ile Pro
                   485                 490                 495
   Glu Gln Phe Arg His Ala Ile Trp Lys Gly Ile Leu Asp His Arg Gln
               500                 505                 510
   Leu His Glu Phe Ser Ser Pro Ser His Leu Leu Arg Thr Pro Ser Ser
           515                 520                 525
   Ala Ser Thr Val Ser Val Gly Ser Ser Glu Thr Arg Gly Glu Arg Val
       530                 535                 540
   Ile Asp Ala Val Arg Phe Thr Leu Arg Gln Thr Ile Ser Phe Pro Pro
   545                 550                 555                 560
   Arg Asp Glu Trp Asn Asp Phe Asn Phe Asp Met Asp Ala Arg Arg Asn
                   565                 570                 575
   Lys Gln Gln Arg Ile Lys Glu Glu Gly Glu
               580                 585
<210> 153
   <211> 2007
   <212> DNA
   <213> Homo sapien
<400> 153
<210> 154
   <211> 2148
   <212> DNA
   <213> Homo sapien
<400> 154
   gaattcgtcg ctgctccagg gaaagttctg ttactccact gactctctct tttcctgata     60
   acatggccag caagaaagta attacagtgt ttggagcaac aggagctcaa ggtggctctg    120
   tggccagggc aattttggag agcaaaaaat ttgcagtgag agcagtgacc agggatgtga    180
   cttgaccaaa tgccctggag ctccagcgcc ttggagctga ggtggtcaaa ggtgacctga    240
   atgataaagc atcggtggac agtgccttaa aaggggaagc tggtggcaga ctccgccaag    300
   cacctgggtc tgaagcacgt ggtgtacagc ggcctggaga acgtcaagcg actgacggat    360
   ggcaagctgg aggtgccgca ctttgacagc aagggcgagg tggaggagta cttctggtcc    420
   attggcatcc ccatgaccag tgtccgcgtg gcggcctact ttgaaaactt tctcgcggcg    480
   tggcggcccg tgaaagcctc tgatggagat tactacacct tggctgtacc gatgggagat    540
   gtaccaatgg atggtatctc tgttgctgat attggagcag ccgtctctag catttttaat    600
   tctccagagg aatttttagg caaggccgtg gggctcagtg cagaagcact aacaatacag    660
   caatatgctg atgttttgtc caaggctttg gggaaagaag tccgagatgc aaagactatc    720
   tgtgctatag atgaccagaa aacagtggaa gaaggtttca tggaagacgt gggcttgagt    780
   tggtccttga gggaacatga ccatgtatag acagaggagg catcaagaag gctggcctgg    840
   ctaattctgg aataaacacg acaaaccaga ggcagtacgg gaaggaggca aattctggct    900
   ctgcctctat ccttgattac cccggaagct ttcgagaagc tgggattccc tgcagcaaag    960
   gaaatagcca atatgtgtcg tttctatgaa atgaagccag accgagatgt caatctcacc   1020
   caccaactaa atcccaaagt caaaagcttc agccatttta tctcagagaa ccagggagcc   1080
   ttcaagggca tgtagaaaat cagctgttca gataggcctc tgcaccacac agcctctttc   1140
   ctctctgatc cttttcctct ttacggcaca acattcatgt tgacagaaca tgctggaatg   1200
   caattgtttg caacaccgaa ggatttcctg cggtcgcctc ttcagtagga agcactgcat   1260
   tggtgatagg acacggtaat ttgattcaca tttaacttgc tagttagtga taagggtggt   1320
   acaactgttt ggtaaaatga gaagcctcgg aacttggagc ttctctccta ccactaatgg   1380
   gagggcagat tatactggga tttctcctgg gtgagtaatt tcaagcccta atgctgaaat   1440
   tcccctaggc agctccagtt ttctcaactg cattgcaaaa ttcccagtga acttttaagt   1500
   acttttaact taaaaaaatg aacatctttg tagagaattt tctggggaac atggtgttca   1560
   atgaacaagc acaagcattg gaaatgctaa aattcagttt tgcctcaaga ttggaagttt   1620
   attttctgac tcattcatga agtcatctat tgagccacca ttcaattatt catctattaa   1680
   ttccttgatc cttcatttat ccattctgca aacttttctt gagcaccagc acgggtggcc   1740
   atttgtggac ttctcttcat tcctatgtgt tttcttatca aagtgatcca ctctcgaaag   1800
   gctcctttcc agtctgtggt tgggttcaag tcatgccagg gccagggggc ccatctcctc   1860
   gtttagctct aggcaaaatc caggggatct gcagtgggga gcgggggcag gaagctggag   1920
   ggaaggcctg tgaagggtag ggatgtggaa agacaaggtg acagaaggac ccaataggac   1980
   ctttctatat ctctggctta gcattttcta catcatattg taatcgtctt atttgctagt   2040
   tttcttcctt actgtgagtg actaacagtc atctttatcc cagtgcctgg tacataataa   2100
   gtgatcaata aatgttgatt gactaaatga aaaaaaaaaa aaaaaaaa                  2148
<210> 155
   <211> 153
   <212> PRT
   <213> Homo sapien
<400> 155
   Met Thr Ser Val Arg Val Ala Ala Tyr Phe Glu Asn Phe Leu Ala Ala
    1               5                  10                  15
   Trp Arg Pro Val Lys Ala Ser Asp Gly Asp Tyr Tyr Thr Leu Ala Val
               20                  25                  30
   Pro Met Gly Asp Val Pro Met Asp Gly Ile Ser Val Ala Asp Ile Gly
           35                  40                  45
   Ala Ala Val Ser Ser Ile Phe Asn Ser Pro Glu Glu Phe Leu Gly Lys
       50                  55                  60
   Ala Val Gly Leu Ser Ala Glu Ala Leu Thr Ile Gln Gln Tyr Ala Asp
   65                  70                  75                  80
   Val Leu Ser Lys Ala Leu Gly Lys Glu Val Arg Asp Ala Lys Ile Thr
                   85                  90                  95
   Pro Glu Ala Phe Glu Lys Leu Gly Phe Pro Ala Ala Lys Glu Ile Ala
               100                 105                 110
   Asn Met Cys Arg Phe Tyr Glu Met Lys Pro Asp Arg Asp Val Asn Leu
           115                 120                 125
   Thr His Gln Leu Asn Pro Lys Val Lys Ser Phe Ser Gln Phe Ile Ser
       130                 135                 140
   Glu Asn Gln Gly Ala Phe Lys Gly Met
   145                 150
<210> 156
   <211> 128
   <212> PRT
   <213> Homo sapien
<400> 156
   Met Thr Ser Val Arg Val Ala Ala Tyr Phe Glu Asn Phe Leu Ala Ala
    1               5                  10                  15
   Trp Arg Pro Val Lys Ala Ser Asp Gly Asp Tyr Tyr Thr Leu Ala Val
               20                  25                  30
   Pro Met Gly Asp Val Pro Met Asp Gly Ile Ser Val Ala Asp Ile Gly
           35                  40                  45
   Ala Ala Val Ser Ser Ile Phe Asn Ser Pro Glu Glu Phe Leu Gly Lys
       50                  55                  60
   Ala Val Gly Leu Ser Ala Glu Ala Leu Thr Ile Gln Gln Tyr Ala Asp
   65                  70                  75                  80
   Val Leu Ser Lys Ala Leu Gly Lys Glu Val Arg Asp Ala Lys Thr Ile
                   85                  90                  95
   Cys Ala Ile Asp Asp Gln Lys Thr Val Glu Glu Gly Phe Met Glu Asp
               100                 105                 110
   Val Gly Leu Ser Trp Ser Leu Arg Glu His Asp His Val Ala Gly Ala
           115                 120                 125
<210> 157
   <211> 424
   <212> DNA
   <213> Homo sapien
<220>
   <221> misc_feature
   <222> (1)...(424)
   <223> n = A,T,C or G
<400> 157
   ctgcagcccg ggggatccac tagtccagtg tggtggaatt cattggtctt tacaagactt      60
   ggatacatta cagcagacat ggaaatataa ttttaaaaaa tttctctcca acctccttca     120
   aattcagtca ccactgttat attaccttct ccaggaaccc tccagtgggg aaggctgcga     180
   tattagattt ccttgtatgc aaagtttttg ttgaaagctg tgctcagagg aggtgagagg     240
   agaggaagga gaaaactgca tcataacttt acagaattga atctagagtc ttccccgaaa     300
   agcccagaaa cttctctgcn gnatctggct tgtccatctg gtctaaggtg gctgcttctt     360
   ccccagccat cgagtcagtt tgtgcccatg aataatacac gacctgctat ttcccatgac     420
   tgct                                                                  424
<210> 158
   <211> 2099
   <212> DNA
   <213> Homo sapien
<400> 158
<210> 159
   <211> 291
   <212> PRT
   <213> Homo sapien
<400> 159
<210> 160
   <211> 3951
   <212> DNA
   <213> Homo sapien
<400> 160
   tctgcatcca tattgaaaac ctgacacaat gtatgcagca ggctcagtgt gagtgaactg      60
   gaggcttctc tacaacatga cccaaaggag cattgcaggt cctatttgca acctgaagtt    120
   tgtgactctc ctggttgcct taagttcaga actcccattc ctgggagctg gagtacagct    180
   tcaagacaat gggtataatg gattgctcat tgcaattaat cctcaggtac ctgagaatca    240
   gaacctcatc tcaaacatta aggaaatgat aactgaagct tcattttacc tatttaatgc    300
   taccaagaga agagtatttt tcagaaatat aaagatttta atacctgcca catggaaagc    360
   taataataac agcaaaataa aacaagaatc atatgaaaag gcaaatgtca tagtgactga    420
   ctggtatggg gcacatggag atgatccata caccctacaa tacagagggt gtggaaaaga    480
   gggaaaatac attcatttca cacctaattt cctactgaat gataacttaa cagctggcta    540
   cggatcacga ggccgagtgt ttgtccatga atgggcccac ctccgttggg gtgtgttcga    600
   tgagtataac aatgacaaac ctttctacat aaatgggcaa aatcaaatta aagtgacaag    660
   gtgttcatct gacatcacag gcatttttgt gtgtgaaaaa ggtccttgcc cccaagaaaa    720
   ctgtattatt agtaagcttt ttaaagaagg atgcaccttt atctacaata gcacccaaaa    780
   tgcaactgca tcaataatgt tcatgcaaag tttatcttct gtggttgaat tttgtaatgc    840
   aagtacccac aaccaagaag caccaaacct acagaaccag atgtgcagcc tcagaagtgc    900
   atgggatgta atcacagact ctgctgactt tcaccacagc tttcccatga acgggactga    960
   gcttccacct cctcccacat tctcgcttgt agaggctggt gacaaagtgg tctgtttagt   1020
   gctggatgtg tccagcaaga tggcagaggc tgacagactc cttcaactac aacaagccgc   1080
   agaattttat ttgatgcaga ttgttgaaat tcataccttc gtgggcattg ccagtttcga   1140
   cagcaaagga gagatcagag cccagctaca ccaaattaac agcaatgatg atcgaaagtt   1200
   gctggtttca tatctgccca ccactgtatc agctaaaaca gacatcagca tttgttcagg   1260
   gcttaagaaa ggatttgagg tggttgaaaa actgaatgga aaagcttatg gctctgtgat   1320
   gatattagtg accagcggag atgataagct tcttggcaat tgcttaccca ctgtgctcag   1380
   cagtggttca acaattcact ccattgccct gggttcatct gcagccccaa atctggagga   1440
   attatcacgt cttacaggag gtttaaagtt ctttgttcca gatatatcaa actccaatag   1500
   catgattgat gctttcagta gaatttcctc tggaactgga gacattttcc agcaacatat   1560
   tcagcttgaa agtacaggtg aaaatgtcaa acctcaccat caattgaaaa acacagtgac   1620
   tgtggataat actgtgggca acgacactat gtttctagtt acgtggcagg ccagtggtcc   1680
   tcctgagatt atattatttg atcctgatgg acgaaaatac tacacaaata attttatcac   1740
   caatctaact tttcggacag ctagtctttg gattccagga acagctaagc ctgggcactg   1800
   gacttacacc ctgaacaata cccatcattc tctgcaagcc ctgaaagtga cagtgacctc   1860
   tcgcgcctcc aactcagctg tgcccccagc cactgtggaa gcctttgtgg aaagagacag   1920
   cctccatttt cctcatcctg tgatgattta tgccaatgtg aaacagggat tttatcccat   1980
   tcttaatgcc actgtcactg ccacagttga gccagagact ggagatcctg ttacgctgag   2040
   actccttgat gatggagcag gtgctgatgt tataaaaaat gatggaattt actcgaggta   2100
   ttttttctcc tttgctgcaa atggtagata tagcttgaaa gtgcatgtca atcactctcc    2160
   cagcataagc accccagccc actctattcc agggagtcat gctatgtatg taccaggtta   2220
   cacagcaaac ggtaatattc agatgaatgc tccaaggaaa tcagtaggca gaaatgagga   2280
   ggagcgaaag tggggcttta gccgagtcag ctcaggaggc tccttttcag tgctgggagt   2340
   tccagctggc ccccaccctg atgtgtttcc accatgcaaa attattgacc tggaagctgt   2400
   aaaagtagaa gaggaattga ccctatcttg gacagcacct ggagaagact ttgatcaggg   2460
   ccaggctaca agctatgaaa taagaatgag taaaagtcta cagaatatcc aagatgactt   2520
   taacaatgct attttagtaa atacatcaaa gcgaaatcct cagcaagctg gcatcaggga   2580
   gatatttacg ttctcacccc aaatttccac gaatggacct gaacatcagc caaatggaga   2640
   aacacatgaa agccacagaa tttatgttgc aatacgagca atggatagga actccttaca   2700
   gtctgctgta tctaacattg cccaggcgcc tctgtttatt ccccccaatt ctgatcctgt   2760
   acctgccaga gattatctta tattgaaagg agttttaaca gcaatgggtt tgataggaat   2820
   catttgcctt attatagttg tgacacatca tactttaagc aggaaaaaga gagcagacaa   2880
   gaaagagaat ggaacaaaat tattataaat aaatatccaa agtgtcttcc ttcttagata   2940
   taagacccat ggccttcgac tacaaaaaca tactaacaaa gtcaaattaa catcaaaact   3000
   gtattaaaat gcattgagtt tttgtacaat acagataaga tttttacatg gtagatcaac   3060
   aaattctttt tgggggtaga ttagaaaacc cttacacttt ggctatgaac aaataataaa    3120
   aattattctt taaagtaatg tctttaaagg caaagggaag ggtaaagtcg gaccagtgtc   3180
   aaggaaagtt tgttttattg aggtggaaaa atagccccaa gcagagaaaa ggagggtagg   3240
   tctgcattat aactgtctgt gtgaagcaat catttagtta ctttgattaa tttttctttt   3300
   ctccttatct gtgcagaaca ggttgcttgt ttacaactga agatcatgct atatttcata   3360
   tatgaagccc ctaatgcaaa gctctttacc tcttgctatt ttgttatata tattacagat    3420
   gaaatctcac tgctaatgct cagagatctt ttttcactgt aagaggtaac ctttaacaat    3480
   atgggtatta cctttgtctc ttcataccgg ttttatgaca aaggtctatt gaatttattt    3540
   gtttgtaagt ttctactccc atcaaagcag ctttttaagt tattgccttg gttattatgg    3600
   atgatagtta tagcccttat aatgccttaa ctaaggaaga aaagatgtta ttctgagttt    3660
   gttttaatac atatatgaac atatagtttt attcaattaa accaaagaag aggtcagcag    3720
   ggagatacta acctttggaa atgattagct ggctctgttt tttggttaaa taagagtctt    3780
   taatcctttc tccatcaaga gttacttacc aagggcaggg gaagggggat atagaggtcc    3840
   caaggaaata aaaatcatct ttcatcttta attttactcc ttcctcttat ttttttaaaa     3900
   gattatcgaa caataaaatc atttgccttt ttaattaaaa acataaaaaa a             3951
<210> 161
   <211> 943
   <212> PRT
   <213> Homo sapien
<400> 161
<210> 162
   <211> 498
   <212> DNA
   <213> Homo sapien
<400> 162
   tggagaacca cgtggacagc accatgaaca tgttgggcgg gggaggcagt gctggccgga      60
   agcccctcaa gtcgggtatg aaggagctgg ccgtgttccg ggagaaggtc actgagcagc     120
   accggcagat gggcaagggt ggcaagcatc accttggcct ggaggagccc aagaagctgc     180
   gaccaccccc tgccaggact ccctgccaac aggaactgga ccaggtcctg gagcggatct     240
   ccaccatgcg ccttccggat gagcggggcc ctctggagca cctctactcc ctgcacatcc     300
   ccaactgtga caagcatggc ctgtacaacc tcaaacagtg gcaagatgtc tctgaacggg     360
   cagcgtgggg agtgctggtg tgtgaacccc aacaccggga agctgatcca gggagccccc     420
   accatccggg gggaccccga gtgtcatctc ttctacaatg agcagcagga ggctcgcggg     480
   gtgcacaccc cagcggat                                                   498
<210> 163
   <211> 1128
   <212> DNA
   <213> Homo sapien
<400> 163
   gccacctggc cctcctgatc gacgacacac gcacttgaaa cttgttctca gggtgtgtgg     60
   aatcaacttt ccggaagcaa ccagcccacc agaggaggtc ccgagcgcga gcggagacga    120
   tgcagcggag actggttcag cagtggagcg tcgcggtgtt cctgctgagc tacgcggtgc    180
   cctcctgcgg gcgctcggtg gagggtctca gccgccgcct caaaagagct gtgtctgaac    240
   atcagctcct ccatgacaag gggaagtcca tccaagattt acggcgacga ttcttccttc     300
   accatctgat cgcagaaatc cacacagctg aaatcagagc tacctcggag gtgtccccta    360
   actccaagcc ctctcccaac acaaagaacc accccgtccg atttgggtct gatgatgagg    420
   gcagatacct aactcaggaa actaacaagg tggagacgta caaagagcag ccgctcaaga    480
   cacctgggaa gaaaaagaaa ggcaagcccg ggaaacgcaa ggagcaggaa aagaaaaaac    540
   ggcgaactcg ctctgcctgg ttagactctg gagtgactgg gagtgggcta gaaggggacc    600
   acctgtctga cacctccaca acgtcgctgg agctcgattc acggaggcat tgaaattttc    660
   agcagagacc ttccaaggac atattgcagg attctgtaat agtgaacata tggaaagtat    720
   tagaaatatt tattgtctgt aaatactgta aatgcattgg aataaaactg tctcccccat    780
   tgctctatga aactgcacat tggtcattgt gaatattttt ttttttgcca aggctaatcc     840
   aattattatt atcacattta ccataattta ttttgtccat tgatgtattt attttgtaaa    900
   tgtatcttgg tgctgctgaa tttctatatt ttttgtaaca taatgcactt tagatataca    960
   tatcaagtat gttgataaat gacacaatga agtgtctcta ttttgtggtt gattttaatg   1020
   aatgcctaaa tataattatc caaattgatt ttcctttgtg catgtaaaaa taacagtatt   1080
   ttaaatttgt aaagaatgtc taataaaata taatctaatt acatcatg                1128

<210> 164
   <211> 1310
   <212> DNA
   <213> Homo sapien
<400> 164
   gggcctggtt cgcaaagaag ctgacttcag agggggaaac tttcttcttt taggaggcgg     60
   ttagccctgt tccacgaacc caggagaact gctggccaga ttaattagac attgctatgg    120
   gagacgtgta aacacactac ttatcattga tgcatatata aaaccatttt attttcgcta    180
   ttatttcaga ggaagcgcct ctgatttgtt tcttttttcc ctttttgctc tttctggctg    240
   tgtggtttgg agaaagcaca gttggagtag ccggttgcta aataagtccc gagcgcgagc    300
   ggagacgatg cagcggagac tggttcagca gtggagcgtc gcggtgttcc tgctgagcta    360
   cgcggtgccc tcctgcgggc gctcggtgga gggtctcagc cgccgcctca aaagagctgt    420
   gtctgaacat cagctcctcc atgacaaggg gaagtccatc caagatttac ggcgacgatt    480
   cttccttcac catctgatcg cagaaatcca cacagctgaa atcagagcta cctcggaggt    540
   gtcccctaac tccaagccct ctcccaacac aaagaaccac cccgtccgat ttgggtctga    600
   tgatgagggc agatacctaa ctcaggaaac taacaaggtg gagacgtaca aagagcagcc    660
   gctcaagaca cctgggaaga aaaagaaagg caagcccggg aaacgcaagg agcaggaaaa    720
   gaaaaaacgg cgaactcgct ctgcctggtt agactctgga gtgactggga gtgggctaga    780
   aggggaccac ctgtctgaca cctccacaac gtcgctggag ctcgattcac ggaggcattg    840
   aaattttcag cagagacctt ccaaggacat attgcaggat tctgtaatag tgaacatatg    900
   gaaagtatta gaaatattta ttgtctgtaa atactgtaaa tgcattggaa taaaactgtc    960
   tcccccattg ctctatgaaa ctgcacattg gtcattgtga atattttttt ttttgccaag   1020
   gctaatccaa ttattattat cacatttacc ataatttatt ttgtccattg atgtatttat   1080
   tttgtaaatg tatcttggtg ctgctgaatt tctatatttt ttgtaacata atgcacttta   1140
   gatatacata tcaagtatgt tgataaatga cacaatgaag tgtctctatt ttgtggttga   1200
   ttttaatgaa tgcctaaata taattatcca aattgatttt cctttgtgcc cgtaaaaata   1260
   acagtatttt aaatttgtaa agaatgtcta ataaaatata atctaattac              1310
<210> 165
   <211> 177
   <212> PRT
   <213> Homo sapien
<400> 165
<210> 166
   <211> 177
   <212> PRT
   <213> Homo sapien
<400> 166
<210> 167
   <211> 3362
   <212> DNA
   <213> Homo sapien
<400> 167
   cacaatgtat gcagcaggct cagtgtgagt gaactggagg cttctctaca acatgaccca 60
   aaggagcatt gcaggtccta tttgcaacct gaagtttgtg actctcctgg ttgccttaag 120
   ttcagaactc ccattcctgg gagctggagt acagcttcaa gacaatgggt ataatggatt 180
   gctcattgca attaatcctc aggtacctga gaatcagaac ctcatctcaa acattaagga 240
   aatgataact gaagcttcat tttacctatt taatgctacc aagagaagag tatttttcag 300
   aaatataaag attttaatac ctgccacatg gaaagctaat aataacagca aaataaaaca 360
   agaatcatat gaaaaggcaa atgtcatagt gactgactgg tatggggcac atggagatga 420
   tccatacacc ctacaataca gagggtgtgg aaaagaggga aaatacattc atttcacacc 480
   taatttccta ctgaatgata acttaacagc tggctacgga tcacgaggcc gagtgtttgt 540
   ccatgaatgg gcccacctcc gttggggtgt gttcgatgag tataacaatg acaaaccttt 600
   ctacataaat gggcaaaatc aaattaaagt gacaaggtgt tcatctgaca tcacaggcat 660
   ttttgtgtgt gaaaaaggtc cttgccccca agaaaactgt attattagta agctttttaa 720
   agaaggatgc acctttatct acaatagcac ccaaaatgca actgcatcaa taatgttcat 780
   gcaaagttta tcttctgtgg ttgaattttg taatgcaagt acccacaacc aagaagcacc 840
   aaacctacag aaccagatgt gcagcctcag aagtgcatgg gatgtaatca cagactctgc 900
   tgactttcac cacagctttc ccatgaacgg gactgagctt ccacctcctc ccacattctc 960
   gcttgtagag gctggtgaca aagtggtctg tttagtgctg gatgtgtcca gcaagatggc 1020
   agaggctgac agactccttc aactacaaca agccgcagaa ttttatttga tgcagattgt 1080
   tgaaattcat accttcgtgg gcattgccag tttcgacagc aaaggagaga tcagagccca 1140
   gctacaccaa attaacagca atgatgatcg aaagttgctg gtttcatatc tgcccaccac 1200
   tgtatcagct aaaacagaca tcagcatttg ttcagggctt aagaaaggat ttgaggtggt 1260
   tgaaaaactg aatggaaaag cttatggctc tgtgatgata ttagtgacca gcggagatga 1320
   taagcttctt ggcaattgct tacccactgt gctcagcagt ggttcaacaa ttcactccat 1380
   tgccctgggt tcatctgcag ccccaaatct ggaggaatta tcacgtctta caggaggttt 1440
   aaagttcttt gttccagata tatcaaactc caatagcatg attgatgctt tcagtagaat 1500
   ttcctctgga actggagaca ttttccagca acatattcag cttgaaagta caggtgaaaa 1560
   tgtcaaacct caccatcaat tgaaaaacac agtgactgtg gataatactg tgggcaacga 1620
   cactatgttt ctagttacgt ggcaggccag tggtcctcct gagattatat tatttgatcc 1680
   tgatggacga aaatactaca caaataattt tatcaccaat ctaacttttc ggacagctag 1740
   tctttggatt ccaggaacag ctaagcctgg gcactggact tacaccctga tgtgtttcca 1800
   ccatgcaaaa ttattgacct ggaagctgta aaagtagaag aggaattgac cctatcttgg 1860
   acagcacctg gagaagactt tgatcagggc caggctacaa gctatgaaat aagaatgagt 1920
   aaaagtctac agaatatcca agatgacttt aacaatgcta ttttagtaaa tacatcaaag 1980
   cgaaatcctc agcaagctgg catcagggag atatttacgt tctcacccca aatttccacg 2040
   aatggacctg aacatcagcc aaatggagaa acacatgaaa gccacagaat ttatgttgca 2100
   atacgagcaa tggataggaa ctccttacag tctgctgtat ctaacattgc ccaggcgcct 2160
   ctgtttattc cccccaattc tgatcctgta cctgccagag attatcttat attgaaagga 2220
   gttttaacag caatgggttt gataggaatc atttgcctta ttatagttgt gacacatcat 2280
   actttaagca ggaaaaagag agcagacaag aaagagaatg gaacaaaatt attataaata 2340
   aatatccaaa gtgtcttcct tcttagatat aagacccatg gccttcgact acaaaaacat 2400
   actaacaaag tcaaattaac atcaaaactg tattaaaatg cattgagttt ttgtacaata 2460
   cagataagat ttttacatgg tagatcaaca aattcttttt gggggtagat tagaaaaccc 2520
   ttacactttg gctatgaaca aataataaaa attattcttt aaagtaatgt ctttaaaggc 2580
   aaagggaagg gtaaagtcgg accagtgtca aggaaagttt gttttattga ggtggaaaaa 2640
   tagccccaag cagagaaaag gagggtaggt ctgcattata actgtctgtg tgaagcaatc 2700
   atttagttac tttgattaat ttttcttttc tccttatctg tgcagaacag gttgcttgtt 2760
   tacaactgaa gatcatgcta tatttcatat atgaagcccc taatgcaaag ctctttacct 2820
   cttgctattt tgttatatat attacagatg aaatctcact gctaatgctc agagatcttt 2880
   tttcactgta agaggtaacc tttaacaata tgggtattac ctttgtctct tcataccggt 2940
   tttatgacaa aggtctattg aatttatttg tttgtaagtt tctactccca tcaaagcagc 3000
   tttctaagtt attgccttgg ttattatgga tgatagttat agcccttata atgccttaac 3060
   taaggaagaa aagatgttat tctgagtttg ttttaataca tatatgaaca tatagtttta 3120
   ttcaattaaa ccaaagaaga ggtcagcagg gagatactaa cctttggaaa tgattagctg 3180
   gctctgtttt ttggttaaat aagagtcttt aatcctttct ccatcaagag ttacttacca 3240
   agggcagggg aagggggata tagaggtcac aaggaaataa aaatcatctt tcatctttaa 3300
   ttttactcct tcctcttatt tttttaaaag attatcgaac aataaaatca tttgcctttt 3360
   tt 3362
<210> 168
   <211> 2784
   <212> DNA
   <213> Homo sapien
<400> 168
   tctgcatcca tattgaaaac ctgacacaat gtatgcagca ggctcagtgt gagtgaactg     60
   gaggcttctc tacaacatga cccaaaggag cattgcaggt cctatttgca acctgaagtt    120
   tgtgactctc ctggttgcct taagttcaga actcccattc ctgggagctg gagtacagct    180
   tcaagacaat gggtataatg gattgctcat tgcaattaat cctcaggtac ctgagaatca    240
   gaacctcatc tcaaacatta aggaaatgat aactgaagct tcattttacc tatttaatgc    300
   taccaagaga agagtatttt tcagaaatat aaagatttta atacctgcca catggaaagc    360
   taataataac agcaaaataa aacaagaatc atatgaaaag gcaaatgtca tagtgactga    420
   ctggtatggg gcacatggag atgatccata caccctacaa tacagagggt gtggaaaaga    480
   gggaaaatac attcatttca cacctaattt cctactgaat gataacttaa cagctggcta    540
   cggatcacga ggccgagtgt ttgtccatga atgggcccac ctccgttggg gtgtgttcga    600
   tgagtataac aatgacaaac ctttctacat aaatgggcaa aatcaaatta aagtgacaag    660
   gtgttcatct gacatcacag gcatttttgt gtgtgaaaaa ggtccttgcc cccaagaaaa    720
   ctgtattatt agtaagcttt ttaaagaagg atgcaccttt atctacaata gcacccaaaa    780
   tgcaactgca tcaataatgt tcatgcaaag tttatcttct gtggttgaat tttgtaatgc    840
   aagtacccac aaccaagaag caccaaacct acagaaccag atgtgcagcc tcagaagtgc    900
   atgggatgta atcacagact ctgctgactt tcaccacagc tttcccatga acgggactga    960
   gcttccacct cctcccacat tctcgcttgt agaggctggt gacaaagtgg tctgtttagt   1020
   gctggatgtg tccagcaaga tggcagaggc tgacagactc cttcaactac aacaagccgc   1080
   agaattttat ttgatgcaga ttgttgaaat tcataccttc gtgggcattg ccagtttcga   1140
   cagcaaagga gagatcagag cccagctaca ccaaattaac agcaatgatg atcgaaagtt   1200
   gctggtttca tatctgccca ccactgtatc agctaaaaca gacatcagca tttgttcagg   1260
   gcttaagaaa ggatttgagg tggttgaaaa actgaatgga aaagcttatg gctctgtgat   1320
   gatattagtg accagcggag atgataagct tcttggcaat tgcttaccca ctgtgctcag   1380
   cagtggttca acaattcact ccattgccct gggttcatct gcagccccaa atctggagga   1440
   attatcacgt cttacaggag gtttaaagtt ctttgttcca gatatatcaa actccaatag   1500
   catgattgat gctttcagta gaatttcctc tggaactgga gacattttcc agcaacatat   1560
   tcagcttgaa agtacaggtg aaaatgtcaa acctcaccat caattgaaaa acacagtgac   1620
   tgtggataat actgtgggca acgacactat gtttctagtt acgtggcagg ccagtggtcc   1680
   tcctgagatt atattatttg atcctgatgg acgaaaatac tacacaaata attttatcac   1740
   caatctaact tttcggacag ctagtctttg gattccagga acagctaagc ctgggcactg   1800
   gacttacacc ctgaacaata cccatcattc tctgcaagcc ctgaaagtga cagtgacctc   1860
   tcgcgcctcc aactcagctg tgcccccagc cactgtggaa gcctttgtgg aaagagacag   1920
   cctccatttt cctcatcctg tgatgattta tgccaatgtg aaacagggat tttatcccat   1980
   tcttaatgcc actgtcactg ccacagttga gccagagact ggagatcctg ttacgctgag   2040
   actccttgat gatggagcag gtgctgatgt tataaaaaat gatggaattt actcgaggta   2100
   ttttttctcc tttgctgcaa atggtagata tagcttgaaa gtgcatgtca atcactctcc    2160
   cagcataagc accccagccc actctattcc agggagtcat gctatgtatg taccaggtta   2220
   cacagcaaac ggtaatattc agatgaatgc tccaaggaaa tcagtaggca gaaatgagga   2280
   ggagcgaaag tggggcttta gccgagtcag ctcaggaggc tccttttcag tgctgggagt   2340
   tccagctggc ccccaccctg atgtgtttcc accatgcaaa attattgacc tggaagctgt   2400
   aaatagaaga ggaattgacc ctatcttgga cagcacctgg agaagacttt gatcagggcc   2460
   aggctacaag ctatgaaata agaatgagta aaagtctaca gaatatccaa gatgacttta   2520
   acaatgctat tttagtaaat acatcaaagc gaaatcctca gcaagctggc atcagggaga   2580
   tatttacgtt ctcaccccaa atttccacga atggacctga acatcagcca aatggagaaa   2640
   cacatgaaag ccacagaatt tatgttgcaa tacgagcaat ggataggaac tccttacagt   2700
   ctgctgtatc taacattgcc caggcgcctc tgtttattcc ccccaattct gatcctgtac   2760
   ctgccagaga ttatcttata ttga                                            2784
<210> 169
   <211> 592
   <212> PRT
   <213> Homo sapien
<400> 169
   Met Thr Gln Arg Ser Ile Ala Gly Pro Ile Cys Asn Leu Lys Phe Val
   1               5                   10                  15
   Thr Leu Leu Val Ala Leu Ser Ser Glu Leu Pro Phe Leu Gly Ala Gly
               20                  25                  30
   Val Gln Leu Gln Asp Asn Gly Tyr Asn Gly Leu Leu Ile Ala Ile Asn
           35                  40                  45
   Pro Gln Val Pro Glu Asn Gln Asn Leu Ile Ser Asn Ile Lys Glu Met
       50                  55                  60
   Ile Thr Glu Ala Ser Phe Tyr Leu Phe Asn Ala Thr Lys Arg Arg Val
   65                  70                  75                  80
   Phe Phe Arg Asn Ile Lys Ile Leu Ile Pro Ala Thr Trp Lys Ala Asn
                   85                  90                  95
   Asn Asn Ser Lys Ile Lys Gln Glu Ser Tyr Glu Lys Ala Asn Val Ile
               100                 105                 110
   Val Thr Asp Trp Tyr Gly Ala His Gly Asp Asp Pro Tyr Thr Leu Gln
           115                 120                 125
   Tyr Arg Gly Cys Gly Lys Glu Gly Lys Tyr Ile His Phe Thr Pro Asn
       130                 135                 140
   Phe Leu Leu Asn Asp Asn Leu Thr Ala Gly Tyr Gly Ser Arg Gly Arg
   145                 150                 155                 160
   Val Phe Val His Glu Trp Ala His Leu Arg Trp Gly Val Phe Asp Glu
                   165                 170                 175
   Tyr Asn Asn Asp Lys Pro Phe Tyr Ile Asn Gly Gln Asn Gln Ile Lys
               180                 185                 190
   Val Thr Arg Cys Ser Ser Asp Ile Thr Gly Ile Phe Val Cys Glu Lys
           195                 200                 205
   Gly Pro Cys Pro Gln Glu Asn Cys Ile Ile Ser Lys Leu Phe Lys Glu
       210                 215                 220
   Gly Cys Thr Phe Ile Tyr Asn Ser Thr Gln Asn Ala Thr Ala Ser Ile
   225                 230                 235                 240
   Met Phe Met Gln Ser Leu Ser Ser Val Val Glu Phe Cys Asn Ala Ser
                   245                 250                 255
   Thr His Asn Gln Glu Ala Pro Asn Leu Gln Asn Gln Met Cys Ser Leu
               260                 265                 270
   Arg Ser Ala Trp Asp Val Ile Thr Asp Ser Ala Asp Phe His His Ser
           275                 280                 285
   Phe Pro Met Asn Gly Thr Glu Leu Pro Pro Pro Pro Thr Phe Ser Leu
       290                 295                 300
   Val Glu Ala Gly Asp Lys Val Val Cys Leu Val Leu Asp Val Ser Ser
   305                 310                 315                 320
   Lys Met Ala Glu Ala Asp Arg Leu Leu Gln Leu Gln Gln Ala Ala Glu
                   325                 330                 335
   Phe Tyr Leu Met Gln Ile Val Glu Ile His Thr Phe Val Gly Ile Ala
               340                 345                 350
   Ser Phe Asp Ser Lys Gly Glu Ile Arg Ala Gln Leu His Gln Ile Asn
           355                 360                 365
   Ser Asn Asp Asp Arg Lys Leu Leu Val Ser Tyr Leu Pro Thr Thr Val
           370                 375                 380
   Ser Ala Lys Thr Asp Ile Ser Ile Cys Ser Gly Leu Lys Lys Gly Phe
   385                 390                 395                 400
   Glu Val Val Glu Lys Leu Asn Gly Lys Ala Tyr Gly Ser Val Met Ile
                   405                 410                 415
   Leu Val Thr Ser Gly Asp Asp Lys Leu Leu Gly Asn Cys Leu Pro Thr
               420                 425                 430
   Val Leu Ser Ser Gly Ser Thr Ile His Ser Ile Ala Leu Gly Ser Ser
           435                 440                 445
   Ala Ala Pro Asn Leu Glu Glu Leu Ser Arg Leu Thr Gly Gly Leu Lys
       450                 455                 460
   Phe Phe Val Pro Asp Ile Ser Asn Ser Asn Ser Met Ile Asp Ala Phe
   465                 470                 475                 480
   Ser Arg Ile Ser Ser Gly Thr Gly Asp Ile Phe Gln Gln His Ile Gln
                   485                 490                 495
   Leu Glu Ser Thr Gly Glu Asn Val Lys Pro His His Gln Leu Lys Asn
               500                 505                 510
   Thr Val Thr Val Asp Asn Thr Val Gly Asn Asp Thr Met Phe Leu Val
           515                 520                 525
   Thr Trp Gln Ala Ser Gly Pro Pro Glu Ile Ile Leu Phe Asp Pro Asp
       530                 535                 540
   Gly Arg Lys Tyr Tyr Thr Asn Asn Phe Ile Thr Asn Leu Thr Phe Arg
   545                 550                 555                 560
   Thr Ala Ser Leu Trp Ile Pro Gly Thr Ala Lys Pro Gly His Trp Thr
                   565                 570                 575
   Tyr Thr Leu Met Cys Phe His His Ala Lys Leu Leu Thr Trp Lys Leu
               580                 585                 590
<210> 170
   <211> 791
   <212> PRT
   <213> Homo sapien
<400> 170
   Met Thr Gln Arg Ser Ile Ala Gly Pro Ile Cys Asn Leu Lys Phe Val
    1               5                  10                  15
   Thr Leu Leu Val Ala Leu Ser Ser Glu Leu Pro Phe Leu Gly Ala Gly
               20                  25                  30
   Val Gln Leu Gln Asp Asn Gly Tyr Asn Gly Leu Leu Ile Ala Ile Asn
           35                  40                  45
   Pro Gln Val Pro Glu Asn Gln Asn Leu Ile Ser Asn Ile Lys Glu Met
       50                  55                  60
   Ile Thr Glu Ala Ser Phe Tyr Leu Phe Asn Ala Thr Lys Arg Arg Val
   65                  70                  75                  80
   Phe Phe Arg Asn Ile Lys Ile Leu Ile Pro Ala Thr Trp Lys Ala Asn
                   85                  90                  95
   Asn Asn Ser Lys Ile Lys Gln Glu Ser Tyr Glu Lys Ala Asn Val Ile
               100                 105                 110
   Val Thr Asp Trp Tyr Gly Ala His Gly Asp Asp Pro Tyr Thr Leu Gln
           115                 120                 125
   Tyr Arg Gly Cys Gly Lys Glu Gly Lys Tyr Ile His Phe Thr Pro Asn
       130                 135                 140
   Phe Leu Leu Asn Asp Asn Leu Thr Ala Gly Tyr Gly Ser Arg Gly Arg
   145                 150                 155                 160
   Val Phe Val His Glu Trp Ala His Leu Arg Trp Gly Val Phe Asp Glu
                   165                 170                 175
   Tyr Asn Asn Asp Lys Pro Phe Tyr Ile Asn Gly Gln Asn Gln Ile Lys
               180                 185                 190
   Val Thr Arg Cys Ser Ser Asp Ile Thr Gly Ile Phe Val Cys Glu Lys
           195                 200                 205
   Gly Pro Cys Pro Gln Glu Asn Cys Ile Ile Ser Lys Leu Phe Lys Glu
       210                 215                 220
   Gly Cys Thr Phe Ile Tyr Asn Ser Thr Gln Asn Ala Thr Ala Ser Ile
   225                 230                 235                 240
   Met Phe Met Gln Ser Leu Ser Ser Val Val Glu Phe Cys Asn Ala Ser
                   245                 250                 255
   Thr His Asn Gln Glu Ala Pro Asn Leu Gln Asn Gln Met Cys Ser Leu
               260                 265                 270
   Arg Ser Ala Trp Asp Val Ile Thr Asp Ser Ala Asp Phe His His Ser
           275                 280                 285
   Phe Pro Met Asn Gly Thr Glu Leu Pro Pro Pro Pro Thr Phe Ser Leu
       290                 295                 300
   Val Glu Ala Gly Asp Lys Val Val Cys Leu Val Leu Asp Val Ser Ser
   305                 310                 315                 320
   Lys Met Ala Glu Ala Asp Arg Leu Leu Gln Leu Gln Gln Ala Ala Glu
                   325                 330                 335
   Phe Tyr Leu Met Gln Ile Val Glu Ile His Thr Phe Val Gly Ile Ala
               340                 345                 350
   Ser Phe Asp Ser Lys Gly Glu Ile Arg Ala Gln Leu His Gln Ile Asn
           355                 360                 365
   Ser Asn Asp Asp Arg Lys Leu Leu Val Ser Tyr Leu Pro Thr Thr Val
       370                 375                 380
   Ser Ala Lys Thr Asp Ile Ser Ile Cys Ser Gly Leu Lys Lys Gly Phe
   385                 390                 395                 400
   Glu Val Val Glu Lys Leu Asn Gly Lys Ala Tyr Gly Ser Val Met Ile
                   405                 410                 415
   Leu Val Thr Ser Gly Asp Asp Lys Leu Leu Gly Asn Cys Leu Pro Thr
               420                 425                 430
   Val Leu Ser Ser Gly Ser Thr Ile His Ser Ile Ala Leu Gly Ser Ser
           435                 440                 445
   Ala Ala Pro Asn Leu Glu Glu Leu Ser Arg Leu Thr Gly Gly Leu Lys
       450                 455                 460
   Phe Phe Val Pro Asp Ile Ser Asn Ser Asn Ser Met Ile Asp Ala Phe
   465                 470                 475                 480
   Ser Arg Ile Ser Ser Gly Thr Gly Asp Ile Phe Gln Gln His Ile Gln
                   485                 490                 495
   Leu Glu Ser Thr Gly Glu Asn Val Lys Pro His His Gln Leu Lys Asn
               500                 505                 510
   Thr Val Thr Val Asp Asn Thr Val Gly Asn Asp Thr Met Phe Leu Val
           515                 520                 525
   Thr Trp Gln Ala Ser Gly Pro Pro Glu Ile Ile Leu Phe Asp Pro Asp
       530                 535                 540
   Gly Arg Lys Tyr Tyr Thr Asn Asn Phe Ile Thr Asn Leu Thr Phe Arg
   545                 550                 555                 560
   Thr Ala Ser Leu Trp Ile Pro Gly Thr Ala Lys Pro Gly His Trp Thr
                   565                 570                 575
   Tyr Thr Leu Asn Asn Thr His His Ser Leu Gln Ala Leu Lys Val Thr
               580                 585                 590
   Val Thr Ser Arg Ala Ser Asn Ser Ala Val Pro Pro Ala Thr Val Glu
           595                 600                 605
   Ala Phe Val Glu Arg Asp Ser Leu His Phe Pro His Pro Val Met Ile
       610                 615                 620
   Tyr Ala Asn Val Lys Gln Gly Phe Tyr Pro Ile Leu Asn Ala Thr Val
   625                 630                 635                 640
   Thr Ala Thr Val Glu Pro Glu Thr Gly Asp Pro Val Thr Leu Arg Leu
                   645                 650                 655
   Leu Asp Asp Gly Ala Gly Ala Asp Val Ile Lys Asn Asp Gly Ile Tyr
               660                 665                 670
   Ser Arg Tyr Phe Phe Ser Phe Ala Ala Asn Gly Arg Tyr Ser Leu Lys
           675                 680                 685
   Val His Val Asn His Ser Pro Ser Ile Ser Thr Pro Ala His Ser Ile
       690                 695                 700
   Pro Gly Ser His Ala Met Tyr Val Pro Gly Tyr Thr Ala Asn Gly Asn
   705                 710                 715                 720
   Ile Gln Met Asn Ala Pro Arg Lys Ser Val Gly Arg Asn Glu Glu Glu
                   725                 730                 735
   Arg Lys Trp Gly Phe Ser Arg Val Ser Ser Gly Gly Ser Phe Ser Val
               740                 745                 750
   Leu Gly Val Pro Ala Gly Pro His Pro Asp Val Phe Pro Pro Cys Lys
           755                 760                 765
   Ile Ile Asp Leu Glu Ala Val Asn Arg Arg Gly Ile Asp Pro Ile Leu
       770                 775                 780
   Asp Ser Thr Trp Arg Arg Leu
   785                 790
<210> 171
   <211> 1491
   <212> DNA
   <213> Homo sapien
<400> 171
   cctcctgcca gccaagtgaa gacatgctta cttccccttc accttccttc atgatgtggg      60
   aagagtgctg caacccagcc ctagccaacg ccgcatgaga gggagtgtgc cgagggcttc     120
   tgagaaggtt tctctcacat ctagaaagaa gcgcttaaga tgtggcagcc cctcttcttc     180
   aagtggctct tgtcctgttg ccctgggagt tctcaaattg ctgcagcagc ctccacccag     240
   cctgaggatg acatcaatac acagaggaag aagagtcagg aaaagatgag agaagttaca     300
   gactctcctg ggcgaccccg agagcttacc attcctcaga cttcttcaca tggtgctaac     360
   agatttgttc ctaaaagtaa agctctagag gccgtcaaat tggcaataga agccgggttc     420
   caccatattg attctgcaca tgtttacaat aatgaggagc aggttggact ggccatccga     480
   agcaagattg cagatggcag tgtgaagaga gaagacatat tctacacttc aaagctttgg     540
   agcaattccc atcgaccaga gttggtccga ccagccttgg aaaggtcact gaaaaatctt     600
   caattggact atgttgacct ctatcttatt cattttccag tgtctgtaaa gccaggtgag     660
   gaagtgatcc caaaagatga aaatggaaaa atactatttg acacagtgga tctctgtgcc     720
   acatgggagg ccatggagaa gtgtaaagat gcaggattgg ccaagtccat cggggtgtcc     780
   aacttcaacc acaggctgct ggagatgatc ctcaacaagc cagggctcaa gtacaagcct     840
   gtctgcaacc aggtggaatg tcatccttac ttcaaccaga gaaaactgct ggatttctgc     900
   aagtcaaaag acattgttct ggttgcctat agtgctctgg gatcccatcg agaagaacca     960
   tgggtggacc cgaactcccc ggtgctcttg gaggacccag tcctttgtgc cttggcaaaa    1020
   aagcacaagc gaaccccagc cctgattgcc ctgcgctacc agctgcagcg tggggttgtg    1080
   gtcctggcca agagctacaa tgagcagcgc atcagacaga acgtgcaggt gtttgaattc    1140
   cagttgactt cagaggagat gaaagccata gatggcctaa acagaaatgt gcgatatttg    1200
   acccttgata tttttgctgg cccccctaat tatccatttt ctgatgaata ttaacatgga    1260
   gggcattgca tgaggtctgc cagaaggccc tgcgtgtgga tggtgacaca gaggatggct    1320
   ctatgctggt gactggacac atcgcctctg gttaaatctc tcctgcttgg cgacttcagt    1380
   aagctacagc taagcccatc ggccggaaaa gaaagacaat aattttgttt ttcattttga    1440
   aaaaattaaa tgctctctcc taaagattct tcacctaaaa aaaaaaaaaa a              1491
<210> 172
   <211> 364
   <212> PRT
   <213> Homo sapien
<400> 172
   Met Trp Gln Pro Leu Phe Phe Lys Trp Leu Leu Ser Cys Cys Pro Gly
    1               5                  10                  15
   Ser Ser Gln Ile Ala Ala Ala Ala Ser Thr Gln Pro Glu Asp Asp Ile
               20                  25                  30
   Asn Thr Gln Arg Lys Lys Ser Gln Glu Lys Met Arg Glu Val Thr Asp
           35                  40                  45
   Ser Pro Gly Arg Pro Arg Glu Leu Thr Ile Pro Gln Thr Ser Ser His
       50                  55                  60
   Gly Ala Asn Arg Phe Val Pro Lys Ser Lys Ala Leu Glu Ala Val Lys
   65                  70                  75                  80
   Leu Ala Ile Glu Ala Gly Phe His His Ile Asp Ser Ala His Val Tyr
                   85                  90                  95
   Asn Asn Glu Glu Gln Val Gly Leu Ala Ile Arg Ser Lys Ile Ala Asp
               100                 105                 110
   Gly Ser Val Lys Arg Glu Asp Ile Phe Tyr Thr Ser Lys Leu Trp Ser
           115                 120                 125
   Asn Ser His Arg Pro Glu Leu Val Arg Pro Ala Leu Glu Arg Ser Leu
       130                 135                 140
   Lys Asn Leu Gln Leu Asp Tyr Val Asp Leu Tyr Leu Ile His Phe Pro
   145                 150                 155                 160
   Val Ser Val Lys Pro Gly Glu Glu Val Ile Pro Lys Asp Glu Asn Gly
                   165                 170                 175
   Lys Ile Leu Phe Asp Thr Val Asp Leu Cys Ala Thr Trp Glu Ala Met
               180                 185                 190
   Glu Lys Cys Lys Asp Ala Gly Leu Ala Lys Ser Ile Gly Val Ser Asn
           195                 200                 205
   Phe Asn His Arg Leu Leu Glu Met Ile Leu Asn Lys Pro Gly Leu Lys
       210                 215                 220
   Tyr Lys Pro Val Cys Asn Gln Val Glu Cys His Pro Tyr Phe Asn Gln
   225                 230                 235                 240
   Arg Lys Leu Leu Asp Phe Cys Lys Ser Lys Asp Ile Val Leu Val Ala
                   245                 250                 255
   Tyr Ser Ala Leu Gly Ser His Arg Glu Glu Pro Trp Val Asp Pro Asn
               260                 265                 270
   Ser Pro Val Leu Leu Glu Asp Pro Val Leu Cys Ala Leu Ala Lys Lys
           275                 280                 285
   His Lys Arg Thr Pro Ala Leu Ile Ala Leu Arg Tyr Gln Leu Gln Arg
       290                 295                 300
   Gly Val Val Val Leu Ala Lys Ser Tyr Asn Glu Gln Arg Ile Arg Gln
   305                 310                 315                 320
   Asn Val Gln Val Phe Glu Phe Gln Leu Thr Ser Glu Glu Met Lys Ala
                   325                 330                 335
   Ile Asp Gly Leu Asn Arg Asn Val Arg Tyr Leu Thr Leu Asp Ile Phe
               340                 345                 350
   Ala Gly Pro Pro Asn Tyr Pro Phe Ser Asp Glu Tyr
           355                 360
<210> 173
   <211> 1988
   <212> DNA
   <213> Homo sapiens
<400> 173
   cgggagccgc ctccccgcgg cctcttcgct tttgtggcgg cgcccgcgct cgcaggccac 60
   tctctgctgt cgcccgtccc gcgcgctcct ccgacccgct ccgctccgct ccgctcggcc 120
   ccgcgccgcc cgtcaacatg atccgctgcg gcctggcctg cgagcgctgc cgctggatcc 180
   tgcccctgct cctactcagc gccatcgcct tcgacatcat cgcgctggcc ggccgcggct 240
   ggttgcagtc tagcgaccac ggccagacgt cctcgctgtg gtggaaatgc tcccaagagg 300
   gcggcggcag cgggtcctac gaggagggct gtcagagcct catggagtac gcgtggggta 360
   gagcagcggc tgccatgctc ttctgtggct tcatcatcct ggtgatctgt ttcatcctct 420
   ccttcttcgc cctctgtgga ccccagatgc ttgtcttcct gagagtgatt ggaggtctcc 480
   ttgccttggc tgctgtgttc cagatcatct ccctggtaat ttaccccgtg aagtacaccc 540
   agaccttcac ccttcatgcc aaccctgctg tcacttacat ctataactgg gcctacggct 600
   ttgggtgggc agccacgatt atcctgatcg gctgtgcctt cttcttctgc tgcctcccca 660
   actacgaaga tgaccttctg ggcaatgcca agcccaggta cttctacaca tctgcctaac 720
   ttgggaatga atgtgggaga aaatcgctgc tgctgagatg gactccagaa gaagaaactg 780
   tttctccagg cgactttgaa cccatttttt ggcagtgttc atattattaa actagtcaaa 840
   aatgctaaaa taatttggga gaaaatattt tttaagtagt gttatagttt catgtttatc 900
   ttttattatg ttttgtgaag ttgtgtcttt tcactaatta cctatactat gccaatattt 960
   ccttatatct atccataaca tttatactac atttgtaaga gaatatgcac gtgaaactta 1020
   acactttata aggtaaaaat gaggtttcca agatttaata atctgatcaa gttcttgtta 1080
   tttccaaata gaatggactt ggtctgttaa gggctaagga gaagaggaag ataaggttaa 1140
   aagttgttaa tgaccaaaca ttctaaaaga aatgcaaaaa aaaagtttat tttcaagcct 1200
   tcgaactatt taaggaaagc aaaatcattt cctaaatgca tatcatttgt gagaatttct 1260
   cattaatatc ctgaatcatt catttcagct aaggcttcat gttgactcga tatgtcatct 1320
   aggaaagtac tatttcatgg tccaaacctg ttgccatagt tggtaaggct ttcctttaag 1380
   tgtgaaatat ttagatgaaa ttttctcttt taaagttctt tatagggtta gggtgtggga 1440
   aaatgctata ttaataaatc tgtagtgttt tgtgtttata tgttcagaac cagagtagac 1500
   tggattgaaa gatggactgg gtctaattta tcatgactga tagatctggt taagttgtgt 1560
   agtaaagcat taggagggtc attcytgtca caaaagtgcc actaaaacag cctcaggaga 1620
   ataaatgact tgcttttcta aatctcaggt ttatctgggc tctatcatat agacaggctt 1680
   ctgatagttt gcarctgtaa gcagaaacct acatatagtt aaaatcctgg tctttcttgg 1740
   taaacagatt ttaaatgtct gatataaaac atgccacagg agaattcggg gatttgagtt 1800
   tctctgaata gcatatatat gatgcatcgg ataggtcatt atgatttttt accatttcga 1860
   cttacataat gaaaaccaat tcattttaaa tatcagatta ttattttgta agttgtggaa 1920
   aaagctaatt gtagttttca ttatgaagtt ttcccaataa accaggtatt ctaaaaaaaa 1980
   aaaaaaaa                                                          1988
<210> 174
   <211> 238
   <212> PRT
   <213> Homo sapiens
<400> 174
   Gly Ala Ala Ser Pro Arg Pro Leu Arg Phe Cys Gly Gly Ala Arg Ala
                   5                   10                  15
   Arg Arg Pro Leu Ser Ala Val Ala Arg Pro Ala Arg Ser Ser Asp Pro
               20                  25                  30
   Leu Arg Ser Ala Pro Leu Gly Pro Ala Pro Pro Val Asn Met Ile Arg
               35                  40                  45
   Cys Gly Leu Ala Cys Glu Arg Cys Arg Trp Ile Leu Pro Leu Leu Leu
       50                  55                  60
   Leu Ser Ala Ile Ala Phe Asp Ile Ile Ala Leu Ala Gly Arg Gly Trp
   65                  70                  75                  80
   Leu Gln Ser Ser Asp His Gly Gln Thr Ser Ser Leu Trp Trp Lys Cys
                   85                  90                  95
   Ser Gln Glu Gly Gly Gly Ser Gly Ser Tyr Glu Glu Gly Cys Gln Ser
               100                 105                 110
   Leu Met Glu Tyr Ala Trp Gly Arg Ala Ala Ala Ala Met Leu Phe Cys
           115                 120                 125
   Gly Phe Ile Ile Leu Val Ile Cys Phe Ile Leu Ser Phe Phe Ala Leu
       130                 135                 140
   Cys Gly Pro Gln Met Leu Val Phe Leu Arg Val Ile Gly Gly Leu Leu
   145                 150                 155                 160
   Ala Leu Ala Ala Val Phe Gln Ile Ile Ser Leu Val Ile Tyr Pro Val
                   165                 170                 175
   Lys Tyr Thr Gln Thr Phe Thr Leu His Ala Asn Pro Ala Val Thr Tyr
               180                 185                 190
   Ile Tyr Asn Trp Ala Tyr Gly Phe Gly Trp Ala Ala Thr Ile Ile Leu
           195                 200                 205
   Ile Gly Cys Ala Phe Phe Phe Cys Cys Leu Pro Asn Tyr Glu Asp Asp
       210                 215                 220
   Leu Leu Gly Asn Ala Lys Pro Arg Tyr Phe Tyr Thr Ser Ala
   225                 230                 235
<210> 175
   <211> 4181
   <212> DNA
   <213> Homo sapiens
<220>
   <221> unsure
   <222> (3347)
   <223> n=A,T,C or G
   <221> unsure
   <222> (3502)
   <223> n=A,T,C or G
   <221> unsure
   <222> (3506)
   <223> n=A,T,C or G
   <221> unsure
   <222> (3520)
   <223> n=A,T,C or G
   <221> unsure
   <222> (3538)
   <223> n=A,T,C or G
   <221> unsure
   <222> (3549)
   <223> n=A,T,C or G
   <221> unsure
   <222> (3646)
   <223> n=A,T,C or G
   <221> unsure
   <222> (3940)
   <223> n=A,T,C or G
   <221> unsure
   <222> (3968)
   <223> n=A,T,C or G
   <221> unsure
   <222> (3974)
   <223> n=A,T,C or G
   <221> unsure
   <222> (4036)
   <223> n=A,T,C or G
   <221> unsure
   <222> (4056)
   <223> n=A,T,C or G
   <221> unsure
   <222> (4062)
   <223> n=A,T,C or G
   <221> unsure
   <222> (4080)
   <223> n=A,T,C or G
   <221> unsure
   <222> (4088)
   <223> n=A,T,C or G
   <221> unsure
   <222> (4115)
   <223> n=A,T,C or G
<400> 175
   ggtggatgcg tttgggttgt agctaggctt tttcttttct ttctctttta aaacacatct 60
   agacaaggaa aaaacaagcc tcggatctga tttttcactc ctcgttcttg tgcttggttc 120
   ttactgtgtt tgtgtatttt aaaggcgaga agacgagggg aacaaaacca gctggatcca 180
   tccatcaccg tgggtggttt taatttttcg ttttttctcg ttattttttt ttaaacaacc 240
   actcttcaca atgaacaaac tgtatatcgg aaacctcagc gagaacgccg ccccctcgga 300
   cctagaaagt atcttcaagg acgccaagat cccggtgtcg ggacccttcc tggtgaagac 360
   tggctacgcg ttcgtggact gcccggacga gagctgggcc ctcaaggcca tcgaggcgct 420
   ttcaggtaaa atagaactgc acgggaaacc catagaagtt gagcactcgg tcccaaaaag 480
   gcaaaggatt cggaaacttc agatacgaaa tatcccgcct catttacagt gggaggtgct 540
   ggatagttta ctagtccagt atggagtggt ggagagctgt gagcaagtga acactgactc 600
   ggaaactgca gttgtaaatg taacctattc cagtaaggac caagctagac aagcactaga 660
   caaactgaat ggatttcagt tagagaattt caccttgaaa gtagcctata tccctgatga 720
   aatggccgcc cagcaaaacc ccttgcagca gccccgaggt cgccgggggc ttgggcagag 780
   gggctcctca aggcaggggt ctccaggatc cgtatccaag cagaaaccat gtgatttgcc 840
   tctgcgcctg ctggttccca cccaatttgt tggagccatc ataggaaaag aaggtgccac 900
   cattcggaac atcaccaaac agacccagtc taaaatcgat gtccaccgta aagaaaatgc 960
   gggggctgct gagaagtcga ttactatcct ctctactcct gaaggcacct ctgcggcttg 1020
   taagtctatt ctggagatta tgcataagga agctcaagat ataaaattca cagaagagat 1080
   ccccttgaag attttagctc ataataactt tgttggacgt cttattggta aagaaggaag 1140
   aaatcttaaa aaaattgagc aagacacaga cactaaaatc acgatatctc cattgcagga 1200
   attgacgctg tataatccag aacgcactat tacagttaaa ggcaatgttg agacatgtgc 1260
   caaagctgag gaggagatca tgaagaaaat cagggagtct tatgaaaatg atattgcttc 1320
   tatgaatctt caagcacatt taattcctgg attaaatctg aacgccttgg gtctgttccc 1380
   acccacttca gggatgccac ctcccacctc agggccccct tcagccatga ctcctcccta 1440
   cccgcagttt gagcaatcag aaacggagac tgttcatcag tttatcccag ctctatcagt 1500
   cggtgccatc atcggcaagc agggccagca catcaagcag ctttctcgct ttgctggagc 1560
   ttcaattaag attgctccag cggaagcacc agatgctaaa gtgaggatgg tgattatcac 1620
   tggaccacca gaggctcagt tcaaggctca gggaagaatt tatggaaaaa ttaaagaaga 1680
   aaactttgtt agtcctaaag aagaggtgaa acttgaagct catatcagag tgccatcctt 1740
   tgctgctggc agagttattg gaaaaggagg caaaacggtg aatgaacttc agaatttgtc 1800
   aagtgcagaa gttgttgtcc ctcgtgacca gacacctgat gagaatgacc aagtggttgt 1860
   caaaataact ggtcacttct atgcttgcca ggttgcccag agaaaaattc aggaaattct 1920
   gactcaggta aagcagcacc aacaacagaa ggctctgcaa agtggaccac ctcagtcaag 1980
   acggaagtaa aggctcagga aacagcccac cacagaggca gatgccaaac caaagacaga 2040
   ttgcttaacc aacagatggg cgctgacccc ctatccagaa tcacatgcac aagtttttac 2100
   ctagccagtt gtttctgagg accaggcaac ttttgaactc ctgtctctgt gagaatgtat 2160
   actttatgct ctctgaaatg tatgacaccc agctttaaaa caaacaaaca aacaaacaaa 2220
   aaaagggtgg gggagggagg gaaagagaag agctctgcac ttccctttgt tgtagtctca 2280
   cagtataaca gatattctaa ttcttcttaa tattccccca taatgccaga aattggctta 2340
   atgatgcttt cactaaattc atcaaataga ttgctcctaa atccaattgt taaaattgga 2400
   tcagaataat tatcacagga acttaaatgt taagccatta gcatagaaaa actgttctca 2460
   gttttatttt tacctaacac taacatgagt aacctaaggg aagtgctgaa tggtgttggc 2520
   aggggtatta aacgtgcatt tttactcaac tacctcaggt attcagtaat acaatgaaaa 2580
   gcaaaattgt tccttttttt tgaaaatttt atatacttta taatgataga agtccaaccg 2640
   tttttaaaaa aataaattta aaatttaaca gcaatcagct aacaggcaaa ttaagatttt 2700
   tacttctggc tggtgacagt aaagctggaa aattaatttc agggtttttt gaggcttttg 2760
   acacagttat tagttaaatc aaatgttcaa aaatacggag cagtgcctag tatctggaga 2820
   gcagcactac catttattct ttcatttata gttgggaaag tttttgacgg tactaacaaa 2880
   gtggtcgcag gagattttgg aacggctggt ttaaatggct tcaggagact tcagtttttt 2940
   gtttagctac atgattgaat gcataataaa tgctttgtgc ttctgactat caatacctaa 3000
   agaaagtgca tcagtgaaga gatgcaagac tttcaactga ctggcaaaaa gcaagcttta 3060
   gcttgtctta taggatgctt agtttgccac tacacttcag accaatggga cagtcataga 3120
   tggtgtgaca gtgtttaaac gcaacaaaag gctacatttc catggggcca gcactgtcat 3180
   gagcctcact aagctatttt gaagattttt aagcactgat aaattaaaaa aaaaaaaaaa 3240
   aaattagact ccaccttaag tagtaaagta taacaggatt tctgtatact gtgcaatcag 3300
   ttctttgaaa aaaaagtcaa aagatagaga atacaagaaa agttttnggg atataatttg 3360
   aatgactgtg aaaacatatg acctttgata acgaactcat ttgctcactc cttgacagca 3420
   aagcccagta cgtacaattg tgttgggtgt gggtggtctc caaggccacg ctgctctctg 3480
   aattgatttt ttgagttttg gnttgnaaga tgatcacagn catgttacac tgatcttnaa 3540
   ggacatatnt tataaccctt taaaaaaaaa atcccctgcc tcattcttat ttcgagatga 3600
   atttcgatac agactagatg tctttctgaa gatcaattag acattntgaa aatgatttaa 3660
   agtgttttcc ttaatgttct ctgaaaacaa gtttcttttg tagttttaac caaaaaagtg 3720
   ccctttttgt cactggtttc tcctagcatt catgattttt ttttcacaca atgaattaaa 3780
   attgctaaaa tcatggactg gctttctggt tggatttcag gtaagatgtg tttaaggcca 3840
   gagcttttct cagtatttga tttttttccc caatatttga tttttaaaaa atatacacat 3900
   aggagctgca tttaaaacct gctggtttaa attctgtcan atttcacttc tagcctttta 3960
   gtatggcnaa tcanaattta cttttactta agcatttgta atttggagta tctggtacta 4020
   gctaagaaat aattcnataa ttgagttttg tactcnccaa anatgggtca ttcctcatgn 4080
   ataatgtncc cccaatgcag cttcattttc caganacctt gacgcaggat aaattttttc 4140
   atcatttagg tccccaaaaa aaaaaaaaaa aaaaaaaaaa a 4181
<210> 176
   <211> 580
   <212> PRT
   <213> Homo sapiens
<400> 176
<210> 177
   <211> 401
   <212> DNA
   <213> Homo sapiens
<400> 177
   atgccccgta aatgtcttca gtgttcttca gggtagttgg gatctcaaaa gatttggttc 60
   agatccaaac aaatacacat tctgtgtttt agctcagtgt tttctaaaaa aagaaactgc 120
   cacacagcaa aaaattgttt actttgttgg acaaaccaaa tcagttctca aaaaatgacc 180
   ggtgcttata aaaagttata aatatcgagt agctctaaaa caaaccacct gaccaagagg 240
   gaagtgagct tgtgcttagt atttacattg gatgccagtt ttgtaatcac tgacttatgt 300
   gcaaactggt gcagaaattc tataaactct ttgctgtttt tgatacctgc tttttgtttc 360
   attttgtttt gttttgtaaa aatgataaaa cttcagaaaa t 401
<210> 178
   <211> 561
   <212> DNA
   <213> Homo sapiens
<400> 178
   acgcctttca agggtgtacg caaagcactc attgataccc ttttggatgg ctatgaaaca 60
   gcccgctatg ggacaggggt ctttggccag aatgagtacc tacgctatca ggaggccctg 120
   agtgagctgg ccactgcggt taaagcacga attgggagct ctcagcgaca tcaccagtca 180
   gcagccaaag acctaactca gtcccctgag gtctccccaa caaccatcca ggtgacatac 240
   ctcccctcca gtcagaagag taaacgtgcc aagcacttcc ttgaattgaa gagctttaag 300
   gataactata acacattgga gagtactctg tgacggagct gaaggactct tgccgtagat 360
   taagccagtc agttgcaatg tgcaagacag gctgcttgcc gggccgccct cggaacatct 420
   ggcccagcag gcccagactg tatccatcca agttcccgtt gtatccagag ttcttagagc 480
   ttgtgtctaa agggtaattc cccaaccctt ccttatgagc atttttagaa cattggctaa 540
   gactattttc ccccagtagc g 561
<210> 179
   <211> 521
   <212> DNA
   <213> Homo sapiens
<400> 179
   cccaacgcgt ttgcaaatat tcccctggta gcctacttcc ttacccccga atattggtaa 60
   gatcgagcaa tggcttcagg acatgggttc tcttctcctg tgatcattca agtgctcact 120
   gcatgaagac tggcttgtct cagtgtttca acctcaccag ggctgtctct tggtccacac 180
   ctcgctccct gttagtgccg tatgacagcc cccatcaaat gaccttggcc aagtcacggt 240
   ttctctgtgg tcaaggttgg ttggctgatt ggtggaaagt agggtggacc aaaggaggcc 300
   acgtgagcag tcagcaccag ttctgcacca gcagcgcctc cgtcctagtg ggtgttcctg 360
   tttctcctgg ccctgggtgg gctagggcct gattcgggaa gatgcctttg cagggagggg 420
   aggataagtg ggatctacca attgattctg gcaaaacaat ttctaagatt tttttgcttt 480
   atgtgggaaa cagatctaaa tctcatttta tgctgtattt t                    521
<210> 180
   <211> 417
   <212> DNA
   <213> Homo sapiens
<400> 180
   ggtggaattc gccgaagatg gcggaggtgc aggtcctggt gcttgatggt cgaggccatc 60
   tcctgggccg cctggcggcc atcgtggcta aacaggtact gctgggccgg aaggtggtgg 120
   tcgtacgctg tgaaggcatc aacatttctg gcaatttcta cagaaacaag ttgaagtacc 180
   tggctttcct ccgcaagcgg atgaacacca acccttcccg aggcccctac cacttccggg 240
   cccccagccg catcttctgg cggaccgtgc gaggtatgct gccccacaaa accaagcgag 300
   gccaggccgc tctggaccgt ctcaaggtgt ttgacggcat cccaccgccc tacgacaaga 360
   aaaagcggat ggtggttcct gctgccctca aggtcgtgcg tctgaagcct acaagaa    417
<210> 181
   <211> 283
   <212> DNA
   <213> Homo sapiens
<220>
   <221> unsure
   <222> (35)
   <223> n=A,T,C or G
<400> 181
   gatttcttct aaataggatg taaaacttct ttcanattac tcttcctcag tcctgcctgc 60
   caagaactca agtgtaactg tgataaaata acctttccca ggtatattgg caggtatgtg 120
   tgtaatctca gaatacacag gtgacataga tatgatatga caactggtaa tggtggattc 180
   atttacattg tttacacttc tatgaccagg ccttaaggga aggtcagttt tttaaaaaac 240
   caagtagtgt cttcctacct atctccagat acatgtcaaa aaa                  283
<210> 182
   <211> 401
   <212> DNA
   <213> Homo sapiens
<400> 182
   atattcttgc tgcttatgca gctgacattg ttgccctccc taaagcaacc aagtagcctt 60
   tatttcccac agtgaaagaa aacgctggcc tatcagttac attacaaaag gcagatttca 120
   agaggattga gtaagtagtt ggatggcttt cataaaaaca agaattcaag aagaggattc 180
   atgctttaag aaacatttgt tatacattcc tcacaaatta tacctgggat aaaaactatg 240
   tagcaggcag tgtgttttcc ttccatgtct ctctgcacta cctgcagtgt gtcctctgag 300
   gctgcaagtc tgtcctatct gaattcccag cagaagcact aagaagctcc accctatcac 360
   ctagcagata aaactatggg gaaaacttaa atctgtgcat a                     401
<210> 183
   <211> 366
   <212> DNA
   <213> Homo sapiens
<220>
   <221> unsure
   <222> (325)
   <223> n=A,T,C or G
<400> 183
   accgtgtcca agtttttaga acccttgtta gccagaccga ggtgtcctgg tcaccgtttc 60
   accatcatgc tttgatgttc ccctgtcttt ctctcttctg ctctcaagag caaaggttaa 120
   tttaaggaca aagatgaagt cactgtaaac taatctgtca ttgtttttac cttccttttc 180
   tttttcagtg cagaaattaa aagtaagtat aaagcaccgt gattgggagt gtttttgcgt 240
   gtgtcggaat cactggtaaa tgttggctga gaacaatccc tccccttgca cttgtgaaaa 300
   cactttgagc gctttaagag attancctga gaaataatta aatatctttt ctcttcaaaa 360
   aaaaaa                                                            366
<210> 184
   <211> 370
   <212> DNA
   <213> Homo sapiens
<400> 184
   tcttacttca aaagaaaaat aaacataaaa aataagttgc tggttcctaa caggaaaaat 60
   tttaataatt gtactgagag aaactgctta cgtacacatt gcagatcaaa tatttggagt 120
   taaaatgtta gtctacatag atgggtgatt gtaactttat tgccattaaa agatttcaaa 180
   ttgcattcat gcttctgtgt acacataatg aaaaatgggc aaataatgaa gatctctcct 240
   tcagtctgct ctgtttaatt ctgctgtctg ctcttctcta atgctgcgtc cctaattgta 300
   cacagtttag tgatatctag gagtataaag ttgtcgccca tcaataaaaa tcacaaagtt 360
   ggtttaaaaa                                                        370
<210> 185
   <211> 107
   <212> DNA
   <213> Homo sapiens
<400> 185
   ctcatattat tttccttttg agaaattgga aactctttct gttgctatta tattaataaa 60
   gttggtgttt attttctggt agtcaccttc cccatttaaa aaaaaaa                107
<210> 186
   <211> 309
   <212> DNA
   <213> Homo sapiens
<400> 186
   gaaaggatgg ctctggttgc cacagagctg ggacttcatg ttcttctaga gagggccaca 60
   agagggccac aggggtggcc gggagttgtc agctgatgcc tgctgagagg caggaattgt 120
   gccagtgagt gacagtcatg agggagtgtc tcttcttggg gaggaaagaa ggtagagcct 180
   ttctgtctga atgaaaggcc aaggctacag tacagggccc cgccccagcc agggtgttaa 240
   tgcccacgta gtggaggcct ctggcagatc ctgcattcca aggtcactgg actgtacgtt 300
   tttatggtt                                                         309
<210> 187
   <211> 477
   <212> DNA
   <213> Homo sapiens
<400> 187
   ttcagtccta gcaagaagcg agaattctga gatcctccag aaagtcgagc agcacccacc 60
   tccaacctcg ggccagtgtc ttcaggcttt actggggacc tgcgagctgg cctaatgtgg 120
   tggcctgcaa gccaggccat ccctgggcgc cacagacgag ctccgagcca ggtcaggctt 180
   cggaggccac aagctcagcc tcaggcccag gcactgattg tggcagaggg gccactaccc 240
   aaggtctagc taggcccaag acctagttac ccagacagtg agaagcccct ggaaggcaga 300
   aaagttggga gcatggcaga cagggaaggg aaacattttc agggaaaaga catgtatcac 360
   atgtcttcag aagcaagtca ggtttcatgt aaccgagtgt cctcttgcgt gtccaaaagt 420
   agcccagggc tgtagcacag gcttcacagt gattttgtgt tcagccgtga gtcacac    477
<210> 188
   <211> 220
   <212> DNA
   <213> Homo sapiens
<400> 188
   taaatatggt agatattaat attcctctta gatgaccagt gattccaatt gtcccaagtt 60
   ttaaataagt accctgtgag tatgagataa attagtgaca atcagaacaa gtttcagtat 120
   cagatgttca agaggaagtt gctattgcat tgattttaat atttgtacat aaacactgat 180
   ttttttgagc attattttgt atttgttgta ctttaatacc                        220
<210> 189
   <211> 417
   <212> DNA
   <213> Homo sapiens
<220>
   <221> unsure
   <222> (76)
   <223> n=A,T,C or G
   <221> unsure
   <222> (77)
   <223> n=A,T,C or G
<400> 189
   accatcttga cagaggatac atgctcccaa aacgtttgtt accacactta aaaatcactg 60
   ccatcattaa gcatcnnttt caaaattata gccattcatg atttactttt tccagatgac 120
   tatcattatt ctagtccttt gaatttgtaa ggggaaaaaa aacaaaaaca aaaacttacg 180
   atgcactttt ctccagcaca tcagatttca aattgaaaat taaagacatg ctatggtaat 240
   gcacttgcta gtactacaca ctttgtacaa caaaaaacag aggcaagaaa caacggaaag 300
   agaaaagcct tcctttgttg gcccttaaac tgagtcaaga tctgaaatgt agagatgatc 360
   tctgacgata cctgtatgtt cttattgtgt aaataaaatt gctggtatga aatgaca    417
<210> 190
   <211> 497
   <212> DNA
   <213> Homo sapiens
<400> 190
   gcactgcggc gctctcccgt cccgcggtgg ttgctgctgc tgccgctgct gctgggcctg 60
   aacgcaggag ctgtcattga ctggcccaca gaggagggca aggaagtatg ggattatgtg 120
   acggtccgca aggatgccta catgttctgg tggctctatt atgccaccaa ctcctgcaag 180
   aacttctcag aactgcccct ggtcatgtgg cttcagggcg gtccaggcgg ttctagcact 240
   ggatttggaa actttgagga aattgggccc cttgacagtg atctcaaacc acggaaaacc 300
   acctggctcc aggctgccag tctcctattt gtggataatc ccgtgggcac tgggttcagt 360
   tatgtgaatg gtagtggtgc ctatgccaag gacctggcta tggtggcttc agacatgatg 420
   gttctcctga agaccttctt cagttgccac aaagaattcc agacagttcc attctacatt 480
   ttctcagagt cctatgg                                                497
<210> 191
   <211> 175
   <212> DNA
   <213> Homo sapiens
<400> 191
   atgttgaata ttttgcttat taactttgtt tattgtcttc tccctcgatt agaatattag 60
   ctacttgagt acaaggattt gagcctgtta cattcactgc tgaattttag gctcctggaa 120
   gatacccagc attcaataga gaccacacaa taaatatatg tcaaataaaa aaaaa      175
<210> 192
   <211> 526
   <212> DNA
   <213> Homo sapiens
<400> 192
   agtaaacatt attatttttt ttatatttgc aaaggaaaca tatctaatcc ttcctataga 60
   aagaacagta ttgctgtaat tccttttctt ttcttcctca tttcctctgc cccttaaaag 120
   attgaagaaa gagaaacttg tcaactcata tccacgttat ctagcaaagt acataagaat 180
   ctatcactaa gtaatgtatc cttcagaatg tgttggttta ccagtgacac cccatattca 240
   tcacaaaatt aaagcaagaa gtccatagta atttatttgc taatagtgga tttttaatgc 300
   tcagagtttc tgaggtcaaa ttttatcttt tcacttacaa gctctatgat cttaaataat 360
   ttacttaatg tattttggtg tattttcctc aaattaatat tggtgttcaa gactatatct 420
   aattcctctg atcactttga gaaacaaact tttattaaat gtaaggcact tttctatgaa 480
   ttttaaatat aaaaataaat attgttctga ttattactga aaaaaa                526
<210> 193
   <211> 553
   <212> DNA
   <213> Homo sapiens
<220>
   <221> unsure
   <222> (290)
   <223> n=A,T,C or G
   <221> unsure
   <222> (300)
   <223> n=A,T,C or G
   <221> unsure
   <222> (411)
   <223> n=A,T,C or G
   <221> unsure
   <222> (441)
   <223> n=A,T,C or G
<400> 193
   tccattgtgg tggaattcgc tctctggtaa aggcgtgcag gtgttggccg cggcctctga 60
   gctgggatga gccgtgctcc cggtggaagc aagggagccc agccggagcc atggccagta 120
   cagtggtagc agttggactg accattgctg ctgcaggatt tgcaggccgt tacgttttgc 180
   aagccatgaa gcatatggag cctcaagtaa aacaagtttt tcaaagccta ccaaaatctg 240
   ccttcagtgg tggctattat agaggtgggt ttgaacccaa aatgacaaan cgggaagcan 300
   cattaatact aggtgtaagc cctactgcca ataaagggaa aataagagat gctcatcgac 360
   gaattatgct tttaaatcat cctgacaaag gaggatctcc ttatatagca nccaaaatca 420
   atgaagctaa agatttacta naaggtcaag ctaaaaaatg aagtaaatgt atgatgaatt 480
   ttaagttcgt attagtttat gtatatgagt actaagtttt tataataaaa tgcctcagag 540
   ctacaatttt aaa                                                    553

<210> 194
   <211> 320
   <212> DNA
   <213> Homo sapiens
<400> 194
   cccttcccaa tccatcagta aagaccccat ctgccttgtc catgccgttt cccaacaggg 60
   atgtcacttg atatgagaat ctcaaatctc aatgccttat aagcattcct tcctgtgtcc 120
   attaagactc tgataattgt ctcccctcca taggaatttc tcccaggaaa gaaatatatc 180
   cccatctccg tttcatatca gaactaccgt ccccgatatt cccttcagag agattaaaga 240
   ccagaaaaaa gtgagcctct tcatctgcac ctgtaatagt ttcagttcct attttcttcc 300
   attgacccat atttatacct                                             320
<210> 195
   <211> 320
   <212> DNA
   <213> Homo sapiens
<220>
   <221> unsure
   <222> (203)
   <223> n=A,T,C or G
   <221> unsure
   <222> (218)
   <223> n=A,T,C or G
<400> 195
   aagcatgacc tggggaaatg gtcagacctt gtattgtgtt tttggccttg aaagtagcaa 60
   gtgaccagaa tctgccatgg caacaggctt taaaaaagac ccttaaaaag acactgtctc 120
   aactgtggtg ttagcaccag ccagctctct gtacatttgc tagcttgtag ttttctaaga 180
   ctgagtaaac ttcttatttt tanaaagggg aggctggntt gtaactttcc ttgtacttaa 240
   ttgggtaaaa gtcttttcca caaaccacca tctattttgt gaactttgtt agtcatcttt 300
   tatttggtaa attatgaact                                             320
<210> 196
   <211> 357
   <212> DNA
   <213> Homo sapiens
<220>
   <221> unsure
   <222> (36)
   <223> n=A,T,C or G
<400> 196
   atataaaata atacgaaact ttaaaaagca ttggantgtc agtatgttga atcagtagtt 60
   tcactttaac tgtaaacaat ttcttaggac accatttggg ctagtttctg tgtaagtgta 120
   aatactacaa aaacttattt atactgttct tatgtcattt gttatattca tagatttata 180
   tgatgatatg acatctggct aaaaagaaat tattgcaaaa ctaaccacta tgtacttttt 240
   tataaatact gtatggacaa aaaatggcat tttttatatt aaattgttta gctctggcaa 300
   aaaaaaaaaa ttttaagagc tggtactaat aaaggattat tatgactgtt aaaaaaa    357
<210> 197
   <211> 565
   <212> DNA
   <213> Homo sapiens
<220>
   <221> unsure
   <222> (27)
   <223> n=A,T,C or G
<400> 197
   tcagctgagt accatcagga tatttanccc tttaagtgct gttttgggag tagaaaacta 60
   aagcaacaat acttcctctt gacagctttg attggaatgg ggttattaga tcattcacct 120
   tggtcctaca ctttttagga tgcttggtga acataacacc acttataatg aacatccctg 180
   gttcctatat tttgggctat gtgggtagga attgttactt gttactgcag cagcagccct 240
   agaaagtaag cccagggctt cagatctaag ttagtccaaa agctaaatga tttaaagtca 300
   agttgtaatg ctaggcataa gcactctata atacattaaa ttataggccg agcaattagg 360
   gaatgtttct gaaacattaa acttgtattt atgtcactaa aattctaaca caaacttaaa 420
   aaatgtgtct catacatatg ctgtactagg cttcatcatg catttctaaa tttgtgtatg 480
   atttgaatat atgaaagaat ttatacaaga gtgttattta aaattattaa aaataaatgt 540
   atataatttg tacctattgt aaaaa                                       565
<210> 198
   <211> 484
   <212> DNA
   <213> Homo sapiens
<400> 198
   tatgtaagta ttggtgtctg ctttaaaaaa ggagacccag acttcacctg tcctttttaa 60
   acatttgaga acagtgttac tctgagcagt tgggccacct tcaccttatc cgacagctga 120
   ctgttggatg tgtccattgt cgccagtttg gctgttgccc ggacaggaca ggacctccat 180
   tgggcgcagc agcaggtggc aggggtgtgg cttgaggtgg gtggcagcgt ctggtcctcc 240
   tctctggtgc tttctgagag ggtctctaaa gcagagtgtg gttggcctgg gggaaggcag 300
   agcacgtatt tctcccctct agtacctctg catttgtgag tgttccctct ggctttctga 360
   agggcagcag actcttgagt atactgcaga ggacatgctt tatcagtagg tcctgagggc 420
   tccaggggct caactgacca agtaacacag aagttggggt atgtggccta tttgggtcgg 480
   aaac                                                              484
<210> 199
   <211> 429
   <212> DNA
   <213> Homo sapiens
<220>
   <221> unsure
   <222> (77)
   <223> n=A,T,C or G
   <221> unsure
   <222> (88)
   <223> n=A,T,C or G
   <221> unsure
   <222> (134)
   <223> n=A,T,C or G
   <221> unsure
   <222> (151)
   <223> n=A,T,C or G
   <221> unsure
   <222> (189)
   <223> n=A,T,C or G
   <221> unsure
   <222> (227)
   <223> n=A,T,C or G
   <221> unsure
   <222> (274)
   <223> n=A,T,C or G
   <221> unsure
   <222> (319)
   <223> n=A,T,C or G
<400> 199
   gcttatgttt tttgttttaa cttttgtttt ttaacattta gaatattaca ttttgtatta 60
   tacagtacct ttctcanaca ttttgtanaa ttcatttcgg cagctcacta ggattttgct 120
   gaacattaaa aagngtgata gcgatattag ngccaatcaa atggaaaaaa ggtagtctta 180
   ataaacaana cacaacgttt ttatacaaca tactttaaaa tattaanaaa actccttaat 240
   attgtttcct attaagtatt attctttggg caanattttc tgatgctttt gattttctct 300
   caatttagca tttgctttng gtttttttct ctatttagca ttctgttaag gcacaaaaac 360
   tatgtactgt atgggaaatg ttgtaaatat taccttttcc acattttaaa cagacaactt 420
   tgaatccaa                                                         429
<210> 200
   <211> 279
   <212> DNA
   <213> Homo sapiens
<400> 200
   gcttttttga ggaattacag ggaagctcct ggaattgtac atggatatct ttatccctag 60
   ggggaaatca aggagctggg cacccctaat tctttatgga agtgtttaaa actattttaa 120
   ttttattaca agtattacta gagtagtggt tctactctaa gatttcaaaa gtgcatttaa 180
   aatcatacat gttcccgcct gcaaatatat tgttattttg gtggagaaaa aaatagtata 240
   ttctacataa aaaattaaag atattaacta agaaaaaaa                        279
<210> 201
   <211> 569
   <212> DNA
   <213> Homo sapiens
<400> 201
   taggtcagta tttttagaaa ctcttaatag ctcatactct tgataccaaa agcagccctg 60
   attgttaaag cacacacctg cacaagaagc agtgatggtt gcatttacat ttcctgggtg 120
   cacaaaaaaa aattctcaaa aagcaaggac ttacgctttt tgcaaagcct ttgagaagtt 180
   actggatcat aggaagctta taacaagaat ggaagattct taaataactc actttctttg 240
   gtatccagta acagtagatg ttcaaaatat gtagctgatt aataccagca ttgtgaacgc 300
   tgtacaacct tgtggttatt actaagcaag ttactactag cttctgaaaa gtagcttcat 360
   aattaatgtt atttatacac tgccttccat gacttttact ttgccctaag ctaatctcca 420
   aaatctgaaa tgctactcca atatcagaaa aaaaggggga ggtggaatta tatttcctgt 480
   gattttaaga gtacagagaa tcatgcacat ctctgattag ttcatatatg tctagtgtgt 540
   aataaaagtc aaagatgaac tctcaaaaa                                   569
<210> 202
   <211> 501
   <212> DNA
   <213> Homo sapiens
<400> 202
   attaataggc ttaataattg ttggcaagga tccttttgct ttctttggca tgcaagctcc 60
   tagcatctgg cagtggggcc aagaaaataa ggtttatgca tgtatgatgg ttttcttctt 120
   gagcaacatg attgagaacc agtgtatgtc aacaggtgca tttgagataa ctttaaatga 180
   tgtacctgtg tggtctaagc tggaatctgg tcaccttcca tccatgcaac aacttgttca 240
   aattcttgac aatgaaatga agctcaatgt gcatatggat tcaatcccac accatcgatc 300
   atagcaccac ctatcagcac tgaaaactct tttgcattaa gggatcattg caagagcagc 360
   gtgactgaca ttatgaaggc ctgtactgaa gacagcaagc tgttagtaca gaccagatgc 420
   tttcttggca ggctcgttgt acctcttgga aaacctcaat gcaagatagt gtttcagtgc 480
   tggcatattt tggaattctg c                                           501
<210> 203
   <211> 261
   <212> DNA
   <213> Homo sapiens
<220>
   <221> unsure
   <222> (36)
   <223> n=A,T,C or G
   <221> unsure
   <222> (96)
   <223> n=A,T,C or G
<400> 203
   gacaagctcc tggtcttgag atgtcttctc gttaangaga tgggcctttt ggaggtaaag 60
   gataaaatga atgagttctg tcatgattca ctattntata acttgcatga cctttactgt 120
   gttagctctt tgaatgttct tgaaatttta gactttcttt gtaaacaaat gatatgtcct 180
   tatcattgta taaaagctgt tatgtgcaac agtgtggaga ttccttgtct gatttaataa 240
   aatacttaaa cactgaaaaa a                                           261
<210> 204
   <211> 421
   <212> DNA
   <213> Homo sapiens
<400> 204
   agcatctttt ctacaacgtt aaaattgcag aagtagctta tcattaaaaa acaacaacaa 60
   caacaataac aataaatcct aagtgtaaat cagttattct accccctacc aaggatatca 120
   gcctgttttt tccctttttt ctcctgggaa taattgtggg cttcttccca aatttctaca 180
   gcctctttcc tcttctcatg cttgagcttc cctgtttgca cgcatgcgtg tgcaggactg 240
   gcttgtgtgc ttggactcgg ctccaggtgg aagcatgctt tcccttgtta ctgttggaga 300
   aactcaaacc ttcaagccct aggtgtagcc attttgtcaa gtcatcaact gtatttttgt 360
   actggcatta acaaaaaaag aagataaaat attgtaccat taaactttaa taaaacttta 420
   a                                                                 421
<210> 205
   <211> 460
   <212> DNA
   <213> Homo sapiens
<400> 205
   tactctcaca atgaaggacc tggaatgaaa aatctgtgtc taaacaagtc ctctttagat 60
   tttagtgcaa atccagagcc agcgtcggtt gcctcgagta attctttcat gggtaccttt 120
   ggaaaagctc tcaggagacc tcacctagat gcctattcaa gctttggaca gccatcagat 180
   tgtcagccaa gagcctttta tttgaaagct cattcttccc cagacttgga ctctgggtca 240
   gaggaagatg ggaaagaaag gacagatttt caggaagaaa atcacatttg tacctttaaa 300
   cagactttag aaaactacag gactccaaat tttcagtctt atgacttgga cacatagact 360
   gaatgagacc aaaggaaaag cttaacatac tacctcaagg tgaactttta tttaaaagag 420
   agagaatctt atgtttttta aatggagtta tgaattttaa                       460
<210> 206
   <211> 481
   <212> DNA
   <213> Homo sapiens
<400> 206
   tgtggtggaa ttcgggacgc ccccagaccc tgactttttc ctgcgtgggc cgtctcctcc 60
   tgcggaagca gtgacctctg acccctggtg accttcgctt tgagtgcctt ttgaacgctg 120
   gtcccgcggg acttggtttt ctcaagctct gtctgtccaa agacgctccg gtcgaggtcc 180
   cgcctgccct gggtggatac ttgaacccca gacgcccctc tgtgctgctg tgtccggagg 240
   cggccttccc atctgcctgc ccacccggag ctctttccgc cggcgcaggg tcccaagccc 300
   acctcccgcc ctcagtcctg cggtgtgcgt ctgggcacgt cctgcacaca caatgcaagt 360
   cctggcctcc gcgcccgccc gcccacgcga gccgtacccg ccgccaactc tgttatttat 420
   ggtgtgaccc cctggaggtg ccctcggccc accggggcta tttattgttt aatttatttg 480
   t                                                                 481
<210> 207
   <211> 605
   <212> DNA
   <213> Homo sapiens
<400> 207
   accctttttg gattcagggc tcctcacaat taaaatgagt gtaatgaaac aaggtgaaaa 60
   tatagaagca tccctttgta tactgttttg ctacttacag tgtacttggc attgctttat 120
   ctcactggat tctcacggta ggatttctga gatcttaatc taagctccaa agttgtctac 180
   ttttttgatc ctagggtgct ccttttgttt tacagagcag ggtcacttga tttgctagct 240
   ggtggcagaa ttggcaccat tacccaggtc tgactgacca ccagtcagag gcactttatt 300
   tgtatcatga aatgatttga aatcattgta aagcagcgaa gtctgataat gaatgccagc 360
   tttccttgtg ctttgataac aaagactcca aatattctgg agaacctgga taaaagtttg 420
   aagggctaga ttgggatttg aagacaaaat tgtaggaaat cttacatttt tgcaataaca 480
   aacattaatg aaagcaaaac attataaaag taattttaat tcaccacata cttatcaatt 540
   tcttgatgct tccaaatgac atctaccaga tatggttttg tggacatctt tttctgttta 600
   cataa                                                             605
<210> 208
   <211> 655
   <212> DNA
   <213> Homo sapiens
<400> 208
   ggcgttgttc tggattcccg tcgtaactta aagggaaact ttcacaatgt ccggagccct 60
   tgatgtcctg caaatgaagg aggaggatgt ccttaagttc cttgcagcag gaacccactt 120
   aggtggcacc aatcttgact tccagatgga acagtacatc tataaaagga aaagtgatgg 180
   catctatatc ataaatctca agaggacctg ggagaagctt ctgctggcag ctcgtgcaat 240
   tgttgccatt gaaaaccctg ctgatgtcag tgttatatcc tccaggaata ctggccagag 300
   ggctgtgctg aagtttgctg ctgccactgg agccactcca attgctggcc gcttcactcc 360
   tggaaccttc actaaccaga tccaggcagc cttccgggag ccacggcttc ttgtggttac 420
   tgaccccagg gctgaccacc agcctctcac ggaggcatct tatgttaacc tacctaccat 480
   tgcgctgtgt aacacagatt ctcctctgcg ctatgtggac attgccatcc catgcaacaa 540
   caagggagct cactcagtgg gtttgatgtg gtggatgctg gctcgggaag ttctgcgcat 600
   gcgtggcacc atttcccgtg aacacccatg ggaggtcatg cctgatctgt acttc      655
<210> 209
   <211> 621
   <212> DNA
   <213> Homo sapiens
<400> 209
   catttagaac atggttatca tccaagacta ctctaccctg caacattgaa ctcccaagag 60
   caaatccaca ttcctcttga gttctgcagc ttctgtgtaa atagggcagc tgtcgtctat 120
   gccgtagaat cacatgatct gaggaccatt catggaagct gctaaatagc ctagtctggg 180
   gagtcttcca taaagttttg catggagcaa acaaacagga ttaaactagg tttggttcct 240
   tcagccctct aaaagcatag ggcttagcct gcaggcttcc ttgggctttc tctgtgtgtg 300
   tagttttgta aacactatag catctgttaa gatccagtgt ccatggaaac cttcccacat 360
   gccgtgactc tggactatat cagtttttgg aaagcagggt tcctctgcct gctaacaagc 420
   ccacgtggac cagtctgaat gtctttcctt tacacctatg tttttaaata gtcaaacttc 480
   aagaaacaat ctaaacaagt ttctgttgca tatgtgtttg tgaacttgta tttgtattta 540
   gtaggcttct atattgcatt taacttgttt ttgtaactcc tgattcttcc ttttcggata 600
   ctattgatga ataaagaaat t                                            621
<210> 210
   <211> 533
   <212> DNA
   <213> Homo sapiens
<220>
   <221> unsure
   <222> (20)
   <223> n=A,T,C or G
   <221> unsure
   <222> (21)
   <223> n=A,T,C or G
   <221> unsure
   <222> (61)
   <223> n=A,T,C or G
<400> 210
   cgccttgggg agccggcggn ngagtccggg acgtggagac ccggggtccc ggcagccggg 60
   nggcccgcgg gcccagggtg gggatgcacc gccgcggggt gggagctggc gccatcgcca 120
   agaagaaact tgcagaggcc aagtataagg agcgagggac ggtcttggct gaggaccagc 180
   tagcccagat gtcaaagcag ttggacatgt tcaagaccaa cctggaggaa tttgccagca 240
   aacacaagca ggagatccgg aagaatcctg agttccgtgt gcagttccag gacatgtgtg 300
   caaccattgg cgtggatccg ctggcctctg gaaaaggatt ttggtctgag atgctgggcg 360
   tgggggactt ctattacgaa ctaggtgtcc aaattatcga agtgtgcctg gcgctgaagc 420
   atcggaatgg aggtctgata actttggagg aactacatca acaggtgttg aagggaaggg 480
   gcaagttcgc ccaggatgtc agtcaagatg acctgatcag agccatcaag aaa 533
<210> 211
   <211> 451
   <212> DNA
   <213> Homo sapiens
<400> 211
   ttagcttgag ccgagaacga ggcgagaaag ctggagaccg aggagaccgc ctagagcgga 60
   gtgaacgggg aggggaccgt ggggaccggc ttgatcgtgc gcggacacct gctaccaagc 120
   ggagcttcag caaggaagtg gaggagcgga gtagagaacg gccctcccag cctgaggggc 180
   tgcgcaaggc agctagcctc acggaggatc gggaccgtgg gcgggatgcc gtgaagcgag 240
   aagctgccct acccccagtg agccccctga aggcggctct ctctgaggag gagttagaga 300
   agaaatccaa ggctatcatt gaggaatatc tccatctcaa tgacatgaaa gaggcagtcc 360
   agtgcgtgca ggagctggcc tcaccctcct tgctcttcat ctttgtacgg catggtgtcg 420
   agtctacgct ggagcgcagt gccattgctc g 451
<210> 212
   <211> 471
   <212> DNA
   <213> Homo sapiens
<220>
   <221> unsure
   <222> (54)
   <223> n=A,T,C or G
<400> 212
   gtgattattc ttgatcaggg agaagatcat ttagatttgt tttgcattcc ttanaatgga 60
   gggcaacatt ccacagctgc cctggctgtg atgagtgtcc ttgcaggggc cggagtagga 120
   gcactggggt gggggcggaa ttggggttac tcgatgtaag ggattccttg ttgttgtgtt 180
   gagatccagt gcagttgtga tttctgtgga tcccagcttg gttccaggaa ttttgtgtga 240
   ttggcttaaa tccagttttc aatcttcgac agctgggctg gaacgtgaac tcagtagctg 300
   aacctgtctg acccggtcac gttcttggat cctcagaact ctttgctctt gtcggggtgg 360
   gggtgggaac tcacgtgggg agcggtggct gagaaaatgt aaggattctg gaatacatat 420
   tccatgggac tttccttccc tctcctgctt cctcttttcc tgctccctaa c 471
<210> 213
   <211> 511
   <212> DNA
   <213> Homo sapiens
<220>
   <221> unsure
   <222> (27)
   <223> n=A,T,C or G
   <221> unsure
   <222> (63)
   <223> n=A,T,C or G
   <221> unsure
   <222> (337)
   <223> n=A,T,C or G
   <221> unsure
   <222> (442)
   <223> n=A,T,C or G
<400> 213
   ctaattagaa acttgctgta ctttttnttt tcttttaggg gtcaaggacc ctctttatag 60
   ctnccatttg cctacaataa attattgcag cagtttgcaa tactaaaata ttttttatag 120
   actttatatt tttccttttg ataaagggat gctgcatagt agagttggtg taattaaact 180
   atctcagccg tttccctgct ttcccttctg ctccatatgc ctcattgtcc ttccagggag 240
   ctcttttaat cttaaagttc tacatttcat gctcttagtc aaattctgtt acctttttaa 300
   taactcttcc cactgcatat ttccatcttg aattggnggt tctaaattct gaaactgtag 360
   ttgagataca gctatttaat atttctggga gatgtgcatc cctcttcttt gtggttgccc 420
   aaggttgttt tgcgtaactg anactccttg atatgcttca gagaatttag gcaaacactg 480
   gccatggccg tgggagtact gggagtaaaa t                                511
<210> 214
   <211> 521
   <212> DNA
   <213> Homo sapiens
<400> 214
   agcattgcca aataatccct aattttccac taaaaatata atgaaatgat gttaagcttt 60
   ttgaaaagtt taggttaaac ctactgttgt tagattaatg tatttgttgc ttccctttat 120
   ctggaatgtg gcattagctt ttttatttta accctcttta attcttattc aattccatga 180
   cttaaggttg gagagctaaa cactgggatt tttggataac agactgacag ttttgcataa 240
   ttataatcgg cattgtacat agaaaggata tggctacctt ttgttaaatc tgcactttct 300
   aaatatcaaa aaagggaaat gaagtataaa tcaatttttg tataatctgt ttgaaacatg 360
   agttttattt gcttaatatt agggctttgc cccttttctg taagtctctt gggatcctgt 420
   gtagaagctg ttctcattaa acaccaaaca gttaagtcca ttctctggta ctagctacaa 480
   attcggtttc atattctact taacaattta aataaactga a                     521
<210> 215
   <211> 381
   <212> DNA
   <213> Homo sapiens
<220>
   <221> unsure
   <222> (17)
   <223> n=A,T,C or G
   <221> unsure
   <222> (20)
   <223> n=A,T,C or G
   <221> unsure
   <222> (60)
   <223> n=A,T,C or G
   <221> unsure
   <222> (61)
   <223> n=A,T,C or G
   <221> unsure
   <222> (365)
   <223> n=A,T,C or G
<400> 215
   gagcggagag cggaccngtn agagccctga gcagccccac cgccgccgcc ggcctagttn 60
   ncatcacacc ccgggaggag ccgcagctgc cgcagccggc cccagtcacc atcaccgcaa 120
   ccatgagcag cgaggccgag acccagcagc cgcccgccgc cccccccgcc gcccccgccc 180
   tcagcgccgc cgacaccaag cccggcacta cgggcagcgg cgcagggagc ggtggcccgg 240
   gcggcctcac atcggcggcg cctgccggcg gggacaagaa ggtcatcgca acgaaggttt 300
   tgggaacagt aaaatggttc aatgtaagga acggatatgg tttcatcaac aggaatgaca 360
   ccaangaaga tgtatttgta c                                          381
<210> 216
   <211> 425
   <212> DNA
   <213> Homo sapiens
<400> 216
   ttactaacta ggtcattcaa ggaagtcaag ttaacttaaa catgtcacct aaatgcactt 60
   gatggtgttg aaatgtccac cttcttaaat ttttaagatg aacttagttc taaagaagat 120
   aacaggccaa tcctgaaggt actccctgtt tgctgcagaa tgtcagatat tttggatgtt 180
   gcataagagt cctatttgcc ccagttaatt caacttttgt ctgcctgttt tgtggactgg 240
   ctggctctgt tagaactctg tccaaaaagt gcatggaata taacttgtaa agcttcccac 300
   aattgacaat atatatgcat gtgtttaaac caaatccaga aagcttaaac aatagagctg 360
   cataatagta tttattaaag aatcacaact gtaaacatga gaataactta aggattctag 420
   tttag                                                             425
<210> 217
   <211> 181
   <212> DNA
   <213> Homo sapiens
<400> 217
   gagaaaccaa atgataggtt gtagagcctg atgactccaa acaaagccat cacccgcatt 60
   cttcctcctt cttctggtgc tacagctcca agggcccttc accttcatgt ctgaaatgga 120
   actttggctt tttcagtgga agaatatgtt gaaggtttca ttttgttcta gaaaaaaaaa 180
   a                                                                 181
<210> 218
   <211> 405
   <212> DNA
   <213> Homo sapiens
<400> 218
   caggccttcc agttcactga caaacatggg gaagtgtgcc cagctggctg gaaacctggc 60
   agtgatacca tcaagcctga tgtccaaaag agcaaagaat atttctccaa gcagaagtga 120
   gcgctgggct gttttagtgc caggctgcgg tgggcagcca tgagaacaaa acctcttctg 180
   tatttttttt ttccattagt aaaacacaag acttcagatt cagccgaatt gtggtgtctt 240
   acaaggcagg cctttcctac agggggtgga gagaccagcc tttcttcctt tggtaggaat 300
   ggcctgagtt ggcgttgtgg gcaggctact ggtttgtatg atgtattagt agagcaaccc 360
   attaatcttt tgtagtttgt attaaacttg aactgagaaa aaaaa                 405
<210> 219
   <211> 216
   <212> DNA
   <213> Homo sapiens
<220>
   <221> unsure
   <222> (207)
   <223> n=A,T,C or G
   <221> unsure
   <222> (210)
   <223> n=A,T,C or G
<400> 219
   actccaagag ttagggcagc agagtggagc gatttagaaa gaacatttta aaacaatcag 60
   ttaatttacc atgtaaaatt gctgtaaatg ataatgtgta cagattttct gttcaaatat 120
   tcaattgtaa acttcttgtt aagactgtta cgtttctatt gcttttgtat gggatattgc 180
   aaaaataaaa aggaaagaac cctcttnaan aaaaaa                           216
<210> 220
   <211> 380
   <212> DNA
   <213> Homo sapiens
<400> 220
   cttacaaatt gcccccatgt gtaggggaca cagaaccctt tgagaaaact tagatttttg 60
   tctgtacaaa gtctttgcct ttttccttct tcattttttt ccagtacatt aaatttgtca 120
   atttcatctt tgagggaaac tgattagatg ggttgtgttt gtgttctgat ggagaaaaca 180
   gcaccccaag gactcagaag atgattttaa cagttcagaa cagatgtgtg caatattggt 240
   gcatgtaata atgttgagtg gcagtcaaaa gtcatgattt ttatcttagt tcttcattac 300
   tgcattgaaa aggaaaacct gtctgagaaa atgcctgaca gtttaattta aaactatggt 360
   gtaagtcttt gacaaaaaaa                                             380
<210> 221
   <211> 398
   <212> DNA
   <213> Homo sapiens
<400> 221
   ggttagtaag ctgtcgactt tgtaaaaaag ttaaaaatga aaaaaaaagg aaaaatgaat 60
   tgtatattta atgaatgaac atgtacaatt tgccactggg aggaggttcc tttttgttgg 120
   gtgagtctgc aagtgaattt cactgatgtt gatattcatt gtgtgtagtt ttatttcggt 180
   cccagccccg tttcctttta ttttggagct aatgccagct gcgtgtctag ttttgagtgc 240
   agtaaaatag aatcagcaaa tcactcttat ttttcatcct tttccggtat tttttgggtt 300
   gtttctgtgg gagcagtgta caccaactct tcctgtatat tgcctttttg ctggaaaatg 360
   ttgtatgttg aataaaattt tctataaaaa ttaaaaaa 398
<210> 222
   <211> 301
   <212> DNA
   <213> Homo sapiens
<220>
   <221> unsure
   <222> (49)
   <223> n=A,T,C or G
   <221> unsure
   <222> (64)
   <223> n=A,T,C or G
<400> 222
   ttcgataatt gatctcatgg gctttccctg gaggaaaggt tttttttgnt gtttattttt 60
   taanaacttg aaacttgtaa actgagatgt ctgtagcttt tttgcccatc tgtagtgtat 120
   gtgaagattt caaaacctga gagcactttt tctttgttta gaattatgag aaaggcacta 180
   gatgacttta ggatttgcat ttttcccttt attgcctcat ttcttgtgac gccttgttgg 240
   ggagggaaat ctgtttattt tttcctacaa ataaaaagct aagattctat atcgcaaaaa 300
   a 301
<210> 223
   <211> 200
   <212> DNA
   <213> Homo sapiens
<400> 223
   gtaagtgctt aggaagaaac tttgcaaaca tttaatgagg atacactgtt catttttaaa 60
   attccttcac actgtaattt aatgtgtttt atattctttt gtagtaaaac aacataactc 120
   agatttctac aggagacagt ggttttattt ggattgtctt ctgtaatagg tttcaataaa 180
   gctggatgaa cttaaaaaaa 200
<210> 224
   <211> 385
   <212> DNA
   <213> Homo sapiens
<400> 224
   gaaaggtttg atccggactc aaagaaagca aaggagtgtg agccgccatc tgctggagca 60
   gctgtaactg caagacctgg acaagagatt cgtcagcgaa ctgcagctca aagaaacctt 120
   tctccaacac cagcaagccc taaccagggc cctcctccac aagttccagt atctcctgga 180
   ccaccaaagg acagttctgc ccctggtgga cccccagaaa ggactgttac tccagcccta 240
   tcatcaaatg tgttaccaag acatcttgga tcccctgcta cttcagtgcc tggaatgggt 300
   aaacagagca cttaatgtta tttacagttt atattgtttt ctctggttac caataaaacg 360
   ggccattttc aggtggtaaa aaaaa                                       385
<210> 225
   <211> 560
   <212> PRT
   <213> Homo sapien
<400> 225
   Met Glu Cys Leu Tyr Tyr Phe Leu Gly Phe Leu Leu Leu Ala Ala Arg
    1               5                  10                  15
   Leu Pro Leu Asp Ala Ala Lys Arg Phe His Asp Val Leu Gly Asn Glu
               20                  25                  30
   Arg Pro Ser Ala Tyr Met Arg Glu His Asn Gln Leu Asn Gly Trp Ser
           35                  40                  45
   Ser Asp Glu Asn Asp Trp Asn Glu Lys Leu Tyr Pro Val Trp Lys Arg
       50                  55                  60
   Gly Asp Met Arg Trp Lys Asn Ser Trp Lys Gly Gly Arg Val Gln Ala
   65                  70                  75                  80
   Val Leu Thr Ser Asp Ser Pro Ala Leu Val Gly Ser Asn Ile Thr Phe
                   85                  90                  95
   Ala Val Asn Leu Ile Phe Pro Arg Cys Gln Lys Glu Asp Ala Asn Gly
               100                 105                 110
   Asn Ile Val Tyr Glu Lys Asn Cys Arg Asn Glu Ala Gly Leu Ser Ala
           115                 120                 125
   Asp Pro Tyr Val Tyr Asn Trp Thr Ala Trp Ser Glu Asp Ser Asp Gly
       130                 135                 140
   Glu Asn Gly Thr Gly Gln Ser His His Asn Val Phe Pro Asp Gly Lys
   145                 150                 155                 160
   Pro Phe Pro His His Pro Gly Trp Arg Arg Trp Asn Phe Ile Tyr Val
                   165                 170                 175
   Phe His Thr Leu Gly Gln Tyr Phe Gln Lys Leu Gly Arg Cys Ser Val
               180                 185                 190
   Arg Val Ser Val Asn Thr Ala Asn Val Thr Leu Gly Pro Gln Leu Met
           195                 200                 205
   Glu Val Thr Val Tyr Arg Arg His Gly Arg Ala Tyr Val Pro Ile Ala
               210                     215                     220
   Gln Val Lys Asp Val Tyr Val Val Thr Asp Gln Ile Pro Val Phe Val
   225                     230                     235                 240
   Thr Met Phe Gln Lys Asn Asp Arg Asn Ser Ser Asp Glu Thr Phe Leu
                       245                     250                     255
   Lys Asp Leu Pro Ile Met Phe Asp Val Leu Ile His Asp Pro Ser His
                   260                     265                     270
   Phe Leu Asn Tyr Ser Thr Ile Asn Tyr Lys Trp Ser Phe Gly Asp Asn
           275                     280                     285
   Thr Gly Leu Phe Val Ser Thr Asn His Thr Val Asn His Thr Tyr Val
       290                     295                     300
   Leu Asn Gly Thr Phe Ser Leu Asn Leu Thr Val Lys Ala Ala Ala Pro
   305                     310                     315                 320
   Gly Pro Cys Pro Pro Pro Pro Pro Pro Pro Arg Pro Ser Lys Pro Thr
                       325                     330                     335
   Pro Ser Leu Gly Pro Ala Gly Asp Asn Pro Leu Glu Leu Ser Arg Ile
                   340                     345                     350
   Pro Asp Glu Asn Cys Gln Ile Asn Arg Tyr Gly His Phe Gln Ala Thr
           355                     360                     365
   Ile Thr Ile Val Glu Gly Ile Leu Glu Val Asn Ile Ile Gln Met Thr
       370                     375                     380
   Asp Val Leu Met Pro Val Pro Trp Pro Glu Ser Ser Leu Ile Asp Phe
   385                     390                     395                 400
   Val Val Thr Cys Gln Gly Ser Ile Pro Thr Glu Val Cys Thr Ile Ile
                       405                     410                     415
   Ser Asp Pro Thr Cys Glu Ile Thr Gln Asn Thr Val Cys Ser Pro Val
                   420                     425                     430
   Asp Val Asp Glu Met Cys Leu Leu Thr Val Arg Arg Thr Phe Asn Gly
           435                     440                     445
   Ser Gly Thr Tyr Cys Val Asn Leu Thr Leu Gly Asp Asp Thr Ser Leu
       450                     455                     460
   Ala Leu Thr Ser Thr Leu Ile Ser Val Pro Asp Arg Asp Pro Ala Ser
   465                     470                     475                 480
   Pro Leu Arg Met Ala Asn Ser Ala Leu Ile Ser Val Gly Cys Leu Ala
                       485                     490                     495
   Ile Phe Val Thr Val Ile Ser Leu Leu Val Tyr Lys Lys His Lys Glu
                   500                     505                     510
   Tyr Asn Pro Ile Glu Asn Ser Pro Gly Asn Val Val Arg Ser Lys Gly
           515                     520                     525
   Leu Ser Val Phe Leu Asn Arg Ala Lys Ala Val Phe Phe Pro Gly Asn
       530                     535                     540
   Gln Glu Lys Asp Pro Leu Leu Lys Asn Gln Glu Phe Lys Gly Val Ser
   545                     550                     555                 560
<210> 226
   <211> 9
   <212> PRT
   <213> Homo sapien
<400> 226
   Ile Leu Ile Pro Ala Thr Trp Lys Ala
    1               5
<210> 227
   <211> 9
   <212> PRT
   <213> Homo sapien
<400> 227
   Phe Leu Leu Asn Asp Asn Leu Thr Ala
   1 5
<210> 228
   <211> 9
   <212> PRT
   <213> Homo sapien
<400> 228
   Leu Leu Gly Asn Cys Leu Pro Thr Val
   1 5
<210> 229
   <211> 10
   <212> PRT
   <213> Homo sapien
<400> 229
   Lys Leu Leu Gly Asn Cys Leu Pro Thr Val
   1 5 10
<210> 230
   <211> 10
   <212> PRT
   <213> Homo sapien
<400> 230
   Arg Leu Thr Gly Gly Leu Lys Phe Phe Val
   1 5 10
<210> 231
   <211> 9
   <212> PRT
   <213> Homo sapien
<400> 231
   Ser Leu Gln Ala Leu Lys Val Thr Val
   1 5
<210> 232
   <211> 20
   <212> PRT
   <213> Homo sapiens
<400> 232
   Ala Gly Ala Asp Val Ile Lys Asn Asp Gly Ile Tyr Ser Arg Tyr Phe
   5 10 15
   Phe Ser Phe Ala
   20
<210> 233
   <211> 21
   <212> PRT
   <213> Homo sapiens
<400> 233
   Phe Phe Ser Phe Ala Ala Asn Gly Arg Tyr Ser Leu Lys Val His Val
   5 10 15
   Asn His Ser Pro Ser
   20
<210> 234
   <211> 20
   <212> PRT
   <213> Homo sapiens
<400> 234
   Phe Leu Val Thr Trp Gln Ala Ser Gly Pro Pro Glu Ile Ile Leu Phe
   5 10 15
   Asp Pro Asp Gly
   20
<210> 235
   <211> 20
   <212> PRT
   <213> Homo sapiens
<400> 235
   Leu Gln Ser Ala Val Ser Asn Ile Ala Gln Ala Pro Leu Phe Ile Pro
   5 10 15
   Pro Asn Ser Asp
   20
<210> 236
   <211> 20
   <212> PRT
   <213> Homo sapiens
<400> 236
   Ile Gln Asp Asp Phe Asn Asn Ala Ile Leu Val Asn Thr Ser Lys Arg
   5 10 15
   Asn Pro Gln Gln
   20
<210> 237
   <211> 21
   <212> PRT
   <213> Homo sapiens
<400> 237
   Arg Asn Ser Leu Gln Ser Ala Val Ser Asn Ile Ala Gln Ala Pro Leu
   5 10 15
   Phe Ile Pro Pro Asn
   20
<210> 238
   <211> 20
   <212> PRT
   <213> Homo sapiens
<400> 238
   Thr His Glu Ser His Arg Ile Tyr Val Ala Ile Arg Ala Met Asp Arg
   5 10 15
   Asn Ser Leu Gln
   20
<210> 239
   <211> 20
   <212> PRT
   <213> Homo sapiens
<400> 239
   Arg Asn Pro Gln Gln Ala Gly Ile Arg Glu Ile Phe Thr Phe Ser Pro
   5 10 15
   Gln Ile Ser Thr
   20
<210> 240
   <211> 21
   <212> PRT
   <213> Homo sapiens
<400> 240
   Gly Gln Ala Thr Ser Tyr Glu Ile Arg Met Ser Lys Ser Leu Gln Asn
   5 10 15
   Ile Gln Asp Asp Phe
   20
<210> 241
   <211> 20
   <212> PRT
   <213> Homo sapiens
<400> 241
   Glu Arg Lys Trp Gly Phe Ser Arg Val Ser Ser Gly Gly Ser Phe Ser
   5 10 15
   Val Leu Gly Val
   20
<210> 242
   <211> 20
   <212> PRT
   <213> Homo sapiens
<400> 242
   Gly Ser His Ala Met Tyr Val Pro Gly Tyr Thr Ala Asn Gly Asn Ile
   5 10 15
   Gln Met Asn Ala
   20
<210> 243
   <211> 20
   <212> PRT
   <213> Homo sapiens
<400> 243
   Val Asn His Ser Pro Ser Ile Ser Thr Pro Ala His Ser Ile Pro Gly
   5 10 15
   Ser His Ala Met
   20
<210> 244
   <211> 20
   <212> PRT
   <213> Homo sapiens
<400> 244
   Ala Val Pro Pro Ala Thr Val Glu Ala Phe Val Glu Arg Asp Ser Leu
                   5                   10                  15
   His Phe Pro His
               20
<210> 245
   <211> 20
   <212> PRT
   <213> Homo sapiens
<400> 245
   Lys Pro Gly His Trp Thr Tyr Thr Leu Asn Asn Thr His His Ser Leu
                   5                   10                  15
   Gln Ala Leu Lys
               20
<210> 246
   <211> 20
   <212> PRT
   <213> Homo sapiens
<400> 246
   Asn Leu Thr Phe Arg Thr Ala Ser Leu Trp Ile Pro Gly Thr Ala Lys
                   5                   10                  15
   Pro Gly His Trp
               20
<210> 247
   <211> 20
   <212> PRT
   <213> Homo sapiens
<400> 247
   Leu His Phe Pro His Pro Val Met Ile Tyr Ala Asn Val Lys Gln Gly
                   5                   10                  15
   Phe Tyr Pro Ile
               20
<210> 248
   <211> 20
   <212> PRT
   <213> Homo sapiens
<400> 248
   Pro Glu Thr Gly Asp Pro Val Thr Leu Arg Leu Leu Asp Asp Gly Ala
   5 10 15
   Gly Ala Asp Val
   20
<210> 249
   <211> 20
   <212> PRT
   <213> Homo sapiens
<400> 249
   Gly Phe Tyr Pro Ile Leu Asn Ala Thr Val Thr Ala Thr Val Glu Pro
   5 10 15
   Glu Thr Gly Asp
   20
<210> 250
   <211> 20
   <212> PRT
   <213> Homo sapiens
<400> 250
   Phe Asp Pro Asp Gly Arg Lys Tyr Tyr Thr Asn Asn Phe Ile Thr Asn
   5 10 15
   Leu Thr Phe Arg
   20
<210> 251
   <211> 20
   <212> PRT
   <213> Homo sapiens
<400> 251
   Leu Gln Ala Leu Lys Val Thr Val Thr Ser Arg Ala Ser Asn Ser Ala
   5 10 15
   Val Pro Pro Ala
   20
<210> 252
   <211> 153
   <212> PRT
   <213> Homo sapien
<400> 252
   Met Ala Ser Val Arg Val Ala Ala Tyr Phe Glu Asn Phe Leu Ala Ala
    1               5                  10                  15
   Trp Arg Pro Val Lys Ala Ser Asp Gly Asp Tyr Tyr Thr Leu Ala Val
               20                  25                  30
   Pro Met Gly Asp Val Pro Met Asp Gly Ile Ser Val Ala Asp Ile Gly
           35                  40                  45
   Ala Ala Val Ser Ser Ile Phe Asn Ser Pro Glu Glu Phe Leu Gly Lys
       50                  55                  60
   Ala Val Gly Leu Ser Ala Glu Ala Leu Thr Ile Gln Gln Tyr Ala Asp
   65                  70                  75                  80
   Val Leu Ser Lys Ala Leu Gly Lys Glu Val Arg Asp Ala Lys Ile Thr
                   85                  90                  95
   Pro Glu Ala Phe Glu Lys Leu Gly Phe Pro Ala Ala Lys Glu Ile Ala
               100                 105                 110
   Asn Met Cys Arg Phe Tyr Glu Met Lys Pro Asp Arg Asp Val Asn Leu
           115                 120                 125
   Thr His Gln Leu Asn Pro Lys Val Lys Ser Phe Ser Gln Phe Ile Ser
       130                 135                 140
   Glu Asn Gln Gly Ala Phe Lys Gly Met
   145                 150
<210> 253
   <211> 462
   <212> DNA
   <213> Homo sapien
<400> 253
   atggccagtg tccgcgtggc ggcctacttt gaaaactttc tcgcggcgtg gcggcccgtg      60
   aaagcctctg atggagatta ctacaccttg gctgtaccga tgggagatgt accaatggat     120
   ggtatctctg ttgctgatat tggagcagcc gtctctagca tttttaattc tccagaggaa     180
   tttttaggca aggccgtggg gctcagtgca gaagcactaa caatacagca atatgctgat     240
   gttttgtcca aggctttggg gaaagaagtc cgagatgcaa agattacccc ggaagctttc     300
   gagaagctgg gattccctgc agcaaaggaa atagccaata tgtgtcgttt ctatgaaatg     360
   aagccagacc gagatgtcaa tctcacccac caactaaatc ccaaagtcaa aagcttcagc     420
   cagtttatct cagagaacca gggagccttc aagggcatgt ag                        462
<210> 254
   <211> 8031
   <212> DNA
   <213> Homo sapien
<400> 254
   tggcgaatgg gacgcgccct gtagcggcgc attaagcgcg gcgggtgtgg tggttacgcg      60
   cagcgtgacc gctacacttg ccagcgccct agcgcccgct cctttcgctt tcttcccttc     120
   ctttctcgcc acgttcgccg gctttccccg tcaagctcta aatcgggggc tccctttagg     180
   gttccgattt agtgctttac ggcacctcga ccccaaaaaa cttgattagg gtgatggttc     240
   acgtagtggg ccatcgccct gatagacggt ttttcgccct ttgacgttgg agtccacgtt     300
   ctttaatagt ggactcttgt tccaaactgg aacaacactc aaccctatct cggtctattc     360
   ttttgattta taagggattt tgccgatttc ggcctattgg ttaaaaaatg agctgattta     420
   acaaaaattt aacgcgaatt ttaacaaaat attaacgttt acaatttcag gtggcacttt     480
   tcggggaaat gtgcgcggaa cccctatttg tttatttttc taaatacatt caaatatgta     540
   tccgctcatg aattaattct tagaaaaact catcgagcat caaatgaaac tgcaatttat     600
   tcatatcagg attatcaata ccatattttt gaaaaagccg tttctgtaat gaaggagaaa     660
   actcaccgag gcagttccat aggatggcaa gatcctggta tcggtctgcg attccgactc     720
   gtccaacatc aatacaacct attaatttcc cctcgtcaaa aataaggtta tcaagtgaga     780
   aatcaccatg agtgacgact gaatccggtg agaatggcaa aagtttatgc atttctttcc     840
   agacttgttc aacaggccag ccattacgct cgtcatcaaa atcactcgca tcaaccaaac     900
   cgttattcat tcgtgattgc gcctgagcga gacgaaatac gcgatcgctg ttaaaaggac     960
   aattacaaac aggaatcgaa tgcaaccggc gcaggaacac tgccagcgca tcaacaatat    1020
   tttcacctga atcaggatat tcttctaata cctggaatgc tgttttcccg gggatcgcag    1080
   tggtgagtaa ccatgcatca tcaggagtac ggataaaatg cttgatggtc ggaagaggca    1140
   taaattccgt cagccagttt agtctgacca tctcatctgt aacatcattg gcaacgctac    1200
   ctttgccatg tttcagaaac aactctggcg catcgggctt cccatacaat cgatagattg    1260
   tcgcacctga ttgcccgaca ttatcgcgag cccatttata cccatataaa tcagcatcca    1320
   tgttggaatt taatcgcggc ctagagcaag acgtttcccg ttgaatatgg ctcataacac    1380
   cccttgtatt actgtttatg taagcagaca gttttattgt tcatgaccaa aatcccttaa    1440
   cgtgagtttt cgttccactg agcgtcagac cccgtagaaa agatcaaagg atcttcttga    1500
   gatccttttt ttctgcgcgt aatctgctgc ttgcaaacaa aaaaaccacc gctaccagcg    1560
   gtggtttgtt tgccggatca agagctacca actctttttc cgaaggtaac tggcttcagc    1620
   agagcgcaga taccaaatac tgtccttcta gtgtagccgt agttaggcca ccacttcaag    1680
   aactctgtag caccgcctac atacctcgct ctgctaatcc tgttaccagt ggctgctgcc    1740
   agtggcgata agtcgtgtct taccgggttg gactcaagac gatagttacc ggataaggcg    1800
   cagcggtcgg gctgaacggg gggttcgtgc acacagccca gcttggagcg aacgacctac    1860
   accgaactga gatacctaca gcgtgagcta tgagaaagcg ccacgcttcc cgaagggaga    1920
   aaggcggaca ggtatccggt aagcggcagg gtcggaacag gagagcgcac gagggagctt    1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatataca tatgcagcat caccaccatc accacggagt 5100
   acagcttcaa gacaatgggt ataatggatt gctcattgca attaatcctc aggtacctga 5160
   gaatcagaac ctcatctcaa acattaagga aatgataact gaagcttcat tttacctatt 5220
   taatgctacc aagagaagag tatttttcag aaatataaag attttaatac ctgccacatg 5280
   gaaagctaat aataacagca aaataaaaca agaatcatat gaaaaggcaa atgtcatagt   5340
   gactgactgg tatggggcac atggagatga tccatacacc ctacaataca gagggtgtgg   5400
   aaaagaggga aaatacattc atttcacacc taatttccta ctgaatgata acttaacagc   5460
   tggctacgga tcacgaggcc gagtgtttgt ccatgaatgg gcccacctcc gttggggtgt   5520
   gttcgatgag tataacaatg acaaaccttt ctacataaat gggcaaaatc aaattaaagt   5580
   gacaaggtgt tcatctgaca tcacaggcat ttttgtgtgt gaaaaaggtc cttgccccca   5640
   agaaaactgt attattagta agctttttaa agaaggatgc acctttatct acaatagcac   5700
   ccaaaatgca actgcatcaa taatgttcat gcaaagttta tcttctgtgg ttgaattttg   5760
   taatgcaagt acccacaacc aagaagcacc aaacctacag aaccagatgt gcagcctcag   5820
   aagtgcatgg gatgtaatca cagactctgc tgactttcac cacagctttc ccatgaacgg   5880
   gactgagctt ccacctcctc ccacattctc gcttgtagag gctggtgaca aagtggtctg   5940
   tttagtgctg gatgtgtcca gcaagatggc agaggctgac agactccttc aactacaaca   6000
   agccgcagaa ttttatttga tgcagattgt tgaaattcat accttcgtgg gcattgccag   6060
   tttcgacagc aaaggagaga tcagagccca gctacaccaa attaacagca atgatgatcg   6120
   aaagttgctg gtttcatatc tgcccaccac tgtatcagct aaaacagaca tcagcatttg   6180
   ttcagggctt aagaaaggat ttgaggtggt tgaaaaactg aatggaaaag cttatggctc   6240
   tgtgatgata ttagtgacca gcggagatga taagcttctt ggcaattgct tacccactgt   6300
   gctcagcagt ggttcaacaa ttcactccat tgccctgggt tcatctgcag ccccaaatct   6360
   ggaggaatta tcacgtctta caggaggttt aaagttcttt gttccagata tatcaaactc   6420
   caatagcatg attgatgctt tcagtagaat ttcctctgga actggagaca ttttccagca   6480
   acatattcag cttgaaagta caggtgaaaa tgtcaaacct caccatcaat tgaaaaacac   6540
   agtgactgtg gataatactg tgggcaacga cactatgttt ctagttacgt ggcaggccag   6600
   tggtcctcct gagattatat tatttgatcc tgatggacga aaatactaca caaataattt   6660
   tatcaccaat ctaacttttc ggacagctag tctttggatt ccaggaacag ctaagcctgg   6720
   gcactggact tacaccctga acaataccca tcattctctg caagccctga aagtgacagt   6780
   gacctctcgc gcctccaact cagctgtgcc cccagccact gtggaagcct ttgtggaaag   6840
   agacagcctc cattttcctc atcctgtgat gatttatgcc aatgtgaaac agggatttta   6900
   tcccattctt aatgccactg tcactgccac agttgagcca gagactggag atcctgttac   6960
   gctgagactc cttgatgatg gagcaggtgc tgatgttata aaaaatgatg gaatttactc   7020
   gaggtatttt ttctcctttg ctgcaaatgg tagatatagc ttgaaagtgc atgtcaatca   7080
   ctctcccagc ataagcaccc cagcccactc tattccaggg agtcatgcta tgtatgtacc   7140
   aggttacaca gcaaacggta atattcagat gaatgctcca aggaaatcag taggcagaaa   7200
   tgaggaggag cgaaagtggg gctttagccg agtcagctca ggaggctcct tttcagtgct   7260
   gggagttcca gctggccccc accctgatgt gtttccacca tgcaaaatta ttgacctgga   7320
   agctgtaaaa gtagaagagg aattgaccct atcttggaca gcacctggag aagactttga   7380
   tcagggccag gctacaagct atgaaataag aatgagtaaa agtctacaga atatccaaga   7440
   tgactttaac aatgctattt tagtaaatac atcaaagcga aatcctcagc aagctggcat   7500
   cagggagata tttacgttct caccccaaat ttccacgaat ggacctgaac atcagccaaa   7560
   tggagaaaca catgaaagcc acagaattta tgttgcaata cgagcaatgg ataggaactc   7620
   cttacagtct gctgtatcta acattgccca ggcgcctctg tttattcccc ccaattctga   7680
   tcctgtacct gccagagatt atcttatatt gaaaggagtt ttaacagcaa tgggtttgat   7740
   aggaatcatt tgccttatta tagttgtgac acatcatact ttaagcagga aaaagagagc   7800
   agacaagaaa gagaatggaa caaaattatt ataatgaatt ctgcagatat ccatcacact   7860
   ggcggccgct cgagcaccac caccaccacc actgagatcc ggctgctaac aaagcccgaa   7920
   aggaagctga gttggctgct gccaccgctg agcaataact agcataaccc cttggggcct   7980
   ctaaacgggt cttgaggggt tttttgctga aaggaggaac tatatccgga t            8031

<210> 255
   <211> 401
   <212> DNA
   <213> Homo sapien
<220>
   <221> misc_feature
   <222> (1)...(401)
   <223> n = A,T,C or G
<400> 255
   gtggccagng actagaaggc gaggcgccgc gggaccatgg cggcggcggc ggacgagcgg 60
   agtccanagg acggagaaga cgaggaagag gaggagcagt tggttctggt ggaattatca 120
   ggaattattg attcagactt cctctcaaaa tgtgaaaata aatgcaaggt tttgggcatt 180
   gacactgaga ggcccattct gcaagtggac agctgtgtct ttgctgggga gtatgaagac 240
   actctangga cctgtgttat atttgaagaa aatgntnaac atgctgatac agaaggcaat 300
   aataaaacag tgctaaaata taaatgccat acaatgaaga agctcagcat gacaagaact 360
   ctcctgacag agaagaagga aggagaagaa aacatangtg g 401
<210> 256
   <211> 401
   <212> DNA
   <213> Homo sapien
<220>
   <221> misc_feature
   <222> (1)...(401)
   <223> n = A,T,C or G
<400> 256
   tggtggncct gggatgggga accgcggtgg cttccgngga ggtttcggca ntggcatccg 60
   gggccggggt cgcggccgng gacggggccg gggccnangc cgnnganctc gcggangcaa 120
   ggccgaggat aaggagtgga tgcccgtcac caacttgggc cgcttgncca aggacatgaa 180
   nancaagccc ctgnaggaga tctatntctt cttccctgcc ccattaagga atcaagagat 240
   catttgattt cttcctgggg gcctctctca aggatnaggt ttttgaagat tatgccagtg 300
   canaaannan accccgttgc ccngtccatc tncacccaac ncttccaagg gcnatttttg 360
   tttaggcctc attncngggg ggaaccttaa cccaatttgg g 401
<210> 257
   <211> 401
   <212> DNA
   <213> Homo sapien
<220>
   <221> misc_feature
   <222> (1)...(401)
   <223> n = A,T,C or G
<400> 257
   atgtatgtaa aacacttcat aaaatgtaaa gggctataac aaatatgtta taaagtgatt 60
   ctctcagccc tgaggtatac agaatcattt gcctcagact gctgttggat tttaaaattt 120
   ttaaaatatc tgctaagtaa tttgctatgt cttctcccac actatcaata tgcctgcttc 180
   taacaggctc cccactttct tttaatgtgc tgttatgagc tttggacatg agataaccgt 240
   gcctgttcag agtgtctaca gtaagagctg gacaaactct ggagggacac agtctttgag 300
   acagctcttt tggttgcttt ccacttttct gaaaggttca cagtaacctt ctagataata 360
   gaaactccca gttaaagcct angctancaa ttttttttag t 401
<210> 258
   <211> 401
   <212> DNA
   <213> Homo sapien
<400> 258
   ggagcgctag gtcggtgtac gaccgagatt agggtgcgtg ccagctccgg gaggccgcgg     60
   tgaggggccg ggcccaagct gccgacccga gccgatcgtc agggtcgcca gcgcctcagc    120
   tctgtggagg agcagcagta gtcggagggt gcaggatatt agaaatggct actccccagt    180
   caattttcat ctttgcaatc tgcattttaa tgataacaga attaattctg gcctcaaaaa    240
   gctactatga tatcttaggt gtgccaaaat cggcatcaga gcgccaaatc aagaaggcct    300
   ttcacaagtt ggccatgaag taccaccctg acaaaaataa gacccagatg ctgaagcaaa    360
   attcagagag attgcagaag catatgaaac actctcagat g                        401
<210> 259
   <211> 401
   <212> DNA
   <213> Homo sapien
<400> 259
   attgggtttg gagggaggat gatgacagag gaatgccctt tggccatcac ggttttgatt     60
   ctccagaata ttgtgggttt gatcatcaat gcagtcatgt taggctgcat tttcatgaaa    120
   acagctcagg ctcacagaag ggcagaaact ttgattttca gccgccatgc tgtgattgcc    180
   gtccgaaatg gcaagctgtg cttcatgttc cgagtgggtg acctgaggaa aagcatgatc    240
   attagtgcct ctgtgcgcat ccaggtggtc aagaaaacaa ctacacctga aggggaggtg    300
   gttcctattc accaactgga cattcctgtt gataacccaa tcgagagcaa taacattttt    360
   ctggtggccc ctttgatcat ctgccacgtg attgacaagc g                        401
<210> 260
   <211> 363
   <212> DNA
   <213> Homo sapien
<220>
   <221> misc_feature
   <222> (1)...(363)
   <223> n = A,T,C or G
<400> 260
   aggaganang gagggggana tgaataggga tggagaggga natagtggat gagcagggca     60
   canggagagg aancagaaag gagaggcaag acagggagac acacancaca nangangana    120
   caggtggggg ctggggtggg gcatggagag cctttnangt cncccaggcc accctgctct    180
   cgctggnctg ttgaaaccca ctccatggct tcctgccact gcagttgggc ccagggctgg    240
   cttattnctg gaatgcaagt ggctgtggct tggagcctcc cctctggnnn anggaaannn    300
   attgctccct tatctgcttg gaatatctga gtttttccan cccggaaata aaacacacac    360
   aca                                                                  363
<210> 261
   <211> 401
   <212> DNA
   <213> Homo sapien
<220>
   <221> misc_feature
   <222> (1) ... (401)
   <223> n = A,T,C or G
<400> 261
   cggctctccg ccgctctccc ggggtttcgg ggcacttggg tcccacagtc tggtcctgct     60
   tcaccttccc ctgacctgag tagtcgccat ggcacaggtt ctcagaggca ctgngactga    120
   cttccctgga tttgatgagc gggctgatgc anaaactctt cggaaggcta tgaaaggctt    180
   gggcacagat gaggagagca tcctgactct gttgacatcc cgaagtaatg ctcagcgcca    240
   ggaaatctct gcagctttta agactctgtt tggcagggat cttctggatg acctgaaatc    300
   agaactaact ggaaaatttg aaaaattaat tgtggctctg atgaaaccct ctcggcttta    360
   tgatgcttat gaactgaaac atgccttgaa gggagctgga a                        401
<210> 262
   <211> 401
   <212> DNA
   <213> Homo sapien
<220>
   <221> misc_feature
   <222> (1)... (401)
   <223> n = A,T,C or G
<400> 262
   agtctanaac atttctaata ttttgngctt tcatatatca aaggagatta tgtgaaacta     60
   tttttaaata ctgtaaagtg acatatagtt ataagatata tttctgtaca gtagagaaag    120
   agtttataac atgaagaata ttgtaccatt atacattttc attctcgatc tcataagaaa    180
   ttcaaaagaa taatgataga ggtgaaaata tgtttacttt ctctaaatca agcctagttg    240
   tcaactcaaa aattatgntg catagtttta ttttgaattt aggttttggg actacttttt    300
   tccancttca atgagaaaat aaaatctaca actcaggagt tactacagaa gttctaanta    360
   tttttttgct aannagcnaa aaatataaac atatgaaaat g                         401
<210> 263
   <211> 401
   <212> DNA
   <213> Homo sapien
<220>
   <221> mise feature
   <222> (1)...(401)
   <223> n = A,T,C or G
<400> 263
   ctgtccgacc aagagaggcc ggccgagccc gaggcttggg cttttgcttt ctggcggagg     60
   gatctgcggc ggtttaggag gcggcgctga tcctgggagg aagaggcagc tacggcggcg    120
   gcggcggtgg cggctagggc ggcggcgaat aaaggggccg ccgccgggtg atgcggtgac    180
   cactgcggca ggcccaggag ctgagtgggc cccggccctc agcccgtccc gncggacccg    240
   ctttcctcaa ctctccatct tctcctgccg accgagatcg ccgaggcggn ctcaggctcc    300
   ctancccctt ccccgtccct tccccncccc cgtccccgcc ccgggggccg ccgccacccg    360
   cctcccacca tggctctgaa ganaatccac aaggaattga a                        401
<210> 264
   <211> 401
   <212> DNA
   <213> Homo sapien
<400> 264
   aacaccagcc actccaggac ccctgaaggc ctctaccagg tcaccagtgt tctgcgccta     60
   aagccacccc ctggcagaaa cttcagctgt gtgttctgga atactcacgt gagggaactt    120
   actttggcca gcattgacct tcaaagtcag atggaaccca ggacccatcc aacttggctg    180
   cttcacattt tcatcccctc ctgcatcatt gctttcattt tcatagccac agtgatagcc    240
   ctaagaaaac aactctgtca aaagctgtat tcttcaaaag acacaacaaa aagacctgtc    300
   accacaacaa agagggaagt gaacagtgct gtgaatctga acctgtggtc ttgggagcca    360
   gggtgacctg atatgacatc taaagaagct tctggactct g                        401
<210> 265
   <211> 271
   <212> DNA
   <213> Homo sapien
<220>
   <221> misc_feature
   <222> (1)...(271)
   <223> n = A,T,C or G
<400> 265
   gccacttcct gtggacatgg gcagagcgct gctgccagtt cctggtagcc ttgaccacna     60
   cgctgggggg tctttgtgat ggtcatgggt ctcatttgca cttgggggtg tgggattcaa    120
   gttagaagtt tctagatctg gccgggcgca gtggctcaca cctgtaatcc cagcacttta    180
   ggaggctgag gcaggcggat catgaggtca ggagatcgag accgtcctgg ctaacacagt    240
   gaaaccccgt ctctactaaa aatacaaaaa a                                   271
<210> 266
   <211> 401
   <212> DNA
   <213> Homo sapien
<220>
   <221> misc_feature
   <222> (1)...(401)
   <223> n = A,T,C or G
<400> 266
   attcataaat ttagctgaaa gatactgatt caatttgtat acagngaata taaatgagac     60
   gacagcaaaa ttttcatgaa atgtaaaata tttttatagt ttgttcatac tatatgaggt    120
   tctattttaa atgactttct ggattttaaa aaatttcttt aaatacaatc atttttgtaa    180
   tatttatttt atgcttatga tctagataat tgcagaatat cattttatct gactctgtct    240
   tcataagaga gctgtggccg aattttgaac atctgttata gggagtgatc aaattagaag    300
   gcaatgtgga aaaacaattc tgggaaagat ttctttatat gaagtccctg ccactagcca    360
   gccatcctaa ttgatgaaag ttatctgttc acaggcctgc a                        401
<210> 267
   <211> 401
   <212> DNA
   <213> Homo sapien
<220>
   <221> misc_feature
   <222> (1)...(401)
   <223> n = A,T,C or G
<400> 267
   gaagaggcat cacctgatcc cggagacctt tggagttaag aggcggcgga agcgagggcc     60
   tgtggagtcg gatcctcttc ggggtgagcc agggtcggcg cgcgcggctg tctcanaact    120
   catgcagctg ttcccgcgag gcctgtttga ggacgcgctg ccgcccatcg tgctgaggag    180
   ccaggtgtac agccttgtgc ctgacaggac cgtggccgac cggcagctga aggagcttca    240
   agagcanggg gagacaaaat cgtccagctg ggcttcnact tggatgccca tggaanttat    300
   tctttcnctt ganggactta cnngggaccc aagaanccct tncaaggggc ccttngtgga    360
   tgggncccga aaccccnnta tttgcccttg gggggnccca a                        401
<210> 268
   <211> 223
   <212> DNA
   <213> Homo sapien
<400> 268
   tcgccatgtt ggccaggctg gtcttgaact cctgacttta agtgatccac ccgcctcaac     60
   ctcccaaagt gctgggatta caggtgtgag ccaccgcgcc tggcctgata catactttta    120
   gaatcaagta gtcacgcact ttttctgttc atttttctaa aaagtaaata tacaaatgtt    180
   ttgttttttg ttttttttgt ttgtttgttt ctgttttttt ttt                      223
<210> 269
   <211> 401
   <212> DNA
   <213> Homo sapien
<400> 269
   actatgtaaa ccacattgta ctttttttta ctttggcaac aaatatttat acatacaaga     60
   tgctagttca tttgaatatt tctcccaact tatccaagga tctccagctc taacaaaatg    120
   gtttattttt atttaaatgt caatagttgt tttttaaaat ccaaatcaga ggtgcaggcc    180
   accagttaaa tgccgtctat caggttttgt gccttaagag actacagagt caaagctcat    240
   ttttaaagga gtaggacaaa gttgtcacag gtttttgttg ttgtttttat tgcccccaaa    300
   attacatgtt aatttccatt tatatcaggg attctattta cttgaagact gtgaagttgc    360
   cattttgtct cattgttttc tttgacataa ctaggatcca t                        401
<210> 270
   <211> 401
   <212> DNA
   <213> Homo sapien
<220>
   <221> misc_feature
   <222> (1)...(401)
   <223> n = A,T,C or G
<400> 270
   tggctgttga ttcacctcag cactgcttgg tatctgcacc ctacctctct ttagaggctg      60
   ccttgtcaac tgaaaaatgc acctgacttc gagcaagact ctttccttag gttctggatc     120
   tgtttgagcc ccatggcact gagctggaat ctgagggtct tgttccaagg atgtgatgat     180
   gtgggagaat gttctttgaa agagcagaaa tccagtctgc atggaaacag cctgtagagn     240
   agaagtttcc agtgataagt gttcactgtt ctaaggaggt acaccacagc tacctgaatt     300
   ttcccaaaat gagtgcttct gtgcgttaca actggccttt gtacttgact gtgatgactt     360
   tgtttttcct tttcaattct anatgaacat gggaaaaaat g                         401
<210> 271
   <211> 329
   <212> DNA
   <213> Homo sapien
<400> 271
   ccacagcctc caagtcaggt ggggtggagt cccagagctg cacagggttt ggcccaagtt      60
   tctaagggag gcacttcctc ccctcgccca tcagtgccag cccctgctgg ctggtgcctg     120
   agcccctcag acagccccct gccccgcagg cctgccttct cagggacttc tgcggggcct     180
   gaggcaagcc atggagtgag acccaggagc cggacacttc tcaggaaatg gcttttccca     240
   acccccagcc cccacccggt ggttcttcct gttctgtgac tgtgtatagt gccaccacag     300
   cttatggcat ctcattgagg acaaaaaaa                                       329
<210> 272
   <211> 401
   <212> DNA
   <213> Homo sapien
<220>
   <221> misc_feature
   <222> (1)...(401)
   <223> n = A,T,C or G
<400> 272
   nggctgntaa cntcggaggt nacttcctgg actatcctgg agaccccctc cgcttccacg      60
   nncatnatat cnctcatngc tgggcccntn angacacnat cccactccaa cacctgngng     120
   atgctggncn cctnggaacc ancntcagaa ngaccctgnt cntntgtnnt ccgcaanctg     180
   aagnnaangc gggntacacc tncntgcant ggnccacnct gcngggaact ntacacacct     240
   acgggatgtg gctgcgccan gagccaagag cntttctgga tgattcccca gcctcttgnn     300
   agggantcta caacattgct nnntaccttt ntccnncngc nnntnntgga ntacaggngn     360
   tnntaacact acatcttttt tactgcnccn tncttggtgg g                         401
<210> 273
   <211> 401
   <212> DNA
   <213> Homo sapien
<220>
   <221> mise feature
   <222> (1) ... (401)
   <223> n = A,T,C or G
<400> 273
   cagcaccatg aagatcaaga tcatcgcacc cccagagcgc aagtactcgg tgtggatcgg     60
   tggctccatc ctggcctcac tgtccacctt ccagcagatg tggattagca agcaggagta    120
   cgacgagtcg ggcccctcca tcgtccaccg caaatgcttc taaacggact cagcagatgc    180
   gtagcatttg ctgcatgggt taattgagaa tagaaatttg cccctggcaa atgcacacac    240
   ctcatgctag cctcacgaaa ctggaataag ccttcgaaaa gaaattgtcc ttgaagcttg    300
   tatctgatat cagcactgga ttgtagaact tgttgctgat tttgaccttg tattgaagtt    360
   aactgttccc cttggtatta acgtgtcagg gctgagtgnt c                        401
<210> 274
   <211> 401
   <212> DNA
   <213> Homo sapien
<400> 274
   ccacccacac ccaccgcgcc ctcgttcgcc tcttctccgg gagccagtcc gcgccaccgc     60
   cgccgcccag gccatcgcca ccctccgcag ccatgtccac caggtccgtg tcctcgtcct    120
   cctaccgcag gatgttcggc ggcccgggca ccgcgagccg gccgagctcc agccggagct    180
   acgtgactac gtccacccgc acctacagcc tgggcagcgc gctgcgcccc agcaccagcc    240
   gcagcctcta cgcctcgtcc ccgggcggcg tgtatgccac gcgctcctct gccgtgcgcc    300
   tgcggagcag cgtgcccggg gtgcggctcc tgcaggactc ggtggacttc tcgctggccg    360
   acgccatcaa caccgagttc aagaacaccc gcaccaacga g                        401
<210> 275
   <211> 401
   <212> DNA
   <213> Homo sapien
<400> 275
   ccacttccac cactttgtgg agcagtgcct tcagcgcaac ccggatgcca ggtatccctg     60
   ctggcctggg cctgggcttc gggagagcag agggtgctca ggagggtaag gccagggtgt    120
   gaagggactt acctcccaaa ggttctgcag gggaatctgg agctacacac aggagggatc    180
   agctcctggg tgtgtcagag gccagcctgg ggagctctgg ccactgcttc ccatgagctg    240
   agggagaggg agaggggacc cgaggctgag gcataagtgg caggatttcg ggaagctggg    300
   gacacggcag tgatgctgcg gtctctcctc ccctttccct ccaggcccag tgccagcacc    360
   ctcctgaacc actctttctt caagcagatc aagcgacgtg c                        401
<210> 276
   <211> 401
   <212> DNA
   <213> Homo sapien
<220>
   <221> misc_feature
   <222> (1)...(401)
   <223> n = A,T,C or G
<400> 276
   tctgatattg ntacccttga gccacctaag ttagaagaaa ttggaaatca agaagttgtc      60
   attgttgaag aagcacagag ttcagaagac tttaacatgg gctcttcctc tagcagccag     120
   tatactttct gtcagccaga aactgtattt tcatctcagc ctagtgatga tgaatcaagt     180
   agtgatgaaa ccagtaatca gcccagtcct gcctttagac gacgccgtgc taggaagaag     240
   accgtttctg cttcagaatc tgaagaccgg ctagttggtg aacaagaaac tgaaccttct     300
   aaggagttga gtaaacgtca gttcagtagt ggtctcaata agtgtgttat acttgctttg     360
   gtgattgcaa tcagcatggg atttggccat ttctatggca c                         401
<210> 277
   <211> 401
   <212> DNA
   <213> Homo sapien
<220>
   <221> misc_feature
   <222> (1)...(401)
   <223> n = A,T,C or G
<400> 277
   aactttggca acatatctca gcaaaaacta cagctatgtt attcatgcca aaataaaagc      60
   tgtgcagagg agtggctgca atgaggtcac aacggtggtg gatgtaaaag agatcttcaa     120
   gtcctcatca cccatccctc gaactcaagt cccgctcatt acaaattctt cttgccagtg     180
   tccacacatc ctgccccatc aagatgttct catcatgtgt tacgagnggc gctcaaggat     240
   gatgcttctt gaaaattgct tagttgaaaa atggagagat cagcttagta aaagatccat     300
   acagtgggaa gagaggctgc aggaacagcg ganaacagtt caggacaaga agaaaacagc     360
   cgggcgcacc agtcgtagta atccccccaa accaaaggga a                         401
<210> 278
   <211> 401
   <212> DNA
   <213> Homo sapien
<220>
   <221> misc_feature
   <222> (1)...(401)
   <223> n = A,T,C or G
<400> 278
   aatgagtgtg agaccacaaa tgaatgccgg gaggatgaaa tgtgttggaa ttatcatggc      60
   ggcttccgtt gttatccacg aaatccttgt caagatccct acattctaac accagagaac     120
   cgatgtgttt gcccagtctc aaatgccatg tgccgagaac tgccccagtc aatagtctac     180
   aaatacatga gcatccgatc tgataggtct gtgccatcag acatcttcca gatacaggcc     240
   acaactattt atgccaacac catcaatact tttcggatta aatctggaaa tgaaaatgga     300
   gagtctacct acgacaacaa anccctgtaa gtgcaatgct tgtgctcgtg aagncattat     360
   caggaccaag agaacatatc gtggacctgg agatgctgac a                         401
<210> 279
   <211> 401
   <212> DNA
   <213> Homo sapien
<220>
   <221> misc_feature
   <222> (1)...(401)
   <223> n = A,T,C or G
<400> 279
   aaattattgc ctctgataca tacctaagtn aacanaacat taatacctaa gtaaacataa      60
   cattacttgg agggttgcag nttctaantg aaactgtatt tgaaactttt aagtatactt     120
   taggaaacaa gcatgaacgg cagtctagaa taccagaaac atctacttgg gtagcttggn     180
   gccattatcc tgtggaatct gatatgtctg gnagcatgtc attgatggga catgaagaca     240
   tctttggaaa tgatgagatt atttcctgtg ttaaaaaaaa aaaaaatctt aaattcctac     300
   aatgtgaaac tgaaactaat aattttgatc ctgatgtatg ggacagcgta tctgtaccag     360
   gctctaaata acaaaagnta gggngacaag nacatgttcc t                         401
<210> 280
   <211> 326
   <212> DNA
   <213> Homo sapien
<400> 280
   gaagtggaat tgtataattc aattcgataa ttgatctcat gggctttccc tggaggaaag      60
   gttttttttg ttgttttttt tttaagaact tgaaacttgt aaactgagat gtctgtagct     120
   tttttgccca tctgtagtgt atgtgaagat ttcaaaacct gagagcactt tttctttgtt     180
   tagaattatg agaaaggcac tagatgactt taggatttgc atttttccct ttattgcctc     240
   atttcttgtg acgccttgtt ggggagggaa atctgtttat tttttcctac aaataaaaag     300
   ctaagattct atatcgcaaa aaaaaa                                          326
<210> 281
   <211> 374
   <212> DNA
   <213> Homo sapien
<400> 281
   caacgcgttt gcaaatattc ccctggtagc ctacttcctt acccccgaat attggtaaga      60
   tcgagcaatg gcttcaggac atgggttctc ttctcctgtg atcattcaag tgctcactgc     120
   atgaagactg gcttgtctca gtgtttcaac ctcaccaggg ctgtctcttg gtccacacct     180
   cgctccctgt tagtgccgta tgacagcccc catcaaatga ccttggccaa gtcacggttt     240
   ctctgtggtc aaggttggtt ggctgattgg tggaaagtag ggtggaccaa aggaggccac     300
   gtgagcagtc agcaccagtt ctgcaccagc agcgcctccg tcctagtggg tgttcctgtt     360
   tctcctggcc ctgg                                                       374
<210> 282
   <211> 404
   <212> DNA
   <213> Homo sapien
<220>
   <221> misc_feature
   <222> (1)...(404)
   <223> n = A,T,C or G
<400> 282
   agtgtggtgg aattcccgca tcctanncgc cgactcacac aaggcagagt ngccatggag      60
   aaaattccag tgtcagcatt cttgctcctt gtggccctct cctacactct ggccagagat     120
   accacagtca aacctgnagc caaaaaggac acaaaggact ctcgacccaa actgccccan     180
   accctctcca gaggttgggg tgaccaactc atctggactc anacatatga agaagctcta     240
   tataaatcca agacaagcaa caaacccttg atgattattc atcacttgga tgagtgccca     300
   cacagtcaag ctttaaagaa agtgtttgct gaaaataaag aaatccagaa attggcagag     360
   cagtttgtcc tcctcaatct ggtttatgaa acaactgaca aaca                      404
<210> 283
   <211> 184
   <212> DNA
   <213> Homo sapien
<220>
   <221> misc_feature
   <222> (1)...(184)
   <223> n = A,T,C or G
<400> 283
   agtgtggtgg aattcacttg cttaanttgt gggcaaaaga gaaaaagaag gattgatcag      60
   agcattgtgc aatacagttt cattaactcc ttccctcgct cccccaaaaa tttgaatttt     120
   tttttcaaca ctcttacacc tgttatggaa aatgtcaacc tttgtaagaa aaccaaaata     180
   aaaa                                                                  184
<210> 284
   <211> 421
   <212> DNA
   <213> Homo sapien
<220>
   <221> misc_feature
   <222> (1)...(421)
   <223> n = A,T,C or G
<400> 284
   ctattaatcc tgccacaata tttttaatta cgtacaaaga tctgacatgt cacccaggga      60
   cccatttcac ccactgctct gtttggccgc cagtcttttg tctctctctt cagcaatggt     120
   gaggcggata ccctttcctc ggggaanana aatccatggt ttgttgccct tgccaataac     180
   aaaaatgttg gaaagtcgag tggcaaagct gttgccattg gcatctttca cgtgaaccac     240
   gtcaaaagat ccagggtgcc tctctctgtt ggtgatcaca ccaattcttc ctaggttagc     300
   acctccagtc accatacaca ggttaccagt gtcgaacttg atgaaatcag taatcttgcc     360
   agtctctaaa tcaatctgaa tggtatcatt caccttgatg aggggatcgg ggtagcggat     420
   g                                                                     421
<210> 285
   <211> 361
   <212> DNA
   <213> Homo sapien
<220>
   <221> misc_feature
   <222> (1)...(361)
   <223> n = A,T,C or G
<400> 285
   ctgggtggta actctttatt tcattgtccg gaanaaagat gggagtggga acagggtgga      60
   cactgtgcag gcttcagctt ccactccggg caggattcag gctatctggg accgcaggga     120
   ctgccaggtg cacagccctg gctcccgagg caggcaggca aggtgacggg actggaagcc     180
   cttttcanag ccttggagga gctggtccgt ccacaagcaa tgagtgccac tctgcagttt     240
   gcaggggatg gataaacagg gaaacactgt gcattcctca cagccaacag tgtaggtctt     300
   ggtgaagccc cggcgctgag ctaagctcag gctgttccag ggagccacga aactgcaggt     360
   a                                                                     361
<210> 286
   <211> 336
   <212> DNA
   <213> Homo sapien
<220>
   <221> misc_feature
   <222> (1)...(336)
   <223> n = A,T,C or G
<400> 286
   tttgagtggc agcgccttta tttgtggggg ccttcaaggn agggtcgtgg ggggcagcgg      60
   ggaggaanag ccganaaact gtgtgaccgg ggcctcaggt ggtgggcatt gggggctcct     120
   cttgcanatg cccattggca tcaccggtgc agccattggt ggcagcgggt accggtcctt     180
   tcttgttcaa catagggtag gtggcagcca cgggtccaac tcgcttgagg ctgggccctg     240
   ggcgctccat tttgtgttcc angagcatgt ggttctgtgg cgggagcccc acgcaggccc     300
   tgaggatgtt ctcgatgcag ctgcgctggc ggaaaa                               336
<210> 287
   <211> 301
   <212> DNA
   <213> Homo sapien
<220>
   <221> misc_feature
   <222> (1)...(301)
   <223> n = A,T,C or G
<400> 287
   tgggtaccaa atttntttat ttgaaggaat ggnacaaatc aaanaactta agnggatgtt      60
   ttggtacaac ttatanaaaa ggnaaaggaa accccaacat gcatgcnctg ccttggngac     120
   cagggaagtc accccacggc tatggggaaa ttancccgag gcttancttt cattatcact     180
   gtctcccagg gngngcttgt caaaaanata ttccnccaag ccaaattcgg gcgctcccat     240
   nttgcncaag ttggtcacgt ggtcacccaa ttctttgatg gctttcacct gctcattcag     300
   g                                                                     301
<210> 288
   <211> 358
   <212> DNA
   <213> Homo sapien
<220>
   <221> mise feature
   <222> (1)...(358)
   <223> n = A,T,C or G
<400> 288
   aagtttttaa actttttatt tgcatattaa aaaaattgng cattccaata attaaaatca      60
   tttgaacaaa aaaaaaaatg gcactctgat taaactgcat tacagcctgc aggacacctt     120
   gggccagctt ggttttactc tanatttcac tgtcgtccca ccccacttct tccaccccac     180
   ttcttccttc accaacatgc aagttctttc cttccctgcc agccanatag atagacagat     240
   gggaaaggca ggcgcggcct tcgttgtcag tagttctttg atgtgaaagg ggcagcacag     300
   tcatttaaac ttgatccaac ctctttgcat cttacaaagt taaacagcta aaagaagt       358
<210> 289
   <211> 462
   <212> DNA
   <213> Homo sapien
<220>
   <221> misc_feature
   <222> (1)...(462)
   <223> n = A,T,C or G
<400> 289
   ggcatcagaa atgctgttta tttctctgct gctcccaagc tggctggcct ttgcagagga      60
   gcagacaaca gatgcatagt tgggganaaa gggaggacag gttccaggat agagggtgca     120
   ggctgaggga ggaagggtaa naggaaggaa ggccatcctg gatccccaca tttcagtctc     180
   anatgaggac aaagggactc ccaagccccc aaatcatcan aaaacaccaa ggagcaggag     240
   gagcttgagc aggccccagg gagcctcana gccataccag ccactgtcta cttcccatcc     300
   tcctctccca ttccctgtct gcttcanacc acctcccagc taagccccag ctccattccc     360
   ccaatcctgg cccttgccag cttgacagtc acagtgcctg gaattccacc actgaggctt     420
   ctcccagttg gattaggacg tcgccctgtt agcatgctgc cc                        462
<210> 290
   <211> 481
   <212> DNA
   <213> Homo sapien
<220>
   <221> misc_feature
   <222> (1)...(481)
   <223> n = A,T,C or G
<400> 290
   tactttccta aactttatta aagaaaaaag caataagcaa tggnggtaaa tctctanaac 60
   atacccaatt ttctgggctt cctcccccga gaatgtgaca ttttgatttc caaacatgcc 120
   anaagtgtat ggttcccaac tgtactaaag taggtganaa gctgaagtcc tcaagtgttc 180
   atcttccaac ttttcccagt ctgtggtctg tctttggatc agcaataatt gcctgaacag 240
   ctactatggc ttcgttgatt tttgtctgta gctctctgag ctcctctatg tgcagcaatc 300
   gcanaatttg agcagcttca ttaanaactg catctcctgt gtcaaaacca anaatatgtt 360
   tgtctaaagc aacaggtaag ccctcttttg tttgatttgc cttancaact gcatcctgtg 420
   tcaggcgctc ctgaaccaaa atccgaattg ccttaagcat taccaggtaa tcatcatgac 480
   g 481
<210> 291
   <211> 381
   <212> DNA
   <213> Homo sapien
<220>
   <221> misc_feature
   <222> (1)...(381)
   <223> n = A,T,C or G
<400> 291
   tcatagtaat gtaaaaccat ttgtttaatt ctaaatcaaa tcactttcac aacagtgaaa 60
   attagtgact ggttaaggng tgccactgta catatcatca ttttctgact ggggtcagga 120
   cctggtccta gtccacaagg gtggcaggag gagggtggag gctaanaaca cagaaaacac 180
   acaaaanaaa ggaaagctgc cttggcanaa ggatgaggng gtgagcttgc cgaaggatgg 240
   tgggaagggg gctccctgtt ggggccgagc caggagtccc aagtcagctc tcctgcctta 300
   cttagctcct ggcanagggt gagtggggac ctacgaggtt caaaatcaaa tggcatttgg 360
   ccagcctggc tttactaaca g 381
<210> 292
   <211> 371
   <212> DNA
   <213> Homo sapien
<220>
   <221> misc_feature
   <222> (1)...(371)
   <223> n = A,T,C or G
<400> 292
   gaaaaaataa tccgtttaat tgaaaaacct gnaggatact attccactcc cccanatgag 60
   gaggctgagg anaccaaacc cctacatcac ctcgtagcca cttctgatac tcttcacgag 120
   gcagcaggca aagacaattc ccaaaacctc nacaaaagca attccaaggg ctgctgcagc 180
   taccaccanc acatttttcc tcagccagcc cccaatcttc tccacacagc cctccttatg 240
   gatcgccttc tcgttgaaat taatcccaca gcccacagta acattaatgc ancaggagtc 300
   ggggactcgg ttcttcgaca tggaagggat tttctcccaa tctgtgtagt tagcagcccc 360
   acagcactta a 371
<210> 293
   <211> 361
   <212> DNA
   <213> Homo sapien
<220>
   <221> misc_feature
   <222> (1)...(361)
   <223> n = A,T,C or G
<400> 293
   gatttaaaag aaaacacttt attgttcagc aattaaaagt tagccaaata tgtatttttc     60
   tccataattt attgngatgt tatcaacatc aagtaaaatg ctcattttca tcatttgctt    120
   ctgttcatgt tttcttgaac acgtcttcaa ttttccttcc aaaatgctgc atgccacact    180
   tgaggtaacg aagcanaagt atttttaaac atgacagcta anaacattca tctacagcaa    240
   cctatatgct caatacatgc cgcgtgatcc tagtagtttt ttcacaacct tctacaagtt    300
   tttggaaaac atctgttatg atgactttca tacaccttca cctcaaaggc tttcttgcac    360
   c                                                                    361
<210> 294
   <211> 391
   <212> DNA
   <213> Homo sapien
<220>
   <221> misc_feature
   <222> (1)...(391)
   <223> n = A,T,C or G
<400> 294
   tattttaaag tttaattatg attcanaaaa aatcgagcga ataactttct ctgaaaaaat     60
   atattgactc tgtatanacc acagttattg gggganaagg gctggtaggt taaattatcc    120
   tattttttat tctgaaaatg atattaatan aaagtcccgt ttccagtctg attataaaga    180
   tacatatgcc caaaatggct ganaataaat acaacaggaa atgcaaaagc tgtaaagcta    240
   agggcatgca ananaaaatc tcanaatacc caaagnggca acaaggaacg tttggctgga    300
   atttgaagtt atttcagtca tctttgtctt tggctccatg tttcaggatg cgtgtgaact    360
   cgatgtaatt gaaattcccc tttttatcaa t                                  391
<210> 295
   <211> 343
   <212> DNA
   <213> Homo sapien
<220>
   <221> mise feature
   <222> (1)...(343)
   <223> n = A,T,C or G
<400> 295
   ttcttttgtt ttattgataa cagaaactgt gcataattac agatttgatg aggaatctgc     60
   aaataataaa gaatgtgtct actgccagca aaatacaatt attccatgcc ctctcaacat    120
   acaaatatag agttcttcac accanatggc tctggtgtaa caaagccatt ttanatgttt    180
   aattgtgctt ctacaaaacc ttcanagcat gaggtagttt cttttaccta cnatattttc    240
   cacatttcca ttattacact tttagtgagc taaaatcctt ttaacatagc ctgcggatga    300
   tctttcacaa aagccaagcc tcatttacaa agggtttatt tct                      343
<210> 296
   <211> 241
   <212> DNA
   <213> Homo sapien
<220>
   <221> misc_feature
   <222> (1)...(241)
   <223> n = A,T,C or G
<400> 296
   ttcttggata ttggttgttt ttgtgaaaaa gtttttgttt ttcttctcag tcaactgaat      60
   tatttctcta ctttgccctc ctgatgccca catgananaa cttaanataa tttctaacag     120
   cttccacttt ggaaaaaaaa aaaacctgtt ttcctcatgg aaccccagga gttgaaagtg     180
   gatanatcgc tctcaaaatc taaggctctg ttcagcttta cattatgtta cctgacgttt     240
   t                                                                     241
<210> 297
   <211> 391
   <212> DNA
   <213> Homo sapien
<220>
   <221> misc_feature
   <222> (1) ... (391)
   <223> n = A,T,C or G
<400> 297
   gttgtggctg anaatgctgg agatgctcag ttctctccct cacaaggtag gccacaaatt      60
   cttggtggtg ccctcacatc tggggtcttc aggcaccagc catgcctgcc gaggagtgct     120
   gtcaggacan accatgtccg tgctaggccc aggcacagcc caaccactcc tcatccaagt     180
   ctctcccagg tttctggtcc cgatgggcaa ggatgacccc tccagtggct ggtaccccac     240
   catcccacta cccctcacat gctctcactc tccatcaggt ccccaatcct ggcttccctc     300
   ttcacgaact ctcaaagaaa aggaaggata aaacctaaat aaaccagaca gaagcagctc     360
   tggaaaagta caaaaagaca gccagaggtg t                                    391
<210> 298
   <211> 321
   <212> DNA
   <213> Homo sapien
<220>
   <221> misc_feature
   <222> (1)...(321)
   <223> n = A,T,C or G
<400> 298
   caagccaaac tgtntccagc tttattaaan atactttcca taaacaatca tggtatttca      60
   ggcaggacat gggcanacaa tcgttaacag tatacaacaa ctttcaaact cccttnttca   120
   atggactacc aaaaatcaaa aagccactat aaaacccaat gaagtcttca tctgatgctc     180
   tgaacaggga aagtttaaag ngagggttga catttcacat ttagcatgtt gtttaacaac     240
   ttttcacaag ccgaccctga ctttcaggaa gtgaaatgaa aatggcanaa tttatctgaa     300
   natccacaat ctaaaaatgg a                                               321
<210> 299
   <211> 401
   <212> DNA
   <213> Homo sapien
<220>
   <221> misc_feature
   <222> (1) ... (401)
   <223> n = A,T,C or G
<400> 299
   tatcataaag agtgttgaag tttatttatt atagcaccat tgagacattt tgaaattgga      60
   attggtaaaa aaataaaaca aaaagcattt gaattgtatt tggnggaaca gcaaaaaaag     120
   agaagtatca tttttctttg tcaaattata ctgtttccaa acattttgga aataaataac     180
   tggaattttg tcggtcactt gcactggttg acaagattag aacaagagga acacatatgg     240
   agttaaattt tttttgttgg gatttcanat agagtttggt ttataaaaag caaacagggc     300
   caacgtccac accaaattct tgatcaggac caccaatgtc atagggngca atatctacaa     360
   taggtagtct cacagccttg cgtgttcgat attcaaagac t                         401
<210> 300
   <211> 188
   <212> DNA
   <213> Homo sapien
<220>
   <221> misc_feature
   <222> (1)...(188)
   <223> n = A,T,C or G
<400> 300
   tgaatgcttt gtcatattaa gaaagttaaa gtgcaataat gtttgaanac aataagtggt      60
   ggtgtatctt gtttctaata agataaactt ttttgtcttt gctttatctt attagggagt     120
   tgtatgtcag tgtataaaac atactgtgtg gtataacagg cttaataaat tctttaaaag     180
   gaaaaaaa                                                              188
<210> 301
   <211> 291
   <212> DNA
   <213> Homo sapien
<400> 301
   aagattttgt tttattttat tatggctaga aagacactgt tatagccaaa atcggcaatg      60
   acactaaaga aatcctctgt gcttttcaat atgcaaatat atttcttcca agagttgccc     120
   tggtgtgact tcaagagttc atgttaactt cttttctgga aacttccttt tcttagttgt     180
   tgtattcttg aagagcctgg gccatgaaga gcttgcctaa gttttgggca gtgaactcct     240
   tgatgttctg gcagtaagtg tttatctggc ctgcaatgag cagcgagtcc a              291
<210> 302
   <211> 341
   <212> DNA
   <213> Homo sapien
<220>
   <221> misc_feature
   <222> (1)...(341)
   <223> n = A,T,C or G
<400> 302
   tgatttttca taattttatt aaatnatcac tgggaaaact aatggttcgc gtatcacaca      60
   attacactac aatctgatag gagtggtaaa accagccaat ggaatccagg taaagtacaa     120
   aaacgccacc ttttattgtc ctgtcttatt tctcgggaag gagggttcta ctttacacat     180
   ttcatgagcc agcagtggac ttgagttaca atgtgtaggt tccttgtggt tatagctgca     240
   gaagaagcca tcaaattctt gaggacttga catctctcgg aaagaagcaa actagtggat     300
   cccccgggct gcaggaattc gatatcaagc ttatcgatac c                         341
<210> 303
   <211> 361
   <212> DNA
   <213> Homo sapien
<220>
   <221> mise feature
   <222> (1)...(361)
   <223> n = A,T,C or G
<400> 303
   tgcagacagt aaatnaattt tatttgngtt cacagaacat actaggcgat ctcgacagtc      60
   gctccgtgac agcccaccaa cccccaaccc tntacctcgc agccacccta aaggcgactt     120
   caanaanatg gaaggatctc acggatctca ttcctaatgg tccgccgaag tctcacacag    180
   tanacagacg gagttganat gctggaggat gcagtcacct cctaaactta cgacccacca     240
   ccanacttca tcccagccgg gacgtcctcc cccacccgag tcctccccat ttcttctcct     300
   actttgccgc agttccaggn gtcctgcttc caccagtccc acaaagctca ataaatacca     360
   a                                                                     361
<210> 304
   <211> 301
   <212> DNA
   <213> Homo sapien
<220>
   <221> misc_feature
   <222> (1)...(301)
   <223> n = A,T,C or G
<400> 304
   ctctttacaa cagcctttat ttncggccct tgatcctgct cggatgctgg tggaggccct      60
   tagctccgcc cgccaggctc tgtgccgcct ccccgcaggc gcanattcat gaacacggtg     120
   ctcaggggct tgaggccgta ctcccccagc gggagctggt cctccagggg cttcccctcg     180
   aaggtcagcc anaacaggtc gtcctgcaca ccctccagcc cgctcacttg ctgcttcagg     240
   tgggccacgg tctgcgtcag ccgcacctcg taggtgctgc tgcggccctt gttattcctc     300
   a                                                                     301
<210> 305
   <211> 331
   <212> DNA
   <213> Homo sapien
<220>
   <221> mise feature
   <222> (1)...(331)
   <223> n = A,T,C or G
<400> 305
   ganaggctag taacatcagt tttattgggt tggggnggca accatagcct ggctgggggn      60
   ggggctggcc ctcacaggtt gttgagttcc agcagggtct ggtccaaggt ctggtgaatc     120
   tcgacgttct cctccttggc actggccaag gtctcttcta ggtcatcgat ggttttctcc     180
   aactttgcca canacctctc ggcaaactct gctcgggtct cancctcctt cagcttctcc    240
   tccaacagtt tgatctcctc ttcatattta tcttctttgg gggaatactc ctcctctgag     300
   gccatcaggg acttgagggc ctggtccatg g                                    331
<210> 306
   <211> 457
   <212> DNA
   <213> Homo sapien
<400> 306
   aatatgtaaa ggtaataact tttattatat taaagacaat gcaaacgaaa aacagaattg      60
   agcagtgcaa aatttaaagg actgttttgt tctcaaagtt gcaagtttca aagccaaaag     120
   aattatatgt atcaaatata taagtaaaaa aaagttagac tttcaagcct gtaatcccag     180
   cactttggga ggctgaggca ggtggatcac taacattaaa aagacaacat tagattttgt     240
   cgatttatag caattttata aatatataac tttgtcactt ggatcctgaa gcaaaataat     300
   aaagtgaatt tgggattttt gtacttggta aaaagtttaa caccctaaat tcacaactag     360
   tggatccccc gggctgcagg aattcgatat caagcttatc gataccgtcg acctcgaggg     420
   ggggcccggt acccaattcg ccctatagtg agtcgta                              457
<210> 307
   <211> 491
   <212> DNA
   <213> Homo sapien
<400> 307
   gtgcttggac ggaacccggc gctcgttccc caccccggcc ggccgcccat agccagccct      60
   ccgtcacctc ttcaccgcac cctcggactg ccccaaggcc cccgccgccg ctccagcgcc     120
   gcgcagccac cgccgccgcc gccgcctctc cttagtcgcc gccatgacga ccgcgtccac     180
   ctcgcaggtg cgccagaact accaccagga ctcagaggcc gccatcaacc gccagatcaa     240
   cctggagctc tacgcctcct acgtttacct gtccatgtct tactactttg accgcgatga     300
   tgtggctttg aagaactttg ccaaatactt tcttcaccaa tctcatgagg agagggaaca     360
   tgctgagaaa ctgatgaagc tgcagaacca acgaggtggc cgaatcttcc ttcaggatat     420
   caagaaacca gactgtgatg actgggagag cgggctgaat gcaatggagt gtgcattaca     480
   tttggaaaaa a                                                          491
<210> 308
   <211> 421
   <212> DNA
   <213> Homo sapien
<400> 308
   ctcagcgctt cttctttctt ggtttgatcc tgactgctgt catggcgtgc cctctggaga      60
   aggccctgga tgtgatggtg tccaccttcc acaagtactc gggcaaagag ggtgacaagt     120
   tcaagctcaa caagtcagaa ctaaaggagc tgctgacccg ggagctgccc agcttcttgg     180
   ggaaaaggac agatgaagct gctttccaga agctgatgag caacttggac agcaacaggg     240
   acaacgaggt ggacttccaa gagtactgtg tcttcctgtc ctgcatcgcc atgatgtgta     300
   acgaattctt tgaaggcttc ccagataagc agcccaggaa gaaatgaaaa ctcctctgat     360
   gtggttgggg ggtctgccag ctggggccct ccctgtcgcc agtgggcact tttttttttc     420
   c                                                                     421
<210> 309
   <211> 321
   <212> DNA
   <213> Homo sapien
<400> 309
   accaaatggc ggatgacgcc ggtgcagcgg gggggcccgg gggccctggt ggccctggga      60
   tggggaaccg cggtggcttc cgcggaggtt tcggcagtgg catccggggc cggggtcgcg     120
   gccgtggacg gggccggggc cgaggccgcg gagctcgcgg aggcaaggcc gaggataagg     180
   agtggatgcc cgtcaccaag ttgggccgct tggtcaagga catgaagatc aagtccctgg     240
   aggagatcta tctcttctcc ctgcccatta aggaatcaga gatcattgat ttcttcctgg     300
   gggcctctct caaggatgag g                                               321
<210> 310
   <211> 381
   <212> DNA
   <213> Homo sapien
<400> 310
   ttaaccagcc atattggctc aataaatagc ttcggtaagg agttaatttc cttctagaaa      60
   tcagtgccta tttttcctgg aaactcaatt ttaaatagtc caattccatc tgaagccaag     120
   ctgttgtcat tttcattcgg tgacattctc tcccatgaca cccagaaggg gcagaagaac     180
   cacatttttc atttatagat gtttgcatcc tttgtattaa aattattttg aaggggttgc     240
   ctcattggat ggcttttttt tttttcctcc agggagaagg ggagaaatgt acttggaaat     300
   taatgtatgt ttacatctct ttgcaaattc ctgtacatag agatatattt tttaagtgtg     360
   aatgtaacaa catactgtga a                                               381
<210> 311
   <211> 538
   <212> DNA
   <213> Homo sapien
<400> 311
   tttgaattta caccaagaac ttctcaataa aagaaaatca tgaatgctcc acaatttcaa 60
   cataccacaa gagaagttaa tttcttaaca ttgtgttcta tgattatttg taagaccttc 120
   accaagttct gatatctttt aaagacatag ttcaaaattg cttttgaaaa tctgtattct 180
   tgaaaatatc cttgttgtgt attaggtttt taaataccag ctaaaggatt acctcactga 240
   gtcatcagta ccctcctatt cagctcccca agatgatgtg tttttgctta ccctaagaga 300
   ggttttcttc ttatttttag ataattcaag tgcttagata aattatgttt tctttaagtg 360
   tttatggtaa actcttttaa agaaaattta atatgttata gctgaatctt tttggtaact 420
   ttaaatcttt atcatagact ctgtacatat gttcaaatta gctgcttgcc tgatgtgtgt 480
   atcatcggtg ggatgacaga acaaacatat ttatgatcat gaataatgtg ctttgtaa 538
<210> 312
   <211> 176
   <212> DNA
   <213> Homo sapien
<400> 312
   ggaggagcag ctgagagata gggtcagtga atgcggttca gcctgctacc tctcctgtct 60
   tcatagaacc attgccttag aattattgta tgacacgttt tttgttggtt aagctgtaag 120
   gttttgttct ttgtgaacat gggtattttg aggggagggt ggagggagta gggaag 176
<210> 313
   <211> 396
   <212> DNA
   <213> Homo sapien
<400> 313
   ccagcacccc caggccctgg gggacctggg ttctcagact gccaaagaag ccttgccatc 60
   tggcgctccc atggctcttg caacatctcc ccttcgtttt tgagggggtc atgccggggg 120
   agccaccagc ccctcactgg gttcggagga gagtcaggaa gggccaagca cgacaaagca 180
   gaaacatcgg atttggggaa cgcgtgtcaa tcccttgtgc cgcagggctg ggcgggagag 240
   actgttctgt tccttgtgta actgtgttgc tgaaagacta cctcgttctt gtcttgatgt 300
   gtcaccgggg caactgcctg ggggcgggga tgggggcagg gtggaagcgg ctccccattt 360
   tataccaaag gtgctacatc tatgtgatgg gtgggg 396
<210> 314
   <211> 311
   <212> DNA
   <213> Homo sapien
<400> 314
   cctcaacatc ctcagagagg actggaagcc agtccttacg ataaactcca taatttatgg 60
   cctgcagtat ctcttcttgg agcccaaccc cgaggaccca ctgaacaagg aggccgcaga 120
   ggtcctgcag aacaaccggc ggctgtttga gcagaacgtg cagcgctcca tgcggggtgg 180
   ctacatcggc tccacctact ttgagcgctg cctgaaatag ggttggcgca tacccacccc 240
   cgccacggcc acaagccctg gcatcccctg caaatattta ttgggggcca tgggtagggg 300
   tttggggggc g 311
<210> 315
   <211> 336
   <212> DNA
   <213> Homo sapien
<400> 315
   tttagaacat ggttatcatc caagactact ctaccctgca acattgaact cccaagagca      60
   aatccacatt cctcttgagt tctgcagctt ctgtgtaaat agggcagctg tcgtctatgc     120
   cgtagaatca catgatctga ggaccattca tggaagctgc taaatagcct agtctgggga     180
   gtcttccata aagttttgca tggagcaaac aaacaggatt aaactaggtt tggttccttc     240
   agccctctaa aagcataggg cttagcctgc aggcttcctt gggctttctc tgtgtgtgta     300
   gttttgtaaa cactatagca tctgttaaga tccagt                               336
<210> 316
   <211> 436
   <212> DNA
   <213> Homo sapien
<400> 316
   aacatggtct gcgtgcctta agagagacgc ttcctgcaga acaggacctg actacaaaga      60
   atgtttccat tggaattgtt ggtaaagact tggagtttac aatctatgat gatgatgatg     120
   tgtctccatt cctggaaggt cttgaagaaa gaccacagag aaaggcacag cctgctcaac     180
   ctgctgatga acctgcagaa aaggctgatg aaccaatgga acattaagtg ataagccagt     240
   ctatatatgt attatcaaat atgtaagaat acaggcacca catactgatg acaataatct     300
   atactttgaa ccaaaagttg cagagtggtg gaatgctatg ttttaggaat cagtccagat     360
   gtgagttttt tccaagcaac ctcactgaaa cctatataat ggaatacatt tttctttgaa     420
   agggtctgta taatca                                                     436
<210> 317
   <211> 196
   <212> DNA
   <213> Homo sapien
<400> 317
   tattccttgt gaagatgata tactattttt gttaagcgtg tctgtattta tgtgtgagga      60
   gctgctggct tgcagtgcgc gtgcacgtgg agagctggtg cccggagatt ggacggcctg     120
   atgctccctc ccctgccctg gtccagggaa gctggccgag ggtcctggct cctgaggggc     180
   atctgcccct ccccca                                                     196

<210> 318
   <211> 381
   <212> DNA
   <213> Homo sapien
<220>
   <221> misc_feature
   <222> (1)...(381)
   <223> n = A,T,C or G
<400> 318
   gacgcttnng ccgtaacgat gatcggagac atcctgctgt tcgggacgtt gctgatgaat 60
   gccggggcgg tgctgaactt taagctgaaa aagaaggaca cncagggctt tggggaggag 120
   tncagggagc ccaacacagg tgacaacatc cgggaattct tgctgancct cagatacttt 180
   cnaatcttca tcnccctgtg gaacatcttc atgatgttct gcatgattgt gctgntcggc 240
   tcttgaatcc cancgatgaa accannaact cactttcccg ggatgccgan tctccattcc 300
   tccattcctg atgacttcaa naatgttttt gaccaaaaaa ccgacaacct tcccagaaag 360
   tccaagctcg tggtgggngg a 381
<210> 319
   <211> 506
   <212> DNA
   <213> Homo sapien
<400> 319
   ctaagcttta cgaatggggt gacaacttat gataaaaact agagctagtg aattagccta 60
   tttgtaaata cctttgttat aattgatagg atacatcttg gacatggaat tgttaagcca 120
   cctctgagca gtgtatgtca ggacttgttc attaggttgg cagcagaggg gcagaaggaa 180
   ttatacaggt agagatgtat gcagatgtgt ccatatatgt ccatatttac attttgatag 240
   ccattgatgt atgcatctct tggctgtact ataagaacac attaattcaa tggaaataca 300
   ctttgctaat attttaatgg tatagatctg ctaatgaatt ctcttaaaaa catactgtat 360
   tctgttgctg tgtgtttcat tttaaattga gcattaaggg aatgcagcat ttaaatcaga 420
   actctgccaa tgcttttatc tagaggcgtg ttgccatttt tgtcttatat gaaatttctg 480
   tcccaagaaa ggcaggatta catctt 506
<210> 320
   <211> 351
   <212> DNA
   <213> Homo sapien
<400> 320
   ctgacctgca ggacgaaacc atgaagagcc tgatccttct tgccatcctg gccgccttag 60
   cggtagtaac tttgtgttat gaatcacatg aaagcatgga atcttatgaa cttaatccct 120
   tcattaacag gagaaatgca aataccttca tatcccctca gcagagatgg agagctaaag 180
   tccaagagag gatccgagaa cgctctaagc ctgtccacga gctcaatagg gaagcctgtg 240
   atgactacag actttgcgaa cgctacgcca tggtttatgg atacaatgct gcctataatc 300
   gctacttcag gaagcgccga gggaccaaat gagactgagg gaagaaaaaa a 351
<210> 321
   <211> 421
   <212> DNA
   <213> Homo sapien
<400> 321
   ctcggaggcg ttcagctgct tcaagatgaa gctgaacatc tccttcccag ccactggctg 60
   ccagaaactc attgaagtgg acgatgaacg caaacttcgt actttctatg agaagcgtat 120
   ggccacagaa gttgctgctg acgctctggg tgaagaatgg aagggttatg tggtccgaat 180
   cagtggtggg aacgacaaac aaggtttccc catgaagcag ggtgtcttga cccatggccg 240
   tgtccgcctg ctactgagta aggggcattc ctgttacaga ccaaggagaa ctggagaaag 300
   aaagagaaaa tcagttcgtg gttgcattgt ggatgcaaat ctgagcgttc tcaacttggt 360
   tattgtaaaa aaaggagaga aggatattcc tggactgact gatactacag tgcctcgccg 420
   c 421
<210> 322
   <211> 521
   <212> DNA
   <213> Homo sapien
<400> 322
   agcagctctc ctgccacagc tcctcacccc ctgaaaatgt tcgcctgctc caagtttgtc      60
   tccactccct ccttggtcaa gagcacctca cagctgctga gccgtccgct atctgcagtg     120
   gtgctgaaac gaccggagat actgacagat gagagcctca gcagcttggc agtctcatgt     180
   ccccttacct cacttgtctc tagccgcagc ttccaaacca gcgccatttc aagggacatc     240
   gacacagcag ccaagttcat tggagctggg gctgccacag ttggggtggc tggttctggg     300
   gctgggattg gaactgtgtt tgggagcctc atcattggtt atgccaggaa cccttctctg     360
   aagcaacagc tcttctccta cgccattctg ggctttgccc tctcggaggc catggggctc     420
   ttttgtctga tggtagcctt tctcatcctc tttgccatgt gaaggagccg tctccacctc     480
   ccatagttct cccgcgtctg gttggccccg tgtgttcctt t                         521
<210> 323
   <211> 435
   <212> DNA
   <213> Homo sapien
<400> 323
   ccgaggtcgc acgcgtgaga cttctccgcc gcagacgccg ccgcgatgcg ctacgtcgcc      60
   tcctacctgc tggctgccct agggggcaac tcctccccca gcgccaagga catcaagaag     120
   atcttggaca gcgtgggtat cgaggcggac gacgaccggc tcaacaaggt tatcagtgag     180
   ctgaatggaa aaaacattga agacgtcatt gcccagggta ttggcaagct tgccagtgta     240
   cctgctggtg gggctgtagc cgtctctgct gccccaggct ctgcagcccc tgctgctggt     300
   tctgcccctg ctgcagcaga ggagaagaaa gatgagaaga aggaggagtc tgaagagtca     360
   gatgatgaca tgggatttgg cctttttgat taaattcctg ctcccctgca aataaagcct     420
   ttttacacat ctcaa                                                      435
<210> 324
   <211> 521
   <212> DNA
   <213> Homo sapien
<400> 324
   aggagatcga ctttcggtgc ccgcaagacc agggctggaa cgccgagatc acgctgcaga      60
   tggtgcagta caagaatcgt caggccatcc tggcggtcaa atccacgcgg cagaagcagc     120
   agcacctggt ccagcagcag cccccctcgc agccgcagcc gcagccgcag ctccagcccc     180
   aaccccagcc tcagcctcag ccgcaacccc agccccaatc acaaccccag cctcagcccc     240
   aacccaagcc tcagccccag cagctccacc cgtatccgca tccacatcca catccacact     300
   ctcatcctca ctcgcaccca caccctcacc cgcacccgca tccgcaccaa ataccgcacc     360
   cacacccaca gccgcactcg cagccgcacg ggcaccggct tctccgcagc acctccaact     420
   ctgcctgaaa ggggcagctc ccgggcaaga caaggttttg aggacttgag gaagtgggac     480
   gagcacattt ctattgtctt cacttggatc aaaagcaaaa c                         521
<210> 325
   <211> 451
   <212> DNA
   <213> Homo sapien
<400> 325
   attttcattt ccattaacct ggaagctttc atgaatattc tcttctttta aaacatttta      60
   acattattta aacagaaaaa gatgggctct ttctggttag ttgttacatg atagcagaga     120
   tatttttact tagattactt tgggaatgag agattgttgt cttgaactct ggcactgtac     180
   agtgaatgtg tctgtagttg tgttagtttg cattaagcat gtataacatt caagtatgtc     240
   atccaaataa gaggcatata cattgaattg tttttaatcc tctgacaagt tgactcttcg     300
   acccccaccc ccacccaaga cattttaata gtaaatagag agagagagaa gagttaatga     360
   acatgaggta gtgttccact ggcaggatga cttttcaata gctcaaatca atttcagtgc     420
   ctttatcact tgaattatta acttaatttg a                                    451
<210> 326
   <211> 421
   <212> DNA
   <213> Homo sapien
<220>
   <221> mise feature
   <222> (1)...(421)
   <223> n = A,T,C or G
<400> 326
   cgcggtcgta agggctgagg atttttggtc cgcacgctcc tgctcctgac tcaccgctgt      60
   tcgctctcgc cgaggaacaa gtcggtcagg aagcccgcgc gcaacagcca tggcttttaa     120
   ggataccgga aaaacacccg tggagccgga ggtggcaatt caccgaattc gaatcaccct     180
   aacaagccgc aacgtaaaat ccttggaaaa ggtgtgtgct gacttgataa gaggcgcaaa     240
   agaaaagaat ctcaaagtga aaggaccagt tcgaatgcct accaagactt tgagantcac     300
   tacaagaaaa actccttgtg gtgaaggttc taagacgtgg gatcgtttcc agatgagaat     360
   tcacaagcga ctcattgact tgcacagtcc ttctgagatt gttaagcaga ttacttccat     420
   c                                                                     421
<210> 327
   <211> 456
   <212> DNA
   <213> Homo sapien
<400> 327
   atcttgacga ggctgcggtg tctgctgcta ttctccgagc ttcgcaatgc cgcctaagga      60
   cgacaagaag aagaaggacg ctggaaagtc ggccaagaaa gacaaagacc cagtgaacaa     120
   atccgggggc aaggccaaaa agaagaagtg gtccaaaggc aaagttcggg acaagctcaa     180
   taacttagtc ttgtttgaca aagctaccta tgataaactc tgtaaggaag ttcccaacta     240
   taaacttata accccagctg tggtctctga gagactgaag attcgaggct ccctggccag     300
   ggcagccctt caggagctcc ttagtaaagg acttatcaaa ctggtttcaa agcacagagc     360
   tcaagtaatt tacaccagaa ataccaaggg tggagatgct ccagctgctg gtgaagatgc     420
   atgaataggt ccaaccagct gtacatttgg aaaaat                               456
<210> 328
   <211> 471
   <212> DNA
   <213> Homo sapien
<400> 328
   gtggaagtga catcgtcttt aaaccctgcg tggcaatccc tgacgcaccg ccgtgatgcc      60
   cagggaagac agggcgacct ggaagtccaa ctacttcctt aagatcatcc aactattgga     120
   tgattatccg aaatgtttca ttgtgggagc agacaatgtg ggctccaagc agatgcagca     180
   gatccgcatg tcccttcgcg ggaaggctgt ggtgctgatg ggcaagaaca ccatgatgcg     240
   caaggccatc cgagggcacc tggaaaacaa cccagctctg gagaaactgc tgcctcatat     300
   ccgggggaat gtgggctttg tgttcaccaa ggaggacctc actgagatca gggacatgtt     360
   gctggccaat aaggtgccag ctgctgcccg tgctggtgcc attgccccat gtgaagtcac     420
   tgtgccagcc cagaacactg gtctcgggcc cgagaagacc tcctttttcc a              471
<210> 329
   <211> 278
   <212> DNA
   <213> Homo sapien
<220>
   <221> misc_feature
   <222> (1)...(278)
   <223> n = A,T,C or G
<400> 329
   gtttaaactt aagcttggta ccgagctcgg atccactagt ccagtgtggt ggaattctag      60
   aaattgagat gcccccccag gccagcaaat gttccttttt gttcaaagtc tatttttatt     120
   ccttgatatt tttctttttt tttttttttt ttgnggatgg ggacttgtga atttttctaa     180
   aggtgctatt taacatggga gganagcgtg tgcggctcca gcccagcccg ctgctcactt     240
   tccaccctct ctccacctgc ctctggcttc tcaggcct                             278
<210> 330
   <211> 338
   <212> DNA
   <213> Homo sapien
<400> 330
   ctcaggcttc aacatcgaat acgccgcagg ccccttcgcc ctattcttca tagccgaata      60
   cacaaacatt attataataa acaccctcac cactacaatc ttcctaggaa caacatatga     120
   cgcactctcc cctgaactct acacaacata ttttgtcacc aagaccctac ttctaacctc     180
   cctgttctta tgaattcgaa cagcataccc ccgattccgc tacgaccaac tcatacacct     240
   cctatgaaaa aacttcctac cactcaccct agcattactt atatgatatg tctccatacc     300
   cattacaatc tccagcattc cccctcaaac ctaaaaaa                             338
<210> 331
   <211> 2820
   <212> DNA
   <213> Homo sapiens
<400> 331
   tggcaaaatc ctggagccag aagaaaggac agcagcattg atcaatctta cagctaacat 60
   gttgtacctg gaaaacaatg cccagactca atttagtgag ccacagtaca cgaacctggg 120
   gctcctgaac agcatggacc agcagattcg gaacggctcc tcgtccacca gtccctataa 180
   cacagaccac gcgcagaaca gcgtcacggc gccctcgccc tacgcacagc ccagccccac 240
   cttcgatgct ctctctccat cacccgccat cccctccaac accgactacc caggcccgca 300
   cagttccgac gtgtccttcc agcagtcgag caccgccaag tcggccacct ggacgtattc 360
   cactgaactg aagaaactct actgccaaat tgcaaagaca tgccccatcc agatcaaggt 420
   gatgacccca cctcctcagg gagctgttat ccgcgccatg cctgtctaca aaaaagctga 480
   gcacgtcacg gaggtggtga agcggtgccc caaccatgag ctgagccgtg agttcaacga 540
   gggacagatt gcccctccta gtcatttgat tcgagtagag gggaacagcc atgcccagta 600
   tgtagaagat cccatcacag gaagacagag tgtgctggta ccttatgagc caccccaggt 660
   tggcactgaa ttcacgacag tcttgtacaa tttcatgtgt aacagcagtt gtgttggagg 720
   gatgaaccgc cgtccaattt taatcattgt tactctggaa accagagatg ggcaagtcct 780
   gggccgacgc tgctttgagg cccggatctg tgcttgccca ggaagagaca ggaaggcgga 840
   tgaagatagc atcagaaagc agcaagtttc ggacagtaca aagaacggtg atggtacgaa 900
   gcgcccgttt cgtcagaaca cacatggtat ccagatgaca tccatcaaga aacgaagatc 960
   cccagatgat gaactgttat acttaccagt gaggggccgt gagacttatg aaatgctgtt 1020
   gaagatcaaa gagtccctgg aactcatgca gtaccttcct cagcacacaa ttgaaacgta 1080
   caggcaacag caacagcagc agcaccagca cttacttcag aaacagacct caatacagtc 1140
   tccatcttca tatggtaaca gctccccacc tctgaacaaa atgaacagca tgaacaagct 1200
   gccttctgtg agccagctta tcaaccctca gcagcgcaac gccctcactc ctacaaccat 1260
   tcctgatggc atgggagcca acattcccat gatgggcacc cacatgccaa tggctggaga 1320
   catgaatgga ctcagcccca cccaggcact ccctccccca ctctccatgc catccacctc 1380
   ccactgcaca cccccacctc cgtatcccac agattgcagc attgtcagtt tcttagcgag 1440
   gttgggctgt tcatcatgtc tggactattt cacgacccag gggctgacca ccatctatca 1500
   gattgagcat tactccatgg atgatctggc aagtctgaaa atccctgagc aatttcgaca 1560
   tgcgatctgg aagggcatcc tggaccaccg gcagctccac gaattctcct ccccttctca 1620
   tctcctgcgg accccaagca gtgcctctac agtcagtgtg ggctccagtg agacccgggg 1680
   tgagcgtgtt attgatgctg tgcgattcac cctccgccag accatctctt tcccaccccg 1740
   agatgagtgg aatgacttca actttgacat ggatgctcgc cgcaataagc aacagcgcat 1800
   caaagaggag ggggagtgag cctcaccatg tgagctcttc ctatccctct cctaactgcc 1860
   agccccctaa aagcactcct gcttaatctt caaagccttc tccctagctc ctccccttcc 1920
   tcttgtctga tttcttaggg gaaggagaag taagaggcta cctcttacct aacatctgac 1980
   ctggcatcta attctgattc tggctttaag ccttcaaaac tatagcttgc agaactgtag 2040
   ctgccatggc taggtagaag tgagcaaaaa agagttgggt gtctccttaa gctgcagaga 2100
   tttctcattg acttttataa agcatgttca cccttatagt ctaagactat atatataaat 2160
   gtataaatat acagtataga tttttgggtg gggggcattg agtattgttt aaaatgtaat 2220
   ttaaatgaaa gaaaattgag ttgcacttat tgaccatttt ttaatttact tgttttggat 2280
   ggcttgtcta tactccttcc cttaaggggt atcatgtatg gtgataggta tctagagctt 2340
   aatgctacat gtgagtgcga tgatgtacag attctttcag ttctttggat tctaaataca 2400
   tgccacatca aacctttgag tagatccatt tccattgctt attatgtagg taagactgta 2460
   gatatgtatt cttttctcag tgttggtata ttttatatta ctgacatttc ttctagtgat 2520
   gatggttcac gttggggtga tttaatccag ttataagaag aagttcatgt ccaaacggtc 2580
   ctctttagtt tttggttggg aatgaggaaa attcttaaaa ggcccatagc agccagttca 2640
   aaaacacccg acgtcatgta tttgagcata tcagtaaccc ccttaaattt aatacccaga 2700
   taccttatct tacaatgttg attgggaaaa catttgctgc ccattacaga ggtattaaaa 2760
   ctaaatttca ctactagatt gactaactca aatacacatt tgctactgtt gtaagaattc 2820
<210> 332
   <211> 2270
   <212> DNA
   <213> Homo sapiens
<400> 332
   tcgttgatat caaagacagt tgaaggaaat gaattttgaa acttcacggt gtgccaccct 60
   acagtactgc cctgaccctt acatccagcg tttcgtagaa acccagctca tttctcttgg 120
   aaagaaagtt attaccgatc caccatgtcc cagagcacac agacaaatga attcctcagt 180
   ccagaggttt tccagcatat ctgggatttt ctggaacagc ctatatgttc agttcagccc 240
   attgacttga actttgtgga tgaaccatca gaagatggtg cgacaaacaa gattgagatt 300
   agcatggact gtatccgcat gcaggactcg gacctgagtg accccatgtg gccacagtac 360
   acgaacctgg ggctcctgaa cagcatggac cagcagattc agaacggctc ctcgtccacc 420
   agtccctata acacagacca cgcgcagaac agcgtcacgg cgccctcgcc ctacgcacag 480
   cccagctcca ccttcgatgc tctctctcca tcacccgcca tcccctccaa caccgactac 540
   ccaggcccgc acagtttcga cgtgtccttc cagcagtcga gcaccgccaa gtcggccacc 600
   tggacgtatt ccactgaact gaagaaactc tactgccaaa ttgcaaagac atgccccatc 660
   cagatcaagg tgatgacccc acctcctcag ggagctgtta tccgcgccat gcctgtctac 720
   aaaaaagctg agcacgtcac ggaggtggtg aagcggtgcc ccaaccatga gctgagccgt 780
   gaattcaacg agggacagat tgcccctcct agtcatttga ttcgagtaga ggggaacagc 840
   catgcccagt atgtagaaga tcccatcaca ggaagacaga gtgtgctggt accttatgag 900
   ccaccccagg ttggcactga attcacgaca gtcttgtaca atttcatgtg taacagcagt 960
   tgtgttggag ggatgaaccg ccgtccaatt ttaatcattg ttactctgga aaccagagat 1020
   gggcaagtcc tgggccgacg ctgctttgag gcccggatct gtgcttgccc aggaagagac 1080
   aggaaggcgg atgaagatag catcagaaag cagcaagttt cggacagtac aaagaacggt 1140
   gatggtacga agcgcccgtt tcgtcagaac acacatggta tccagatgac atccatcaag 1200
   aaacgaagat ccccagatga tgaactgtta tacttaccag tgaggggccg tgagacttat 1260
   gaaatgctgt tgaagatcaa agagtccctg gaactcatgc agtaccttcc tcagcacaca 1320
   attgaaacgt acaggcaaca gcaacagcag cagcaccagc acttacttca gaaacagacc 1380
   tcaatacagt ctccatcttc atatggtaac agctccccac ctctgaacaa aatgaacagc 1440
   atgaacaagc tgccttctgt gagccagctt atcaaccctc agcagcgcaa cgccctcact 1500
   cctacaacca ttcctgatgg catgggagcc aacattccca tgatgggcac ccacatgcca 1560
   atggctggag acatgaatgg actcagcccc acccaggcac tccctccccc actctccatg 1620
   ccatccacct cccactgcac acccccacct ccgtatccaa cagattgcag cattgtcggt 1680
   ttcttagcga ggttgggctg ttcatcatgt ctggactatt tcacgaccca ggggctgacc 1740
   accatctatc agattgagca ttactccatg gatgatctgg caagtctgaa aatccctgag 1800
   caatttcgac atgcgatctg gaagggcatc ctggaccacc ggcagctcca cgaattctcc 1860
   tccccttctc atctcctgcg gaccccaagc agtgcctcta cagtcagtgt gggctccagt 1920
   gagacccggg gtgagcgtgt tattgatgct gtgcgattca ccctccgcca gaccatctct 1980
   ttcccacccc gagatgagtg gaatgacttc aactttgaca tggatgctcg ccgcaataag 2040
   caacagcgca tcaaagagga gggggagtga gcctcaccat gtgagctctt cctatccctc 2100
   tcctaactgc cagcccccta aaagcactcc tgcttaatct tcaaagcctt ctccctagct 2160
   cctccccttc ctcttgtctg atttcttagg ggaaggagaa gtaagaggct acctcttacc 2220
   taacatctga cctggcatct aattctgatt ctggctttaa gccttcaaaa 2270
<210> 333
   <211> 2816
   <212> DNA
   <213> Homo sapiens
<400> 333
   tcgttgatat caaagacagt tgaaggaaat gaattttgaa acttcacggt gtgccaccct 60
   acagtactgc cctgaccctt acatccagcg tttcgtagaa acccagctca tttctcttgg 120
   aaagaaagtt attaccgatc caccatgtcc cagagcacac agacaaatga attcctcagt 180
   ccagaggttt tccagcatat ctgggatttt ctggaacagc ctatatgttc agttcagccc 240
   attgacttga actttgtgga tgaaccatca gaagatggtg cgacaaacaa gattgagatt 300
   agcatggact gtatccgcat gcaggactcg gacctgagtg accccatgtg gccacagtac 360
   acgaacctgg ggctcctgaa cagcatggac cagcagattc agaacggctc ctcgtccacc 420
   agtccctata acacagacca cgcgcagaac agcgtcacgg cgccctcgcc ctacgcacag 480
   cccagctcca ccttcgatgc tctctctcca tcacccgcca tcccctccaa caccgactac 540
   ccaggcccgc acagtttcga cgtgtccttc cagcagtcga gcaccgccaa gtcggccacc 600
   tggacgtatt ccactgaact gaagaaactc tactgccaaa ttgcaaagac atgccccatc 660
   cagatcaagg tgatgacccc acctcctcag ggagctgtta tccgcgccat gcctgtctac 720
   aaaaaagctg agcacgtcac ggaggtggtg aagcggtgcc ccaaccatga gctgagccgt 780
   gaattcaacg agggacagat tgcccctcct agtcatttga ttcgagtaga ggggaacagc 840
   catgcccagt atgtagaaga tcccatcaca ggaagacaga gtgtgctggt accttatgag 900
   ccaccccagg ttggcactga attcacgaca gtcttgtaca atttcatgtg taacagcagt 960
   tgtgttggag ggatgaaccg ccgtccaatt ttaatcattg ttactctgga aaccagagat 1020
   gggcaagtcc tgggccgacg ctgctttgag gcccggatct gtgcttgccc aggaagagac 1080
   aggaaggcgg atgaagatag catcagaaag cagcaagttt cggacagtac aaagaacggt 1140
   gatggtacga agcgcccgtt tcgtcagaac acacatggta tccagatgac atccatcaag 1200
   aaacgaagat ccccagatga tgaactgtta tacttaccag tgaggggccg tgagacttat 1260
   gaaatgctgt tgaagatcaa agagtccctg gaactcatgc agtaccttcc tcagcacaca 1320
   attgaaacgt acaggcaaca gcaacagcag cagcaccagc acttacttca gaaacatctc 1380
   ctttcagcct gcttcaggaa tgagcttgtg gagccccgga gagaaactcc aaaacaatct 1440
   gacgtcttct ttagacattc caagccccca aaccgatcag tgtacccata gagccctatc 1500
   tctatatttt aagtgtgtgt gttgtatttc catgtgtata tgtgagtgtg tgtgtgtgta 1560
   tgtgtgtgcg tgtgtatcta gccctcataa acaggacttg aagacacttt ggctcagaga 1620
   cccaactgct caaaggcaca aagccactag tgagagaatc ttttgaaggg actcaaacct 1680
   ttacaagaaa ggatgttttc tgcagatttt gtatccttag accggccatt ggtgggtgag 1740
   gaaccactgt gtttgtctgt gagctttctg ttgtttcctg ggagggaggg gtcaggtggg 1800
   gaaaggggca ttaagatgtt tattggaacc cttttctgtc ttcttctgtt gtttttctaa 1860
   aattcacagg gaagcttttg agcaggtctc aaacttaaga tgtcttttta agaaaaggag 1920
   aaaaaagttg ttattgtctg tgcataagta agttgtaggt gactgagaga ctcagtcaga 1980
   cccttttaat gctggtcatg taataatatt gcaagtagta agaaacgaag gtgtcaagtg 2040
   tactgctggg cagcgaggtg atcattacca aaagtaatca actttgtggg tggagagttc 2100
   tttgtgagaa cttgcattat ttgtgtcctc ccctcatgtg taggtagaac atttcttaat 2160
   gctgtgtacc tgcctctgcc actgtatgtt ggcatctgtt atgctaaagt ttttcttgta 2220
   catgaaaccc tggaagacct actacaaaaa aactgttgtt tggcccccat agcaggtgaa 2280
   ctcattttgt gcttttaata gaaagacaaa tccaccccag taatattgcc cttacgtagt 2340
   tgtttaccat tattcaaagc tcaaaataga atttgaagcc ctctcacaaa atctgtgatt 2400
   aatttgctta attagagctt ctatccctca agcctaccta ccataaaacc agccatatta 2460
   ctgatactgt tcagtgcatt tagccaggag acttacgttt tgagtaagtg agatccaagc 2520
   agacgtgtta aaatcagcac tcctggactg gaaattaaag attgaaaggg tagactactt 2580
   ttcttttttt tactcaaaag tttagagaat ctctgtttct ttccatttta aaaacatatt 2640
   ttaagataat agcataaaga ctttaaaaat gttcctcccc tccatcttcc cacacccagt 2700
   caccagcact gtattttctg tcaccaagac aatgatttct tgttattgag gctgttgctt 2760
   ttgtggatgt gtgattttaa ttttcaataa acttttgcat cttggtttaa aagaaa 2816
<210> 334
   <211> 2082
   <212> DNA
   <213> Homo sapiens
<400> 334
   agatgctaca gcgactgcac acccaggctg tatgatacag cctattgctc ccgggctgca 60
   aacctgtcca gcatgtgatg tggtgggata ctgaattgaa taccgaatac tgtaggcaat 120
   tgtaacacag tggtaagtct ttgtgtatct aaacatagct aaacaccaaa aggtatagta 180
   agaatatggt attataatct tatggaacta tcattgtata tgtggtttgt caaccagaat 240
   gtagttatac agcacaggac tgtgcttatg atgtgccaag cacagctctc agtactaact 300
   cctttaatct tcatatcaac cctaggaggt aacttcttaa gtagattcat attgtaaggg 360
   tctcggggtg ggggggttgg caaaatcctg gagccagaag aaaggacagc agcattgatc 420
   aatcttacag ctaacatgtt gtacctggaa aacaatgccc agactcaatt tagtgagcca 480
   cagtacacga acctggggct cctgaacagc atggaccagc agattcagaa cggctcctcg 540
   tccaccagtc cctataacac agaccacgcg cagaacagcg tcacggcgcc ctcgccctac 600
   gcacagccca gctccacctt cgatgctctc tctccatcac ccgccatccc ctccaacacc 660
   gactacccag gcccgcacag tttcgacgtg tccttccagc agtcgagcac cgccaagtcg 720
   gccacctgga cgtattccac tgaactgaag aaactctact gccaaattgc aaagacatgc 780
   cccatccaga tcaaggtgat gaccccacct cctcagggag ctgttatccg cgccatgcct 840
   gtctacaaaa aagctgagca cgtcacggag gtggtgaagc ggtgccccaa ccatgagctg 900
   agccgtgaat tcaacgaggg acagattgcc cctcctagtc atttgattcg agtagagggg 960
   aacagccatg cccagtatgt agaagatccc atcacaggaa gacagagtgt gctggtacct 1020
   tatgagccac cccaggttgg cactgaattc acgacagtct tgtacaattt catgtgtaac 1080
   agcagttgtg ttggagggat gaaccgccgt ccaattttaa tcattgttac tctggaaacc 1140
   agagatgggc aagtcctggg ccgacgctgc tttgaggccc ggatctgtgc ttgcccagga 1200
   agagacagga aggcggatga agatagcatc agaaagcagc aagtttcgga cagtacaaag 1260
   aacggtgatg gtacgaagcg cccgtctcgt cagaacacac atggtatcca gatgacatcc 1320
   atcaagaaac gaagatcccc agatgatgaa ctgttatact taccagtgag gggccgtgag 1380
   acttatgaaa tgctgttgaa gatcaaagag tccctggaac tcatgcagta ccttcctcag 1440
   cacacaattg aaacgtacag gcaacagcaa cagcagcagc accagcactt acttcagaaa 1500
   cagtgagtgt atcaacgtgt cattttagga ggcatgagtg acggtgactt tatttggatc 1560
   agcaataggg tgattgatga gcaatgtgga acataatggg agatagcaga ttgtcataga 1620
   ttcagatgac ctggtatggc aaccctcttt cagttgcaac cttttttacg tgtcttatta 1680
   taaccttccc ttcagaattc cacttatgtt ctgaaattaa atacaaacca tttctggtga 1740
   attacaaaga aactcacact aacagttctc ttctctatat gcctggtcca tacacactaa 1800
   cagtaagtac acactctatt tggtagtgat gtgtatattt gaaaacatga aatcttttct 1860
   catcccaatg gattgtctta taaatctcct gggatgcaca ctatccactt ttgggaataa 1920
   cactgtagac cagggatagc aaataggctt tactataata taaagtgact tgtttgaatg 1980
   ctgtaatgag aagaattctg agacctagtg catgataatt ggggaaatat ctgggtgcag 2040
   aaggataagg tagcatcatg ttgccgtatt ttagcatctc tg 2082
<210> 335
   <211> 4849
   <212> DNA
   <213> Homo sapiens
<400> 335
   cgttgatatc aaagacagtt gaaggaaatg aattttgaaa cttcacggtg tgccacccta 60
   cagtactgcc ctgaccctta catccagcgt ttcgtagaaa ccccagctca tttctcttgg 120
   aaagaaagtt attaccgatc caccatgtcc cagagcacac agacaaatga attcctcagt 180
   ccagaggttt tccagcatat ctgggatttt ctggaacagc ctatatgttc agttcagccc 240
   attgacttga actttgtgga tgaaccatca gaagatggtg cgacaaacaa gattgagatt 300
   agcatggact gtatccgcat gcaggactcg gacctgagtg accccatgtg gccacagtac 360
   acgaacctgg ggctcctgaa cagcatggac cagcagattc agaacggctc ctcgtccacc 420
   agtccctata acacagacca cgcgcagaac agcgtcacgg cgccctcgcc ctacgcacag 480
   cccagctcca ccttcgatgc tctctctcca tcacccgcca tcccctccaa caccgactac 540
   ccaggcccgc acagtttcga cgtgtccttc cagcagtcga gcaccgccaa gtcggccacc 600
   tggacgtatt ccactgaact gaagaaactc tactgccaaa ttgcaaagac atgccccatc 660
   cagatcaagg tgatgacccc acctcctcag ggagctgtta tccgcgccat gcctgtctac 720
   aaaaaagctg agcacgtcac ggaggtggtg aagcggtgcc ccaaccatga gctgagccgt 780
   gaattcaacg agggacagat tgcccctcct agtcatttga ttcgagtaga ggggaacagc 840
   catgcccagt atgtagaaga tcccatcaca ggaagacaga gtgtgctggt accttatgag 900
   ccaccccagg ttggcactga attcacgaca gtcttgtaca atttcatgtg taacagcagt 960
   tgtgttggag ggatgaaccg ccgtccaatt ttaatcattg ttactctgga aaccagagat 1020
   gggcaagtcc tgggccgacg ctgctttgag gcccggatct gtgcttgccc aggaagagac 1080
   aggaaggcgg atgaagatag catcagaaag cagcaagttt cggacagtac aaagaacggt 1140
   gatggtacga agcgcccgtt tcgtcagaac acacatggta tccagatgac atccatcaag 1200
   aaacgaagat ccccagatga tgaactgtta tacttaccag tgaggggccg tgagacttat 1260
   gaaatgctgt tgaagatcaa agagtccctg gaactcatgc agtaccttcc tcagcacaca 1320
   attgaaacgt acaggcaaca gcaacagcag cagcaccagc acttacttca gaaacagacc 1380
   tcaatacagt ctccatcttc atatggtaac agctccccac ctctgaacaa aatgaacagc 1440
   atgaacaagc tgccttctgt gagccagctt atcaaccctc agcagcgcaa cgccctcact 1500
   cctacaacca ttcctgatgg catgggagcc aacattccca tgatgggcac ccacatgcca 1560
   atggctggag acatgaatgg actcagcccc acccaggcac tccctccccc actctccatg 1620
   ccatccacct cccagtgcac acccccacct ccgtatccca cagattgcag cattgtcagt 1680
   ttcttagcga ggttgggctg ttcatcatgt ctggactatt tcacgaccca ggggctgacc 1740
   accatctatc agattgagca ttactccatg gatgatctgg caagtctgaa aatccctgag 1800
   caatttcgac atgcgatctg gaagggcatc ctggaccacc ggcagctcca cgaattctcc 1860
   tccccttctc atctcctgcg gaccccaagc agtgcctcta cagtcagtgt gggctccagt 1920
   gagacccggg gtgagcgtgt tattgatgct gtgcgattca ccctccgcca gaccatctct 1980
   ttcccacccc gagatgagtg gaatgacttc aactttgaca tggatgctcg ccgcaataag 2040
   caacagcgca tcaaagagga gggggagtga gcctcaccat gtgagctctt cctatccctc 2100
   tcctaactgc cagcycccta aaagcactcc tgcttaatct tcaaagcctt ctccctagct 2160
   cctccccttc ctcttgtctg atttcttagg ggaaggagaa gtaagaggct acctcttacc 2220
   taacatctga cctggcatct aattctgatt ctggctttaa gccttcaaaa ctatagcttg 2280
   cagaactgta gctgccatgg ctaggtagaa gtgagcaaaa aagagttggg tgtctcctta 2340
   agctgcagag atttctcatt gacttttata aagcatgttc acccttatag tctaagacta 2400
   tatatataaa tgtataaata tacagtatag atttttgggt ggggggcatt gagtattgtt 2460
   taaaatgtaa tttaaatgaa agaaaattga gttgcactta ttgaccattt tttaatttac 2520
   ttgttttgga tggcttgtct atactccttc ccttaagggg tatcatgtat ggtgataggt 2580
   atctagagct taatgctaca tgtgagtgac gatgatgtac agattctttc agttctttgg 2640
   attctaaata catgccacat caaacctttg agtagatcca tttccattgc ttattatgta 2700
   ggtaagactg tagatatgta ttcttttctc agtgttggta tattttatat tactgacatt 2760
   tcttctagtg atgatggttc acgttggggt gatttaatcc agttataaga agaagttcat 2820
   gtccaaacgt cctctttagt ttttggttgg gaatgaggaa aattcttaaa aggcccatag 2880
   cagccagttc aaaaacaccc gacgtcatgt atttgagcat atcagtaacc cccttaaatt 2940
   taataccaga taccttatct tacaatattg attgggaaaa catttgctgc cattacagag 3000
   gtattaaaac taaatttcac tactagattg actaactcaa atacacattt gctactgttg 3060
   taagaattct gattgatttg attgggatga atgccatcta tctagttcta acagtgaagt 3120
   tttactgtct attaatattc agggtaaata ggaatcattc agaaatgttg agtctgtact 3180
   aaacagtaag atatctcaat gaaccataaa ttcaactttg taaaaatctt ttgaagcata 3240
   gataatattg tttggtaaat gtttcttttg tttggtaaat gtttctttta aagaccctcc 3300
   tattctataa aactctgcat gtagaggctt gtttaccttt ctctctctaa ggtttacaat 3360
   aggagtggtg atttgaaaaa tataaaatta tgagattggt tttcctgtgg cataaattgc 3420
   atcactgtat cattttcttt tttaaccggt aagagtttca gtttgttgga aagtaactgt 3480
   gagaacccag tttcccgtcc atctccctta gggactaccc atagacatga aaggtcccca 3540
   cagagcaaga gataagtctt tcatggctgc tgttgcttaa accacttaaa cgaagagttc 3600
   ccttgaaact ttgggaaaac atgttaatga caatattcca gatctttcag aaatataaca 3660
   catttttttg catgcatgca aatgagctct gaaatcttcc catgcattct ggtcaagggc 3720
   tgtcattgca cataagcttc cattttaatt ttaaagtgca aaagggccag cgtggctcta 3780
   aaaggtaatg tgtggattgc ctctgaaaag tgtgtatata ttttgtgtga aattgcatac 3840
   tttgtatttt gattattttt tttttcttct tgggatagtg ggatttccag aaccacactt 3900
   gaaacctttt tttatcgttt ttgtattttc atgaaaatac catttagtaa gaataccaca 3960
   tcaaataaga aataatgcta caattttaag aggggaggga agggaaagtt tttttttatt 4020
   atttttttaa aattttgtat gttaaagaga atgagtcctt gatttcaaag ttttgttgta 4080
   cttaaatggt aataagcact gtaaacttct gcaacaagca tgcagctttg caaacccatt 4140
   aaggggaaga atgaaagctg ttccttggtc ctagtaagaa gacaaactgc ttcccttact 4200
   ttgctgaggg tttgaataaa cctaggactt ccgagctatg tcagtactat tcaggtaaca 4260
   ctagggcctt ggaaattcct gtactgtgtc tcatggattt ggcactagcc aaagcgaggc 4320
   acccttactg gcttacctcc tcatggcagc ctactctcct tgagtgtatg agtagccagg 4380
   gtaaggggta aaaggatagt aagcatagaa accactagaa agtgggctta atggagttct 4440
   tgtggcctca gctcaatgca gttagctgaa gaattgaaaa gtttttgttt ggagacgttt 4500
   ataaacagaa atggaaagca gagttttcat taaatccttt tacctttttt ttttcttggt 4560
   aatcccctaa aataacagta tgtgggatat tgaatgttaa agggatattt tttttctatt 4620
   atttttataa ttgtacaaaa ttaagcaaat gttaaaagtt ttatatgctt tattaatgtt 4680
   ttcaaaaggt attatacatg tgatacattt tttaagcttc agttgcttgt cttctggtac 4740
   tttctgttat gggcttttgg ggagccagaa gccaatctac aatctctttt tgtttgccag 4800
   gacatgcaat aaaatttaaa aaataaataa aaactaatta agaaataaa 4849
<210> 336
   <211> 1386
   <212> DNA
   <213> Homo sapiens
<400> 336
   atgttgtacc tggaaaacaa tgcccagact caatttagtg agccacagta cacgaacctg 60
   gggctcctga acagcatgga ccagcagatt cagaacggct cctcgtccac cagtccctat 120
   aacacagacc acgcgcagaa cagcgtcacg gcgccctcgc cctacgcaca gcccagctcc 180
   accttcgatg ctctctctcc atcacccgcc atcccctcca acaccgacta cccaggcccg 240
   cacagtttcg acgtgtcctt ccagcagtcg agcaccgcca agtcggccac ctggacgtat 300
   tccactgaac tgaagaaact ctactgccaa attgcaaaga catgccccat ccagatcaag 360
   gtgatgaccc cacctcctca gggagctgtt atccgcgcca tgcctgtcta caaaaaagct 420
   gagcacgtca cggaggtggt gaagcggtgc cccaaccatg agctgagccg tgaattcaac 480
   gagggacaga ttgcccctcc tagtcatttg attcgagtag aggggaacag ccatgcccag 540
   tatgtagaag atcccatcac aggaagacag agtgtgctgg taccttatga gccaccccag 600
   gttggcactg aattcacgac agtcttgtac aatttcatgt gtaacagcag ttgtgttgga 660
   gggatgaacc gccgtccaat tttaatcatt gttactctgg aaaccagaga tgggcaagtc 720
   ctgggccgac gctgctttga ggcccggatc tgtgcttgcc caggaagaga caggaaggcg 780
   gatgaagata gcatcagaaa gcagcaagtt tcggacagta caaagaacgg tgatggtacg 840
   aagcgcccgt ttcgtcagaa cacacatggt atccagatga catccatcaa gaaacgaaga 900
   tccccagatg atgaactgtt atacttacca gtgaggggcc gtgagactta tgaaatgctg 960
   ttgaagatca aagagtccct ggaactcatg cagtaccttc ctcagcacac aattgaaacg 1020
   tacaggcaac agcaacagca gcagcaccag cacttacttc agaaacagac ctcaatacag 1080
   tctccatctt catatggtaa cagctcccca cctctgaaca aaatgaacag catgaacaag 1140
   ctgccttctg tgagccagct tatcaaccct cagcagcgca acgccctcac tcctacaacc 1200
   attcctgatg gcatgggagc caacattccc atgatgggca cccacatgcc aatggctgga 1260
   gacatgaatg gactcagccc cacccaggca ctccctcccc cactctccat gccatccacc 1320
   tcccactgca cacccccacc tccgtatccc acagattgca gcattgtcag gatctggcaa 1380
   gtctga 1386
<210> 337
   <211> 1551
   <212> DNA
   <213> Homo sapiens
<400> 337
   atgtcccaga gcacacagac aaatgaattc ctcagtccag aggttttcca gcatatctgg 60
   gattttctgg aacagcctat atgttcagtt cagcccattg acttgaactt tgtggatgaa 120
   ccatcagaag atggtgcgac aaacaagatt gagattagca tggactgtat ccgcatgcag 180
   gactcggacc tgagtgaccc catgtggcca cagtacacga acctggggct cctgaacagc 240
   atggaccagc agattcagaa cggctcctcg tccaccagtc cctataacac agaccacgcg 300
   cagaacagcg tcacggcgcc ctcgccctac gcacagccca gctccacctt cgatgctctc 360
   tctccatcac ccgccatccc ctccaacacc gactacccag gcccgcacag tttcgacgtg 420
   tccttccagc agtcgagcac cgccaagtcg gccacctgga cgtattccac tgaactgaag 480
   aaactctact gccaaattgc aaagacatgc cccatccaga tcaaggtgat gaccccacct 540
   cctcagggag ctgttatccg cgccatgcct gtctacaaaa aagctgagca cgtcacggag 600
   gtggtgaagc ggtgccccaa ccatgagctg agccgtgaat tcaacgaggg acagattgcc 660
   cctcctagtc atttgattcg agtagagggg aacagccatg cccagtatgt agaagatccc 720
   atcacaggaa gacagagtgt gctggtacct tatgagccac cccaggttgg cactgaattc 780
   acgacagtct tgtacaattt catgtgtaac agcagttgtg ttggagggat gaaccgccgt 840
   ccaattttaa tcattgttac tctggaaacc agagatgggc aagtcctggg ccgacgctgc 900
   tttgaggccc ggatctgtgc ttgcccagga agagacagga aggcggatga agatagcatc 960
   agaaagcagc aagtttcgga cagtacaaag aacggtgatg gtacgaagcg cccgtttcgt 1020
   cagaacacac atggtatcca gatgacatcc atcaagaaac gaagatcccc agatgatgaa 1080
   ctgttatact taccagtgag gggccgtgag acttatgaaa tgctgttgaa gatcaaagag 1140
   tccctggaac tcatgcagta ccttcctcag cacacaattg aaacgtacag gcaacagcaa 1200
   cagcagcagc accagcactt acttcagaaa cagacctcaa tacagtctcc atcttcatat 1260
   ggtaacagct ccccacctct gaacaaaatg aacagcatga acaagctgcc ttctgtgagc 1320
   cagcttatca accctcagca gcgcaacgcc ctcactccta caaccattcc tgatggcatg 1380
   ggagccaaca ttcccatgat gggcacccac atgccaatgg ctggagacat gaatggactc 1440
   agccccaccc aggcactccc tcccccactc tccatgccat ccacctccca ctgcacaccc 1500
   ccacctccgt atcccacaga ttgcagcatt gtcaggatct ggcaagtctg a          1551
<210> 338
   <211> 586
   <212> PRT
   <213> Homo sapiens
<400> 338
<210> 339
   <211> 641
   <212> PRT
   <213> Homo sapiens
<400> 339
<210> 340
   <211> 448
   <212> PRT
   <213> Homo sapiens
<400> 340
   Met Ser Gln Ser Thr Gln Thr Asn Glu Phe Leu Ser Pro Glu Val Phe
   5 10 15
   Gln His Ile Trp Asp Phe Leu Glu Gln Pro Ile Cys Ser Val Gln Pro
   20 25 30
   Ile Asp Leu Asn Phe Val Asp Glu Pro Ser Glu Asp Gly Ala Thr Asn
   35 40 45
   Lys Ile Glu Ile Ser Met Asp Cys Ile Arg Met Gln Asp Ser Asp Leu
        50                  55                  60
   Ser Asp Pro Met Trp Pro Gln Tyr Thr Asn Leu Gly Leu Leu Asn Ser
    65                  70                  75                  80
   Met Asp Gln Gln Ile Gln Asn Gly Ser Ser Ser Thr Ser Pro Tyr Asn
                    85                  90                  95
   Thr Asp His Ala Gln Asn Ser Val Thr Ala Pro Ser Pro Tyr Ala Gln
               100                 105                 110
   Pro Ser Ser Thr Phe Asp Ala Leu Ser Pro Ser Pro Ala Ile Pro Ser
           115                 120                 125
   Asn Thr Asp Tyr Pro Gly Pro His Ser Phe Asp Val Ser Phe Gln Gln
       130                 135                 140
   Ser Ser Thr Ala Lys Ser Ala Thr Trp Thr Tyr Ser Thr Glu Leu Lys
   145                 150                 155                 160
   Lys Leu Tyr Cys Gln Ile Ala Lys Thr Cys Pro Ile Gln Ile Lys Val
                   165                 170                 175
   Met Thr Pro Pro Pro Gln Gly Ala Val Ile Arg Ala Met Pro Val Tyr
               180                 185                 190
   Lys Lys Ala Glu His Val Thr Glu Val Val Lys Arg Cys Pro Asn His
           195                 200                 205
   Glu Leu Ser Arg Glu Phe Asn Glu Gly Gln Ile Ala Pro Pro Ser His
       210                 215                 220
   Leu Ile Arg Val Glu Gly Asn Ser His Ala Gln Tyr Val Glu Asp Pro
   225                 230                 235                 240
   Ile Thr Gly Arg Gln Ser Val Leu Val Pro Tyr Glu Pro Pro Gln Val
                   245                 250                 255
   Gly Thr Glu Phe Thr Thr Val Leu Tyr Asn Phe Met Cys Asn Ser Ser
               260                 265                 270
   Cys Val Gly Gly Met Asn Arg Arg Pro Ile Leu Ile Ile Val Thr Leu
           275                 280                 285
   Glu Thr Arg Asp Gly Gln Val Leu Gly Arg Arg Cys Phe Glu Ala Arg
       290                 295                 300
   Ile Cys Ala Cys Pro Gly Arg Asp Arg Lys Ala Asp Glu Asp Ser Ile
   305                 310                 315                 320
   Arg Lys Gln Gln Val Ser Asp Ser Thr Lys Asn Gly Asp Gly Thr Lys
                   325                 330                 335
   Arg Pro Phe Arg Gln Asn Thr His Gly Ile Gln Met Thr Ser Ile Lys
   340 345 350
   Lys Arg Arg Ser Pro Asp Asp Glu Leu Leu Tyr Leu Pro Val Arg Gly
   355 360 365
   Arg Glu Thr Tyr Glu Met Leu Leu Lys Ile Lys Glu Ser Leu Glu Leu
   370 375 380
   Met Gln Tyr Leu Pro Gln His Thr Ile Glu Thr Tyr Arg Gln Gln Gln
   385 390 395 400
   Gln Gln Gln His Gln His Leu Leu Gln Lys His Leu Leu Ser Ala Cys
   405 410 415
   Phe Arg Asn Glu Leu Val Glu Pro Arg Arg Glu Thr Pro Lys Gln Ser
   420 425 430
   Asp Val Phe Phe Arg His Ser Lys Pro Pro Asn Arg Ser Val Tyr Pro
   435 440 445
<210> 341
   <211> 356
   <212> PRT
   <213> Homo sapiens
<400> 341
<210> 342
   <211> 680
   <212> PRT
   <213> Homo sapiens
<400> 342
   Met Asn Phe Glu Thr Ser Arg Cys Ala Thr Leu Gln Tyr Cys Pro Asp
   5 10 15
   Pro Tyr Ile Gln Arg Phe Val Glu Thr Pro Ala His Phe Ser Trp Lys
   20 25 30
   Glu Ser Tyr Tyr Arg Ser Thr Met Ser Gln Ser Thr Gln Thr Asn Glu
   35 40 45
   Phe Leu Ser Pro Glu Val Phe Gln His Ile Trp Asp Phe Leu Glu Gln
   50 55 60
   Pro Ile Cys Ser Val Gln Pro Ile Asp Leu Asn Phe Val Asp Glu Pro
   65 70 75 80
   Ser Glu Asp Gly Ala Thr Asn Lys Ile Glu Ile Ser Met Asp Cys Ile
   85 90 95
   Arg Met Gln Asp Ser Asp Leu Ser Asp Pro Met Trp Pro Gln Tyr Thr
   100 105 110
   Asn Leu Gly Leu Leu Asn Ser Met Asp Gln Gln Ile Gln Asn Gly Ser
   115 120 125
   Ser Ser Thr Ser Pro Tyr Asn Thr Asp His Ala Gln Asn Ser Val Thr
   130 135 140
   Ala Pro Ser Pro Tyr Ala Gln Pro Ser Ser Thr Phe Asp Ala Leu Ser
   145 150 155 160
   Pro Ser Pro Ala Ile Pro Ser Asn Thr Asp Tyr Pro Gly Pro His Ser
   165 170 175
   Phe Asp Val Ser Phe Gln Gln Ser Ser Thr Ala Lys Ser Ala Thr Trp
   180 185 190
   Thr Tyr Ser Thr Glu Leu Lys Lys Leu Tyr Cys Gln Ile Ala Lys Thr
   195 200 205
   Cys Pro Ile Gln Ile Lys Val Met Thr Pro Pro Pro Gln Gly Ala Val
   210 215 220
   Ile Arg Ala Met Pro Val Tyr Lys Lys Ala Glu His Val Thr Glu Val
   225 230 235 240
   Val Lys Arg Cys Pro Asn His Glu Leu Ser Arg Glu Phe Asn Glu Gly
   245 250 255
   Gln Ile Ala Pro Pro Ser His Leu Ile Arg Val Glu Gly Asn Ser His
   260 265 270
   Ala Gln Tyr Val Glu Asp Pro Ile Thr Gly Arg Gln Ser Val Leu Val
   275 280 285
   Pro Tyr Glu Pro Pro Gln Val Gly Thr Glu Phe Thr Thr Val Leu Tyr
   290 295 300
   Asn Phe Met Cys Asn Ser Ser Cys Val Gly Gly Met Asn Arg Arg Pro
   305 310 315 320
   Ile Leu Ile Ile Val Thr Leu Glu Thr Arg Asp Gly Gln Val Leu Gly
   325 330 335
   Arg Arg Cys Phe Glu Ala Arg Ile Cys Ala Cys Pro Gly Arg Asp Arg
   340 345 350
   Lys Ala Asp Glu Asp Ser Ile Arg Lys Gln Gln Val Ser Asp Ser Thr
   355 360 365
   Lys Asn Gly Asp Gly Thr Lys Arg Pro Phe Arg Gln Asn Thr His Gly
   370 375 380
   Ile Gln Met Thr Ser Ile Lys Lys Arg Arg Ser Pro Asp Asp Glu Leu
   385 390 395 400
   Leu Tyr Leu Pro Val Arg Gly Arg Glu Thr Tyr Glu Met Leu Leu Lys
   405 410 415
   Ile Lys Glu Ser Leu Glu Leu Met Gln Tyr Leu Pro Gln His Thr Ile
   420 425 430
   Glu Thr Tyr Arg Gln Gln Gln Gln Gln Gln His Gln His Leu Leu Gln
   435 440 445
   Lys Gln Thr Ser Ile Gln Ser Pro Ser Ser Tyr Gly Asn Ser Ser Pro
   450 455 460
   Pro Leu Asn Lys Met Asn Ser Met Asn Lys Leu Pro Ser Val Ser Gln
   465 470 475 480
   Leu Ile Asn Pro Gln Gln Arg Asn Ala Leu Thr Pro Thr Thr Ile Pro
   485 490 495
   Asp Gly Met Gly Ala Asn Ile Pro Met Met Gly Thr His Met Pro Met
   500 505 510
   Ala Gly Asp Met Asn Gly Leu Ser Pro Thr Gln Ala Leu Pro Pro Pro
   515 520 525
   Leu Ser Met Pro Ser Thr Ser Gln Cys Thr Pro Pro Pro Pro Tyr Pro
   530 535 540
   Thr Asp Cys Ser Ile Val Ser Phe Leu Ala Arg Leu Gly Cys Ser Ser
   545 550 555 560
   Cys Leu Asp Tyr Phe Thr Thr Gln Gly Leu Thr Thr Ile Tyr Gln Ile
   565 570 575
   Glu His Tyr Ser Met Asp Asp Leu Ala Ser Leu Lys Ile Pro Glu Gln
   580 585 590
   Phe Arg His Ala Ile Trp Lys Gly Ile Leu Asp His Arg Gln Leu His
   595 600 605
   Glu Phe Ser Ser Pro Ser His Leu Leu Arg Thr Pro Ser Ser Ala Ser
   610 615 620
   Thr Val Ser Val Gly Ser Ser Glu Thr Arg Gly Glu Arg Val Ile Asp
   625 630 635 640
   Ala Val Arg Phe Thr Leu Arg Gln Thr Ile Ser Phe Pro Pro Arg Asp
   645 650 655
   Glu Trp Asn Asp Phe Asn Phe Asp Met Asp Ala Arg Arg Asn Lys Gln
   660 665 670
   Gln Arg Ile Lys Glu Glu Gly Glu
   675 680

<210> 343
   <211> 461
   <212> PRT
   <213> Homo sapiens
<400> 343
   Met Leu Tyr Leu Glu Asn Asn Ala Gln Thr Gln Phe Ser Glu Pro Gln
   5 10 15
   Tyr Thr Asn Leu Gly Leu Leu Asn Ser Met Asp Gln Gln Ile Gln Asn
   20 25 30
   Gly Ser Ser Ser Thr Ser Pro Tyr Asn Thr Asp His Ala Gln Asn Ser
   35 40 45
   Val Thr Ala Pro Ser Pro Tyr Ala Gln Pro Ser Ser Thr Phe Asp Ala
   50 55 60
   Leu Ser Pro Ser Pro Ala Ile Pro Ser Asn Thr Asp Tyr Pro Gly Pro
   65 70 75 80
   His Ser Phe Asp Val Ser Phe Gln Gln Ser Ser Thr Ala Lys Ser Ala
   85 90 95
   Thr Trp Thr Tyr Ser Thr Glu Leu Lys Lys Leu Tyr Cys Gln Ile Ala
   100 105 110
   Lys Thr Cys Pro Ile Gln Ile Lys Val Met Thr Pro Pro Pro Gln Gly
   115 120 125
   Ala Val Ile Arg Ala Met Pro Val Tyr Lys Lys Ala Glu His Val Thr
   130 135 140
   Glu Val Val Lys Arg Cys Pro Asn His Glu Leu Ser Arg Glu Phe Asn
   145 150 155 160
   Glu Gly Gln Ile Ala Pro Pro Ser His Leu Ile Arg Val Glu Gly Asn
   165 170 175
   Ser His Ala Gln Tyr Val Glu Asp Pro Ile Thr Gly Arg Gln Ser Val
   180 185 190
   Leu Val Pro Tyr Glu Pro Pro Gln Val Gly Thr Glu Phe Thr Thr Val
   195 200 205
   Leu Tyr Asn Phe Met Cys Asn Ser Ser Cys Val Gly Gly Met Asn Arg
       210                 215                 220
   Arg Pro Ile Leu Ile Ile Val Thr Leu Glu Thr Arg Asp Gly Gln Val
   225                 230                 235                 240
   Leu Gly Arg Arg Cys Phe Glu Ala Arg Ile Cys Ala Cys Pro Gly Arg
                   245                 250                 255
   Asp Arg Lys Ala Asp Glu Asp Ser Ile Arg Lys Gln Gln Val Ser Asp
               260                 265                 270
   Ser Thr Lys Asn Gly Asp Gly Thr Lys Arg Pro Phe Arg Gln Asn Thr
           275                 280                 285
   His Gly Ile Gln Met Thr Ser Ile Lys Lys Arg Arg Ser Pro Asp Asp
       290                 295                 300
   Glu Leu Leu Tyr Leu Pro Val Arg Gly Arg Glu Thr Tyr Glu Met Leu
   305                 310                 315                 320
   Leu Lys Ile Lys Glu Ser Leu Glu Leu Met Gln Tyr Leu Pro Gln His
                   325                 330                 335
   Thr Ile Glu Thr Tyr Arg Gln Gln Gln Gln Gln Gln His Gln His Leu
               340                 345                 350
   Leu Gln Lys Gln Thr Ser Ile Gln Ser Pro Ser Ser Tyr Gly Asn Ser
           355                 360                 365
   Ser Pro Pro Leu Asn Lys Met Asn Ser Met Asn Lys Leu Pro Ser Val
       370                 375                 380
   Ser Gln Leu Ile Asn Pro Gln Gln Arg Asn Ala Leu Thr Pro Thr Thr
   385                 390                 395                 400
   Ile Pro Asp Gly Met Gly Ala Asn Ile Pro Met Met Gly Thr His Met
                   405                 410                 415
   Pro Met Ala Gly Asp Met Asn Gly Leu Ser Pro Thr Gln Ala Leu Pro
               420                 425                 430
   Pro Pro Leu Ser Met Pro Ser Thr Ser His Cys Thr Pro Pro Pro Pro
           435                 440                 445
   Tyr Pro Thr Asp Cys Ser Ile Val Arg Ile Trp Gln Val
       450                 455                 460
<210> 344
   <211> 516
   <212> PRT
   <213> Homo sapiens
<400> 344
   Met Ser Gln Ser Thr Gln Thr Asn Glu Phe Leu Ser Pro Glu Val Phe
   5 10 15
   Gln His Ile Trp Asp Phe Leu Glu Gln Pro Ile Cys Ser Val Gln Pro
   20 25 30
   Ile Asp Leu Asn Phe Val Asp Glu Pro Ser Glu Asp Gly Ala Thr Asn
   35 40 45
   Lys Ile Glu Ile Ser Met Asp Cys Ile Arg Met Gln Asp Ser Asp Leu
   50 55 60
   Ser Asp Pro Met Trp Pro Gln Tyr Thr Asn Leu Gly Leu Leu Asn Ser
   65 70 75 80
   Met Asp Gln Gln Ile Gln Asn Gly Ser Ser Ser Thr Ser Pro Tyr Asn
   85 90 95
   Thr Asp His Ala Gln Asn Ser Val Thr Ala Pro Ser Pro Tyr Ala Gln
   100 105 110
   Pro Ser Ser Thr Phe Asp Ala Leu Ser Pro Ser Pro Ala Ile Pro Ser
   115 120 125
   Asn Thr Asp Tyr Pro Gly Pro His Ser Phe Asp Val Ser Phe Gln Gln
   130 135 140
   Ser Ser Thr Ala Lys Ser Ala Thr Trp Thr Tyr Ser Thr Glu Leu Lys
   145 150 155 160
   Lys Leu Tyr Cys Gln Ile Ala Lys Thr Cys Pro Ile Gln Ile Lys Val
   165 170 175
   Met Thr Pro Pro Pro Gln Gly Ala Val Ile Arg Ala Met Pro Val Tyr
   180 185 190
   Lys Lys Ala Glu His Val Thr Glu Val Val Lys Arg Cys Pro Asn His
   195 200 205
   Glu Leu Ser Arg Glu Phe Asn Glu Gly Gln Ile Ala Pro Pro Ser His
   210 215 220
   Leu Ile Arg Val Glu Gly Asn Ser His Ala Gln Tyr Val Glu Asp Pro
   225 230 235 240
   Ile Thr Gly Arg Gln Ser Val Leu Val Pro Tyr Glu Pro Pro Gln Val
   245 250 255
   Gly Thr Glu Phe Thr Thr Val Leu Tyr Asn Phe Met Cys Asn Ser Ser
   260 265 270
   Cys Val Gly Gly Met Asn Arg Arg Pro Ile Leu Ile Ile Val Thr Leu
   275 280 285
   Glu Thr Arg Asp Gly Gln Val Leu Gly Arg Arg Cys Phe Glu Ala Arg
       290                 295                 300
   Ile Cys Ala Cys Pro Gly Arg Asp Arg Lys Ala Asp Glu Asp Ser Ile
   305                 310                 315                 320
   Arg Lys Gln Gln Val Ser Asp Ser Thr Lys Asn Gly Asp Gly Thr Lys
                   325                 330                 335
   Arg Pro Phe Arg Gln Asn Thr His Gly Ile Gln Met Thr Ser Ile Lys
               340                 345                 350
   Lys Arg Arg Ser Pro Asp Asp Glu Leu Leu Tyr Leu Pro Val Arg Gly
           355                 360                 365
   Arg Glu Thr Tyr Glu Met Leu Leu Lys Ile Lys Glu Ser Leu Glu Leu
       370                 375                 380
   Met Gln Tyr Leu Pro Gln His Thr Ile Glu Thr Tyr Arg Gln Gln Gln
   385                 390                 395                 400
   Gln Gln Gln His Gln His Leu Leu Gln Lys Gln Thr Ser Ile Gln Ser
                   405                 410                 415
   Pro Ser Ser Tyr Gly Asn Ser Ser Pro Pro Leu Asn Lys Met Asn Ser
               420                 425                 430
   Met Asn Lys Leu Pro Ser Val Ser Gln Leu Ile Asn Pro Gln Gln Arg
           435                 440                 445
   Asn Ala Leu Thr Pro Thr Thr Ile Pro Asp Gly Met Gly Ala Asn Ile
       450                 455                 460
   Pro Met Met Gly Thr His Met Pro Met Ala Gly Asp Met Asn Gly Leu
   465                 470                 475                 480
   Ser Pro Thr Gln Ala Leu Pro Pro Pro Leu Ser Met Pro Ser Thr Ser
                   485                 490                 495
   His Cys Thr Pro Pro Pro Pro Tyr Pro Thr Asp Cys Ser Ile Val Arg
               500                 505                 510
   Ile Trp Gln Val
           515
<210> 345
   <211> 1800
   <212> DNA
   <213> Homo sapiens
<400> 345
   gcgcctcatt gccactgcag tgactaaagc tgggaagacg ctggtcagtt cacctgcccc 60
   actggttgtt ttttaaacaa attctgatac aggcgacatc ctcactgacc gagcaaagat 120
   tgacattcgt atcatcactg tgcaccattg gcttctaggc actccagtgg ggtaggagaa 180
   ggaggtctga aaccctcgca gagggatctt gccctcattc tttgggtctg aaacactggc 240
   agtcgttgga aacaggactc agggataaac cagcgcaatg gattggggga cgctgcacac 300
   tttcatcggg ggtgtcaaca aacactccac cagcatcggg aaggtgtgga tcacagtcat 360
   ctttattttc cgagtcatga tcctagtggt ggctgcccag gaagtgtggg gtgacgagca 420
   agaggacttc gtctgcaaca cactgcaacc gggatgcaaa aatgtgtgct atgaccactt 480
   tttcccggtg tcccacatcc ggctgtgggc cctccagctg atcttcgtct ccaccccagc 540
   gctgctggtg gccatgcatg tggcctacta caggcacgaa accactcgca agttcaggcg 600
   aggagagaag aggaatgatt tcaaagacat agaggacatt aaaaagcaca aggttcggat 660
   agaggggtcg ctgtggtgga cgtacaccag cagcatcttt ttccgaatca tctttgaagc 720
   agcctttatg tatgtgtttt acttccttta caatgggtac cacctgccct gggtgttgaa 780
   atgtgggatt gacccctgcc ccaaccttgt tgactgcttt atttctaggc caacagagaa 840
   gaccgtgttt accattttta tgatttctgc gtctgtgatt tgcatgctgc ttaacgtggc 900
   agagttgtgc tacctgctgc tgaaagtgtg ttttaggaga tcaaagagag cacagacgca 960
   aaaaaatcac cccaatcatg ccctaaagga gagtaagcag aatgaaatga atgagctgat 1020
   ttcagatagt ggtcaaaatg caatcacagg tttcccaagc taaacatttc aaggtaaaat 1080
   gtagctgcgt cataaggaga cttctgtctt ctccagaagg caataccaac ctgaaagttc 1140
   cttctgtagc ctgaagagtt tgtaaatgac tttcataata aatagacact tgagttaact 1200
   ttttgtagga tacttgctcc attcatacac aacgtaatca aatatgtggt ccatctctga 1260
   aaacaagaga ctgcttgaca aaggagcatt gcagtcactt tgacaggttc cttttaagtg 1320
   gactctctga caaagtgggt actttctgaa aatttatata actgttgttg ataaggaaca 1380
   tttatccagg aattgatacg tttattagga aaagatattt ttataggctt ggatgttttt 1440
   agttccgact ttgaatttat ataaagtatt tttataatga ctggtcttcc ttacctggaa 1500
   aaacatgcga tgttagtttt agaattacac cacaagtatc taaatttcca acttacaaag 1560
   ggtcctatct tgtaaatatt gttttgcatt gtctgttggc aaatttgtga actgtcatga 1620
   tacgcttaag gtgggaaagt gttcattgca caatatattt ttactgcttt ctgaatgtag 1680
   acggaacagt gtggaagcag aaggcttttt taactcatcc gtttggccga tcgttgcaga 1740
   ccactgggag atgtggatgt ggttgcctcc ttttgctcgt ccccgtggct taacccttct 1800
<210> 346
   <211> 261
   <212> PRT
   <213> Homo sapiens
<400> 346
   Met Asp Trp Gly Thr Leu His Thr Phe Ile Gly Gly Val Asn Lys His
                   5                   10                  15
   Ser Thr Ser Ile Gly Lys Val Trp Ile Thr Val Ile Phe Ile Phe Arg
               20                  25                  30
   Val Met Ile Leu Val Val Ala Ala Gln Glu Val Trp Gly Asp Glu Gln
           35                  40                  45
   Glu Asp Phe Val Cys Asn Thr Leu Gln Pro Gly Cys Lys Asn Val Cys
       50                  55                  60
   Tyr Asp His Phe Phe Pro Val Ser His Ile Arg Leu Trp Ala Leu Gln
   65                  70                  75                  80
   Leu Ile Phe Val Ser Thr Pro Ala Leu Leu Val Ala Met His Val Ala
                   85                  90                  95
   Tyr Tyr Arg His Glu Thr Thr Arg Lys Phe Arg Arg Gly Glu Lys Arg
               100                 105                 110
   Asn Asp Phe Lys Asp Ile Glu Asp Ile Lys Lys His Lys Val Arg Ile
           115                 120                 125
   Glu Gly Ser Leu Trp Trp Thr Tyr Thr Ser Ser Ile Phe Phe Arg Ile
       130                 135                 140
   Ile Phe Glu Ala Ala Phe Met Tyr Val Phe Tyr Phe Leu Tyr Asn Gly
   145                 150                 155                 160
   Tyr His Leu Pro Trp Val Leu Lys Cys Gly Ile Asp Pro Cys Pro Asn
                   165                 170                 175
   Leu Val Asp Cys Phe Ile Ser Arg Pro Thr Glu Lys Thr Val Phe Thr
               180                 185                 190
   Ile Phe Met Ile Ser Ala Ser Val Ile Cys Met Leu Leu Asn Val Ala
           195                 200                 205
   Glu Leu Cys Tyr Leu Leu Leu Lys Val Cys Phe Arg Arg Ser Lys Arg
       210                 215                 220
   Ala Gln Thr Gln Lys Asn His Pro Asn His Ala Leu Lys Glu Ser Lys
   225                 230                 235                 240
   Gln Asn Glu Met Asn Glu Leu Ile Ser Asp Ser Gly Gln Asn Ala Ile
                   245                 250                 255
   Thr Gly Phe Pro Ser
               260
<210> 347
   <211> 1740
   <212> DNA
   <213> Homo sapiens
<400> 347
   atgaacaaac tgtatatcgg aaacctcagc gagaacgccg ccccctcgga cctagaaagt 60
   atcttcaagg acgccaagat cccggtgtcg ggacccttcc tggtgaagac tggctacgcg 120
   ttcgtggact gcccggacga gagctgggcc ctcaaggcca tcgaggcgct ttcaggtaaa 180
   atagaactgc acgggaaacc catagaagtt gagcactcgg tcccaaaaag gcaaaggatt 240
   cggaaacttc agatacgaaa tatcccgcct catttacagt gggaggtgct ggatagttta 300
   ctagtccagt atggagtggt ggagagctgt gagcaagtga acactgactc ggaaactgca 360
   gttgtaaatg taacctattc cagtaaggac caagctagac aagcactaga caaactgaat 420
   ggatttcagt tagagaattt caccttgaaa gtagcctata tccctgatga aacggccgcc 480
   cagcaaaacc ccttgcagca gccccgaggt cgccgggggc ttgggcagag gggctcctca 540
   aggcaggggt ctccaggatc cgtatccaag cagaaaccat gtgatttgcc tctgcgcctg 600
   ctggttccca cccaatttgt tggagccatc ataggaaaag aaggtgccac cattcggaac 660
   atcaccaaac agacccagtc taaaatcgat gtccaccgta aagaaaatgc gggggctgct 720
   gagaagtcga ttactatcct ctctactcct gaaggcacct ctgcggcttg taagtctatt 780
   ctggagatta tgcataagga agctcaagat ataaaattca cagaagagat ccccttgaag 840
   attttagctc ataataactt tgttggacgt cttattggta aagaaggaag aaatcttaaa 900
   aaaattgagc aagacacaga cactaaaatc acgatatctc cattgcagga attgacgctg 960
   tataatccag aacgcactat tacagttaaa ggcaatgttg agacatgtgc caaagctgag 1020
   gaggagatca tgaagaaaat cagggagtct tatgaaaatg atattgcttc tatgaatctt 1080
   caagcacatt taattcctgg attaaatctg aacgccttgg gtctgttccc acccacttca 1140
   gggatgccac ctcccacctc agggccccct tcagccatga ctcctcccta cccgcagttt 1200
   gagcaatcag aaacggagac tgttcatctg tttatcccag ctctatcagt cggtgccatc 1260
   atcggcaagc agggccagca catcaagcag ctttctcgct ttgctggagc ttcaattaag 1320
   attgctccag cggaagcacc agatgctaaa gtgaggatgg tgattatcac tggaccacca 1380
   gaggctcagt tcaaggctca gggaagaatt tatggaaaaa ttaaagaaga aaactttgtt 1440
   agtcctaaag aagaggtgaa acttgaagct catatcagag tgccatcctt tgctgctggc 1500
   agagttattg gaaaaggagg caaaacggtg aatgaacttc agaatttgtc aagtgcagaa 1560
   gttgttgtcc ctcgtgacca gacacctgat gagaatgacc aagtggttgt caaaataact 1620
   ggtcacttct atgcttgcca ggttgcccag agaaaaattc aggaaattct gactcaggta 1680
   aagcagcacc aacaacagaa ggctctgcaa agtggaccac ctcagtcaag acggaagtaa 1740
<210> 348
   <211> 579
   <212> PRT
   <213> Homo sapiens
<400> 348
   Met Asn Lys Leu Tyr Ile Gly Asn Leu Ser Glu Asn Ala Ala Pro Ser
   5 10 15
   Asp Leu Glu Ser Ile Phe Lys Asp Ala Lys Ile Pro Val Ser Gly Pro
   20 25 30
   Phe Leu Val Lys Thr Gly Tyr Ala Phe Val Asp Cys Pro Asp Glu Ser
   35 40 45
   Trp Ala Leu Lys Ala Ile Glu Ala Leu Ser Gly Lys Ile Glu Leu His
   50 55 60
   Gly Lys Pro Ile Glu Val Glu His Ser Val Pro Lys Arg Gln Arg Ile
   65 70 75 80
   Arg Lys Leu Gln Ile Arg Asn Ile Pro Pro His Leu Gln Trp Glu Val
   85 90 95
   Leu Asp Ser Leu Leu Val Gln Tyr Gly Val Val Glu Ser Cys Glu Gln
   100 105 110
   Val Asn Thr Asp Ser Glu Thr Ala Val Val Asn Val Thr Tyr Ser Ser
   115 120 125
   Lys Asp Gln Ala Arg Gln Ala Leu Asp Lys Leu Asn Gly Phe Gln Leu
   130 135 140
   Glu Asn Phe Thr Leu Lys Val Ala Tyr Ile Pro Asp Glu Thr Ala Ala
   145 150 155 160
   Gln Gln Asn Pro Leu Gln Gln Pro Arg Gly Arg Arg Gly Leu Gly Gln
   165 170 175
   Arg Gly Ser Ser Arg Gln Gly Ser Pro Gly Ser Val Ser Lys Gln Lys
                   180                 185                 190
   Pro Cys Asp Leu Pro Leu Arg Leu Leu Val Pro Thr Gln Phe Val Gly
           195                 200                 205
   Ala Ile Ile Gly Lys Glu Gly Ala Thr Ile Arg Asn Ile Thr Lys Gln
       210                 215                 220
   Thr Gln Ser Lys Ile Asp Val His Arg Lys Glu Asn Ala Gly Ala Ala
   225                 230                 235                 240
   Glu Lys Ser Ile Thr Ile Leu Ser Thr Pro Glu Gly Thr Ser Ala Ala
                   245                 250                 255
   Cys Lys Ser Ile Leu Glu Ile Met His Lys Glu Ala Gln Asp Ile Lys
               260                 265                 270
   Phe Thr Glu Glu Ile Pro Leu Lys Ile Leu Ala His Asn Asn Phe Val
           275                 280                 285
   Gly Arg Leu Ile Gly Lys Glu Gly Arg Asn Leu Lys Lys Ile Glu Gln
       290                 295                 300
   Asp Thr Asp Thr Lys Ile Thr Ile Ser Pro Leu Gln Glu Leu Thr Leu
   305                 310                 315                 320
   Tyr Asn Pro Glu Arg Thr Ile Thr Val Lys Gly Asn Val Glu Thr Cys
                   325                 330                 335
   Ala Lys Ala Glu Glu Glu Ile Met Lys Lys Ile Arg Glu Ser Tyr Glu
               340                 345                 350
   Asn Asp Ile Ala Ser Met Asn Leu Gln Ala His Leu Ile Pro Gly Leu
           355                 360                 365
   Asn Leu Asn Ala Leu Gly Leu Phe Pro Pro Thr Ser Gly Met Pro Pro
       370                 375                 380
   Pro Thr Ser Gly Pro Pro Ser Ala Met Thr Pro Pro Tyr Pro Gln Phe
   385                 390                 395                 400
   Glu Gln Ser Glu Thr Glu Thr Val His Leu Phe Ile Pro Ala Leu Ser
                   405                 410                 415
   Val Gly Ala Ile Ile Gly Lys Gln Gly Gln His Ile Lys Gln Leu Ser
               420                 425                 430
   Arg Phe Ala Gly Ala Ser Ile Lys Ile Ala Pro Ala Glu Ala Pro Asp
           435                 440                 445
   Ala Lys Val Arg Met Val Ile Ile Thr Gly Pro Pro Glu Ala Gln Phe
       450                 455                 460
   Lys Ala Gln Gly Arg Ile Tyr Gly Lys Ile Lys Glu Glu Asn Phe Val
   465                 470                 475                 480
   Ser Pro Lys Glu Glu Val Lys Leu Glu Ala His Ile Arg Val Pro Ser
   485 490 495
   Phe Ala Ala Gly Arg Val Ile Gly Lys Gly Gly Lys Thr Val Asn Glu
   500 505 510
   Leu Gln Asn Leu Ser Ser Ala Glu Val Val Val Pro Arg Asp Gln Thr
   515 520 525
   Pro Asp Glu Asn Asp Gln Val Val Val Lys Ile Thr Gly His Phe Tyr
   530 535 540
   Ala Cys Gln Val Ala Gln Arg Lys Ile Gln Glu Ile Leu Thr Gln Val
   545 550 555 560
   Lys Gln His Gln Gln Gln Lys Ala Leu Gln Ser Gly Pro Pro Gln Ser
   565 570 575
   Arg Arg Lys
<210> 349
   <211> 207
   <212> DNA
   <213> Homo sapiens
<400> 349
   atgtggcagc ccctcttctt caagtggctc ttgtcctgtt gccctgggag ttctcaaatt 60
   gctgcagcag cctccaccca gcctgaggat gacatcaata cacagaggaa gaagagtcag 120
   gaaaagatga gagaagttac agactctcct gggcgacccc gagagcttac cattcctcag 180
   acttcttcac atggtgctaa cagattt 207
<210> 350
   <211> 69
   <212> PRT
   <213> Homo sapiens
<400> 350
   Met Trp Gln Pro Leu Phe Phe Lys Trp Leu Leu Ser Cys Cys Pro Gly
                   5                   10                  15
   Ser Ser Gln Ile Ala Ala Ala Ala Ser Thr Gln Pro Glu Asp Asp Ile
               20                  25                  30
   Asn Thr Gln Arg Lys Lys Ser Gln Glu Lys Met Arg Glu Val Thr Asp
           35                  40                  45
   Ser Pro Gly Arg Pro Arg Glu Leu Thr Ile Pro Gln Thr Ser Ser His
       50                  55                  60
   Gly Ala Asn Arg Phe
   65

## Description

### TECHNICAL FIELD

The present invention relates generally to diagnosis of cancer, such as lung cancer. The invention is more specifically related to polypeptides comprising at least a portion of a lung tumor protein, and to polynucleotides encoding such polypeptides. Such polypeptides and polynucleotides may be used in vaccines and pharmaceutical compositions for prevention and treatment of lung cancer, and for the diagnosis and monitoring of such cancers.

### BACKGROUND OF THE INVENTION

Lung cancer is the primary cause of cancer death among both men and women in the U.S., with an estimated 172,000 new cases being reported in 1994. The five-year survival rate among all lung cancer patients, regardless of the stage of disease at diagnosis, is only 13%. This contrasts with a five-year survival rate of 46% among cases detected while the disease is still localized. However, only 16% of lung cancers are discovered before the disease has spread.

Early detection is difficult since clinical symptoms are often not seen until the disease has reached an advanced stage. Currently, diagnosis is aided by the use of chest x-rays, analysis of the type of cells contained in sputum and fiberoptic examination of the bronchial passages. Treatment regimens are determined by the type and stage of the cancer, and include surgery, radiation therapy and/or chemotherapy. In spite of considerable research into therapies for the disease, lung cancer remains difficult to treat.

Accordingly, there remains a need in the art for improved diagnostic techniques for lung cancer.

### SUMMARY OF THE INVENTION

Briefly stated, the present invention provides compositions and their use in methods for the diagnosis of lung cancer.

In a first aspect of the present invention there is provided a method for detecting the presence of a lung cancer in a patient, comprising the steps of:
(a) contacting a biological sample obtained from the patient with an antibody, or antigen binding fragment thereof, that binds to the polypeptide set forth in SEQ ID NO:176;
(b) detecting in the sample an amount of polypeptide that binds to the binding agent; and
(c) comparing the amount of polypeptide to a predetermined cut-off value and therefrom determining the presence of lung cancer in the patient.

In a second aspect of the present invention there is provided the use of an antibody, or antigen binding fragment thereof, in an *in vitro* method to detect the presence of a lung cancer in a biological sample, wherein the antibody can detect the presence of the polypeptide set forth in SEQ ID NO: 176 in the biological sample.

In a third aspect of the present invention there is provided the use of the polypeptide set forth in SEQ ID NO: 176 in an *in vitro* method to detect the presence of lung cancer in a biological sample, wherein the polypeptide is used to detect the presence of T cells that specifically react therewith in the biological sample.

In a fourth aspect of the present invention there is provided a method for detecting the presence of lung cancer in a patient, the method comprising the steps of:
a) incubating an isolated biological sample comprising CD4+ and/or CD8+ T cells with the polypeptide set forth in SEQ ID NO:176 or a polynucleotide encoding same; and
b) detecting the presence or absence of specific activation of the T cells.

There is also provided the use of oligonucleotide primers in a polymerase chain reaction based assay comprising at least 15 contiguous nucleotides of a DNA molecule set forth in SEQ ID NO: 175 to detect for the presence of lung cancer in a biological sample.

Furthermore, there is also provided a lung cancer diagnostic kit comprising a monoclonal antibody or antigen binding fragment thereof that specifically binds to the polypeptide set forth in SEQ ID NO:176 and a detection reagent, wherein the detection reagent comprises a reporter group.

In a further aspect of the present invention there is provided a lung cancer diagnostic kit comprising at least one oligonucleotide comprising 15-40 contiguous nucleotides of the polynucleotide set forth in SEQ ID NO: 175 and a diagnostic reagent for use in a polymerase chain reaction.

In another aspect of the present invention there is provided a method for determining the presence of a lung cancer in a patient, comprising the steps of:
(a) contacting a biological sample obtained from the patient with an oligonucleotide that hybridizes to the sequence recited in SEQ ID NO:175;
(b) detecting in the sample an amount of a polynucleotide that hybridizes to the oligonucleotide; and
(c) comparing the amount of polynucleotide that hybridizes to the oligonucleotide to a predetermined cut-off value, and therefrom determining the presence of the lung cancer in the patient.

There is also disclosed polypeptides comprising at least a portion of a lung tumor protein, or a variant thereof. Certain portions and other variants are immunogenic, such that the ability of the variant to react with antigen-specific antisera is not substantially diminished. Within certain disclosed embodiments, the polypeptide comprises a sequence that is encoded by a polynucleotide sequence selected from the group consisting of: (a) sequences recited in any one of SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347 and 349; (b) variants of a sequence recited in any one of SEQ ID NO: 1-3, 6-8,10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347 and 349; and (c) complements of a sequence of (a) or (b). In specific embodiments, the polypeptides of the present disclosure comprise at least a portion of a tumor protein that includes an amino acid sequence selected from the group consisting of sequences recited in any one of SEQ ID NO: 152,155,156,165,166,169,170,172,174,176, 226-252,338-344 and 346, and variants thereof.

The present disclosure further provides polynucleotides that encode a polypeptide as described above, or a portion thereof (such as a portion encoding at least 15 amino acid residues of a lung tumor protein), expression vectors comprising such polynucleotides and host cells transformed or transfected with such expression vectors.

Within other aspects, the present disclosure provides pharmaceutical compositions comprising a polypeptide or polynucleotide as described above and a physiologically acceptable carrier.

Within a related aspect of the present disclosure, vaccines for prophylactic or therapeutic use are provided. Such vaccines comprise a polypeptide or polynucleotide as described above and an immunostimulant.

The present disclosure further provides pharmaceutical compositions that comprise: (a) an antibody or antigen-binding fragment thereof that specifically binds to a-lung tumor protein; and (b) a physiologically acceptable carrier.

Within-further aspects, the present disclosure provides pharmaceutical compositions comprising: (a) an antigen presenting cell that expresses a polypeptide as described above and (b) a pharmaceutically acceptable carrier or excipient Antigen presenting cells include dendritic cells, macrophages, monocytes, fibroblasts and B cells.

Within related disclosed aspects, vaccines are provided that comprise: (a) an antigen presenting cell that expresses a polypeptide as described above, and (b) an immunostimulant.

The present disclosure further provides, in other aspects, fusion proteins that comprise at least one polypeptide as described above, as well as polynucleotides encoding such fusion proteins.

Within related disclosed aspects, pharmaceutical compositions comprising a fusion protein, or a polynucleotide encoding a fusion protein, In combination with a physiologically acceptable carrier are provided.

Vaccines are disclosed that comprise a fusion protein, or a polynucleotide encoding a fusion protein, in combination with an immunostimulant.

Within further aspects, the present disclosure provides methods for inhibiting the development of a cancer in a patient, comprising administering to a patient a pharmaceutical composition or vaccine as recited above.

The present disclosure further provides, methods for removing tumor cells from a biological sample, comprising contacting a biological sample with T cells that specifically react with a lung tumor protein, wherein the step of contacting is performed under conditions and for a time sufficient to permit the removal of cells expressing the protein from the sample.

Methods are also disclosed for inhibiting the development of a cancer in a patient, comprising administering to a patient a biological sample treated as described above.

Methods are disclosed, for stimulating and/or expanding T cells specific for a lung tumor protein, comprising contacting T cells with one or more of (i) a polypeptide as described above; (ii) a polynucleotide encoding such a polypeptide; and/or (iii) an antigen presenting cell that expresses such a polypeptide; under conditions and for a time sufficient to permit the stimulation and/or expansion of T cells. Determined T cell populations comprising T cells prepared as described above are also provided.

Methods for inhibiting the development of a cancer in a patient are disclosed, comprising administering to a patient an effective amount of a T cell population as described above.

Methods for inhibiting the development of a cancer in a patient are also disclosed, comprising the steps of: (a) incubating CD4⁺ and/or CD8⁺ T cells determined from a patient with one or more of: (i) a polypeptide comprising at least an immunogenic portion of a lung tumor protein; (ii) a polynucleotide encoding such a polypeptide; and (iii) an antigen-presenting cell that expressed such a polypeptide; and (b) administering to the patient an effective amount of the proliferated T cells, and thereby inhibiting the development of a cancer in the patient Proliferated cells may, but need not, be cloned prior to administration to the patient

Within further aspects, the present invention provides methods for determining the presence or absence of lung cancer in a patient, comprising: (a) contacting a biological sample obtained, from a patient with an antibody that binds to the polypeptide of the present invention; (b) detecting in the sample an amount of polypeptide that binds to the binding agent; and (c) comparing the amount of polypeptide with a predetermined cut-off value, and therefrom determining the presence or absence of a cancer in the patient. Within preferred embodiments, the binding agent is preferably a monoclonal antibody.

The present invention also provides, within other aspects, methods for monitoring the progression of lung cancer in a patient. Such methods comprise the steps of: (a) contacting a biological sample obtained from a patient at a first point in time with a antibody that binds to a polypeptide as recited above; (b) detecting in the sample an amount of polypeptide that binds to the binding agent; (c) repeating steps (a) and (b) using a biological sample obtained from the patient at a subsequent point in time; and (d) comparing the amount of polypeptide detected in step (c) with the amount detected in step (b) and therefrom monitoring the progression of the cancer in the patient

The present invention further provides, within other aspects, methods for determining the presence or absence of lung cancer in a patient, comprising the steps of: (a) contacting a biological sample obtained from a patient with an oligonucleotide that hybridizes to a polynucleotide that encodes a lung tumor protein of the present invention; (b) detecting in the sample a level of a polynucleotide, preferably mRNA, that hybridizes to the oligonucleotide; and (c) comparing the level of polynucleotide that hybridizes to the oligonucleotide with a predetermined cut-off value, and therefrom determining the presence or absence of a cancer in the patient. Within certain embodiments, the amount of mRNA is detected via polymerase chain reaction using, for example, at least one oligonucleotide primer that hybridizes to a polynucleotide encoding a polypeptide as recited above, or a complement of such a polynucleotide. Within other embodiments, the amount of mRNA is detected using a hybridization technique, employing an oligonucleotide probe that hybridizes to a polynucleotide that encodes a polypeptide as recited above, or a complement of such a polynucleotide.

In related aspects, methods are provided for monitoring the progression of lung cancer in a patient, comprising the steps of: (a) contacting a biological sample obtained from a patient with an oligonucleotide that hybridizes to a polynucleotide that encodes a lung tumor protein of the present invention; (b) detecting in the sample an amount of a polynucleotide that hybridizes to the oligonucleotide; (c) repeating steps (a) and (b) using a biological sample obtained from the patient at a subsequent point in time; and (d) comparing the amount of polynucleotide detected in step (c) with the amount detected in step (b) and therefrom monitoring the progression of the cancer in the patient.

Within further aspects, the present disclosure provides antibodies, such as monoclonal antibodies, that bind to a polypeptide as described above, as well as diagnostic kits comprising such antibodies. Diagnostic kits comprising one or more oligonucleotide probes or primers as described above are also provided. These and other aspects of the present invention will become apparent upon reference to the following detailed description and attached drawings.

### SEQUENCE IDENTIFIERS

SEQ ID NO: 1 is the determined cDNA sequence for LST-S1-2
SEQ ID NO: 2 is the determined cDNA sequence for LST-S1-28
SEQ ID NO: 3 is the determined cDNA sequence for LST-S1-90
SEQ ID NO: 4 is the determined cDNA sequence for LST-S1-144
SEQ ID NO: 5 is the determined cDNA sequence for LST-S1-133
SEQ ID NO: 6 is the determined cDNA sequence for LST-S1-169
SEQ ID NO: 7 is the determined cDNA sequence for LST-S2-6
SEQ ID NO: 8 is the determined cDNA sequence for LST-S2-11
SEQ ID NO: 9 is the determined cDNA sequence for LST-S2-17
SEQ ID NO: 10 is the determined cDNA sequence for LST-S2-25
SEQ ID NO: 11 is the determined cDNA sequence for LST-S2-39
SEQ ID NO: 12 is a first determined cDNA sequence for LST-S2-43
SEQ ID NO: 13 is a second determined cDNA sequence for LST-S2-43
SEQ ID NO: 14 is the determined cDNA sequence for LST-S2-65
SEQ ID NO: 15 is the determined cDNA sequence for LST-S2-68
SEQ ID NO: 16 is the determined cDNA sequence for LST-S2-72
SEQ ID NO: 17 is the determined cDNA sequence for LST-S2-74
SEQ ID NO: 18 is the determined cDNA sequence for LST-S2-103
SEQ ID NO: 19 is the determined cDNA sequence for LST-S2-N1-1F
SEQ ID NO: 20 is the determined cDNA sequence for LST-S2-N1-2A
SEQ ID NO: 21 is the determined cDNA sequence for LST-S2-N1-4H
SEQ ID NO: 22 is the determined cDNA sequence for LST-S2-N1-5A
SEQ ID NO: 23 is the determined cDNA sequence for LST-S2-N1-6B
SEQ ID NO: 24 is the determined cDNA sequence for LST-S2-N1-7B
SEQ ID NO: 25 is the determined cDNA sequence for LST-S2-N1-7H
SEQ ID NO: 26 is the determined cDNA sequence for LST-S2-N1-8A
SEQ ID NO: 27 is the determined cDNA sequence for LST-S2-N1-8D
SEQ ID NO: 28 is the determined cDNA sequence for LST-S2-N1-9A
SEQ ID NO: 29 is the determined cDNA sequence for LST-S2-N1-9E
SEQ ID NO: 30 is the determined cDNA sequence for LST-S2-N1-10A
SEQ ID NO: 31 is the determined cDNA sequence for LST-S2-N1-10G
SEQ ID NO: 32 is the determined cDNA sequence for LST-S2-N1-11A
SEQ ID NO: 33 is the determined cDNA sequence for LST-S2-N1-12C
SEQ ID NO: 34 is the determined cDNA sequence for LST-S2-N1-12E
SEQ ID NO: 35 is the determined cDNA sequence for LST-S2-B1-3D
SEQ ID NO: 36 is the determined cDNA sequence for LST-S2-B1-6C
SEQ ID NO: 37 is the determined cDNA sequence for LST-S2-B1-SD
SEQ ID NO: 38 is the determined cDNA sequence for LST-S2-B1-SF
SEQ ID NO: 39 is the determined cDNA sequence for LST-S2-B1-6G
SEQ ID NO: 40 is the determined cDNA sequence for LST-S2-B1-8A
SEQ ID NO: 41 is the determined cDNA sequence for LST-S2-B1-8D
SEQ ID NO: 42 is the determined cDNA sequence for LST-S2-B1-10A
SEQ ID NO: 43 is the determined cDNA sequence for LST-S2-B1-9B
SEQ ID NO: 44 is the determined cDNA sequence for LST-S2-B1-9F
SEQ ID NO: 45 is the determined cDNA sequence for LST-S2-B1-12D
SEQ ID NO: 46 is the determined cDNA sequence for LST-S2-I2-2B
SEQ ID NO: 47 is the determined cDNA sequence for LST-S2-I2-5F
SEQ ID NO: 48 is the determined cDNA sequence for LST-S2-I2-6B
SEQ ID NO: 49 is the determined cDNA sequence for LST-S2-I2-7F
SEQ ID NO: 50 is the determined cDNA sequence for LST-S2-I2-8G
SEQ ID NO: 51 is the determined cDNA sequence for LST-S2-I2-9E
SEQ ID NO: 52 is the determined cDNA sequence for LST-S2-I2-12B
SEQ ID NO: 53 is the determined cDNA sequence for LST-S2-H2-2C
SEQ ID NO: 54 is the determined cDNA sequence for LST-S2-H2-1G
SEQ ID NO: 55 is the determined cDNA sequence for LST-S2-H2-4G
SEQ ID NO: 56 is the determined cDNA sequence for LST-S2-H2-3H
SEQ ID NO: 57 is the determined cDNA sequence for LST-S2-H2-5G
SEQ ID NO: 58 is the determined cDNA sequence for LST-S2-H2-9B
SEQ ID NO: 59 is the determined cDNA sequence for LST-S2-H2-10H
SEQ ID NO: 60 is the determined cDNA sequence for LST-S2-H2-12D
SEQ ID NO: 61 is the determined cDNA sequence for LST-S3-2
SEQ ID NO: 62 is the determined cDNA sequence for LST-S3-4
SEQ ID NO: 63 is the determined cDNA sequence for LST-S3-7
SEQ ID NO: 64 is the determined cDNA sequence for LST-S3-8
SEQ ID NO: 65 is the determined cDNA sequence for LST-S3-12
SEQ ID NO: 66 is the determined cDNA sequence for LST-S3-13
SEQ ID NO: 67 is the determined cDNA sequence for LST-S3-14
SEQ ID NO: 68 is the determined cDNA sequence for LST-S3-16
SEQ ID NO: 69 is the determined cDNA sequence for LST-S3-21
SEQ ID NO: 70 is the determined cDNA sequence for LST-S3-22
SEQ ID NO: 71 is the determined cDNA sequence for LST-S1-7
SEQ ID NO: 72 is the determined cDNA sequence for LST-S1-A-1E
SEQ ID NO: 73 is the determined cDNA sequence for LST-S1-A-1G
SEQ ID NO: 74 is the determined cDNA sequence for LST-S1-A-3E
SEQ ID NO: 75 is the determined cDNA sequence for LST-S1-A-4E
SEQ ID NO: 76 is the determined cDNA sequence for LST-S1-A-6D
SEQ ID NO: 77 is the determined cDNA sequence for LST-S1-A-8D
SEQ ID NO: 78 is the determined cDNA sequence for LST-S1-A-10A
SEQ ID NO: 79 is the determined cDNA sequence for LST-S1-A-10C
SEQ ID NO: 80 is the determined cDNA sequence for LST-S1-A-9D
SEQ ID NO: 81 is the determined cDNA sequence for LST-S1-A-10D
SEQ ID NO: 82 is the determined cDNA sequence for LST-S1-A-9H
SEQ ID NO: 83 is the determined cDNA sequence for LST-S1-A-11D
SEQ ID NO: 84 is the determined cDNA sequence for LST-S1-A-12D
SEQ ID NO: 85 is the determined cDNA sequence for LST-S1-A-11E
SEQ ID NO: 86 is the determined cDNA sequence for LST-S1-A-12E
SEQ ID NO: 87 is the determined cDNA sequence for L513S (T3).
SEQ ID NO: 88 is the determined cDNA sequence for L513S contig 1.
SEQ ID NO: 89 is a first determined cDNA sequence for L514S.
SEQ ID NO: 90 is a second determined cDNA sequence for L514S.
SEQ ID NO: 91 is a first determined cDNA sequence for L516S.
SEQ ID NO: 92 is a second determined cDNA sequence for L516S.
SEQ ID NO: 93 is the determined cDNA sequence for L517S.
SEQ ID NO: 94 is the extended cDNA sequence for LST-S1-169 (also known as L519S).
SEQ ID NO: 95 is a first determined cDNA sequence for L520S.
SEQ ID NO: 96 is a second determined cDNA sequence for L520S.
SEQ ID NO: 97 is a first determined cDNA sequence for L521S.
SEQ ID NO: 98 is a second determined cDNA sequence for L521 S.
SEQ ID NO: 99 is the determined cDNA sequence for L522S.
SEQ ID NO: 100 is the determined cDNA sequence for L523S.
SEQ ID NO: 101 is the determined cDNA sequence for L524S.
SEQ ID NO: 102 is the determined cDNA sequence for L525S.
SEQ ID NO: 103 is the determined cDNA sequence for L526S.
SEQ ID NO: 104 is the determined cDNA sequence for L527S.
SEQ ID NO: 105 is the determined cDNA sequence for L528S.
SEQ ID NO: 106 is the determined cDNA sequence for L529S.
SEQ ID NO: 107 is a first determined cDNA sequence for L530S.
SEQ ID NO: 108 is a second determined cDNA sequence for L530S.
SEQ ID NO: 109 is the determined full-length cDNA sequence for L531 S short form
SEQ ID NO: 110 is the predicted amino acid sequence encoded by SEQ ID NO: 109.
SEQ ID NO: 111 is the determined full-length cDNA sequence for L531S long form
SEQ ID NO: 112 is the predicted amino acid sequence encoded by SEQ ID NO: 111.
SEQ ID NO: 113 is the determined full-length cDNA sequence for L520S.
SEQ ID NO: 114 is the predicted amino acid sequence encoded by SEQ ID NO: 113.
SEQ ID NO: 115 is the determined cDNA sequence for contig 1.
SEQ ID NO: 116 is the determined cDNA sequence for contig 3.
SEQ ID NO: 117 is the determined cDNA sequence for contig 4.
SEQ ID NO: 118 is the determined cDNA sequence for contig 5.
SEQ ID NO: 119 is the determined cDNA sequence for contig 7.
SEQ ID NO: 120 is the determined cDNA sequence for contig 8.
SEQ ID NO: 121 is the determined cDNA sequence for contig 9.
SEQ ID NO: 122 is the determined cDNA sequence for contig 10.
SEQ ID NO: 123 is the determined cDNA sequence for contig 12.
SEQ ID NO: 124 is the determined cDNA sequence for contig 11.
SEQ ID NO: 125 is the determined cDNA sequence for contig 13.
SEQ ID NO: 126 is the determined cDNA sequence for contig 15.
SEQ ID NO: 127 is the determined cDNA sequence for contig 16.
SEQ ID NO: 128 is the determined cDNA sequence for contig 17.
SEQ ID NO: 129 is the determined cDNA sequence for contig 19.
SEQ ID NO: 130 is the determined cDNA sequence for contig 20.
SEQ ID NO: 131 is the determined cDNA sequence for contig 22.
SEQ ID NO: 132 is the determined cDNA sequence for contig 24.
SEQ ID NO: 133 is the determined cDNA sequence for contig 29.
SEQ ID NO: 134 is the determined cDNA sequence for contig 31.
SEQ ID NO: 135 is the determined cDNA sequence for contig 33.
SEQ ID NO: 136 is the determined cDNA sequence for contig 38.
SEQ ID NO: 137 is the determined cDNA sequence for contig 39.
SEQ ID NO: 138 is the determined cDNA sequence for contig 41.
SEQ ID NO: 139 is the determined cDNA sequence for contig 43.
SEQ ID NO: 140 is the determined cDNA sequence for contig 44.
SEQ ID NO: 141 is the determined cDNA sequence for contig 45.
SEQ ID NO: 142 is the determined cDNA sequence for contig 47.
SEQ ID NO: 143 is the determined cDNA sequence for contig 48.
SEQ ID NO: 144 is the determined cDNA sequence for contig 49.
SEQ ID NO: 145 is the determined cDNA sequence for contig 50.
SEQ ID NO: 146 is the determined cDNA sequence for contig 53.
SEQ ID NO: 147 is the determined cDNA sequence for contig 54.
SEQ ID NO: 148 is the determined cDNA sequence for contig 56.
SEQ ID NO: 149 is the determined cDNA sequence for contig 57.
SEQ ID NO: 150 is the determined cDNA sequence for contig 58.
SEQ ID NO: 151 is the full-length cDNA sequence for L530S.
SEQ ID NO: 152 is the amino acid sequence encoded by SEQ ID NO: 151
SEQ ID NO: 153 is the full-length cDNA sequence of a first variant of L514S
SEQ ID NO: 154 is the full-length cDNA sequence of a second variant of L514S
SEQ ID NO: 155 is the amino acid sequence encoded by SEQ ID NO: 153.
SEQ ID NO: 156 is the amino acid sequence encoded by SEQ ID NO: 154.
SEQ ID NO: 157 is the determined cDNA sequence for contig 59.
SEQ ID NO: 158 is the full-length cDNA sequence for L763P (also referred to as contig 22).
SEQ ID NO: 159 is the amino acid sequence encoded by SEQ ID NO: 158.
SEQ ID NO: 160 is the full-length cDNA sequence for L762P (also referred to as contig 17).
SEQ ID NO: 161 is the amino acid sequence encoded by SEQ ID NO: 160.
SEQ ID NO: 162 is the determined cDNA sequence for L515S.
SEQ ID NO: 163 is the full-length cDNA sequence of a first variant of L524S.
SEQ ID NO: 164 is the full-length cDNA sequence of a second variant of L524S.
SEQ ID NO: 165 is the amino acid sequence encoded by SEQ ID NO: 163.
SEQ ID NO: 166 is the amino acid sequence encoded by SEQ ID NO: 164.
SEQ ID NO: 167 is the full-length cDNA sequence of a first variant of L762P.
SEQ ID NO: 168 is the full-length cDNA sequence of a second variant of L762P.
SEQ ID NO: 169 is the amino acid sequence encoded by SEQ ID NO: 167.
SEQ ID NO: 170 is the amino acid sequence encoded by SEQ ID NO: 168.
SEQ ID NO: 171 is the full-length cDNA sequence for L773P (also referred to as contig 56).
SEQ ID NO: 172 is the amino acid sequence encoded by SEQ ID NO: 171.
SEQ ID NO: 173 is an extended cDNA sequence for L519S.
SEQ ID NO: 174 is the predicted amino acid sequence encoded by SEQ ID NO: 174.
SEQ ID NO: 175 is the full-length cDNA sequence for L523S.
SEQ ID NO: 176 is the predicted amino acid sequence encoded by SEQ ID NO: 175.
SEQ ID NO: 177 is the determined cDNA sequence for LST-sub5-7A.
SEQ ID NO: 178 is the determined cDNA sequence for LST-sub5-8G.
SEQ ID NO: 179 is the determined cDNA sequence for LST-sub5-8H.
SEQ ID NO: 180 is the determined cDNA sequence for LST-sub5-10B.
SEQ ID NO: 181 is the determined cDNA sequence for LST-sub5-10H.
SEQ ID NO: 182 is the determined cDNA sequence for LST-sub5-12B.
SEQ ID NO: 183 is the determined cDNA sequence for LST-sub5-11C.
SEQ ID NO: 184 is the determined cDNA sequence for LST-sub6-1c.
SEQ ID NO: 185 is the determined cDNA sequence for LST-sub6-2f.
SEQ ID NO: 186 is the determined cDNA sequence for LST-sub6-2G.
SEQ ID NO: 187 is the determined cDNA sequence for LST-sub6-4d.
SEQ ID NO: 188 is the determined cDNA sequence for LST-sub6-4e.
SEQ ID NO: 189 is the determined cDNA sequence for LST-sub6-4f.
SEQ ID NO: 190 is the determined cDNA sequence for LST-sub6-3h.
SEQ ID NO: 191 is the determined cDNA sequence for LST-sub6-5d.
SEQ ID NO: 192 is the determined cDNA sequence for LST-sub6-5h.
SEQ ID NO: 193 is the determined cDNA sequence for LST-sub6-6h.
SEQ ID NO: 194 is the determined cDNA sequence for LST-sub6-7a.
SEQ ID NO: 195 is the determined cDNA sequence for LST-sub6-8a.
SEQ ID NO: 196 is the determined cDNA sequence for LST-sub6-7d.
SEQ ID NO: 197 is the determined cDNA sequence for LST-sub6-7e.
SEQ ID NO: 198 is the determined cDNA sequence for LST-sub6-8e.
SEQ ID NO: 199 is the determined cDNA sequence for LST-sub6-7g.
SEQ ID NO: 200 is the determined cDNA sequence for LST-sub6-9f.
SEQ ID NO: 201 is the determined cDNA sequence for LST-sub6-9h.
SEQ ID NO: 202 is the determined cDNA sequence for LST-sub6-11b.
SEQ ID NO: 203 is the determined cDNA sequence for LST-sub6-11c.
SEQ ID NO: 204 is the determined cDNA sequence for LST-sub6-12c.
SEQ ID NO: 205 is the determined cDNA sequence for LST-sub6-12e.
SEQ ID NO: 206 is the determined cDNA sequence for LST-sub6-12f.
SEQ ID NO: 207 is the determined cDNA sequence for LST-sub6-11g.
SEQ ID NO: 208 is the determined cDNA sequence for LST-sub6-12g.
SEQ ID NO: 209 is the determined cDNA sequence for LST-sub6-12h.
SEQ ID NO: 210 is the determined cDNA sequence for LST-sub6-II-1a.
SEQ ID NO: 211 is the determined cDNA sequence for LST-sub6-II-2b.
SEQ ID NO: 212 is the determined cDNA sequence for LST-sub6-II-2g.
SEQ ID NO: 213 is the determined cDNA sequence for LST-sub6-II-1h.
SEQ ID NO: 214 is the determined cDNA sequence for LST-sub6-II-4a.
SEQ ID NO: 215 is the determined cDNA sequence for LST-sub6-II-4b.
SEQ ID NO: 216 is the determined cDNA sequence for LST-sub6-II-3e.
SEQ ID NO: 217 is the determined cDNA sequence for LST-sub6-II-4f.
SEQ ID NO: 218 is the determined cDNA sequence for LST-sub6-II-4g.
SEQ ID NO: 219 is the determined cDNA sequence for LST-sub6-II-4h.
SEQ ID NO: 220 is the determined cDNA sequence for LST-sub6-II-5c.
SEQ ID NO: 221 is the determined cDNA sequence for LST-sub6-II-5e.
SEQ ID NO: 222 is the determined cDNA sequence for LST-sub6-II-6f.
SEQ ID NO: 223 is the determined cDNA sequence for LST-sub6-II-5g.
SEQ ID NO: 224 is the determined cDNA sequence for LST-sub6-II-6g.
SEQ ID NO: 225 is the amino acid sequence for L528S.
SEQ ID NO: 226-251 are synthetic peptides derived from L762P.
SEQ ID NO: 252 is the expressed amino acid sequence of L514S.
SEQ ID NO: 253 is the DNA sequence corresponding to SEQ ID NO: 252.
SEQ ID NO: 254 is the DNA sequence of a L762P expression construct.
SEQ ID NO: 255 is the determined cDNA sequence for clone 23785.
SEQ ID NO: 256 is the determined cDNA sequence for clone 23786.
SEQ ID NO: 257 is the determined cDNA sequence for clone 23788.
SEQ ID NO: 258 is the determined cDNA sequence for clone 23790.
SEQ ID NO: 259 is the determined cDNA sequence for clone 23793.
SEQ ID NO: 260 is the determined cDNA sequence for clone 23794.
SEQ ID NO: 261 is the determined cDNA sequence for clone 23795.
SEQ ID NO: 262 is the determined cDNA sequence for clone 23796.
SEQ ID NO: 263 is the determined cDNA sequence for clone 23797.
SEQ ID NO: 264 is the determined cDNA sequence for clone 23798.
SEQ ID NO: 265 is the determined cDNA sequence for clone 23799.
SEQ ID NO: 266 is the determined cDNA sequence for clone 23800.
SEQ ID NO: 267 is the determined cDNA sequence for clone 23802.
SEQ ID NO: 268 is the determined cDNA sequence for clone 23803.
SEQ ID NO: 269 is the determined cDNA sequence for clone 23804.
SEQ ID NO: 270 is the determined cDNA sequence for clone 23805.
SEQ ID NO: 271 is the determined cDNA sequence for clone 23806.
SEQ ID NO: 272 is the determined cDNA sequence for clone 23807.
SEQ ID NO: 273 is the determined cDNA sequence for clone 23808.
SEQ ID NO: 274 is the determined cDNA sequence for clone 23809.
SEQ ID NO: 275 is the determined cDNA sequence for clone 23810.
SEQ ID NO: 276 is the determined cDNA sequence for clone 23811.
SEQ ID NO: 277 is the determined cDNA sequence for clone 23812.
SEQ ID NO: 278 is the determined cDNA sequence for clone 23813.
SEQ ID NO: 279 is the determined cDNA sequence for clone 23815.
SEQ ID NO: 280 is the determined cDNA sequence for clone 25298.
SEQ ID NO: 281 is the determined cDNA sequence for clone 25299.
SEQ ID NO: 282 is the determined cDNA sequence for clone 25300.
SEQ ID NO: 283 is the determined cDNA sequence for clone 25301
SEQ ID NO: 284 is the determined cDNA sequence for clone 25304
SEQ ID NO: 285 is the determined cDNA sequence for clone 25309.
SEQ ID NO: 286 is the determined cDNA sequence for clone 25312.
SEQ ID NO: 287 is the determined cDNA sequence for clone 25317.
SEQ ID NO: 288 is the determined cDNA sequence for clone 25321.
SEQ ID NO: 289 is the determined cDNA sequence for clone 25323.
SEQ ID NO: 290 is the determined cDNA sequence for clone 25327.
SEQ ID NO: 291 is the determined cDNA sequence for clone 25328.
SEQ ID NO: 292 is the determined cDNA sequence for clone 25332.
SEQ ID NO: 293 is the determined cDNA sequence for clone 25333.
SEQ ID NO: 294 is the determined cDNA sequence for clone 25336.
SEQ ID NO: 295 is the determined cDNA sequence for clone 25340.
SEQ ID NO: 296 is the determined cDNA sequence for clone 25342.
SEQ ID NO: 297 is the determined cDNA sequence for clone 25356.
SEQ ID NO: 298 is the determined cDNA sequence for clone 25357.
SEQ ID NO: 299 is the determined cDNA sequence for clone 25361.
SEQ ID NO: 300 is the determined cDNA sequence for clone 25363.
SEQ ID NO: 301 is the determined cDNA sequence for clone 25397.
SEQ ID NO: 302 is the determined cDNA sequence for clone 25402.
SEQ ID NO: 303 is the determined cDNA sequence for clone 25403.
SEQ ID NO: 304 is the determined cDNA sequence for clone 25405.
SEQ ID NO: 305 is the determined cDNA sequence for clone 25407.
SEQ ID NO: 306 is the determined cDNA sequence for clone 25409.
SEQ ID NO: 307 is the determined cDNA sequence for clone 25396.
SEQ ID NO: 308 is the determined cDNA sequence for clone 25414.
SEQ ID NO: 309 is the determined cDNA sequence for clone 25410.
SEQ ID NO: 310 is the determined cDNA sequence for clone 25406.
SEQ ID NO: 311 is the determined cDNA sequence for clone 25306.
SEQ ID NO: 312 is the determined cDNA sequence for clone 25362.
SEQ ID NO: 313 is the determined cDNA sequence for clone 25360.
SEQ ID NO: 314 is the determined cDNA sequence for clone 25398.
SEQ ID NO: 315 is the determined cDNA sequence for clone 25355.
SEQ ID NO: 316 is the determined cDNA sequence for clone 25351.
SEQ ID NO: 317 is the determined cDNA sequence for clone 25331.
SEQ ID NO: 318 is the determined cDNA sequence for clone 25338.
SEQ ID NO: 319 is the determined cDNA sequence for clone 25335.
SEQ ID NO: 320 is the determined cDNA sequence for clone 25329.
SEQ ID NO: 321 is the determined cDNA sequence for clone 25324.
SEQ ID NO: 322 is the determined cDNA sequence for clone 25322.
SEQ ID NO: 323 is the determined cDNA sequence for clone 25319.
SEQ ID NO: 324 is the determined cDNA sequence for clone 25316.
SEQ ID NO: 325 is the determined cDNA sequence for clone 25311.
SEQ ID NO: 326 is the determined cDNA sequence for clone 25310.
SEQ ID NO: 327 is the determined cDNA sequence for clone 25302.
SEQ ID NO: 328 is the determined cDNA sequence for clone 25315.
SEQ ID NO: 329 is the determined cDNA sequence for clone 25308.
SEQ ID NO: 330 is the determined cDNA sequence for clone 25303.
SEQ ID NO: 331-337 are the cDNA sequences of isoforms of the p53 tumor suppressor homologue, p63 (also referred to as L530S).
SEQ ID NO: 338-344 are the amino acid sequences encoded by SEQ ID NO: 331-337, respectively.
SEQ ID NO: 345 is a second cDNA sequence for the antigen L763P.
SEQ ID NO: 346 is the amino acid sequence encoded by the sequence of SEQ ID NO: 345.
SEQ ID NO: 347 is a determined full-length cDNA sequence for L523S.
SEQ ID NO: 348 is the predicted amino acid sequence encoded by SEQ ID NO: 347.
SEQ ID NO: 349 is the cDNA sequence encoding the N-terminal portion of L773P.
SEQ ID NO: 350 is the amino acid sequence of the N-terminal portion of L773P.

### DETAILED DESCRIPTION OF THE INVENTION

As noted above, the present invention is generally directed to methods for the diagnosis of lung cancer and the use of composition in such methods. The compositions described herein may include lung tumor polypeptides, polynucleotides encoding such polypeptides, binding agents such as antibodies, antigen presenting cells (APCs) and/or immune system cells (*e.g*., T cells). Polypeptides of the present invention generally comprise at least a portion (such as an immunogenic portion) of a lung tumor protein or a variant thereof. A "lung tumor protein" is a protein that is expressed in lung tumor cells at a level that is at least two fold, and preferably at least five fold, greater than the level of expression in a normal tissue, as determined using a representative assay provided herein. Certain lung tumor proteins are tumor proteins that react detectably (within an immunoassay, such as an ELISA or Western blot) with antisera of a patient afflicted with lung cancer. Polynucleotides of the subject invention generally comprise a DNA or RNA sequence that encodes all or a portion of such a polypeptide, or that is complementary to such a sequence. Antibodies are generally immune system proteins, or antigen-binding fragments thereof, that are capable of binding to a polypeptide as described above. Antigen presenting cells include dendritic cells, macrophages, monocytes, fibroblasts and B-cells that express a polypeptide as described above. T cells that may be employed within such compositions are generally T cells that are specific for a polypeptide as described above.

The present invention is based on the discovery human lung tumor proteins. The Sequence of the polynucleotide encoding the specific tumor protein of the invention is provided in SEQ ID NO: 175.

### LUNG TUMOR PROTEIN POLYNUCLEOTIDES

Any polynucleotide that encodes a lung tumor protein or a portion or other variant thereof as described herein is encompassed by the present disclosure. Preferred polynucleotides comprise at least 15 consecutive nucleotides, preferably at least 30 consecutive nucleotides and more preferably at least 45 consecutive nucleotides, that encode a portion of a lung tumor protein. More preferably, a polynucleotide encodes an immunogenic portion of a lung tumor protein. Polynucleotides complementary to any such sequences are also encompassed by the present invention. Polynucleotides may be single-stranded (coding or antisense) or double-stranded, and may be DNA (genomic, cDNA or synthetic) or RNA molecules. RNA molecules include HnRNA, molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*i*.*e*., an endogenous sequence that encodes a lung tumor protein or a portion thereof) or may comprise a variant of such a sequence. Polynucleotide variants may contain one or more substitutions, additions, deletions ared/or insertions such that the immunogenicity of the encoded polypeptide is not diminished, relative to a native tumor protein. The effect on the immunogenicity of the encoded polypeptide may generally be assessed as described herein. Variants preferably exhibit at least about 70% identity, more preferably at least about 80% identity and most preferably at least about 90% identity to a polynucleotide sequence that encodes a native lung tumor protein or a portion thereof. The term "variants" also encompasses homologous genes of xenogenic origin.

Two polynucleotide or polypeptide sequences are said to be "identical" if the sequence of nucleotides or amino acids in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W. and Muller W. (1988) CABIOS 4:11-17; Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl. Acad., Sci. USA 80:726-730.

Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (i.e. gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e. the window size) and multiplying the results by 100 to yield the percentage of sequence identity.

Variants may also, or alternatively, be substantially homologous to a native gene, or a portion or complement thereof. Such polynucleotide variants are capable of hybridizing under moderately stringent conditions to a naturally occurring DNA sequence encoding a native lung tumor protein (or a complementary sequence). Suitable moderately stringent conditions include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1 % SDS.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Polynucleotides may be prepared using any of a variety of techniques. For example, a polynucleotide may be identified, as described in more detail below, by screening a microarray of cDNAs for tumor-associated expression (*i.e*., expression that is at least two fold greater in a lung tumor than in normal tissue, as determined using a representative assay provided herein). Such screens may be performed using a Synteni microarray (Palo Alto, CA) according to the manufacturer's instructions (and essentially as described by Schena et al., Proc. Natl. Acad. Sci. USA 93:10614-10619, 1996 and Heller et al., Proc. Natl. Acad. Sci. USA 94:2150-2155, 1997). Alternatively, polypeptides may be amplified from cDNA prepared from cells expressing the proteins described herein, such as lung tumor cells. Such polynucleotides may be amplified via polymerase chain reaction (PCR). For this approach, sequence-specific primers may be designed based on the sequences provided herein, and may be purchased or synthesized.

An amplified portion may be used to isolate a full length gene from a suitable library (*e.g*., a lung tumor cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (*e.g*., by nick-translation or end-labeling with ³²P) using well known techniques. A bacterial or bacteriophage library is then screened by hybridizing filters containing denatured bacterial colonies (or lawns containing phage plaques) with the labeled probe *(see* Sambrook et al., Molecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using, for example, software well known in the art. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR *(see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridizes to a polyA region or vector sequence, to identify sequences that are 5' and 3' of a known sequence. Additional techniques include capture PCR (Lagerstrom et al., PCR Methods Applic. 1:111-19, 1991) and walking PCR (Parker et al., Nucl. Acids. Res. 19:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (*e.g*., NCBI BLAST searches), and such ESTs may be used to generate a contiguous full length sequence. Full length DNA sequences may also be obtained by analysis of genomic fragments.

Certain nucleic acid sequences of cDNA molecules encoding portions of lung tumor proteins are provided in SEQ ID NO: 1-109, 111, 113, 115-151, 153, 154,157, 158, 160, 162-164, 167, 168, 171, 173, 175, 177-224, 255-337, 345, 347 and 349.

Polynucleotide variants may generally be prepared by any method known in the art, including chemical synthesis by, for example, solid phase phosphoramidite chemical synthesis. Modifications in a polynucleotide sequence may also be introduced using standard mutagenesis techniques, such as oligonucleotide-directed site-specific mutagenesis (*see* Adelman et al., DNA 2:183, 1983). Alternatively, RNA molecules may be generated by *in vitro* or *in vivo* transcription of DNA sequences encoding a lung tumor protein, or portion thereof, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo* (*e.g.,* by transfecting antigen-presenting cells, such as dendritic cells, with a cDNA construct encoding a lung tumor polypeptide, and administering the transfected cells to the patient).

A portion of a sequence complementary to a coding sequence (*i.e*., an antisense polynucleotide) may also be used as a probe or to modulate gene expression. cDNA constructs that can be transcribed into antisense RNA may also be introduced into cells of tissues to facilitate the production of antisense RNA. An antisense polynucleotide may be used, as described herein, to inhibit expression of a tumor protein. Antisense technology can be used to control gene expression through triple-helix formation, which compromises the ability of the double helix to open sufficiently for the binding of polymerases, transcription factors or regulatory molecules *(see* Gee et al., *In* Huber and Carr, Molecular and Immunologic Approaches, Futura Publishing Co. (Mt. Kisco, NY; 1994)). Alternatively, an antisense molecule may be designed to hybridize with a control region of a gene (*e.g.*, promoter, enhancer or transcription initiation site), and block transcription of the gene; or to block translation by inhibiting binding of a transcript to ribosomes.

A portion of a coding sequence, or of a complementary sequence, may also be designed as a probe or primer to detect gene expression. Probes may be labeled with a variety of reporter groups, such as radionuclides and enzymes, and are preferably at least 10 nucleotides in length, more preferably at least 20 nucleotides in length and still more preferably at least 30 nucleotides in length. Primers, as noted above, are preferably 22-30 nucleotides in length.

Any polynucleotide may be further modified to increase stability *in vivo*. Possible modifications include, but are not limited to, the addition of flanking sequences at the 5' and/or 3' ends; the use of phosphorothioate or 2' 0-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Within certain embodiments, polynucleotides may be formulated so as to permit entry into a cell of a mammal, and expression therein. Such formulations are particularly useful for therapeutic purposes, as described below. Those of ordinary skill in the art will appreciate that there are many ways to achieve expression of a polynucleotide in a target cell, and any suitable method may be employed. For example, a polynucleotide may be incorporated into a viral vector such as, but not limited to, adenovirus, adeno-associated virus, retrovirus, or vaccinia or other pox virus (*e.g.*, avian pox virus).). The polynucleotides may also be administered as naked plasmid vectors. Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome (*i.e.*, an artificial membrane vesicle). The preparation and use of such systems is well known in the art.

### LUNG TUMOR POLYPEPTIDES

Within the context of the present disclosure, polypeptides may comprise at least an immunogenic portion of a lung tumor protein or a variant thereof, as described herein. As noted above, a "lung tumor protein" is a protein that is expressed by lung tumor cells Proteins that are lung tumor proteins also react detectably within an immunoassay (such as an ELISA) with antisera from a patient with lung cancer. Polypeptides as described herein may be of any length. Additional sequences derived from the native protein and/or heterologous sequences may be present, and such sequences may (but need not) possess further immunogenic or antigenic properties.

An "immunogenic portion," as used herein is a portion of a protein that is recognized (*i*,*e*., specifically bound) by a B-cell and/or T-cell surface antigen receptor. Such immunogenic portions generally comprise at least 5 amino acid residues, more preferably at least 10, and still more preferably at least 20 amino acid residues of a lung tumor protein or a variant thereof. Certain preferred immunogenic portions include peptides in which an N-terminal leader sequence and/or transmembrane domain have been deleted. Other preferred immunogenic portions may contain a small N- and/or C-terminal deletion (*e.g.*, 1-30 amino acids, preferably 5-15 amino acids), relative to the mature protein.

Immunogenic portions may generally be identified using well known techniques, such as those summarized in Paul, Fundamental Immunology, 3rd ed., 243-247 (Raven Press, 1993) and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e*., they react with the protein in an ELISA or other immunoassay, and do not react detectably with unrelated proteins). Such antisera and antibodies may be prepared as described herein, and using well known techniques. An immunogenic portion of a native lung tumor protein is a portion that reacts with such antisera and/or T-cells at a level that is not substantially less than the reactivity of the full length polypeptide (*e.g*., in an ELISA and/or T-cell reactivity assay). Such immunogenic portions may react within such assays at a level that is similar to or greater than the reactivity of the full length polypeptide. Such screens may generally be performed using methods well known to those of ordinary skill in the art, such as those described in Harlow and Lane, Antibodies: A Laboratory Manual, Cold Spring Harbor Laboratory, 1988. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As noted above, a composition may comprise a variant of a native lung tumor protein. A polypeptide "variant," as used herein, is a polypeptide that differs from a native lung tumor protein in one or more substitutions, deletions, additions and/or insertions, such that the immunogenicity of the polypeptide is not substantially diminished. In other words, the ability of a variant to react with antigen-specific antisera may be enhanced or unchanged, relative to the native protein, or may be diminished by less than 50%, and preferably less than 20%, relative to the native protein. Such variants may generally be identified by modifying one of the above polypeptide sequences and evaluating the reactivity of the modified polypeptide with antigen-specific antibodies or antisera as described herein. Preferred variants include those in which one or more portions, such as an N-terminal leader sequence or transmembrane domain, have been removed. Other preferred variants include variants in which a small portion (*e.g.*, 1-30 amino acids, preferably 5-15 amino acids) has been removed from the N- and/or C-terminal of the mature protein.

Polypeptide variants preferably exhibit at least about 70%, more preferably at least about 90% and most preferably at least about 95% identity (determined as described above) to the identified polypeptides.

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g.*, poly-His), or to enhance binding of the polypeptide to a solid support. For example, a polypeptide may be conjugated to an immunoglobulin Fc region.

Polypeptides may be prepared using any of a variety of well known techniques. Recombinant polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast, higher eukaryotic and plant cells. Preferably, the host cells employed are *E. coli,* yeast or a mammalian cell line such as COS or CHO. Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Portions and other variants having fewer than about 100 amino acids, and generally fewer than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Perkin Elmer/Applied BioSystems Division (Foster City, CA), and may be operated according to the manufacturer's instructions.

Within certain specific embodiments, a polypeptide may be a fusion protein that comprises multiple polypeptides as described herein, or that comprises at least one polypeptide as described herein and an unrelated sequence, such as a known tumor protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion protein that retains the biological activity of both component polypeptides.

A peptide linker sequence may be employed to separate the first and the second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Fusion proteins are also provided that comprise a polypeptide of the present invention together with an unrelated immunogenic protein. Preferably the immunogenic protein is capable of eliciting a recall response. Examples of such proteins include tetanus, tuberculosis and hepatitis proteins (*see,* for example, Stoute et al. New Engl. J. Med., 336:86-91, 1997).

Within preferred embodiments, an immunological fusion partner is derived from protein D, a surface protein of the gram-negative bacterium Haemophilus influenza B (WO 91/18926). Preferably, a protein D derivative comprises approximately the first third of the protein (*e.g.*, the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E. coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae,* which synthesizes an N-acetyl-L-alanine amidase known as amidase LYTA (encoded by the LytA gene; Gene 43:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described (*see* Biotechnology 10:795-798, 1992). Within a preferred embodiment, a repeat portion of LYTA, may be incorporated into a fusion protein. A repeat portion is found in the C-terminal region starting at residue 178. A particularly preferred repeat portion incorporates residues 188-305.

In general, polypeptides (including fusion proteins) and polynucleotides as described herein are isolated. An "isolated" polypeptide or polynucleotide is one that is removed from its original environment For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

### BINDING AGENTS

The present disclosure further provides agents, such as antibodies and antigen-binding fragments thereof, that specifically bind to a lung tumor protein. As used herein, an antibody, or antigen-binding fragment thereof, is said to "specifically bind" to a lung tumor protein if it reacts at a detectable level (within, for example, an ELISA) with a lung tumor protein, and does not react detectably with unrelated proteins under similar conditions. As used herein, "binding" refers to a noncovalent association between two separate molecules such that a complex is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about 10³ L/mol- The binding constant may be determined using methods well known in the art.

Binding agents may be further capable of differentiating between patients with and without a cancer, such as lung cancer, using the representative assays provided herein. In other words, antibodies or other binding agents that bind to a lung tumor protein will generate a signal indicating the presence of a cancer in at least about 20% of patients with the disease, and will generate a negative signal indicating the absence of the disease in at least about 90% of individuals without the cancer. To determine whether a binding agent satisfies this requirement, biological samples (*e.g.*, blood, sera, sputum urine and/or tumor biopsies) from patients with and without a cancer (as determined using standard clinical tests) may be assayed as described herein for the presence of polypeptides that bind to the binding agent. It will be apparent that a statistically significant number of samples with and without the disease should be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g.,* Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (*e.g.*, mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, Antibodies: A Laboratory Manual, Cold Spring Harbor Laboratory, 1988) and digested by papain to yield Fab and Fc fragments. The Fab and Fc fragments may be separated by affinity chromatography on protein A bead columns.

Monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (*e.g.*, covalently bonded) to a suitable monoclonal antibody either directly or indirectly (*e.g.*, via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e.g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g.*, U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e.g*., U.S. Patent No. 4,489,710, to Spitler), by irradiation of a photolabile bond (*e.g*., U.S. Patent No. 4,625,014, to Senter et al.), by hydrolysis of derivatized amino acid side chains (*e.g*., U.S. Patent No. 4,638,045, to Kohn et al.), by serum complement-mediated hydrolysis (*e.g*., U.S. Patent No. 4,671,958, to Rodwell et al.), and acid-catalyzed hydrolysis (*e.g*., U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (*e.g.*, U.S. Patent No. 4,507,234, to Kato et al.), peptides and polysaccharides such as aminodextran (*e.g*., U.S. Patent No. 4,699,784, to Shih et al.). A carrier may also bear an agent by noncovalent bonding or by encapsulation, such as within a liposome vesicle (*e.g*., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

### T CELLS

Immunotherapeutic compositions may also, or alternatively, comprise T cells specific for a lung tumor protein. Such cells may generally be prepared *in vitro* or *ex vivo,* using standard procedures. For example, T cells may be isolated from bone marrow, peripheral blood, or a fraction of bone marrow or peripheral blood of a patient, using a commercially available cell separation system, such as the Isolex^{™} System, available from Nexell Therapeutics, Inc. Irvine, CA (see also U.S. Patent No. 5,240,856; U.S. Patent No. 5,215,926; WO 89/06280; WO 91/16116 and WO 92/07243). Alternatively, T cells may be derived from related or unrelated humans, non-human mammals, cell lines or cultures.

T cells may be stimulated with a lung tumor polypeptide, polynucleotide encoding a lung tumor polypeptide and/or an antigen presenting cell (APC) that expresses such a polypeptide. Such stimulation is performed under conditions and for a time sufficient to permit the generation of T cells that are specific for the polypeptide. Preferably, a lung tumor polypeptide or polynucleotide is present within a delivery vehicle, such as a microsphere, to facilitate the generation of specific T cells.

T cells are considered to be specific for a lung tumor polypeptide if the T cells specifically proliferate, secrete cytokines or kill target cells coated with the polypeptide or expressing a gene encoding the polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070, 1994. Alternatively, detection of the proliferation of T cells may be accomplished by a variety of known techniques. For example, T cell proliferation can be detected by measuring an increased rate of DNA synthesis (*e*.*g*., by pulse-labeling cultures of T cells with tritiated thymidine and measuring the amount of tritiated thymidine incorporated into DNA). Contact with a lung tumor polypeptide (100 ng/ml - 100 µg/ml, preferably 200 µg/ml - 25 µg/ml) for 3 - 7 days should result in at least a two fold increase in proliferation of the T cells. Contact as described above for 2-3 hours should result in activation of the T cells, as measured using standard cytokine assays in which a two fold increase in the level of cytokine release *(e.g.,* TNF or IFN-γ) is indicative of T cell activation (*see* Coligan et al., Current Protocols in Immunology, vol. 1, Wiley Interscience (Greene 1998)). T cells that have been activated in response to a lung tumor polypeptide, polynucleotide or polypeptide-expressing APC may be CD4⁺ and/or CD8⁺. Lung tumor protein-specific T cells may be expanded using standard techniques. Within preferred embodiments, the T cells are derived from either a patient or a related, or unrelated, donor and are administered to the patient following stimulation and expansion.

For therapeutic purposes, CD4⁺ or CD8⁺ T cells that proliferate in response to a lung tumor polypeptide, polynucleotide or APC can be expanded in number either *in vitro* or *in vivo*. Proliferation of such T cells *in vitro* may be accomplished in a variety of ways. For example, the T cells can be re-exposed to a lung tumor polypeptide, or a short peptide corresponding to an immunogenic portion of such a polypeptide, with or without the addition of T cell growth factors, such as interleukin-2, and/or stimulator cells that synthesize a lung tumor polypeptide. Alternatively, one or more T cells that proliferate in the presence of a lung tumor protein can be expanded in number by cloning. Methods for cloning cells are well known in the art, and include limiting dilution.

### PHARMACEUTICAL COMPOSITIONS AND VACCINES

Polypeptides, polynucleotides, T cells and/or binding agents disclosed herein may be incorporated into pharmaceutical compositions or immunogenic compositions (*i.e.,* vaccines). Pharmaceutical compositions comprise one or more such compounds and a physiologically acceptable carrier. Vaccines may comprise one or more such compounds and an immunostimulant. An immunostimulant may be any substance that enhances or potentiates an immune response to an exogenous antigen. Examples of immunostimulants include adjuvants, biodegradable microspheres (*e.g*., polylactic galactide) and liposomes (into which the compound is incorporated; *see e.g.,* Fullerton, U.S. Patent No. 4,235,877). Vaccine preparation is generally described in, for example, M.F. Powell and M.J. Newman, eds., "Vaccine Design (the subunit and adjuvant approach)," Plenum Press (NY, 1995). Pharmaceutical compositions and vaccines within the scope of the present invention may also contain other compounds, which may be biologically active or inactive. For example, one or more immunogenic portions of other tumor antigens may be present, either incorporated into a fusion polypeptide or as a separate compound, within the composition or vaccine.

A pharmaceutical composition or vaccine may contain DNA encoding one or more of the polypeptides as described above, such that the polypeptide is generated *in situ*. As noted above, the DNA may be present within any of a variety of delivery systems known to those of ordinary skill in the art, including nucleic acid expression systems, bacteria and viral expression systems. Numerous gene delivery techniques are well known in the art, such as those described by Rolland, Crit. Rev. Therap. Drug Carrier Systems 15:143-198, 1998, and references cited therein. Appropriate nucleic acid expression systems contain the necessary DNA sequences for expression in the patient (such as a suitable promoter and terminating signal). Bacterial delivery systems involve the administration of a bacterium (such as *Bacillus-Calmette-Guerrin*) that expresses an immunogenic portion of the polypeptide on its cell surface or secretes such an epitope. In a preferred embodiment, the DNA may be introduced using a viral expression system (*e.g*., vaccinia or other pox virus, retrovirus, or adenovirus), which may involve the use of a non-pathogenic (defective), replication competent virus. Suitable systems are disclosed, for example, in Fisher-Hoch et al., Proc. Natl. Acad Sci. USA 86:317-321, 1989; Flexner et al., Ann. N.Y. Acad Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad Sci. USA 91:215-219, 1994; Kass-Eisler et al., Proc. Natl. Acad. Sci. USA 90:11498-11502,1993; Guzman et al., Circulation 88:2838-2848, 1993; and Guzman et al., Cir. Res. 73:1202-1207, 1993. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g.,* polylactate polyglycolate) may also be employed as carriers for the pharmaceutical compositions of this invention. Suitable biodegradable microspheres are disclosed, for example, in U.S. Patent Nos. 4,897,268 and 5,075,109.

Such compositions may also comprise buffers (*e.g*., neutral buffered saline or phosphate buffered saline), carbohydrates (*e*.*g*., glucose, mannose, sucrose or dextrans), mannitol, proteins, polypeptides or amino acids such as glycine, antioxidants, chelating agents such as EDTA or glutathione, adjuvants (*e.g*., aluminum hydroxide) and/or preservatives. Alternatively, compositions of the present invention may be formulated as a lyophilizate. Compounds may also be encapsulated within liposomes using well known technology.

Any of a variety of immunostimulants may be employed in the vaccines of this invention. For example, an adjuvant may be included. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

Within the vaccines provided herein, the adjuvant composition is preferably designed to induce an immune response predominantly of the Th1 type. High levels of Th1-type cytokines (*e.g.*, IFN-γ, TNFα, IL-2 and IL-12) tend to favor the induction of cell mediated immune responses to an administered antigen. In contrast, high levels of Th2-type cytokines (*e.g.*, IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Thl-type, the level of Th1type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Ribi ImmunoChem Research Inc. (Hamilton, MT) (*see* US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555 and WO 99/33488. Immunostimulatory DNA sequences are also described, for example, by Sato et al., Science 273:352, 1996. Another preferred adjuvant is a saponin, preferably QS21 (Aquila Biopharmaceuticals Inc., Framingham, MA), which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprises an oil-in-water emulsion and tocopherol. A particularly potent adjuvant formulation involving QS21, 3D-MPL and tocopherol in an oil-in-water emulsion is described in WO 95/17210.

Other preferred adjuvants include Montanide ISA 720 (Seppic, France), SAF (Chiron, California, United States), ISCOMS (CSL), MF-59 (Chiron), the SBAS series of adjuvants (*e.g*., SBAS-2 or SBAS-4, available from SmithKline Beecham, Rixensart, Belgium), Detox (Ribi ImmunoChem Research Inc., Hamilton, MT), RC-529 (Ribi ImmunoChem Research Inc., Hamilton, MT) and Aminoalkyl glucosaminide 4-phosphates (AGPs).

Any vaccine provided herein may be prepared using well known methods that result in a combination of antigen, immune response enhancer and a suitable carrier or excipient. The compositions described herein may be administered as part of a sustained release formulation (*i.e.,* a formulation such as a capsule, sponge or gel (composed of polysaccharides, for example) that effects a slow release of compound following administration). Such formulations may generally be prepared using well known technology (*see, e.g.* Coombes et al., Vaccine 14:1429-1438, 1996) and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane.

Carriers for use within such formulations are biocompatible, and may also be biodegradable; preferably the formulation provides a relatively constant level of active component release. Such carriers include microparticles of poly(lactide-co-glycolide), as well as polyacrylate, latex, starch, cellulose and dextran. Other delayed-release carriers include supramolecular biovectors, which comprise a non-liquid hydrophilic core (*e.g*., a cross-linked polysaccharide or oligosaccharide) and, optionally, an external layer comprising an amphiphilic compound, such as a phospholipid (*see e.g.*, U.S. Patent No- 5,151,254 and PCT applications WO 94/20078, WO/94/23701 and WO 96/06638). The amount of active compound contained within a sustained release formulation depends upon the site of implantation, the rate and expected duration of release and the nature of the condition to be treated or prevented.

Any of a variety of delivery vehicles may be employed within pharmaceutical compositions and vaccines to facilitate production of an antigen-specific immune response that targets tumor cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver (*i*.*e*., matched HLA haplotype). APCs may generally be isolated from any of a variety of biological fluids and organs, including tumor and peritumoral tissues, and may be autologous, allogeneic, syngeneic or xenogeneic cells.

Certain preferred embodiments of the present disclosure use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic antitumor immunity (*see* Timmerman and Levy, Ann Rev. Med. 50:507-529, 1999). In general, dendritic cells may be identified based on their typical shape (stellate *in situ,* with marked cytoplasmic processes (dendrites) visible *in vitro*), their ability to take up, process and present antigens with high efficiency, and their ability to activate naive T cell responses. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine (*see* Zitvogel et al., Nature Med. 4:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e.g*., CD54 and CD11) and costimulatory molecules (*e.g*., CD40, CD80, CD86 and 4-1BB).

APCs may generally be transfected with a polynucleotide encoding a lung tumor protein (or portion or other variant thereof) such that the lung tumor polypeptide, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the lung tumor polypeptide, DNA (naked or within a plasmid vector) or RNA; or with antigen-expressing recombinant bacterium or viruses (*e*.*g*., vaccinia, fowlpox, adenovirus or lentivirus vectors) Prior to loading, the polypeptide may be covalently conjugated to an immunological partner that provides T cell help (*e.g*., a carrier molecule). Alternatively, a dendritic cell may be pulsed with a non-conjugated immunological partner, separately or in the presence of the polypeptide.

Vaccines and pharmaceutical compositions may be presented in unit-dose or multi-dose containers, such as sealed ampoules or vials. Such containers are preferably hermetically sealed to preserve sterility of the formulation until use. In general, formulations may be stored as suspensions, solutions or emulsions in oily or aqueous vehicles. Alternatively, a vaccine or pharmaceutical composition may be stored in a freeze-dried condition requiring only the addition of a sterile liquid carrier immediately prior to use

### CANCER THERAPY

The compositions described herein may be used for immunotherapy of cancer, such as lung cancer. Within such methods, pharmaceutical compositions and vaccines are typically administered to a patient. As used herein, a "patient" refers to any warm-blooded animal, preferably a human. A patient may or may not be afflicted with cancer. Accordingly, the above pharmaceutical compositions and vaccines may be used to prevent the development of a cancer or to treat a patient afflicted with a cancer. A cancer may be diagnosed using criteria generally accepted in the art, including the presence of a malignant tumor. Pharmaceutical compositions and vaccines may be administered either prior to or following surgical removal of primary tumors and/or treatment such as administration of radiotherapy or conventional chemotherapeutic drugs.

Within certain embodiments, immunotherapy may be active immunotherapy, in which treatment relies on the *in vivo* stimulation of the endogenous host immune system to react against tumors with the administration of immune response-modifying agents (such as polypeptides and polynucleotides disclosed herein).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T cells as discussed above, T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a polypeptide provided herein. T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage, monocyte, fibroblast and/or B cells, may be pulsed with immunoreactive polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow in vivo and to survive long term in substantial numbers by repeated stimulation with antigen supplemented with IL-2 (*see*, for example, Cheever et al., Immunological Reviews 157:177, 1997).

Alternatively, a vector expressing a polypeptide recited herein may be introduced into antigen presenting cells taken from a patient and clonally propagated *ex vivo* for transplant back into the same patient. Transfected cells may be reintroduced into the patient using any means known in the art, preferably in sterile form by intravenous, intracavitary, intraperitoneal or intratumor administration.

Routes and frequency of administration of the therapeutic compositions disclosed herein, as well as dosage, will vary from individual to individual, and may be readily established using standard techniques. In general, the pharmaceutical compositions and vaccines may be administered by injection (*e.g*., intracutaneous, intramuscular, intravenous or subcutaneous), intranasally (*e.g.*, by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i.e*., untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e.g*., more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 25 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (*e.g*., more frequent remissions, complete or partial, or longer disease-free survival) in treated patients as compared to non-treated patients. Increases in preexisting immune responses to a lung tumor protein generally correlate with an improved clinical outcome. Such immune responses may generally be evaluated using standard proliferation, cytotoxicity or cytokine assays, which may be performed using samples obtained from a patient before and after treatment.

### METHODS FOR DETECTING CANCER

In general, a cancer may be detected in a patient based on the presence of one or more lung tumor proteins and/or polynucleotides encoding such proteins in a biological sample (for example, blood, sera, sputum urine and/or tumor biopsies) obtained from the patient. In other words, such proteins may be used as markers to indicate the presence or absence of a cancer such as lung cancer. In addition, such proteins may be useful for the detection of other cancers. The binding agents provided herein generally permit detection of the level of antigen that binds to the agent in the biological sample. Polynucleotide primers and probes may be used to detect the level of mRNA encoding a tumor protein, which is also indicative of the presence or absence of a cancer. In general, a lung tumor sequence should be present at a level that is at least three fold higher in tumor tissue than in normal tissue

There are a variety of assay formats known to those of ordinary skill in the art for using a binding agent to detect polypeptide markers in a sample. *See, e.g.,* Harlow and Lane, Antibodies: A Laboratory Manual, Cold Spring Harbor Laboratory, 1988. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length lung tumor proteins and portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*see, e.g.,* Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20^{™} (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i.e.*, incubation time) is a period of time that is sufficient to detect the presence of polypeptide within a sample obtained from an individual with lung cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20^{™}. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as lung cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value may be determined from a plot of pairs of true positive rates (*i.e.*, sensitivity) and false positive rates (100%-specificity) that correspond to each possible cut-off value for the diagnostic test result. The cut-off value on the plot that is the closest to the upper left-hand corner (*i.e.,* the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use lung tumor polypeptides to detect antibodies that bind to such polypeptides in a biological sample. The detection of such lung tumor protein specific antibodies may correlate with the presence of a cancer.

A cancer may also, or alternatively, be detected based on the presence of T cells that specifically react with a lung tumor protein in a biological sample. Within certain methods, a biological sample comprising CD4⁺ and/or CD8⁺ T cells isolated from a patient is incubated with a lung tumor polypeptide, a polynucleotide encoding such a polypeptide and/or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with polypeptide (*e.g.*, 5-25 µg/ml). It may be desirable to incubate another aliquot of a T cell sample in the absence of lung tumor polypeptide to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding a lung tumor protein in a biological sample. For example, at least two oligonucleotide primers may be employed in a polymerase chain reaction (PCR) based assay to amplify a portion of a lung tumor cDNA derived from a biological sample, wherein at least one of the oligonucleotide primers is specific for (*i.e*., hybridizes to) a polynucleotide encoding the lung tumor protein. The amplified cDNA is then separated and detected using techniques well known in the art, such as gel electrophoresis. Similarly, oligonucleotide probes that specifically hybridize to a polynucleotide encoding a lung tumor protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a lung tumor protein that is at least 10 nucleotides, and preferably at least 20 nucleotides, in length. Preferably, oligonucleotide primers and/or probes will hybridize to a polynucleotide encoding a polypeptide disclosed herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence recited in SEQ ID NO: 1-109, 111, 113, 115-151, 153, 154, 157, 158, 160, 162-164, 167, 168, 171, 173, 175, 177-224, 255-337, 345, 347 and 349. Techniques for both PCR based assays and hybridization assays are well known in the art *(see,* for example, Mullis et al., *Cold Spring Harbor Symp. Quant. Biol., 51*:263, 1987; Erlich ed., *PCR Technology,* Stockton Press, NY, 1989).

One preferred assay employs RT-PCR, in which PCR is applied in conjunction with reverse transcription. Typically, RNA is extracted from a biological sample, such as biopsy tissue, and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another embodiment, the disclosed compositions may be used as markers for the progression of cancer. In this embodiment, assays as described above for the diagnosis of a cancer may be performed over time, and the change in the level of reactive polypeptide(s) or polynucleotide evaluated. For example, the assays may be performed every 24-72 hours for a period of 6 months to 1 year, and thereafter performed as needed. In general, a cancer is progressing in those patients in whom the level of polypeptide or polynucleotide detected increases over time. In contrast, the cancer is not progressing when the level of reactive polypeptide or polynucleotide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple lung tumor protein markers may be assayed within a given sample. It will be apparent that binding agents specific for different proteins provided herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

### DIAGNOSTIC KITS

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a lung tumor protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding a lung tumor protein in a biological sample. Such kits generally comprise at least one oligonucleotide probe or primer, as described above, that hybridizes to a polynucleotide encoding a lung tumor protein. Such an oligonucleotide may be used, for example, within a PCR or hybridization assay. Additional components that may be present within such kits include a second oligonucleotide and/or a diagnostic reagent or container to facilitate the detection of a polynucleotide encoding a lung tumor protein.

The following Examples are offered by way of illustration and not by way of limitation.

### EXAMPLE 1

### ISOLATION AND CHARACTERIZATION OF cDNA SEQUENCES ENCODING LUNG TUMOR POLYPEPTIDES

This example illustrates the isolation of cDNA molecules encoding lung tumor-specific polypeptides from lung tumor cDNA libraries.

### A. ISOLATION OF cDNA SEQUENCES FROM A LUNG SQUAMOUS CELL CARCINOMA LIBRARY

A human lung squamous cell carcinoma cDNA expression library was constructed from poly A⁺ RNA from a pool of two patient tissues using a Superscript Plasmid System for cDNA Synthesis and Plasmid Cloning kit (BRL Life Technologies, Gaithersburg, MD) following the manufacturer's protocol. Specifically, lung carcinoma tissues were homogenized with polytron (Kinematica, Switzerland) and total RNA was extracted using Trizol reagent (BRL Life Technologies) as directed by the manufacturer. The poly A⁺ RNA was then purified using an oligo dT cellulose column as described in Sambrook et al., Molecular Cloning: A Laboratory Manual, Cold Spring Harbor Laboratories, Cold Spring Harbor, NY, 1989. First-strand cDNA was synthesized using the NotI/Oligo-dT18 primer. Double-stranded cDNA was synthesized, ligated with BstXI/EcoRI adaptors (Invitrogen, San Diego, CA) and digested with NotI. Following size fractionation with cDNA size fractionation columns (BRL Life Technologies), the cDNA was ligated into the BstXI/NotI site of pcDNA3.1 (Invitrogen) and transformed into ElectroMax *E. coli* DH10B cells (BRL Life Technologies) by electroporation.

Using the same procedure, a normal human lung cDNA expression library was prepared from a pool of four tissue specimens. The cDNA libraries were characterized by determining the number of independent colonies, the percentage of clones that carried insert, the average insert size and by sequence analysis. The lung squamous cell carcinoma library contained 2.7 x 10⁶ independent colonies, with 100% of clones having an insert and the average insert size being 2100 base pairs. The normal lung cDNA library contained 1.4 x 10⁶ independent colonies, with 90% of clones having inserts and the average insert size being 1800 base pairs. For both libraries, sequence analysis showed that the majority of clones had a full length cDNA sequence and were synthesized from mRNA

cDNA library subtraction was performed using the above lung squamous cell carcinoma and normal lung cDNA libraries, as described by Hara et al. (Blood, 84:189-199, 1994) with some modifications. Specifically, a lung squamous cell carcinoma-specific subtracted cDNA library was generated as follows. Normal tissue cDNA library (80 µg) was digested with BamHI and XhoI, followed by a filling-in reaction with DNA polymerase Klenow fragment. After phenol-chloroform extraction and ethanol precipitation, the DNA was dissolved in 133 µl of H₂O, heat-denatured and mixed with 133 µl (133 µg) of Photoprobe biotin (Vector Laboratories, Burlingame, CA). As recommended by the manufacturer, the resulting mixture was irradiated with a 270 W sunlamp on ice for 20 minutes. Additional Photoprobe biotin (67 µl) was added and the biotinylation reaction was repeated. After extraction with butanol five times, the DNA was ethanol-precipitated and dissolved in 23 µl H₂O to form the driver DNA.

To form the tracer DNA, 10 µg lung squamous cell carcinoma cDNA library was digested with NotI and SpeI, phenol chloroform extracted and passed through Chroma spin-400 columns (Clontech, Palo Alto, CA). Typically, 5 µg of cDNA was recovered after the sizing column. Following ethanol precipitation, the tracer DNA was dissolved in 5 µl H₂O. Tracer DNA was mixed with 15 µl driver DNA and 20 µl of 2 x hybridization buffer (1.5 M NaCl/10 mM EDTA/50 mM HEPES pH 7.5/0.2% sodium dodecyl sulfate), overlaid with mineral oil, and heat-denatured completely. The sample was immediately transferred into a 68 °C water bath and incubated for 20 hours (long hybridization [LH]). The reaction mixture was then subjected to a streptavidin treatment followed by phenol/chloroform extraction. This process was repeated three more times. Subtracted DNA was precipitated, dissolved in 12 µl H₂O, mixed with 8 µl driver DNA and 20 µl of 2 x hybridization buffer, and subjected to a hybridization at 68 °C for 2 hours (short hybridization [SH]). After removal of biotinylated double-stranded DNA, subtracted cDNA was ligated into NotI/SpeI site of chloramphenicol resistant pBCSK⁺ (Stratagene, La Jolla, CA) and transformed into ElectroMax *E. coli* DH10B cells by electroporation to generate a lung squamous cell carcinoma specific subtracted cDNA library (herein after referred to as "lung subtraction I").

A second lung squamous cell carcinoma specific subtracted cDNA library (referred to as "lung subtraction II") was generated in a similar way to the lung subtraction library I, except that eight frequently recovered genes from lung subtraction I were included in the driver DNA, and 24,000 independent clones were recovered.

To analyze the subtracted cDNA libraries, plasmid DNA was prepared from 320 independent clones, randomly picked from the subtracted lung squamous cell carcinoma specific libraries. Representative cDNA clones were further characterized by DNA sequencing with a Perkin Elmer/Applied Biosystems Division Automated Sequencer Model 373A and/or Model 377 (Foster City, CA). The cDNA sequences for sixty isolated clones are provided in SEQ ID NO: 1-60. These sequences were compared to known sequences in the gene bank using the EMBL and GenBank databases (release 96). No significant homologies were found to the sequences provided in SEQ ID NO: 2, 3, 19, 38 and 46. The sequences of SEQ ID NO: 1, 6-8, 10-13, 15, 17, 18, 20-27, 29, 30, 32, 34-37, 39-45, 47-49, 51, 52, 54, 55 and 57-59 were found to show some homology to previously identified expressed sequence tags (ESTs). The sequences of SEQ ID NO: 9, 28, 31 and 33 were found to show some homology to previously identified non-human gene sequences and the sequences of SEQ ID NO: 4, 5, 14, 50, 53, 56 and 60 were found to show some homology to gene sequences previously identified in humans.

The subtraction procedure described above was repeated using the above lung squamous cell carcinoma cDNA library as the tracer DNA, and the above normal lung tissue cDNA library and a cDNA library from normal liver and heart (constructed from a pool of one sample of each tissue as described above), plus twenty other cDNA clones that were frequently recovered in lung subtractions I and II, as the driver DNA (lung subtraction III). The normal liver and heart cDNA library contained 1.76 x 10⁶ independent colonies, with 100% of clones having inserts and the average insert size being 1600 base pairs. Ten additional clones were isolated (SEQ ID NO: 61-70). Comparison of these cDNA sequences with those in the gene bank as described above, revealed no significant homologies to the sequences provided in SEQ ID NO: 62 and 67. The sequences of SEQ ID NO: 61, 63-66, 68 and 69 were found to show some homology to previously isolated ESTs and the sequence provided in SEQ ID NO: 70 was found to show some homology to a previously identified rat gene.

In further studies, the subtraction procedure described above was repeated using the above lung squamous cell carcinoma cDNA library as the tracer DNA, and a cDNA library from a pool of normal lung, kidney, colon, pancreas, brain, resting PBMC, heart, skin and esophagus as the driver DNA, with esophagus cDNAs making up one third of the driver material. Since esophagus is enriched in normal epithelial cells, including differentiated squamous cells, this procedure is likely to enrich genes that are tumor specific rather than tissues specific. The cDNA sequences of 48 clones determined in this subtraction are provided in SEQ ID NO: 177-224. The sequences of SEQ ID NO: 177, 178, 180, 181, 183, 187, 192, 195-197, 208, 211, 212, 215, 216, 218 and 219 showed some homology to previously identified genes. The sequences of SEQ ID NO: 179, 182, 184-186, 188-191, 193, 194, 198-207, 209 210, 213, 214, 217, 220 and 224 showed some homology to previously determined ESTs. The sequence of SEQ ID NO: 221-223 showed no homology to any previously determined sequence.

### B. ISOLATION OF cDNA SEQUENCES FROM A LUNG ADENOCARCINOMA LIBRARY

A human lung adenocarcinoma cDNA expression library was constructed as described above. The library contained 3.2 x 10⁶ independent colonies, with 100% of clones having an insert and the average insert size being 1500 base pairs. Library subtraction was performed as described above using the normal lung and normal liver and heart cDNA expression libraries described above as the driver DNA. Twenty-six hundred independent clones were recovered.

Initial cDNA sequence analysis from 100 independent clones revealed many ribosomal protein genes. The cDNA sequences for fifteen clones isolated in this subtraction are provided in SEQ ID NO: 71-86. Comparison of these sequences with those in the gene bank as described above revealed no significant homologies to the sequence provided in SEQ ID NO: 84. The sequences of SEQ ID NO: 71, 73, 74, 77, 78 and 80-82 were found to show some homology to previously isolated ESTs, and the sequences of SEQ ID NO: 72, 75, 76, 79, 83 and 85 were found to show some homology to previously identified human genes.

In further studies, a cDNA library (referred to as mets3616A) was constructed from a metastatic lung adenocarcinoma. The determined cDNA sequences of 25 clones sequenced at random from this library are provided in SEQ ID NO: 255-279. The mets3616A cDNA library was subtracted against a cDNA library prepared from a pool of normal lung, liver, pancreas, skin, kidney, brain and resting PBMC. To increase the specificity of the subtraction, the driver was spiked with genes that were determined to be most abundant in the mets3616A cDNA library, such as EF1-alpha, integrin-beta and anticoagulant protein PP4, as well as with cDNAs that were previously found to be differentially expressed in subtracted lung adenocarcinoma cDNA libraries. The determined cDNA sequences of 51 clones isolated from the subtracted library (referred to as mets3616A-S1) are provided in SEQ ID NO: 280-330.

Comparison of the sequences of SEQ ID NO: 255-330 with those in the public databases revealed no significant homologies to the sequences of SEQ ID NO: 255-258, 260, 262-264, 270, 272, 275, 276, 279, 281, 287, 291, 296, 300 and 310. The sequences of SEQ ID NO: 259, 261, 265-269, 271, 273, 274, 277, 278, 282-285, 288-290, 292, 294, 297-299, 301, 303-309, 313, 314, 316, 320-324 and 326-330 showed some homology to previously identified gene sequences, while the sequences of SEQ ID NO: 280, 286, 293, 302, 310, 312, 315, 317-319 and 325 showed some homology to previously isolated expressed sequence tags (ESTs).

### EXAMPLE 2

### DETERMINATION OF TISSUE SPECIFICITY OF LUNG TUMOR POLYPEPTIDES

Using gene specific primers, mRNA expression levels for seven representative lung tumor polypeptides described in Example 1 were examined in a variety of normal and tumor tissues using RT-PCR.

Briefly, total RNA was extracted from a variety of normal and tumor tissues using Trizol reagent as described above. First strand synthesis was carried out using 2 µg of total RNA with SuperScript II reverse transcriptase (BRL Life Technologies) at 42 °C for one hour. The cDNA was then amplified by PCR with gene-specific primers. To ensure the semi-quantitative nature of the RT-PCR, β-actin was used as an internal control for each of the tissues examined. 1 µl of 1:30 dilution of cDNA was employed to enable the linear range amplification of the β-actin template and was sensitive enough to reflect the differences in the initial copy numbers. Using these conditions, the β-actin levels were determined for each reverse transcription reaction from each tissue. DNA contamination was minimized by DNase treatment and by assuring a negative PCR result when using first strand cDNA that was prepared without adding reverse transcriptase.

mRNA Expression levels were examined in five different types of tumor tissue (lung squamous cell carcinoma from 3 patients, lung adenocarcinoma, colon tumor from 2 patients, breast tumor and prostate tumor), and thirteen different normal tissues (lung from 4 donors, prostate, brain, kidney, liver, ovary, skeletal muscle, skin, small intestine, stomach, myocardium, retina and testes). Using a 10-fold amount of cDNA, the antigen LST-S1-90 (SEQ ID NO: 3) was found to be expressed at high levels in lung squamous cell carcinoma and in breast tumor, and at low to undetectable levels in the other tissues examined.

The antigen LST-S2-68 (SEQ ID NO: 15) appears to be specific to lung and breast tumor, however, expression was also detected in normal kidney. Antigens LST-S1-169 (SEQ ID NO: 6) and LST-S1-133 (SEQ ID NO: 5) appear to be very abundant in lung tissues (both normal and tumor), with the expression of these two genes being decreased in most of the normal tissues tested. Both LST-S1-169 and LST-S1-133 were also expressed in breast and colon tumors. Antigens LST-S1-6 (SEQ ID NO: 7) and LST-S2-I2-5F (SEQ ID NO: 47) did not show tumor or tissue specific expression, with the expression of LST-S1-28 being rare and only detectable in a few tissues. The antigen LST-S3-7 (SEQ ID NO: 63) showed lung and breast tumor specific expression, with its message only being detected in normal testes when the PCR was performed for 30 cycles. Lower level expression was detected in some normal tissues when the cycle number was increased to 35. Antigen LST-S3-13 (SEQ ID NO: 66) was found to be expressed in 3 out of 4 lung tumors, one breast tumor and both colon tumor samples. Its expression in normal tissues was lower compared to tumors, and was only detected in 1 out of 4 normal lung tissues and in normal tissues from kidney, ovary and retina. Expression of antigens LST-S3-4 (SEQ ID NO: 62) and LST-S3-14 (SEQ ID NO: 67) was rare and did not show any tissue or tumor specificity. Consistent with Northern blot analyses, the RT-PCT results on antigen LAT-S1-A-10A (SEQ ID NO: 78) suggested that its expression is high in lung, colon, stomach and small intestine tissues, including lung and colon tumors, whereas its expression was low or undetectable in other tissues.

A total of 2002 cDNA fragments isolated in lung subtractions I, II and III, described above, were colony PCR amplified and their mRNA expression levels in lung tumor, normal lung, and various other normal and tumor tissues were determined using microarray technology (Synteni, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity. Seventeen non-redundant cDNA clones showed over-expression in lung squamous tumors, with expression in normal tissues tested (lung, skin, lymph node, colon, liver, pancreas, breast, heart, bone marrow, large intestine, kidney, stomach, brain, small intestine, bladder and salivary gland) being either undetectable, or 10-fold less compared to lung squamous tumors. The determined partial cDNA sequences for the clone L513S are provided in SEQ ID NO: 87 and 88; those for L514S are provided in SEQ ID NO: 89 and 90; those for L516S in SEQ ID NO: 91 and 92; that for L517S in SEQ ID NO: 93; that for L519S in SEQ ID NO: 94; those for L520S in SEQ ID NO: 95 and 96; those for L521 S in SEQ ID NO: 97 and 98; that for L522S in SEQ ID NO: 99; that for L523S in SEQ ID NO: 100; that for L524S in SEQ ID NO: 101; that for L525S in SEQ ID NO: 102; that for L526S in SEQ ID NO: 103; that for L527S in SEQ ID NO: 104; that for L528S in SEQ ID NO: 105; that for L529S in SEQ ID NO: 106; and those for L530S in SEQ ID NO: 107 and 108. Additionally, the full-length cDNA sequence for L530S is provided in SEQ ID NO: 151, with the corresponding predicted amino acid sequence being provided in SEQ ID NO: 152. L530S shows homology to a splice variant of a p53 tumor suppressor homologue, p63. The cDNA sequences of 7 known isoforms of p63 are provided in SEQ ID NO: 331-337, with the corresponding predicted amino acid sequences being provided in SEQ ID NO: 338-344, respectively.

Due to polymorphisms, the clone L531S appears to have two forms. A first determined full-length cDNA sequence for L531S is provided in SEQ ID NO: 109, with the corresponding predicted amino acid sequence being provided in SEQ ID NO: 110. A second determined full-length cDNA sequence for L531S is provided in SEQ ID NO: 111, with the corresponding predicted amino acid sequence being provided in SEQ ID NO: 112. The sequence of SEQ ID NO: 111 is identical to that of SEQ ID NO: 109, except that it contains a 27 bp insertion. Similarly, L514S also has two alternatively spliced forms; the first variant cDNA is listed as SEQ ID NO: 153, with the corresponding amino acid sequence being provided in SEQ ID NO: 155. The second variant form of L514S full-length cDNA is provided in SEQ ID NO: 154, with its corresponding amino acid sequence being provided in SEQ ID NO: 156.

Full length cloning for L524S (SEQ ID NO: 101) yielded two variants (SEQ ID NO: 163 and 164) with the corresponding predicted amino acid sequences of SEQ ID NO: 165 and 166, respectively. Both variants have been shown to encode parathyroid hormone-related peptide.

Attempts to isolate the full-length cDNA for L519S, resulted in the isolation of the extended cDNA sequence provided in SEQ ID NO: 173, which contains a potential open reading frame. The predicted amino acid sequence encoded by the sequence of SEQ ID NO: 173 is provided in SEQ ID NO: 174. Additionally, the full-length cDNA sequence for the clone of SEQ ID NO: 100 (known as L523S), a known gene, is provided in SEQ ID NO: 175, with the corresponding predicted amino acid sequence provided in SEQ ID NO: 176. In further studies, a full-length cDNA sequence for L523S was isolated from a L523S-positive tumor cDNA library by PCR amplification using gene specific primers designed from the sequence of SEQ ID NO: 175. The determined cDNA sequence is provided in SEQ ID NO: 347. The amino acid sequence encoded by this sequence is provided in SEQ ID NO: 348. This protein sequence differs from the previously published protein sequence at two amino acid positions, namely at positions 158 and 410.

Comparison of the sequences of L514S and L531S (SEQ ID NO: 87 and 88, 89 and 90, and 109, respectively) with those in the gene bank, as described above, revealed no significant homologies to known sequences. The sequences of L513S, L516S, L517S, L519S, L520S and L530S (SEQ ID NO: 87 and 88,91 and 92, 93, 94, 95 and 96, 107 and 108, respectively) were found to show some homology to previously identified ESTs. The sequences of L521S, L522S, L523S, L524S, L525S, L526S, L527S, L528S and L529S (SEQ ID NO: 97 and 98, 99, 99, 101, 102, 103, 104, 105, and 106, respectively) were found to represent known genes. The determined full-length cDNA sequences for L520S is provided in SEQ ID NO: 113, with the corresponding predicted amino acid sequence being provided in SEQ ID NO: 114. Subsequent microarray analysis has shown L520S to be overexpressed in breast tumors in addition to lung squamous tumors.

Further analysis has demonstrated that L529S (SEQ ID NO: 106 and 115), L525S (SEQ ID NO: 102 and 120) and L527S (SEQ ID NO: 104) are cytoskeletal components and potentially squamous cell specific proteins. L529S is connexin 26, a gap junction protein. It is highly expressed in lung squamous tumor 9688T, and moderately over-expressed in two others. However, lower level expression of connexin 26 is also detectable in normal skin, colon, liver and stomach. The over-expression of connexin 26 in some breast tumors has been reported and a mutated form of L529S may result in over-expression in lung tumors. L525S is plakophilin 1, a desmosomal protein found in plaque-bearing adhering junctions of the skin. Expression levels for L525S mRNA is highly elevated in three out of four lung squamous tumors tested, and in normal skin. L527S has been identified as keratin 6 isoform, type II 58 Kd keratin, and cytokeratin 13 and shows over-expression in squamous tumors and low expression in normal skin, breast and colon tissues. Notably, keratin and keratin-related genes have been extensively documented as potential markers for lung cancer including CYFRA2.1 (Pastor, A., et al, Eur. Respir. J., 10:603-609, 1997). L513S (SEQ ID NO: 87 and 88) shows moderate over-expression in several tumor tissues tested, and encodes a protein that was first isolated as a pemphigus vulgaris antigen.

L520S (SEQ ID NO: 95 and 96) and L521 S (SEQ ID NO: 97 and 98) are highly expressed in lung squamous tumors, and L520S is up-regulated in normal salivary gland and L521 S is over-expressed in normal skin. Both belong to a family of small proline rich proteins and represent markers for fully differentiated squamous cells. L521 S has been described as a specific marker for lung squamous tumor (Hu, R., et al, Lung Cancer, 20:25-30, 1998). L515S (SEQ ID NO: 162) encodes IGF-β2 and L516S is an aldose reductase homologue and both are moderately expressed in lung squamous tumors and in normal colon. Notably, L516S (SEQ ID NO: 91 and 92) is up-regulated in metastatic tumors but not primary lung adenocarcinoma, an indication of its potential role in metatasis and a potential prognostic marker. L522S (SEQ ID NO: 99) is moderately over-expressed in lung squamous tumors with minimum expression in normal tissues. L522S has been shown to belong to a class IV alcohol dehydrogenase, ADH7, and its expression profile suggests it is a squamous cell specific antigen. L523S (SEQ ID NO: 100) is moderately over-expressed in lung squamous tumor, human pancreatic cancer cell lines and pancreatic cancer tissues, suggesting this gene may be a shared antigen between pancreatic and lung squamous cell cancer.

L524S (SEQ ID NO: 101) is over-expressed in the majority of squamous tumors tested and is homologous with parathyroid hormone-related peptide (PTHrP), which is best known to cause humoral hypercalcaemia associated with malignant tumors such as leukemia, prostate and breast cancer. It is also believed that PTHrP is most commonly associated with squamous carcinoma of lung and rarely with lung adenocarcinoma (Davidson, L.A., et al, J. Pathol., 178: 398-401, 1996). L528S (SEQ ID NO: 105) is highly over-expressed in two lung squamous tumors with moderate expression in two other squamous tumors, one lung adenocarcinoma and some normal tissues, including skin, lymph nodes, heart, stomach and lung. It encodes the NMB gene that is similar to the precursor of melanocyte specific gene Pmel17, which is reported to be preferentially expressed in low-metastatic potential melanoma cell lines. This suggests that L528S may be a shared antigen in both melanoma and lung squamous cell carcinoma. L526S (SEQ ID NO: 103) is overexpressed in all lung squamous cell tumor tissues tested and has been shown to share homology with a gene (ATM) in which a mutation causes ataxia telangiectasia, a genetic disorder in humans causing a predisposition to cancer, among other symptoms. ATM encodes a protein that activates p53 mediated cell-cycle checkpoint through direct binding and phosphorylation of the p53 molecule. Approximately 40% of lung cancer is associated with p53 mutations, and it is speculated that over-expression of ATM is a result of compensation for loss of p53 function, but it is unknown whether over-expression is the cause of result of lung squamous cell carcinoma. Additionally, expression of L526S (ATM) is also detected in a metastatic but not lung adenocarcinoma, suggesting a role in metastasis.

Expression of L523S (SEQ ID NO: 175), was also examined by real time RT-PCR as described above. In a first study using a panel of lung squamous tumors, L523S was found to be expressed in 4/7 lung squamous tumors, 2/3 head and neck squamous tumors and 2/2 lung adenocarcinomas, with low level expression being observed in skeletal muscle, soft palate and tonsil. In a second study using a lung adenocarcinoma panel, expression of L523S was observed in 4/9 primary adenocarcinomas, 2/2 lung pleural effusions, 1/1 metastatic lung adenocarcinomas and 2/2 lung squamous tumors, with little expression being observed in normal tissues.

Expression of L523S in lung tumors and various normal tissues was also examined by Northern blot analysis, using standard techniques. In a first study, L523S was found to be expressed in a number of lung adenocarcinomas and squamous cell carcinomas, as well as normal tonsil. No expression was observed in normal lung. In a second study using a normal tissue blot (HB-12) from Clontech, no expression was observed in brain, skeletal muscle, colon, thymus, spleen, kidney, liver, small intestine, lung or PBMC, although there was strong expression in placenta.

### EXAMPLE 3

### ISOLATION AND CHARACTERIZATION OF LUNG TUMOR POLYPEPTIDES BY PCR-BASED SUBTRACTION

Eight hundred and fifty seven clones from a cDNA subtraction library, containing cDNA from a pool of two human lung squamous tumors subtracted against eight normal human tissue cDNAs including lung, PBMC, brain, heart, kidney, liver, pancreas, and skin, (Clontech, Palo Alto, CA) were derived and submitted to a first round of PCR amplification. This library was subjected to a second round of PCR amplification, following the manufacturer's protocol. The resulting cDNA fragments were subcloned into the vector P7- Adv vector (Clontech, Palo Alto, CA) and transformed into DH5α *E. coli* (Gibco, BRL). DNA was isolated from independent clones and sequenced using a Perkin Elmer/Applied Biosystems Division Automated Sequencer Model 373A.

One hundred and sixty two positive clones were sequenced. Comparison of the DNA sequences of these clones with those in the the EMBL and GenBank databases, as described above, revealed no significant homologies to 13 of these clones, hereinafter referred to as Contigs 13, 16, 17, 19, 22, 24, 29, 47, 49, 56-59. The determined cDNA sequences for these clones are provided in SEQ ID NO: 125, 127-129, 131-133, 142, 144, 148-150, and 157, respectively. Contigs 1, 3-5, 7-10, 12, 11, 15, 20, 31, 33, 38, 39, 41, 43, 44, 45, 48, 50, 53, 54 (SEQ ID NO: 115-124, 126, 130, 134-141, 143, 145-147, respectively) were found to show some degree of homology to previously identified DNA sequences. Contig 57 (SEQ ID NO: 149) was found to represent the clone L519S (SEQ ID NO: 94) disclosed in US. Patent Application No. 09/123,912, filed July 27, 1998. To the best of the inventors' knowledge, none of these sequences have been previously shown to be differentially over-expressed in lung tumors.

mRNA expression levels for representative clones in lung tumor tissues, normal lung tissues (n=4), resting PBMC, salivary gland, heart, stomach, lymph nodes, skeletal muscle, soft palate, small intestine, large intestine, bronchial, bladder, tonsil, kidney, esophagus, bone marrow, colon, adrenal gland, pancreas, and skin, (all derived from human) were determined by RT-PCR as described above. Expression levels using microarray technology, as described above, were examined in one sample of each tissue type unless otherwise indicated.

Contig 3 (SEQ ID NO: 116) was found to be highly expressed in all head and neck squamous cell tumors tested (17/17), and expressed in the majority (8/12) of lung squamous tumors, (high expression in 7/12, moderate in 2/12, and low in 2/12), while showing negative expression for 2/4 normal lung tissues and low expression in the remaining two samples. Contig 3 showed moderate expression in skin and soft palate, and lowered expression levels in resting PBMC, large intestine, salivary gland, tonsil, pancreas, esophagus, and colon. Contig 11 (SEQ ID NO: 124) was found to be expressed in all head and neck squamous cell tumors tested (17/17): highly expressed in 14/17, and moderately expressed in 3/17. Additionally, expression in lung squamous tumors showed high expression in 3/12 and moderate in 4/12. Contig 11 was negative for 3/4 normal lung samples, with the remaining sample having only low expression. Contig 11 showed low to moderate reactivity to salivary gland, soft palate, bladder, tonsil, skin, esophagus, and large intestine. Contig 13 (SEQ ID NO: 125) was found to be expressed in all head and neck squamous cell tumors tested (17/17): highly expressed in 12/17, and moderately expressed in 5/17. Contig 13 was expressed in 7/12 lung squamous tumors, with high expression in 4/12 and moderate expression in three samples. Analysis of normal lung samples showed negative expression for 2/4 and low to moderate expression in the remaining two samples. Contig 13 did show low to moderate reactivity to resting PBMC, salivary gland, bladder, pancreas, tonsil, skin, esophagus, and large intestine, as well as high expression in soft palate. Contig 16 (SEQ ID NO: 127) was found to be moderately expressed in some head and neck squamous cell tumors (6/17) and one lung squamous tumor; while showing no expression in any normal lung samples tested. Contig 16 did show low reactivity to resting PBMC, large intestine, skin, salivary gland, and soft palate. Contig 17 (SEQ ID NO: 128) was shown to be expressed in all head and neck squamous cell tumors tested (17/17): highly expressed in 5/17, and moderately expressed in 12/17. Expression levels in lung squamous tumors showed one tumor sample with high expression and 3/12 with moderate levels. Contig 17 was negative for 2/4 normal lung samples, with the remaining samples having only low expression. Additionally, low level expression was found in esophagus and soft palate. Contig 19 (SEQ ID NO: 129) was found to be expressed in most head and neck squamous cell tumors tested (11/17); with two samples having high levels, 6/17 showing moderate expression, and low expression being found in 3/17. Testing in lung squamous tumors revealed only moderate expression in 3/12 samples. Expression levels in 2/4 of normal lung samples were negative, the two other samples having only low expression. Contig 19 showed low expression levels in esophagus, resting PBMC, salivary gland, bladder, soft palate and pancreas.

Contig 22 (SEQ ID NO: 131), was shown to be expressed in most head and neck squamous cell tumors tested (13/17) with high expression in four of these samples, moderate expression in 6/17, and low expression in 3/17. Expression levels in lung squamous tumors were found to be moderate to high for 3/12 tissues tested, with negative expression in two normal lung samples and low expression in two other samples (n=4). Contig 22 showed low expression in skin, salivary gland and soft palate. Similarly, Contig 24 (SEQ ID NO: 132) was found to be expressed in most head and neck squamous cell tumors tested (13/17) with high expression in three of these samples, moderate expression in 6/17, and low expression in 4/17. Expression levels in lung squamous tumors were found to be moderate to high for 3/12 tissues tested, with negative expression for three normal lung samples and low expression in one sample (n=4). Contig 24 showed low expression in skin, salivary gland and soft palate. Contig 29 (SEQ ID NO: 133) was expressed in nearly all head and neck squamous cell tumors tested (16/17): highly expressed in 4/17, moderately expressed in 11/17, with low expression in one sample. Also, it was moderately expressed in 3/12 lung squamous tumors, while being negative for 2/4 normal lung samples. Contig 29 showed low to moderate expression in large intestine, skin, salivary gland, pancreas, tonsil, heart and soft palate. Contig 47 (SEQ ID NO: 142) was expressed in most head and neck squamous cell tumors tested (12/17): moderate expression in 10/17, and low expression in two samples. In lung squamous tumors, it was highly expressed in one sample and moderately expressed in two others (n=13). Contig 47 was negative for 2/4 normal lung samples, with the remaining two samples having moderate expression. Also, Contig 47 showed moderate expression in large intestine, and pancreas, and low expression in skin, salivary gland, soft palate, stomach, bladder, resting PBMC, and tonsil.

Contig 48 (SEQ ID NO: 143) was expressed in all head and neck squamous cell tumors tested (17/17): highly expressed in 8/17 and moderately expressed in 7/17, with low expression in two samples. Expression levels in lung squamous tumors were high to moderate in three samples (n=13). Contig 48 was negative for one out of four normal lung samples, the remaining showing low or moderate expression. Contig 48 showed moderate expression in soft palate, large intestine, pancreas, and bladder, and low expression in esophagus, salivary gland, resting PBMC, and heart. Contig 49 (SEQ ID NO: 144) was expressed at low to moderate levels in 6/17 head and neck squamous cell tumors tested. Expression levels in lung squamous tumors were moderate in three samples (n=13). Contig 49 was negative for 2/4 normal lung samples, the remaining samples showing low expression. Moderate expression levels in skin, salivary gland, large intestine, pancreas, bladder and resting PBMC were shown, as well as low expression in soft palate, lymph nodes, and tonsil. Contig 56 (SEQ ID NO: 148) was expressed in low to moderate levels in 3/17 head and neck squamous cell tumors tested, and in lung squamous tumors, showing low to moderate levels in three out of thirteen samples. Notably, low expression levels were detected in one adenocarcinoma lung tumor sample (n=2). Contig 56 was negative for 3/4 normal lung samples, and showed moderate expression levels in only large intestine, and low expression in salivary gland, soft palate, pancreas, bladder, and resting PBMC. Contig 58, also known as L769P, (SEQ ID NO: 150) was expressed at moderate levels in 11/17 head and neck squamous cell tumors tested and low expression in one additional sample. Expression in lung squamous tumors showed low to moderate levels in three out of thirteen samples. Contig 58 was negative for 3/4 normal lung samples, with one sample having low expression. Moderate expression levels in skin, large intestine, and resting PBMC were demonstrated, as well as low expression in salivary gland, soft palate, pancreas, and bladder. Contig 59 (SEQ ID NO: 157) was expressed in some head, neck, and lung squamous tumors. Low level expression of Contig 59 was also detected in salivary gland and large intestine.

The full-length cDNA sequence for Contig 22, also referred to as L763P, is provided in SEQ ID NO: 158, with the corresponding predicted amino acid sequence being provided in SEQ ID NO: 159. Real-time RT-PCR analysis of L763P revealed that it is highly expressed in 3/4 lung squamous tumors as well as 4/4 head and neck squamous tumors, with low level expression being observed in normal brain, skin, soft pallet and trachea. Subsequent database searches revealed that the sequence of SEQ ID NO: 158 contains a mutation, resulting in a frameshift in the corresponding protein sequence. A second cDNA sequence for L763P is provided in SEQ ID NO: 345, with the corresponding amino acid sequence being provided in SEQ ID NO: 346. The sequences of SEQ ID NO: 159 and 346 are identical with the exception of the C-terminal 33 amino acids of SEQ ID NO: 159.

The full-length cDNA sequence incorporating Contigs 17, 19, and 24, referred to as L762P, is provided in SEQ ID NO: 160, with the corresponding predicted amino acid sequence being provided in SEQ ID NO: 161. Further analysis of L762P has determined it to be a type I membrane protein and two additional variants have been sequenced. Variant 1 (SEQ ID NO: 167, with the corresponding amino acid sequence in SEQ ID NO: 169) is an alternatively spliced form of SEQ ID NO: 160 resulting in deletion of 503 nucleotides, as well as deletion of a short segment of the expressed protein. Variant 2 (SEQ ID NO: 168, with the corresponding amino acid sequence in SEQ ID NO: 170) has a two nucleotide deletion at the 3' coding region in comparison to SEQ ID NO: 160, resulting in a secreted form of the expressed protein. Real-time RT-PCR analysis of L762P revealed that is over-expressed in 3/4 lung squamous tumors and 4/4 head & neck tumors, with low level expression being observed in normal skin, soft pallet and trachea.

The full-length cDNA sequence for contig 56 (SEQ ID NO: 148), also referred to as L773P, is provided in SEQ ID NO: 171, with the predicted amino acid sequence in SEQ ID NO: 172. L773P was found to be identical to dihydroxyl dehydrogenase at the 3' portion of the gene, with divergent 5' sequence. As a result, the 69 N-terminal amino acids are unique. The cDNA sequence encoding the 69 N-terminal amino acids is provided in SEQ ID NO: 349, with the N-terminal amino acid sequence being provided in SEQ ID NO: 350. Real-time PCR revealed that L773P is highly expressed in lung squamous tumor and lung adenocarcinoma, with no detectable expression in normal tissues. Subsequent Northern blot analysis of L773P demonstrated that this transcript is differentially over-expressed in squamous tumors and detected at approximately 1.6 Kb in primary lung tumor tissue and approximately 1.3 Kb in primary head and neck tumor tissue.

Subsequent microarray analysis has shown Contig 58, also referred to as L769S (SEQ ID NO: 150), to be overexpressed in breast tumors in addition to lung squamous tumors.

### EXAMPLE 4

### SYNTHESIS OF POLYPEPTIDES

Polypeptides may be synthesized on a Perkin Elmer/Applied Biosystems Division 430A peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1 % trifluoroacetic acid (TFA) and lyophilized prior to purification by C18 reverse phase HPLC. A gradient of 0%-60% acetonitrile (containing 0.1% TFA) in water (containing 0.1% TFA) may be used to elute the peptides. Following lyophilization of the pure fractions, the peptides may be characterized using electrospray or other types of mass spectrometry and by amino acid analysis.

### EXAMPLE 5

### PREPARATION OF ANTIBODIES AGAINST LUNG CANCER ANTIGENS

Polyclonal antibodies against the lung cancer antigens L514S, L528S and L531S (SEQ ID NO: 155, 225 and 112, respectively) were prepared as follows.

Rabbits were immunized with recombinant protein expressed in and purified from *E. coli* as described above. For the initial immunization, 400 µg of antigen combined with muramyl dipeptide (MDP) was injected subcutaneously (S.C.). Animals were boosted S.C. 4 weeks later with 200 µg of antigen mixed with incomplete Freund's Adjuvant (IFA). Subsequent boosts of 100 µg of antigen mixed with IFA were injected S.C. as necessary to induce high antibody titer responses. Serum bleeds from immunized rabbits were tested for antigen-specific reactivity using ELISA assays with purified protein. Polyclonal antibodies against L514S, L528S and L531S were affinity purified from high titer polyclonal sera using purified protein attached to a solid support.

Immunohistochemical analysis using polyclonal antibodies against L514S was performed on a panel of 5 lung tumor samples, 5 normal lung tissue samples and normal colon, kidney, liver, brain and bone marrow. Specifically, tissue samples were fixed in formalin solution for 24 hours and embedded in paraffin before being sliced into 10 micron sections. Tissue sections were permeabilized and incubated with antibody for 1 hr. HRP-labeled anti-mouse followed by incubation with DAB chromogen was used to visualize L514S immunoreactivity. L514S was found to be highly expressed in lung tumor tissue with little or no expression being observed in normal lung, brain or bone marrow. Light staining was observed in colon and kidney. Staining was seen in normal liver but no mRNA has been detected in this tissue making this result suspect.

### EXAMPLE 6

### PEPTIDE PRIMING OF MICE AND PROPAGATION OF CTL LINES

Immunogenic peptides from the lung cancer antigen L762P (SEQ ID NO: 161) for HLA-A2/K^{b}-restricted CD8+ T cells were identified as follows.

The location of HLA-A2 binding peptides within the lung cancer antigen L762P (SEQ ID NO: 161) was predicted using a computer program which predicts peptides sequences likely to being to HLA-A*0201 by fitting to the known peptide binding motif for HLA-A*0201 (Rupert et al. (1993) Cell 74:929; Rammensee et al. (1995) Immunogenetics 41:178-228). A series of 19 synthetic peptides corresponding to a selected subset of the predicted HLA-A*0201 binding peptides was prepared as described above.

Mice expressing the transgene for human HLA A2/K^{b} (provided by Dr L. Sherman, The Scripps Research Institute, La Jolla, CA) were immunized with the synthetic peptides, as described by Theobald et al., Proc. Natl. Acad. Sci. USA 92:11993-11997, 1995 with the following modifications. Mice were immunized with 50µg of L726P peptide and 120µg of an I-A^{b} binding peptide derived from hepatitis B Virus protein emulsified in incomplete Freund's adjuvant. Three weeks later these mice were sacrificed and single cell suspensions prepared. Cells were then resuspended at 7 x 10⁶ cells/ml in complete media (RPMI-1640; Gibco BRL, Gaithersburg, MD) containing 10% FCS, 2mM Glutamine (Gibco BRL), sodium pyruvate (Gibco BRL), non-essential amino acids (Gibco BRL), 2 x 10⁻⁵ M 2-mercaptoethanol, 50U/ml penicillin and streptomycin, and cultured in the presence of irradiated (3000 rads) L762P peptide- (5µg/ml) and 10mg/ml B₂-microglobulin- (3 µg/ml) LPS blasts (A2 transgenic spleens cells cultured in the presence of 7µg/ml dextran sulfate and 25µg/ml LPS for 3 days). After six days, cells (5 x 10⁵/ml) were restimulated with 2.5 x 10⁶/ml peptide pulsed irradiated (20,000 rads) EL4A2Kb cells (Sherman et al, Science 258:815-818, 1992) and 5 x 10⁶/ml irradiated (3000 rads) A2/K^{b}-transgenic spleen feeder cells. Cells were cultured in the presence of 10U/ml IL-2. Cells were restimulated on a weekly basis as described, in preparation for cloning the line.

Peptide-specific cell lines were cloned by limiting dilution analysis with irradiated (20,000 rads) L762P peptide-pulsed EL4 A2Kb tumor cells (1 x 10⁴ cells/well) as stimulators and irradiated (3000 rads) A2/K^{b}-transgenic spleen cells as feeders (5 x 10⁵ cells/ well) grown in the presence of 10U/ml IL-2. On day 7, cells were restimulated as before. On day 14, clones that were growing were isolated and maintained in culture.

Cell lines specific for L762P-87 (SEQ ID NO: 226; corresponding to amino acids 87-95 of SEQ ID NO: 161), L726P-145 (SEQ ID NO: 227; corresponding to amino acids 145-153 of SEQ ID NO: 161), L726P-585 (SEQ ID NO: 228; corresponding to amino acids 585-593 of SEQ ID NO: 161), L762P-425 (SEQ ID NO: 229; corresponding to amino acids 425-433 of SEQ ID NO: 161), L762P(10)-424 (SEQ ID NO: 230; corresponding to amino acids 424-433 of SEQ ID NO: 161) and L762P(10)-458 (SEQ ID NO: 231; corresponding to amino acids 458-467 of SEQ ID NO: 161) demonstrated significantly higher reactivity (as measured by percent specific lysis) against L762P peptide-pulsed EL4-A2/K^{b} tumor target cells than control peptide-pulsed EL4-A2/K^{b} tumor target cells.

### EXAMPLE 7

### IDENTIFICATION OF CD4 IMMUNOGENIC T CELL EPITOPES DERIVED FROM THE LUNG CANCER ANTIGEN L762P

CD4 T cell lines specific for the antigen L762P (SEQ ID NO: 161) were generated as follows.

A series of 28 overlapping peptides were synthesized that spanned approximately 50% of the L762P sequence. For priming, peptides were combined into pools of 4-5 peptides, pulsed at 20 micrograms/ml into dendritic cells for 24 hours. The dendritic cells were then washed and mixed with positively selected CD4+ T cells in 96 well U-bottomed plates. Forty cultures were generated for each peptide pool. Cultures were restimulated weekly with fresh dendritic cells loaded with peptide pools. Following a total of 3 stimulation cycles, cells were rested for an additional week and tested for specificity to antigen presenting cells (APC) pulsed with peptide pools using interferon-gamma ELISA and proliferation assays. For these assays, adherent monocytes loaded with either the relevant peptide pool or an irrelevant peptide were used as APC. T cell lines that appeared to specifically recognize L762P peptide pools both by cytokine release and proliferation were identified for each pool. Emphasis was placed on identifying T cells with proliferative responses. T cell lines that demonstrated either both L762P-specific cytokine secretion and proliferation, or strong proliferation alone were further expanded to be tested for recognition of individual peptides from the pools, as well as for recognition of recombinant L762P. The source of recombinant L762P was *E. coli,* and the material was partially purified and endotoxin positive. These studies employed 10 micrograms of individual peptides, 10 or 2 micrograms of an irrelevant peptide, and 2 or 0.5 micrograms of either L762P protein or an irrelevant, equally impure, *E. coli* generated recombinant protein. Significant interferon-gamma production and CD4 T cell proliferation was induced by a number of L762P-derived peptides in each pool. The amino acid sequences for these peptides are provided in SEQ ID NO: 232-251. These peptides correspond to amino acids 661-680, 676-696, 526-545, 874-893, 811-830, 871-891, 856-875, 826-845, 795-815, 736-755, 706-725, 706-725, 691-710, 601-620, 571-590, 556-575, 616-635, 646-665, 631-650, 541-560 and 586-605, respectively, of SEQ ID NO: 161.

CD4 T cell lines that demonstrated specificity for individual L762P-derived peptides were further expanded by stimulation with the relevant peptide at 10 micrograms/ml. Two weeks post-stimulation, T cell lines were tested using both proliferation and IFN-gamma ELISA assays for recognition of the specific peptide. A number of previously identified T cells continued to demonstrate L762P-peptide specific activity. Each of these lines was further expanded on the relevant peptide and, following two weeks of expansion, tested for specific recognition of the L762P-peptide in titration experiments, as well as for recognition of recombinant *E. coli*-derived L762P protein. For these experiments, autologous adherent monocytes were pulsed with either the relevant L762P-derived peptide, an irrelevant mammaglobin-derived peptide, recombinant *E. coli*-derived L762P (approx. 50% pure), or an irrelevant *E. coli*-derived protein. The majority of T cell lines were found to show low affinity for the relevant peptide, since specific proliferation and IFN-gamma ratios dramatically decreased as L762P peptide was diluted. However, four lines were identified that demonstrated significant activity even at 0.1 micrograms/ml peptide. Each of these lines (referred to as A/D5, D/F5, E/A7 and EB6) also appeared to specifically proliferate in response to the *E. coli*-derived L762P protein preparation, but not in response to the irrelevant protein preparation. The amino acid sequences of the L762P-derived peptides recognized by these lines are provided in SEQ ID NO: 234, 249, 236 and 245, respectively. No protein specific IFN-gamma was detected for any of the lines. Lines A/D5, E/A7 and E/B6 were cloned on autologous adherent monocytes pulsed with the relevant peptide at 0.1 (A/D5 and E/A7) or 1 (D/F5) microgram/ml. Following growth, clones were tested for specificity for the relevant peptide. Numerous clones specific for the relevant peptide were identified for lines A/D5 and E/A7.

### EXAMPLE 8

### PROTEIN EXPRESSION OF LUNG TUMOR-SPECIFIC ANTIGENS

### a) Expression of L514S in E. coli

The lung tumor antigen L514S (SEQ ID NO: 89) was subcloned into the expression vector pE32b at NcoI and NotI sites, and transformed into *E. coli* using standard techniques. The protein was expressed from residues 3-153 of SEQ ID NO: 89. The expressed amino acid sequence and the corresponding DNA sequence are provided in SEQ ID NO: 252 and 253, respectively.

### b) Expression of L762P

Amino acids 32-944 of the lung tumor antigen L762P (SEQ ID NO: 161), with a 6X His Tag, were subcloned into a modified pET28 expression vector, using kanamycin resistance, and transformed into BL21 CodonPlus using standard techniques. Low to moderate levels of expression were observed. The determined DNA sequence of the L762P expression construct is provided in SEQ ID NO: 254.

## Claims

1. A method for detecting the presence of a lung cancer in a patient, comprising the steps of:
(a) contacting a biological sample obtained from the patient with an antibody, or antigen binding fragment thereof, that binds to the polypeptide set forth in SEQ ID NO:176;
(b) detecting in the sample an amount of polypeptide that binds to the binding agent; and
(c) comparing the amount of polypeptide to a predetermined cut-off value and therefrom determining the presence of lung cancer in the patient.

2. The method of claim 1, wherein the antibody is a monoclonal antibody.

3. The use of an antibody, or antigen binding fragment thereof, in an *in vitro* method to detect the presence of a lung cancer in a biological sample, wherein the antibody can detect the presence of the polypeptide set forth in SEQ ID NO: 176 in the biological sample.

4. The use of claim 3, wherein the antibody is immobilised on a solid support to bind to and remove the polypeptide from the remainder of the biological sample.

5. The use of claim 3, wherein the antibody is immobilised on a membrane

6. The use of the polypeptide set forth in SEQ ID NO: 176 in an *in vitro* method to detect the presence of lung cancer in a biological sample, wherein the polypeptide is used to detect the presence of T cells that specifically react therewith in the biological sample.

7. A method for detecting the presence of lung cancer in a patient, the method comprising the steps of:
a) incubating an isolated biological sample comprising CD4+ and/or CD8+ T cells with the polypeptide set forth in SEQ ID NO:176 or a polynucleotide encoding same; and
b) detecting the presence or absence of specific activation of the T cells.

8. The method of claim 7, wherein the polynucleotide has the sequence set forth in SEQ ID NO: 175

9. The method of claims 7 or 8, wherein the polypeptide and biological sample are incubated for 2-9 days, preferably 4 days, at 37°C.

10. The method of claims 7, 8 or 9, wherein activation of the CD4+ T cells is detected by evaluating proliferation of the T cells.

11. The method of claims 7, 8 or 9, wherein activation of the CD8+ T cells is detected by evaluating cytolytic activity.

12. The use of the polypeptide set forth in SEQ ID NO:176 or a polynucleotide encoding same in the method of any one of claims 7 to 11.

13. The use of claim 12, wherein the polynucleotide has the sequence set forth in SEQ ID NO: 175.

14. The use of oligonucleotide primers in a polymerase chain reaction based assay comprising at least 15 contiguous nucleotides of a DNA molecule set forth in SEQ ID NO: 175 to detect for the presence of lung cancer in a biological sample.

15. A lung cancer diagnostic kit comprising a monoclonal antibody or antigen binding fragment thereof that specifically binds to the polypeptide set forth in SEQ ID NO:176 and a detection reagent, wherein the detection reagent comprises a reporter group.

16. A lung cancer diagnostic kit comprising at least one oligonucleotide comprising 15-40 contiguous nucleotides of the polynucleotide set forth in SEQ ID NO: 175 and a diagnostic reagent for use in a polymerase chain reaction.

17. A method for determining the presence of a lung cancer in a patient, comprising the steps of:
(a) contacting a biological sample obtained from the patient with an oligonucleotide that hybridizes to the sequence recited in SEQ ID NO:175;
(b) detecting in the sample an amount of a polynucleotide that hybridizes to the oligonucleotide; and
(c) comparing the amount of polynucleotide that hybridizes to the oligonucleotide to a predetermined cut-off value, and therefrom determining the presence of the lung cancer in the patient.

## Revendications

1. Une méthode permettant de détecter la présence d'un cancer pulmonaire chez un patient, comportant les étapes suivantes :
(a) la mise en contact d'un échantillon biologique obtenu à partir du patient et d'un anticorps, ou d'un fragment de celui-ci lié à un antigène, lequel liant le polypeptide présenté dans le SEQ ID NO : 176 ;
(b) la détection dans un échantillon d'une quantité de polypeptide qui est lié à l'agent liant ; et
(c) la comparaison de la quantité de polypeptide par rapport à la valeur seuil prédéterminée et par la même déterminer la présence d'un cancer pulmonaire chez un patient.

2. La méthode selon la revendication 1, dans laquelle l'anticorps est un anticorps monoclonal.

3. L'utilisation d'un anticorps, ou d'un antigène lié à un fragment de celui-ci, dans une méthode *in vitro* pour détecter la présence d'un cancer pulmonaire dans un échantillon biologique, dans laquelle l'anticorps peut détecter la présence dans l'échantillon biologique du polypeptide présenté dans la SEQ ID NO : 176.

4. L'utilisation de la revendication 3, dans laquelle l'anticorps est immobilisé sur un support solide afin de lier et retirer le polypeptide du reste de l'échantillon biologique.

5. L'utilisation de la revendication 3, dans laquelle l'anticorps est immobilisé sur une membrane.

6. L'utilisation du polypeptide présenté dans la SEQ ID NO : 176 dans une méthode *in vitro* afin de détecter la présence d'un cancer pulmonaire à partir d'un échantillon biologique, dans laquelle le polypeptide est utilisé afin de détecter la présence de cellules T lesquelles réagissent spécifiquement avec celui-ci dans un échantillon biologique.

7. Une méthode pour la détection de la présence d'un cancer pulmonaire chez un patient, la méthode comportant les étapes suivantes :
(a) l'incubation d'un échantillon biologique isolé comportant des cellules T CD4+ et/ou CD8+ et du polypeptide présenté dans la SEQ ID NO : 176 ou d'un polynucléotide encodant celui-ci ; et
(b) la détection de la présence ou l'absence d'une activation spécifique des cellules T.

8. La méthode selon la revendication 7, dans laquelle le polynucléotide possède la séquence présentée dans la SEQ ID NO : 175.

9. La méthode selon la revendication 7 ou 8, dans laquelle le polypeptide et l'échantillon biologique sont incubés entre 2 et 9 jours, préférablement 4 jours, à 37°C.

10. La méthode selon les revendications 7, 8 ou 9, dans laquelle l'activation des cellules T CD4+ est détectée par l'évaluation de la prolifération des cellules T.

11. La méthode selon les revendications 7, 8, ou 9, dans laquelle l'activation des cellules T CD8+ est détectée par l'évaluation de leur activité cytotoxique.

12. L'utilisation du polypeptide présenté dans la SEQ ID NO : 176 ou un polynucléotide encodant pour celui-ci dans une méthode selon l'une quelconque des revendications de 7 à 11.

13. L'utilisation selon la revendication 12, dans laquelle le polynucléotide a la séquence présentée dans la SEQ ID NO : 175.

14. L'utilisation d'une amorce oligonucléotidique dans une analyse par amplification en chaîne par polymérase comportant au moins 15 nucléotides contigus d'une molécule d'ADN présentée dans la SEQ ID NO : 175 pour détecter à partir d'un échantillon biologique la présence d'un cancer du poumon.

15. Un kit de diagnostique du cancer pulmonaire comportant un anticorps monoclonal ou un antigène lié à un fragment de celui-ci lequel se lie de façon spécifique au polypeptide présenté dans le SEQ ID NO : 176 et un réactif de détection, dans lequel le réactif de détection comporte un groupement reporter.

16. Un kit pour le diagnostic du cancer pulmonaire comportant au moins un oligonucléotide constitué de 15-40 nucléotides contigus du polynucléotide présenté dans le SEQ ID NO : 175 et un réactif diagnostic pour une utilisation dans une amplification en chaîne par polymérase.

17. Une méthode pour déterminer la présence d'un cancer pulmonaire chez un patient, comportant les étapes suivantes :
(a) la mise en contact d'un échantillon biologique obtenu à partir d'un patient et d'un olygonucléotide qui s'hybride à la séquence rapportée dans la SEQ ID NO : 175 ;
(b) la détection dans un échantillon d'une quantité de polynucléotide qui s'hybride à l'oligonucléotide ; et
(c) la comparaison de la quantité de polynucléotide qui s'hybride à l'oligonucléotide à la valeur seuil prédéterminée, et de la permettant de déterminer la présence d'un cancer du poumon chez un patient.

## Patentansprüche

1. Verfahren zur Detektion des Vorhandenseins von Lungenkrebs in einem Patienten, das die Schritte umfasst:
(a) eine biologische Probe, welche vom Patienten gewonnen wurde, mit einem Antikörper oder einem Antigen-bindenden Fragment desselben in Kontakt bringen, das das in SEQ ID NO: 176 dargelegte Polypeptid bindet,
(b) in der Probe eine Menge an Polypeptid detektieren, die an das Bindungsreagenz bindet, und
(c) die Menge an Polypeptid mit einem vorbestimmten Grenzwert vergleichen und daraus das Vorhandensein von Lungenkrebs im Patienten bestimmen.

2. Verfahren nach Anspruch 1, wobei der Antikörper ein monoklonaler Antikörper ist.

3. Verwendung eines Antikörpers oder eines Antigen-bindenden Fragments desselben in einem *in-vitro* Verfahren zur Detektion des Vorhandenseins von Lungenkrebs in einer biologischen Probe, wobei der Antikörper das Vorhandensein des in SEQ ID NO: 176 dargelegten Polypeptids in der biologischen Probe detektieren kann.

4. Verwendung nach Anspruch 3, wobei der Antikörper auf einem festen Träger immobilisiert ist, um das Polypeptid aus der übrigen biologischen Probe zu binden und zu entfernen.

5. Verwendung nach Anspruch 3, wobei der Antikörper auf einer Membran immobilisiert ist.

6. Verwendung eines in SEQ ID NO: 176 dargelegten Polypeptids in einem *in-vitro* Verfahren zur Detektion des Vorhandenseins von Lungenkrebs in einer biologischen Probe, wobei das Polypeptid verwendet wird, um das Vorhandensein von T-Zellen zu detektieren, die spezifisch mit demselben in der biologischen Probe reagieren.

7. Verfahren zur Detektion des Vorhandenseins von Lungenkrebs in einem Patienten, wobei das Verfahren die Schritte umfasst:
(a) eine isolierte biologische Probe, die CD4+ und/oder CD8+ T-Zellen aufweist, mit dem in SEQ ID NO: 176 dargelegten Polypeptid oder einem dasselbe kodierenden Polynukleotid inkubieren und
(b) das Vorhandensein oder Fehlen einer spezifischen Aktivierung der T-Zellen detektieren.

8. Verfahren nach Anspruch 7, wobei das Polynukleotid die in SEQ ID NO: 175 dargelegte Sequenz hat.

9. Verfahren nach Ansprüchen 7 oder 8, wobei das Polypeptid und die biologische Probe für 2 - 9 Tage, bevorzugterweise 4 Tage, bei 37 °C inkubiert werden.

10. Verfahren nach Ansprüchen 7, 8 oder 9, wobei die Aktivierung der CD4+ T-Zellen durch Auswerten der Proliferation der T-Zellen detektiert wird.

11. Verfahren nach Ansprüchen 7, 8 oder 9, wobei die Aktivierung der CD8+ T-Zellen durch Auswerten der zytolytischen Aktivität detektiert wird.

12. Verwendung des in SEQ ID NO: 176 dargelegten Polypeptids oder eines dasselbe kodierenden Polynukleotides im Verfahren eines der Ansprüche 7 bis 11.

13. Verwendung nach Anspruch 12, wobei das Polynukleotid die in SEQ ID NO: 175 dargelegte Sequenz hat.

14. Verwendung eines Oligonukleotidprimers, der mindestens 15 zusammenhängende Nukleotide eines in SEQ ID NO: 175 dargelegten DNA-Moleküls aufweist, in einem auf der Polymerasekettenreaktion basierenden Untersuchungsverfahren, um das Vorhandensein von Lungenkrebs in einer biologischen Probe zu detektieren.

15. Lungenkrebsdiagnosekit, der einen monoklonalen Antikörper oder ein Antigen-bindendes Fragment desselben, das das in SEQ ID NO: 176 dargelegte Polypeptid bindet, sowie ein Detektionsreagenz aufweist, wobei das Detektionsreagenz eine Reportergruppe aufweist.

16. Lungenkrebsdiagnosekit, der mindestens ein Oligonukleotid aufweist, das 15 - 40 zusammenhängende Nukleotide des in SEQ ID NO: 175 dargelegten Polynukleotids sowie ein Diagnosereagenz zur Verwendung in einer Polymerasekettenreaktion aufweist.

17. Verfahren zur Bestimmung des Vorhandenseins von Lungenkrebs in einem Patienten, das die Schritte umfasst:
(a) eine biologische Probe, welche vom Patienten gewonnen wurde, mit einem Oligonukleotid in Kontakt bringen, das mit der in SEQ ID NO: 175 rezitierten Sequenz hybridisiert,
(b) in der Probe eine Menge eines Polynukleotids detektieren, die mit dem Oligonukleotid hybridisiert, und
(c) die Menge an Polynukleotid, die mit dem Oligonukleotid hybridisiert, mit einem vorbestimmten Grenzwert vergleichen und daraus das Vorhandensein von Lungenkrebs im Patienten bestimmen.
